# EUROPÄISCHE PATENTSCHRIFT

(11) **EP 3 152 216 B1**
(45) Veröffentlichungstag und Bekanntmachung des Hinweises auf die Patenterteilung: **24.07.2019**
(21) Anmeldenummer: 15724728.9
(22) Anmeldetag: 02.06.2015
(51) Int. Cl.: C07D 513/04, C07D 417/04, C07D 417/14, A01N 43/90, A01P 7/00, A01P 7/04, A61P 33/00

(54) **BICYCLISCHE VERBINDUNGEN ALS SCHÄDLINGSBEKÄMPFUNGSMITTEL**
BICYCLIC COMPOUNDS AS PESTICIDES
COMPOSÉS BICYCLIQUES UTILISABLES COMME PESTICIDES

(30) Priorität: 05.06.2014 EP 14171338
(43) Veröffentlichungstag der Anmeldung: 12.04.2017
(73) Patentinhaber: Bayer CropScience Aktiengesellschaft, 40789 Monheim am Rhein (DE)
(72) Erfinder: CEREZO-GALVEZ, Silvia, 40764 Langenfeld (DE); FISCHER, Reiner, 40789 Monheim (DE); FÜßLEIN, Martin, 40225 Düsseldorf (DE); GREUL, Jörg, 51381 Leverkusen (DE); JESCHKE, Peter, 51467 Bergisch Gladbach (DE); KLUTH, Joachim, 40764 Langenfeld (DE); VOERSTE, Arnd, 50674 Köln (DE); ILG, Kerstin, 50670 Köln (DE); MALSAM, Olga, 51503 Rösrath (DE); LÖSEL, Peter, 51371 Leverkusen (DE); GÖRGENS,Ulrich, 40882 Ratingen (DE)
(74) Vertreter: BIP Patents
(86) Internationale Anmeldenummer: PCT/EP2015/062211
(87) Internationale Veröffentlichungsnummer: WO 2015/185531

(56) Entgegenhaltungen:
- WO-A1-98/57969
- WO-A1-2009/149858
- WO-A1-2010/100189
- US-A- 3 755 581
- US-A- 4 260 765

## Beschreibung

Die vorliegende Anmeldung betrifft neue bicyclische Verbindungen, ihre Verwendung zur nichttherapeutischen Bekämpfung von tierischen Schädlingen sowie Verfahren und Zwischenprodukte zu Ihrer Herstellung.

In WO 2012/102387 A1 sind heterocyclische Verbindungen beschrieben, die insbesondere als Insektizide und Akarizide verwendet werden können.

In den Publikationen WO 2009/149858 A1, US 4 260 765 A, WO 2010/100189 A1, US 3 755 581 A und WO 98/57969 A1 werden Verbindungen zur Verwendung als Pflanzenschutzmittel offenbart, die sich aber strukturell von den hier beanspruchten Verbindungen unterscheiden.

In WO 2003/033476 A1, WO 2004/014916 A1, WO 2008/152390, MedChemComm (2013), 4(2), 456-462, Bioorganic & Medicinal Chemistry Letters (2006), 16(18), 4723-4727, Journal of the American Pharmaceutical Association (1912-1977) (1955), 44, 193-6 and Journal of the Chemical Society (1949), 1064-8 wird über Synthese und pharmakologische Eigenschaften bestimmter [1,3]Thiazolo[4,5-d]pyrimidin-7(6H)-on bzw. [1,3]Oxazolo[4,5-d]pyrimidin-7(6H)-on berichtet.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I) 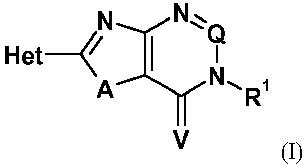 in welcher
Het für einen Rest aus der Reihe 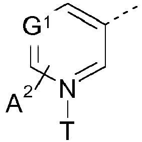 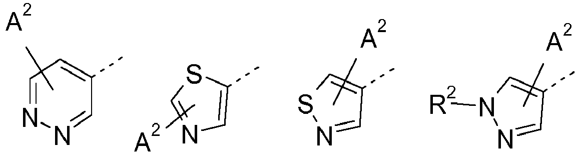 steht, worin die gestrichelte Linie die Bindung zum Kohlenstoffatom in der an Het gebundenen N=C-A-Gruppe darstellt und
- G¹: für N oder C-A¹ steht,
- A¹: für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
- A²: für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
- T: für Sauerstoff oder ein Elektronenpaar steht,
- R²: für Wasserstoff, Alkyl, Halogenalkyl oder gegebenenfalls substituiertes Cycloalkyl steht,
- R¹: für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylsulfanylalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, Dialkylaminosulfanylalkyl, Dialkylaminosulfinylalkyl, Dialkylaminosulfonylalkyl, gegebenenfalls durch Halogen substituiertes Alkoxycarbonyl, gegebenenfalls durch Halogen substituiertes Alkoxycarbonylalkyl, gegebenenfalls durch Halogen substituiertes Alkinyloxy, gegebenenfalls durch Halogen substituiertes Alkinyloxycarbonyl, Dialkylaminocarbonyl, N-Alkyl-N-Cycloalkylaminocarbonyl, Dialkylaminocarbonylalkyl, N-Alkyl-N-Cycloalkylaminocarbonylalkyl, Heterocyclylcarbonylalkyl, Alkylsulfanyl, Halogenalkylsulfanyl, Alkylsulfinyl, Halogenalkylsulfinyl, Alkylsulfonyl, Halogenalkylsulfonyl, gegebenenfalls durch Halogen, Cyano, Nitro, Alkyl, Cycloalkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Nitro, Alkyl, Halogenalkyl, Cycloalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylcarbonyl, gegebenenfalls durch Halogen, Cyano, Nitro, Alkyl, Halogenalkyl, Cycloalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Hydroxy, Alkyl, Halogenalkyl, Cycloalkyl (welches gegebenenfalls substituiert ist), Alkoxy, Halogenalkoxy, Alkylthio, Halogenalkylthio, Alkylsulfinyl, Alkylsulfonyl, Halogenalkylsulfinyl, Halogenalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Hydroxy, Amino, Alkyl, Halogenalkyl, Cycloalkyl (welches gegebenenfalls substituiert ist) Alkoxy oder Halogenalkoxy substituiertes Aryl, gegebenenfalls durch Halogen, Cyano (auch im Alkylteil), Nitro, Hydroxy, Amino, Alkyl, Cycloalkyl (welches gegebenenfalls substituiert ist), Halogenalkyl, Alkoxy oder Halogenalkoxy substituiertes Arylalkyl, gegebenenfalls durch Halogen, Cyano, Nitro, Hydroxy, Alkyl, Halogenalkyl, Cycloalkyl (welches gegebenenfalls substituiert ist), Alkoxy, Halogenalkoxy, Alkylthio, Halogenalkylthio, Alkylsulfinyl, Alkylsulfonyl, Halogenalkylsulfinyl, Halogenalkylsulfonyl, Amino, Alkylamino, Dialkylamino, Alkylaminocarbonyl, Dialkylaminocarbonyl, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Hydroxy, Alkyl, Halogenalkyl, Cycloalkyl (welches gegebenenfalls substituiert ist), Alkoxy, Halogenalkoxy, Alkylthio, Halogenalkylthio, Alkylsulfinyl, Alkylsulfonyl, Halogenalkylsulfinyl, Halogenalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
- R¹: auch für einen Rest aus der Reihe 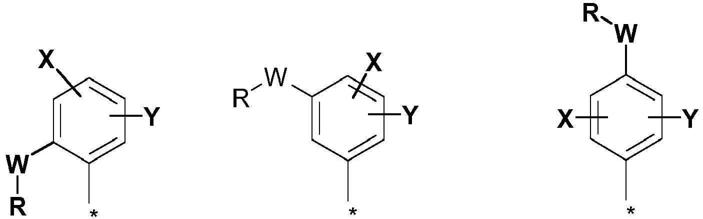 steht,
worin die Bindung zum Stickstoffatom in der C(=V)-N-Q-Gruppe in der Formel (I) mit dem Stern (*) markiert ist,
- R: für NR⁷R⁸ oder für einen jeweils gegebenenfalls substituierten Rest aus der Reihe Alkyl, Alkenyl, Alkinyl, Alkoxyalkyl, Alkyl-S(O)ₘ-alkyl, R⁷-CO-alkyl, NR⁷R⁸-CO-alkyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
- W: für einen Rest aus der Reihe O, S, SO und SO₂ steht,
- X: für einen Rest aus der Reihe Wasserstoff, Halogen, Cyano, Nitro, Alkyl, Halogenalkyl, Alkoxy, Halogenalkoxy und Cycloalkyl steht,
- Y: für einen Rest aus der Reihe Wasserstoff, Halogen, Alkyl, Cycloalkyl, Halogenalkyl, Alkoxy, Halogenalkoxy, Cyano und NR⁵R⁶ steht,
- A: für Sauerstoff oder Schwefel steht
- Q: für Stickstoff oder C-R³ steht, worin
- R³: für einen Rest aus der Reihe Wasserstoff, Alkyl, Cycloalkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
- V: für einen Rest aus der Reihe Sauerstoff, Schwefel und NR⁴ steht,
- R⁴: für einen Rest aus der Reihe Wasserstoff, Cyano, Alkyl, Halogenalkyl und Cycloalkyl steht,
- R⁵: für einen Rest aus der Reihe Wasserstoff, Alkyl, Cycloalkyl und Halogenalkyl steht,
- R⁶: für einen Rest aus der Reihe Wasserstoff, Alkyl, Cycloalkyl und Halogenalkyl steht,
oder
- R⁵ und R⁶: gemeinsam mit dem Stickstoff, an dem sie gebunden sind, für einen gegebenenfalls substituierten und gegebenenfalls ein oder mehrere weitere Heteroatome enthaltenden gesättigten oder ungesättigten 3- bis 6-gliedrigen Ring stehen,
- R⁷: für Wasserstoff, Hydroxy oder einen jeweils gegebenenfalls substituierten Rest aus der Reihe Alkyl, Alkoxy, Alkoxyalkyl, Alkyl-S(O)ₘ-alkyl, Alkylcarbonyl, Alkoxycarbonyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
- R⁸: für Wasserstoff, ein Metallion, ein gegebenenfalls substituiertes Ammoniumion oder einen jeweils gegebenenfalls substituierten Rest aus der Reihe Alkyl, Alkoxy, Alkoxyalkyl, Alkyl-S(O)ₘ-alkyl steht und
- m: für eine Zahl aus der Reihe 0, 1 und 2 steht.

Bevorzugte Substituenten bzw. Bereiche der in den Verbindungen der Formel (I) aufgeführten Reste werden im Folgenden erläutert. Ihre Kombination bildet den Vorzugsbereich (1).

Het steht für einen Rest aus der Reihe 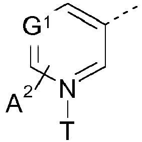 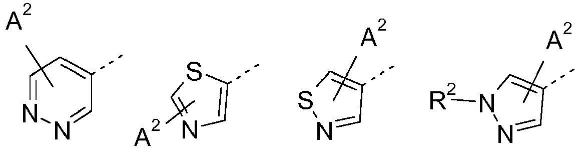 worin die gestrichelte Linie die Bindung zum Kohlenstoffatom in der an Het gebundenen N=C-A-Gruppe darstellt.
- G¹: steht für N oder C-A¹.
- A¹: steht für einen Rest aus der Reihe Wasserstoff, Halogen, C₁-C₆-Alkyl und C₁-C₄-Halogenalkyl.
- A²: steht für einen Rest aus der Reihe Wasserstoff, Halogen, C₁-C₆-Alkyl und C₁-C₄-Halogenalkyl.
- T: steht für Sauerstoff oder ein Elektronenpaar.
- R²: steht für einen Rest aus der Reihe Wasserstoff, C₁-C₆-Alkyl, C₁-C₆-Halogenalkyl und gegebenenfalls einfach bis dreifach durch Halogen, Cyano, Nitro, C₁-C₃-Alkyl, C₁-C₃-Alkoxy und einfach durch C₃-C₆-Cycloalkyl substituiertes C₃-C₆-Cycloalkyl.
- R¹: steht für einen Rest aus der Reihe Wasserstoff, C₁-C₆-Alkyl, C₂-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylsulfanyl-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, Di-(C₁-C₆-alkyl)-aminosulfanyl-C₁-C₆-alkyl, Di-(C₁-C₆)-alkylaminosulfinyl-C₁-C₆-alkyl, Di-(C₁-C₆-alkyl)-aminosulfonyl-C₁-C₆-alkyl, gegebenenfalls durch Halogen substituiertes C₁-C₆-Alkoxycarbonyl, gegebenenfalls durch Halogen substituiertes C₁-C₆-Alkoxycarbonyl-C₁-C₆-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₆-alkyl)-aminocarbonyl, N-C₁-C₆-Alkyl-N-C₃-C₆-cycloalkylaminocarbonyl, Di-(C₁-C₆-alkyl)-aminocarbonyl-C₁-C₆-alkyl, N-C₁-C₆-Alkyl-N-C₃-C₆-cycloalkylaminocarbonyl-C₁-C₆-alkyl, Heterocyclylcarbonyl-C₁-C₆-alkyl, C₁-C₆-Alkylsulfanyl, C₁-C₆-Halogenalkylsulfanyl, C₁-C₆-Alkylsulfinyl, C₁-C₆-Halogenalkylsulfinyl, C₁-C₆-Alkylsulfonyl, C₁-C₆-Halogenalkylsulfonyl, gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, gegebenenfalls durch Halogen, Cyano (auch im C₁-C₆-Alkylteil von Heterocyclyl-C₁-C₆-alkyl), Nitro, Hydroxy, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Alkylsulfonyl, C₁-C₆-Halogenalkylsulfinyl, C₁-C₆-Halogenalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Heterocyclyl-C₁-C₆-alkyl, gegebenenfalls durch Halogen, Cyano, Nitro, Hydroxy, Amino, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy oder C₁-C₆-Halogenalkoxy substituiertes Aryl, gegebenenfalls durch Halogen, Cyano (auch im C₁-C₆-Alkylteil von Aryl-C₁-C₆-alkyl), Nitro, Hydroxy, Amino, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy oder C₁-C₆-Halogenalkoxy substituiertes Aryl-C₁-C₆-alkyl, gegebenenfalls durch Halogen, Cyano, Nitro, Hydroxy, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Alkylsulfonyl, C₁-C₆-Halogenalkylsulfinyl, C₁-C₆-Halogenalkylsulfonyl, Amino, C₁-C₆-Alkylamino, Di-(C₁-C₆-alkyl)-amino, C₁-C₆-Alkylcarbonylamino, C₁-C₆-Alkylaminocarbonyl, Di-(C₁-C₆-alkyl)-aminocarbonyl, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl, gegebenenfalls durch Halogen, Cyano (auch im C₁-C₆-Alkylteil von Hetaryl-C₁-C₆-alkyl), Nitro, Hydroxy, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Alkylsulfonyl, C₁-C₆-Halogenalkylsulfinyl, C₁-C₆-Halogenalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl-C₁-C₆-alkyl.
- R¹: steht auch für einen Rest aus der Reihe 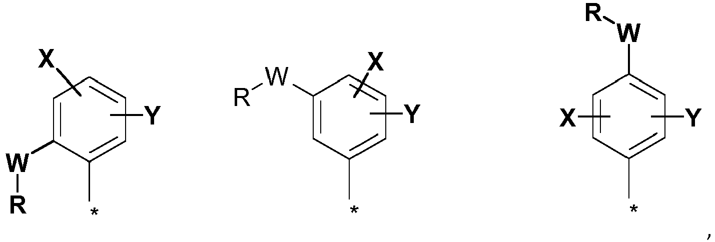 worin die Bindung zum Stickstoffatom in der C(=V)-N-Q-Gruppe in der Formel (I) mit dem Stern (*) markiert ist.
- R: steht für NR⁷R⁸ oder für jeweils gegebenenfalls durch Halogen, Sauerstoff (führt zu C=O) oder Cyano substituiertes C₁-C₆-Alkyl, C₃-C₆-Alkenyl, C₃-C₆-Alkinyl, C₁-C₆-Alkoxy-C₁-C₄-alkyl, C₁-C₆-Alkyl-S(O)ₘ-C₁-C₄-alkyl, für R⁷-CO-C₁-C₄-alkyl, für NR⁷R⁸-CO-C₁-C₄-alkyl, für gegebenenfalls einfach oder zweifach durch Sauerstoff (führt zu C=O), C₁-C₄-Alkyl, C₃-C₈-Cycloalkyl, C₁-C₄-Alkoxy und C₁-C₄-Halogenalkyl substituiertes C₃-C₆-Cycloalkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkenyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₃-C₆-Cycloalkyl, C₁-C₄-Halogenalkyl, C₁-C₄-Alkoxy oder C₁-C₄-Halogenalkoxy substituiertes Phenyl, Phenyl-C₁-C₄-alkyl, Hetaryl und Hetaryl-C₁-C₄-alkyl.
- W: steht für einen Rest aus der Reihe O, S, SO und SO₂.
- X: steht für einen Rest aus der Reihe Wasserstoff, Halogen, Cyano, Nitro, C₁-C₆-Alkyl, C₁-C₆-Halogenalkyl, C₃-C₆-Cycloalkyl, C₁-C₆-Alkoxy und C₁-C₆-Halogenalkoxy.
- Y: steht für aus der Reihe Wasserstoff, Halogen, C₁-C₆-Alkyl, C₃-C₆-Cycloalkyl, C₁-C₆-Halogenalkyl, C₁-C₆-Alkoxy, C₁-C₆-Halogenalkoxy, Cyano und NR⁵R⁶.
- A: steht für Schwefel.
- Q: steht für Stickstoff oder C-R³.
- R³: steht für einen Rest aus der Reihe Wasserstoff, C₁-C₆-Alkyl, C₁-C₆-Halogenalkyl, C₃-C₆-Cycloalkyl, OH, C₁-C₆-Alkoxy, C₁-C₆-Halogenalkoxy, SH, C₁-C₆-Alkylsulfanyl, C₁-C₆-Alkylsulfinyl, C₁-C₆-Alkylsulfonyl, NH₂, C₁-C₆-Alkylamino und Di-(C₁-C₆-alkyl)-amino.
- V: steht für einen Rest aus der Reihe Sauerstoff, Schwefel und NR⁴.
- R⁴: steht für einen Rest aus der Reihe Wasserstoff, Cyano, C₁-C₆-Alkyl, C₂-C₆-Halogenalkyl und C₃-C₆-Cycloalkyl.
- R⁵: steht für einen Rest aus der Reihe Wasserstoff, C₁-C₆-Alkyl, C₃-C₆-Cycloalkyl und C₂-C₆-Halogenalkyl.
- R⁶: steht für einen Rest aus der Reihe Wasserstoff, C₁-C₆-Alkyl, C₃-C₆-Cycloalkyl und C₂-C₆-Halogenalkyl.
- R⁵ und R⁶: können auch gemeinsam mit dem Stickstoffatom, an das sie gebunden sind, für einen gegebenenfalls durch Halogen, Cyano, C₁-C₄-Alkyl, C₁-C₄-Halogenalkyl, C₁-C₄-Alkoxy, C₁-C₄-Halogenalkoxy substituierten gesättigten bis dreifach ungesättigten 3- bis 6-gliedrigen Ring stehen, insbesondere für Aziridinyl, Azirenyl, Diaziridinyl, Diazirenyl, Azetidinyl, Dihydroazetyl, Diazetidinyl, Dihydrodiazetyl, Oxazetidinyl, Oxazetyl, Thiazetidinyl, Thiazetyl, Pyrrolidinyl, Dihydropyrrolyl, Pyrazolidinyl, Dihydropyrazolyl, Imidazolidinyl, Dihydroimidazolyl, Oxazolidinyl, Dihydrooxazolyl, Thiazolidinyl, Dihydrothyazolyl, Piperidinyl, Piperazinyl, Hexahydropyridazinyl, Hexahydropyrimidinyl, Morpholinyl, Dioxazinanyl, Thiomorpholinyl, Dithiazinanyl, Pyrrolyl, Pyrazolyl, Imidazolyl, Triazolyl und Tetrazolyl.
- R⁷: steht für einen Rest aus der Reihe Wasserstoff, Hydroxy, jeweils gegebenenfalls einfach oder mehrfach durch Halogen oder einfach oder zweifach durch Cyano substituiertes C₁-C₆-Alkyl, C₁-C₆-Alkoxy, C₁-C₆-Alkoxy-C₁-C₄-alkyl, C₁-C₆-Alkyl-S(O)ₘ-C₁-C₄-alkyl, C₁-C₆-Alkylcarbonyl, C₁-C₆-Alkoxycarbonyl, C₃-C₆-Cycloalkyl, C₃-C₆-Cycloalkenyl, C₃-C₆-Cycloalkyl-C₁-C₃-alkyl, C₃-C₆-Cycloalkenyl-C₁-C₃-alkyl, Heterocyclyl, Heterocyclyl-C₁-C₃-alkyl und jeweils gegebenenfalls einfach bis vierfach durch C₁-C₄-Alkyl, C₁-C₄-Halogenalkyl, C₁-C₄-Alkoxy, C₁-C₄-Halogenalkoxy, C₃-C₆-Cycloalkyl, Halogen oder Cyano substituiertes Phenyl, Phenyl-C₁-C₃-alkyl, Hetaryl und Hetaryl-C₁-C₃-alkyl.
- R⁸: steht für Wasserstoff, ein Alkali- oder Erdalkalimetallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₄-alkyl und C₁-C₄-Alkyl-S(O)ₘ-C₁-C₄-alkyl.
- m: steht für eine Zahl aus der Reihe 0, 1 und 2.

Besonders bevorzugte Substituenten bzw. Bereiche der in den Verbindungen der Formel (I) aufgeführten Reste werden im Folgenden erläutert. Ihre Kombination bildet den Vorzugsbereich (2).

Het steht für einen Rest aus der Reihe 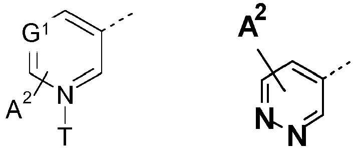 worin die gestrichelte Linie die Bindung zum Kohlenstoffatom in der an Het gebundenen N=C-A-Gruppe darstellt.
- G¹: steht für N oder C-A¹.
- A¹: steht für einen Rest aus der Reihe Wasserstoff, Halogen, C₁-C₄-Alkyl und C₁-C₄-Halogenalkyl.
- A²: steht für einen Rest aus der Reihe Wasserstoff, Halogen, C₁-C₄-Alkyl und C₁-C₄-Halogenalkyl.
- T: steht für Sauerstoff oder ein Elektronenpaar.
- R¹: steht für einen Rest aus der Reihe Wasserstoff, C₁-C₄-Alkyl, C₂-C₄-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylsulfanyl-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, Di-(C₁-C₄-alkyl)-aminosulfanyl-C₁-C₄-alkyl, Di-(C₁-C₄-alkyl)-aminosulfinyl-C₁-C₄-alkyl, Di-(C₁-C₄-alkyl)-aminosulfonyl-C₁-C₄-alkyl, gegebenenfalls durch Halogen substituiertes C₁-C₄-Alkoxycarbonyl, gegebenenfalls durch Halogen substituiertes C₁-C₄-Alkoxycarbonyl-C₁-C₄-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₄-alkyl)-aminocarbonyl, N-C₁-C₄-Alkyl-N-C₃-C₆-cycloalkylaminocarbonyl, Di-(C₁-C₄-alkyl)-aminocarbonyl-C₁-C₄-alkyl, N-C₁-C₄-Alkyl-N-C₃-C₆-cycloalkylaminocarbonyl-C₁-C₄-alkyl, Heterocyclylcarbonyl-C₁-C₄-alkyl, C₁-C₄-Alkylsulfanyl, C₁-C₄-Halogenalkylsulfanyl, C₁-C₄-Alkylsulfinyl, C₁-C₄-Halogenalkylsulfinyl, C₁-C₄-Alkylsulfonyl, C₁-C₄-Halogenalkylsulfonyl, gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkoxycarbonyl, C₁-C₄-Halogenalkoxycarbonyl, Pyridyl, Pyrimidyl, Pyrazanyl, Pyridazinyl, Thiazolyl, Isothiazolyl, Thiadiazolyl, Oxazolyl, Isoxazolyl, Oxadiazolyl, Pyrazolyl, Triazinyl oder Triazolyl (wobei die genannten Hetaryle gegebenenfalls selbst durch C₁-C₄-Alkyl oder Halogen substituiert sind) substituiertes C₃-C₆-Cycloalkyl-C₁-C₄-alkyl, gegebenenfalls durch Halogen, Cyano (auch im C₁-C₄-Alkylteil von Heterocyclyl-C₁-C₄-alkyl), Nitro, Hydroxy, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Heterocyclyl-C₁-C₄-alkyl, gegebenenfalls durch Halogen, Cyano, Nitro, Hydroxy, Amino, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy oder C₁-C₄-Halogenalkoxy substituiertes Aryl, gegebenenfalls durch Halogen, Cyano (auch im C₁-C₄-Alkylteil von Aryl-C₁-C₄-alkyl), Nitro, Hydroxy, Amino, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy oder C₁-C₄-Halogenalkoxy substituiertes Aryl-C₁-C₄-alkyl, gegebenenfalls durch Halogen, Cyano, Nitro, Hydroxy, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkylaminocarbonyl, Di-(C₁-C₄-alkyl)-aminocarbonyl, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl, gegebenenfalls durch Halogen, Cyano (auch im C₁-C₄-Alkylteil von Hetaryl-C₁-C₄-alkyl), Nitro, Hydroxy, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl.
- R¹: steht auch für einen Rest aus der Reihe 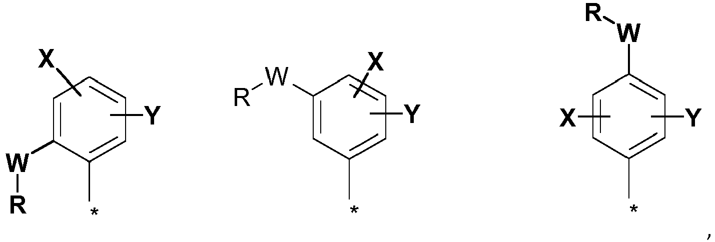 worin die Bindung zum Stickstoffatom in der C(=V)-N-Q-Gruppe in der Formel (I) mit dem Stern (*) markiert ist.
- R: steht für NR⁷R⁸ oder für jeweils gegebenenfalls einfach bis siebenfach durch Halogen, einfach oder zweifach durch Sauerstoff (führt zu C=O) oder einfach oder zweifach durch Cyano substituiertes C₁-C₆-Alkyl, C₃-C₆-Alkenyl, C₃-C₆-Alkinyl, C₁-C₄-Alkoxy-C₁-C₃-alkyl, C₁-C₄-Alkyl-S(O)ₘ-C₁-C₃-alkyl, für R⁷-CO-C₁-C₂-alkyl, für NR⁷R⁸-CO-C₁-C₂-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₈-Cycloalkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkenyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₁-C₄-Halogenalkyl, C₃-C₆-Cycloalkyl, C₁-C₄-Alkoxy oder C₁-C₄-Halogenalkoxy substituiertes Phenyl, Phenyl-C₁-C₄-alkyl, Hetaryl und Hetaryl-C₁-C₄-alkyl.
- W: steht für einen Rest aus der Reihe S, SO und SO₂.
- X: steht für einen Rest aus der Reihe Wasserstoff, Fluor, Chlor, Brom, Jod, Cyano, Nitro, C₁-C₄-Alkyl, C₁-C₄-Halogenalkyl, C₃-C₆-Cycloalkyl, C₁-C₄-Alkoxy und C₁-C₄-Halogenalkoxy.
- Y: steht für einen Rest aus der Reihe Wasserstoff, Fluor, Chlor, Brom, Jod, Cyano, C₁-C₄-Alkyl, C₁-C₄-Halogenalkyl, C₃-C₆-Cycloalkyl, C₁-C₄-Alkoxy und C₁-C₄-Halogenalkoxy.
- A: steht für Schwefel.
- Q: steht für Stickstoff oder C-R³.
- R³: steht für einen Rest aus der Reihe Wasserstoff, C₁-C₄-Alkyl, C₁-C₄-Halogenalkyl, C₃-C₆-Cycloalkyl, OH, C₁-C₄-Alkoxy, C₁-C₄-Halogenalkoxy, SH, C₁-C₄-Alkylsulfanyl, C₁-C₄-Alkylsulfinyl, C₁-C₄-Alkylsulfonyl, NH₂, C₁-C₄-Alkylamino und Di-(C₁-C₄-alkyl)-amino.
- V: steht für Sauerstoff.
- R⁷: steht für einen Rest aus der Reihe Wasserstoff, Hydroxy, jeweils gegebenenfalls einfach oder mehrfach durch Halogen oder einfach oder zweifach durch Cyano substituiertes C₁-C₄-Alkyl, C₁-C₄-Alkoxy, C₁-C₄-Alkoxy-C₁-C₄-alkyl, C₁-C₄-Alkyl-S(O)ₘ-C₁-C₃-alkyl, C₁-C₄-Alkylcarbonyl, C₁-C₄-Alkoxycarbonyl, C₃-C₆-Cycloalkyl, C₃-C₆-Cycloalkyl-C₁-C₃-alkyl, Heterocyclyl, Heterocyclyl-C₁-C₃-alkyl und jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor Chlor, Brom oder Cyano substituiertes Phenyl, Benzyl, Pyridyl, Pyrimidyl, Thiazolyl, Oxazolyl, Pyrazolyl, Thienyl, Furanyl, Pyridinylmethyl und Thiazolylmethyl.
- R⁸: steht für Wasserstoff, für ein Alkali- oder Erdalkalimetallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl.
- m: steht für eine Zahl aus der Reihe 0, 1 und 2.

Ganz besonders bevorzugte Substituenten bzw. Bereiche der in den Verbindungen der Formel (I) aufgeführten Reste werden im Folgenden erläutert. Ihre Kombination bildet den Vorzugsbereich (3).

Het steht für einen Rest aus der Reihe 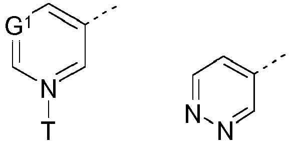 worin die gestrichelte Linie die Bindung zum Kohlenstoffatom in der an Het gebundenen N=C-A-Gruppe darstellt.
- G¹: steht für N oder C-A¹.
- A¹: steht für einen Rest aus der Reihe Wasserstoff, Fluor, Chlor.
- T: steht für Sauerstoff oder ein Elektronenpaar.
- R¹: steht für einen Rest aus der Reihe Methyl, Ethyl, Isopropyl, n-Propyl, n-Butyl, iso-Butyl, sec-Butyl, *tert-Butyl,* 2,2,2-Trifluorethyl, 2,2-Difluorethyl, 2,2-Difluor-n-propyl, Methylsulfanylmethyl, Methylsulfanylethyl, Methylsulfanyl-n-propyl, Trifluormethylsulfonylmethyl, Ethylsulfonylmethyl, 2,2,2-Trifluorethylsulfonylmethyl, 2,2-Difluorethylsulfonylmethyl, Isopropylsulfanylmethyl, Methylsulfinylmethyl, Trifluormethylsulfinylmethyl, Ethylsulfinylmethyl, 2,2,2-Trifluorethylsulfinylmethyl, 2,2-Difluorethylsulfinylmethyl, Isopropylsulfinylmethyl, Methylsulfonylmethyl, Trifluormethylsulfonylmethyl, Ethylsulfonylmethyl, 2,2,2-Trifluorethylsulfonylmethyl, 2,2-Difluorethylsulfonylmethyl, Isopropylsulfonylmethyl, Methoxycarbonyl, Ethoxycarbonyl, Methoxycarbonylmethyl, Ethoxycarbonylmethyl, Methoxycarbonylethyl, Ethoxycarbonylethyl, Dimethylaminocarbonyl, Diethylaminocarbonyl, *N-*Ethyl-*N*-methylaminocarbonyl, *N*-Isopropyl-*N*-methylaminocarbonyl, Dimethylamino-carbonylmethyl, Diethylaminocarbonylmethyl, *N*-Ethyl-*N*-methylaminocarbonylmethyl, *N-*Isopropyl-*N*-methylaminocarbonylmethyl, Dimethylaminocarbonylethyl, Diethylaminocarbonylethyl, *N*-Ethyl-*N*-methylaminocarbonylethyl, *N*-Isopropyl-*N*-methylaminocarbonylethyl, *N*-Cyclopropyl-*N*-methylaminocarbonylmethyl, *N*-Cyclopropyl-*N*-methylaminocarbonylethyl, Methylsulfanyl, Trifluormethylsulfanyl, Ethylsulfanyl, 2,2,2-Trifluorethylsulfanyl, 2,2-Difluorethylsulfanyl, Isopropylsulfanyl, Methylsulfinyl, Trifluormethylsulfinyl, Ethylsulfinyl, 2,2,2-Trifluorethylsulfinyl, 2,2-Difluorethylsulfinyl, Isopropylsulfinyl, Methylsulfonyl, Trifluormethylsulfonyl, Ethylsulfonyl, 2,2,2-Trifluorethylsulfonyl, 2,2-Difluorethylsulfonyl, Isopropylsulfonyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, 2-Cyclopropylcyclopropyl, Cyclobutyl, Cyclopentyl, Cyclohexyl, 4-Trifluormethylcyclohexyl, Cyclopropylmethyl, Cyclopropylethyl, Cyclobutylmethyl, *N*-Cyclopropyl-*N-*methylaminocarbonyl, Morpholin-4-ylcarbonylmethyl, Piperazin-1-ylcarbonylmethyl, 4-Methyl-piperazin-1-ylcarbonylmethyl, jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Heterocyclylmethyl und Heterocyclylethyl, jeweils durch Cyclopropyl substituiertes Heterocyclylmethyl und Heterocyclylethyl, wobei der Cyclopropylrest gegebenenfalls einfach oder zweifach durch Methyl, Fluor, Chlor, Cyano oder einfach durch Cyclopropyl substituiert ist, gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Dimethylaminocarbonyl, substituiertes Hetaryl, durch Cyclopropyl substituiertes Hetaryl, wobei der Cyclopropylrest gegebenenfalls einfach oder zweifach durch Methyl, Fluor, Chlor, Cyano oder einfach durch Cyclopropyl substituiert ist, gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Trifluorethoxy substituiertes Aryl, durch Cyclopropyl substituiertes Aryl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Arylmethyl und Arylethyl, jeweils durch Cyclopropyl substituiertes Arylmethyl und Arylethyl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl, jeweils durch Cyclopropyl substituiertes Hetarylmethyl und Hetarylethyl, wobei der Cyclopropylrest gegebenenfalls einfach oder zweifach durch Methyl, Fluor, Chlor, Cyano oder einfach durch Cyclopropyl substituiert ist.
- R¹: steht auch für einen Rest aus der Reihe 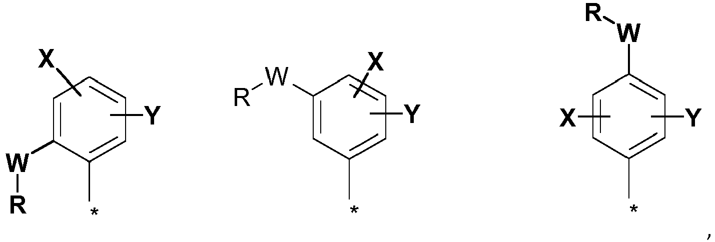 worin die Bindung zum Stickstoffatom in der C(=V)-N-Q-Gruppe in der Formel (I) mit dem Stern (*) markiert ist.
- R: steht für NR⁷R⁸ oder für einen jeweils gegebenenfalls einfach, zweifach, dreifach, vierfach oder fünffach durch Fluor, Chlor oder einfach oder zweifach durch Cyano substituierten Rest aus der Reihe C₁-C₄-Alkyl, C₃-C₄-Alkenyl, C₃-C₄-Alkinyl, C₁-C₂-Alkoxy-C₁-C₂-alkyl und C₁-C₂-Alkyl-S(O)ₘ-C₁-C₂-alkyl, für R⁷-CO-C₁-C₂-alkyl, für NR⁷R⁸-CO-C₁-C₂-alkyl, für gegebenenfalls einfach oder zweifach durch C₁-C₂-Alkyl, C₁-C₂-Alkoxy oder C₁-C₂-Halogenalkyl oder durch ein Sauerstoffatom (führt zu C=O) substituiertes C₃-C₆-Cycloalkyl, für gegebenenfalls einfach oder zweifach durch C₁-C₂-Alkyl, C₁-C₂-Alkoxy oder C₁-C₂-Halogenalkyl oder durch ein Sauerstoffatom (führt zu C=O) substituiertes C₃-C₆-Cycloalkenyl, für gegebenenfalls einfach bis zweifach durch C₁-C₂-Alkyl, C₁-C₂-Alkoxy oder C₁-C₂-Halogenalkyl substituiertes C₃-C₆-Cycloalkyl-C₁-C₂-alkyl, für gegebenenfalls einfach oder zweifach durch C₁-C₂-Alkyl, C₁-C₂-Alkoxy oder C₁-C₂-Halogenalkyl substituiertes C₃-C₆-Cycloalkenyl-C₁-C₂-alkyl, für gegebenenfalls einfach oder zweifach durch C₁-C₂-Alkyl, C₁-C₂-Alkoxy oder C₁-C₂-Halogenalkyl substituiertes Heterocyclyl, für gegebenenfalls einfach oder zweifach durch C₁-C₂-Alkyl, C₁-C₂-Alkoxy oder C₁-C₂-Halogenalkyl substituiertes Heterocyclyl-C₁-C₂-alkyl oder für jeweils gegebenenfalls einfach oder zweifach durch Fluor, Chlor, Brom, Cyano, Methyl, Ethyl, Difluormethyl, Trifluormethyl, Methoxy, Ethoxy, Difluormethoxy oder Trifluormethoxy substituiertes Phenyl, Benzyl, Pyridyl, Pyrimidyl, Thiazolyl, Oxazolyl, Pyrazolyl, Thienyl, Furanyl, Pyridinylmethyl oder Thiazolylmethyl.
- W: steht für einen Rest aus der Reihe S, SO und SO₂.
- X: steht für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy.
- Y: steht für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy.
- A: steht für Schwefel.
- Q: steht für Stickstoff oder C-R³.
- R³: steht für Wasserstoff.
- V: steht für Sauerstoff.
- R⁷: steht für einen Rest aus der Reihe Wasserstoff, Hydroxy, für jeweils gegebenenfalls einfach, zweifach, dreifach, vierfach oder fünffach durch Fluor, Chlor oder einfach oder zweifach durch Cyano substituiertes C₁-C₄-Alkyl, C₁-C₄-Alkoxy, C₁-C₄-Alkoxy-C₁-C₄-alkyl, C₁-C₄-Alkyl-S(O)ₘ-C₁-C₂-alkyl, C₁-C₄-Alkylcarbonyl, C₁-C₄-Alkoxycarbonyl, C₃-C₆-Cycloalkyl, C₃-C₆-Cycloalkyl-C₁-C₃-alkyl, Heterocyclyl und Heterocyclyl-C₁-C₃-alkyl und jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor, Chlor, Brom oder Cyano substituiertes Phenyl, Benzyl, Pyridyl, Pyrimidyl, Thiazolyl, Oxazolyl, Pyrazolyl, Thienyl, Furanyl, Pyridinylmethyl und Thiazolylmethyl.
- R⁸: steht für Wasserstoff, ein Alkali- oder Erdalkaliion, für ein gegebenenfalls einfach bis vierfach durch C₁-C₄-Alkyl substituiertes Ammoniumion oder für einen jeweils gegebenenfalls einfach, zweifach, dreifach, vierfach oder fünffach durch Fluor, Chlor oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl.
- m: steht für eine Zahl aus der Reihe 0, 1 und 2.

Inbesonders bevorzugte Substituenten bzw. Bereiche der in den Verbindungen der Formel (I) aufgeführten Reste werden im Folgenden erläutert. Ihre Kombination bildet den Vorzugsbereich (4).

Het steht für einen Rest aus der Reihe 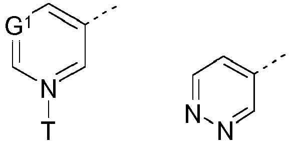 worin die gestrichelte Linie die Bindung zum Kohlenstoffatom in der an Het gebundenen N=C-A-Gruppe darstellt.
- G¹: steht für N oder C-A¹.
- A¹: steht für einen Rest aus der Reihe Wasserstoff, Fluor, Chlor.
- T: steht für Sauerstoff oder ein Elektronenpaar.
- R¹: steht für einen Rest aus der Reihe Methyl, Ethyl, Isopropyl, n-Propyl, n-Butyl, iso-Butyl, sec-Butyl, *tert-*Butyl, 2,2,2-Trifluorethyl, 2,2-Difluorethyl, 2,2-Difluor-n-propyl, Methylsulfanyl-methyl, Methylsulfanylethyl, Methylsulfanyl-n-propyl, Trifluormethylsulfonylmethyl, Ethylsulfonylmethyl, 2,2,2-Trifluorethylsulfonylmethyl, 2,2-Difluorethylsulfonylmethyl, Isopropylsulfanylmethyl, Methylsulfinylmethyl, Trifluormethylsulfinylmethyl, Ethylsulfinyl-methyl, 2,2,2-Trifluorethylsulfinylmethyl, 2,2-Difluorethylsulfinylmethyl, Isopropylsulfinyl-methyl, Methylsulfonylmethyl, Trifluormethylsulfonylmethyl, Ethylsulfonylmethyl, 2,2,2-Trifluorethylsulfonylmethyl, 2,2-Difluorethylsulfonylmethyl, Isopropylsulfonylmethyl, Methoxycarbonyl, Ethoxycarbonyl, Methoxycarbonylmethyl, Ethoxycarbonylmethyl, Methoxycarbonylethyl, Ethoxycarbonylethyl, Dimethylaminocarbonyl, Diethylaminocarbonyl, *N-*Ethyl-*N*-methylaminocarbonyl, *N*-Isopropyl-*N*-methylaminocarbonyl, Dimethylaminocarbonyl-methyl, Diethylaminocarbonylmethyl, *N*-Ethyl-*N*-methylaminocarbonylmethyl, *N*-Isopropyl-*N-*methylaminocarbonylmethyl, Dimethylaminocarbonylethyl, Diethylaminocarbonylethyl, *N-*Ethyl-*N*-methylaminocarbonylethyl, *N*-Isopropyl-*N*-methylaminocarbonylethyl, *N*-Cyclopropyl-*N-*methylaminocarbonylmethyl, *N*-Cyclopropyl-*N*-methylaminocarbonylethyl, Methylsulfanyl, Trifluormethylsulfanyl, Ethylsulfanyl, 2,2,2-Trifluorethylsulfanyl, 2,2-Difluorethylsulfanyl, Isopropylsulfanyl, Methylsulfinyl, Trifluormethylsulfinyl, Ethylsulfinyl, 2,2,2-Trifluorethylsulfinyl, 2,2-Difluorethylsulfinyl, Isopropylsulfinyl, Methylsulfonyl, Trifluormethylsulfonyl, Ethylsulfonyl, 2,2,2-Trifluorethylsulfonyl, 2,2-Difluorethylsulfonyl, Isopropylsulfonyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, 2-Cyclopropylcyclopropyl, Cyclobutyl, Cyclopentyl, Cyclohexyl, 4-Trifluormethylcyclohexyl, Cyclopropylmethyl, Cyclopropylethyl, Cyclobutylmethyl *N*-Cyclopropyl-*N-*methylaminocarbonyl, Morpholin-4-ylcarbonylmethyl, Piperazin-1-ylcarbonylmethyl, 4-Methyl-piperazin-1-ylcarbonylmethyl,
jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Heterocyclylmethyl und Heterocyclylethyl, jeweils durch Cyclopropyl substituiertes Heterocyclylmethyl und Heterocyclylethyl, wobei der Cyclopropylrest gegebenenfalls einfach oder zweifach durch Methyl, Fluor, Chlor, Cyano oder einfach durch Cyclopropyl substituiert ist, gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Dimethylaminocarbonyl substituiertes Hetaryl, durch Cyclopropyl substituiertes Hetaryl, wobei der Cyclopropylrest gegebenenfalls einfach oder zweifach durch Methyl, Fluor, Chlor, Cyano oder einfach durch Cyclopropyl substituiert ist, gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Trifluorethoxy substituiertes Aryl, durch Cyclopropyl substituiertes Aryl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Arylmethyl und Arylethyl, jeweils durch Cyclopropyl substituiertes Arylmethyl und Arylethyl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl, jeweils durch Cyclopropyl substituiertes Hetarylmethyl und Hetarylethyl, wobei der Cyclopropylrest gegebenenfalls einfach oder zweifach durch Methyl, Fluor, Chlor, Cyano oder einfach durch Cyclopropyl substituiert ist.
- R¹: steht auch für den Rest der Formel 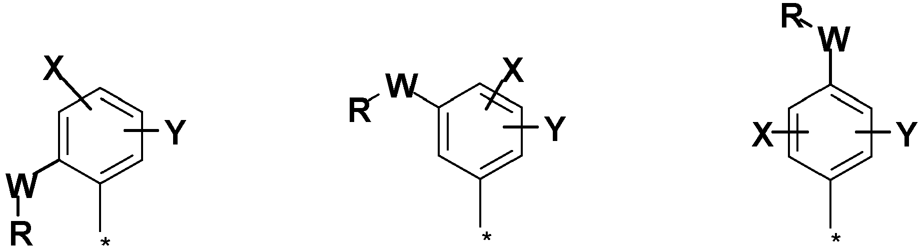 worin die Bindung zum Stickstoffatom in der C(=V)-N-Q-Gruppe in der Formel (I) mit dem Stern (*) markiert ist.
- R: steht für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Methyl, Ethyl, Methoxy, Ethoxy oder Trifluormethyl substituiertes C₃-C₆-Cycloalkyl, für gegebenenfalls einfach durch Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethlyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl.
- W: steht für einen Rest aus der Reihe S, SO und SO₂.
- X: steht für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy.
- Y: steht für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy.
- A: steht für Schwefel.
- Q: steht für Stickstoff oder C-R³.
- R³: steht für Wasserstoff oder Methyl.
- V: steht für Sauerstoff.

Eine weitere bevorzugte Ausführungsform der Erfindung betrifft Verbindungen der Formel (I), in welcher die Substituenten folgende Bedeutungen haben. Ihre Kombination bildet den Vorzugsbereich (5).

Het steht für 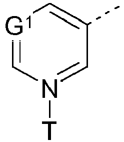 worin die gestrichelte Linie die Bindung zum Kohlenstoffatom in der an Het gebundenen N=C-A-Gruppe darstellt.
- G¹: steht für C-A¹.
- A¹: steht für Wasserstoff.
- T: steht für Sauerstoff oder ein Elektronenpaar.
- R¹: steht für
Methyl, Ethyl, Isopropyl, n-Propyl, n-Butyl, iso-Butyl, sec-Butyl, *tert-Butyl,* 2,2,2-Trifluorethyl, 2,2-Difluorethyl, 2,2-Difluor-n-propyl, Dimethylaminocarbonyl, Diethylaminocarbonyl, *N*-Ethyl-*N*-methylaminocarbonyl, *N*-Isopropyl-*N*-methylaminocarbonyl, Dimethylaminocarbonylmethyl, Diethylaminocarbonylmethyl, *N*-Ethyl-*N*-methylaminocarbonylmethyl, *N*-Isopropyl-*N-*methylaminocarbonylmethyl, Dimethylaminocarbonylethyl, Diethylaminocarbonylethyl, *N*-Ethyl-*N*-methylaminocarbonylethyl, *N*-Isopropyl-*N*-methylaminocarbonylethyl, *N*-Cyclopropyl-*N-*methylaminocarbonylmethyl, *N*-Cyclopropyl-*N*-methylaminocarbonylethyl, Cyclopropyl, Cyclobutyl, Cyclopentyl, Cyclohexyl, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl.
- R¹: steht auch für 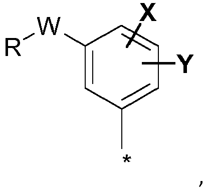 worin die Bindung zum Stickstoffatom in der C(=V)-N-Q-Gruppe in der Formel (I) mit dem Stern (*) markiert ist.
- R: steht für gegebenenfalls einfach bis dreifach durch Fluor oder Chlor substituiertes C₁-C₄-Alkyl.
- W: steht für einen Rest aus der Reihe S und SO.
- X: steht für einen Rest aus der Reihe Wasserstoff, Fluor, Chlor, Brom, Methyl, Ethyl, Difluormethyl und Trifluormethyl.
- Y: steht für einen Rest aus der Reihe Wasserstoff, Fluor, Chlor, Brom, Jod (insbesondere für Fluor), Methyl und Ethyl.
- A: steht für Schwefel.
- Q: steht für Stickstoff oder C-R³.
- R³: steht für Wasserstoff.
- V: steht für Sauerstoff.

Eine weitere bevorzugte Ausführungsform der Erfindung betrifft Verbindungen der Formel (I), in welcher die Substituenten folgende Bedeutungen haben. Ihre Kombination bildet den Vorzugsbereich (6).

Het steht für 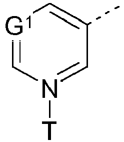 worin die gestrichelte Linie die Bindung zum Kohlenstoffatom in der an Het gebundenen N=C-A-Gruppe darstellt.
- G¹: steht für C-A¹ oder N.
- A¹: steht für Wasserstoff oder Fluor.
- T: steht für Sauerstoff oder ein Elektronenpaar.
- R¹: steht für
Methyl, Ethyl, Isopropyl, n-Propyl, n-Butyl, iso-Butyl, sec-Butyl, *tert-Butyl,* 2,2,2-Trifluorethyl, 2,2-Difluorethyl, 2,2-Difluor-n-propyl, Dimethylaminocarbonyl, Diethylaminocarbonyl, *N*-Ethyl-*N*-methylaminocarbonyl, *N*-Isopropyl-*N*-methylaminocarbonyl, Dimethylaminocarbonylmethyl, Diethylaminocarbonylmethyl, *N*-Ethyl-*N*-methylaminocarbonylmethyl, *N*-Isopropyl-*N-*methylaminocarbonylmethyl, Dimethylaminocarbonylethyl, Diethylaminocarbonylethyl, *N*-Ethyl-*N*-methylaminocarbonylethyl, *N*-Isopropyl-*N*-methylaminocarbonylethyl, *N*-Cyclopropyl-*N-*methylaminocarbonylmethyl, *N*-Cyclopropyl-*N*-methylaminocarbonylethyl, Cyclopropyl, Cyclobutyl, Cyclopentyl, Cyclohexyl, Cyclopropylmethyl, gegebenenfalls durch Dimethylaminocarbonyl substituiertes Benzothienyl, gegebenenfalls durch Halogen, Methyl oder Trifluorethoxy substituiertes Phenyl, gegebenenfalls durch Methyl, Methoxy, Difluormethoxy oder Halogen substituiertes Phenylmethyl, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl.
- R¹: steht auch für 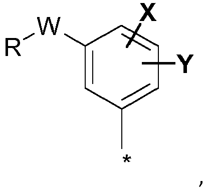 worin die Bindung zum Stickstoffatom in der C(=V)-N-Q-Gruppe in der Formel (I) mit dem Stern (*) markiert ist.
- R: steht für gegebenenfalls einfach bis dreifach durch Fluor oder Chlor substituiertes C₁-C₄-Alkyl.
- W: steht für einen Rest aus der Reihe S,SO und SO₂.
- X: steht für einen Rest aus der Reihe Wasserstoff, Fluor, Chlor, Brom, Methyl, Ethyl, Difluormethyl und Trifluormethyl.
- Y: steht für einen Rest aus der Reihe Wasserstoff, Fluor, Chlor, Brom, Jod (insbesondere für Fluor), Methyl und Ethyl.
- A: steht für Schwefel.
- Q: steht für Stickstoff oder C-R³.
- R³: steht für Wasserstoff oder Methyl.
- V: steht für Sauerstoff.

In den bevorzugten Definitionen, deren Kombination den Vorzugsbereich (1) bildet, ist, sofern nichts anderes angegeben ist,
Alkaliion ausgewählt aus der Reihe Lithium, Natrium, Kalium, Rubidium, Cäsium, bevorzugt aus der Reihe Lithium, Natrium und Kalium,
Erdalkaliion ausgewählt aus der Reihe Beryllium, Magnesium, Calcium, Strontium, Barium, bevorzugt aus der Reihe Magnesium und Calcium,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl ein gesättigter 4-, 5- oder 6-Ring, der 1 oder 2 Stickstoffatome und/oder ein Sauerstoffatom und/oder ein Schwefelatom enthält, beispielsweise Azetidinyl, Azolidinyl, Azinanyl, Oxetanyl, Oxolanyl, Oxanyl, Dioxanyl, Thiethanyl, Thiolanyl, Thianyl, Tetrahydrofuryl, Piperazinyl, Morpholinyl.

In den besonders bevorzugten Definitionen, deren Kombination den Vorzugsbereich (2) bildet, ist, sofern nichts anderes angegeben ist,
Alkaliion ausgewählt aus der Reihe Lithium, Natrium, Kalium, Rubidium, Cäsium, bevorzugt aus der Reihe Lithium, Natrium und Kalium,
Erdalkaliion ausgewählt aus der Reihe Beryllium, Magnesium, Calcium, Strontium, Barium, bevorzugt aus der Reihe Magnesium und Calcium,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl und Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Pyrazolyl, Imidazolyl, 1,2,3-Triazolyl, 1,2,4-Triazolyl, Oxazolyl, Isoxazolyl, Thiazolyl, Isothiazolyl, Pyridyl, Pyrimidinyl, Pyridazinyl, Pyrazinyl, 1,2,3-Triazinyl, 1,2,4-Triazinyl, 1,3,5-Triazinyl, Benzothienyl,
Heterocyclyl ausgewählt aus der Reihe Azetidinyl, Azolidinyl, Azinanyl, Oxetanyl, Oxolanyl, Oxanyl, Dioxanyl, Thiethanyl, Thiolanyl, Thianyl, Tetrahydrofuryl, Piperazinyl, Morpholinyl.

In den ganz besonders bevorzugten Definitionen bzw. den insbesondere bevorzugten Definitionen, deren Kombination den Vorzugsbereich (3) bzw. den Vorzugsbereich (4) bilden, steht, sofern nichts anderes angegeben ist,

Alkaliion für ein Ion aus der Reihe Lithium, Natrium, Kalium, Rubidium, Cäsium, bevorzugt aus der Reihe Lithium, Natrium, Kalium,
Erdalkaliion für ein Ion aus der Reihe Beryllium, Magnesium, Calcium, Strontium und Barium, bevorzugt aus der Reihe Magnesium und Calcium,
Halogen für Fluor, Chlor, Brom und Iod, bevorzugt wiederum für Fluor, Chlor und Brom,
Heterocyclyl für einen Rest aus der Reihe Oxetanyl, Thiethanyl, Tetrahydrofuryl und Morpholinyl, Aryl für Phenyl und
Hetaryl (gleichbedeutend mit Heteroaryl, auch als Teil einer größeren Einheit, wie beispielsweise Hetarylalkyl) für einen Rest aus der Reihe Pyridyl, Pyrimidyl, Pyrazinyl, Pyridazinyl, Thiazolyl, Pyrazolyl und Benzothienyl,.
In den Definitionen, die den Vorzugsbereich (5) bzw. den Vorzugsbereich (6) bilden, steht
Halogen für Fluor, Chlor, Brom und Iod, bevorzugt wiederum für Fluor, Chlor und Brom und
Hetaryl für Pyridyl, Pyrimidinyl, Thiazolyl, Pyrazolyl und Benzothienyl.

Durch Halogen substituierte Reste, z.B. Halogenalkyl (= Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Wenn T im Rest Het der Formel 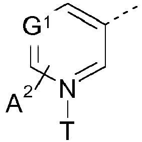 für ein Elektronenpaar steht, liegt der Rest als Pyridinderivat der Formel 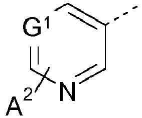 vor.

Wenn T im Rest Het der Formel 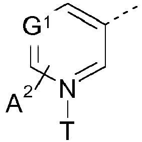 für Sauerstoff steht, liegt der Rest als Pyridin-N-Oxid-derivat der Formel 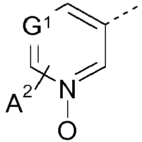 vor. Auf die Darstellung der Formalladungen (+ am Stickstoff und - am Sauerstoff) wurde hier verzichtet (vgl. aber Beispiel 24 in Tabelle 1).

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt sind Verbindungen der Formel (I), in welchen eine Kombination der vorstehend als bevorzugt aufgeführten Bedeutungen vorliegt (Vorzugsbereich (1)).

Erfindungsgemäß besonders bevorzugt sind Verbindungen der Formel (I), in welchen eine Kombination der vorstehend als besonders bevorzugt aufgeführten Bedeutungen vorliegt (Vorzugsbereich (2)).

Erfindungsgemäß ganz besonders bevorzugt sind Verbindungen der Formel (I), in welchen eine Kombination der vorstehend als ganz besonders bevorzugt aufgeführten Bedeutungen vorliegt (Vorzugsbereich (3)).

Erfindungsgemäß insbesonders bevorzugt sind Verbindungen der Formel (I), in welchen eine Kombination der vorstehend als insbesonders bevorzugt aufgeführten Bedeutungen vorliegt (Vorzugsbereich (4)).

Eine weitere Gruppe von herausgehobenen Verbindungen der Formel (I) sind diejenigen, die den Vorzugsbereich (5) bilden.

Eine weitere Gruppe von herausgehobenen Verbindungen der Formel (I) sind diejenigen, die den Vorzugsbereich (6) bilden.

Eine bevorzugte Ausführungsform der Erfindung betrifft Verbindungen der Formel (I), in welchen Het für den Rest der Formel 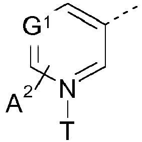 steht.

Eine weitere bevorzugte Ausführungsform der Erfindung betrifft Verbindungen der Formel (I), in welchen Het für Pyridin-3-yl steht.

Eine weitere bevorzugte Ausführungsform der Erfindung betrifft Verbindungen der Formel (I), in welchen Het für 5-Fluor-pyridin-3-yl steht.

Eine weitere bevorzugte Ausführungsform der Erfindung betrifft Verbindungen der Formel (I), in welchen Het für Pyrimidin-5-yl steht.

Eine weitere bevorzugte Ausführungsform der Erfindung betrifft Verbindungen der Formel (I), in welchen Het für Pyridazin-4-yl steht.

Eine weitere bevorzugte Ausführungsform der Erfindung betrifft Verbindungen der Formel (I), in welchen R¹ für den Rest 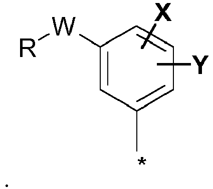 steht.

Eine weitere bevorzugte Ausführungsform der Erfindung betrifft Verbindungen der Formel (I), in welchen Q für C-H steht.

Die Verbindungen der Formel (I) können gegebenenfalls auch in Abhängigkeit von der Art der Substituenten als Stereoisomere, d.h. als geometrische und/oder als optische Isomere oder Isomerengemische in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren Formen der Verbindungen der Formel (I) und deren Salze erfindungsgemäß verwendet.

Die Erfindung betrifft daher sowohl die reinen Enantiomeren und Diastereomeren, als auch deren Gemische zur Bekämpfung von tierischen Schädlingen, zu denen Arthropoden und insbesondere Insekten zählen.

Weiter wurde gefunden, dass sich die neuen Verbindungen der Formel (I) nach den im Folgenden beschriebenen Verfahren herstellen lassen.

Die Verbindungen der Formel (I) können beispielsweise nach Verfahren A in zwei Schritten hergestellt werden, wie im folgenden Schema dargestellt. Die dafür benötigten Aminothiazole der Formel (III) können beispielweise nach dem Verfahren B aus Heteroaryl-thioamiden hergestellt werden.

Die Verbindungen der Formel (II) sind ebenfalls Gegenstand der Erfindung und können wie in den nachfolgenden Verfahren beschrieben hergestellt werden. 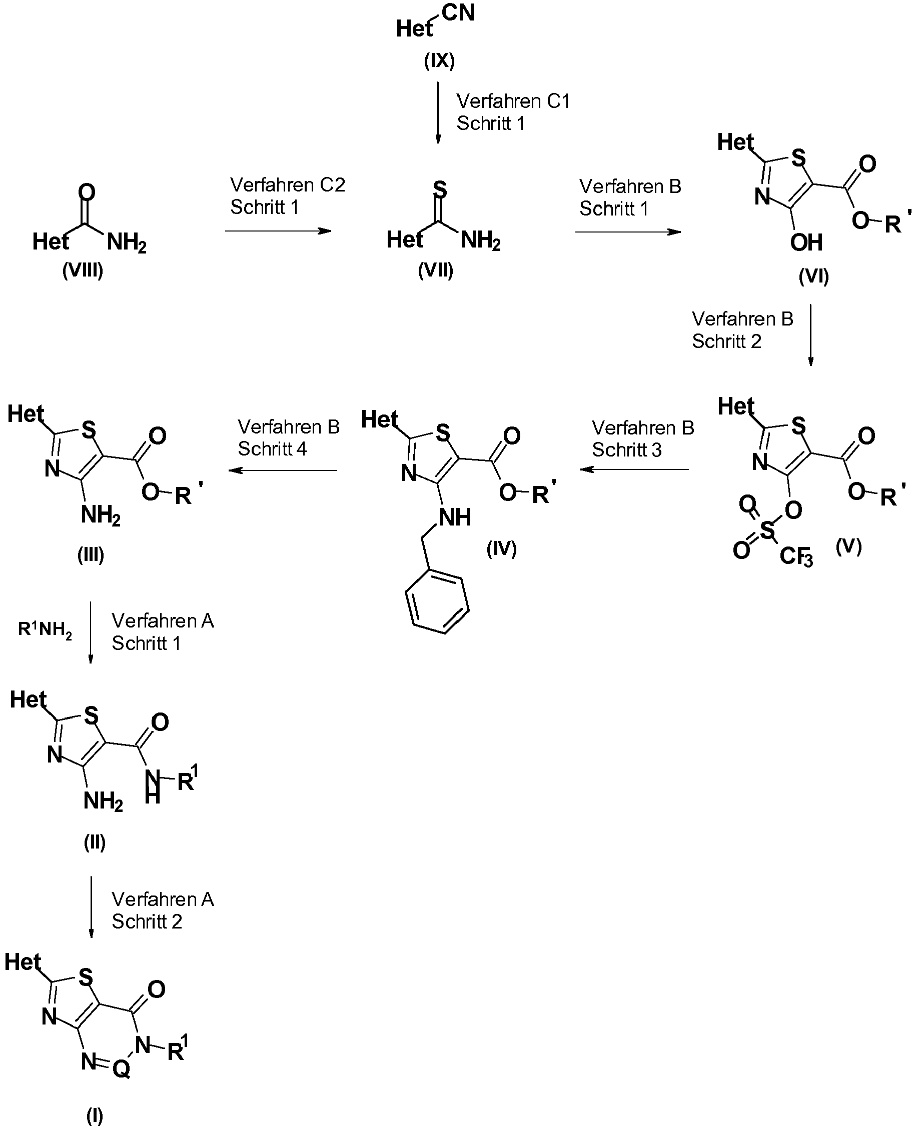 wobei Het, Q und R¹ die oben angegebenen Bedeutungen haben und R' für Wasserstoff oder Alkyl (insbesondere Methyl und Ethyl) steht.

### Verfahren A

Die Synthese der Verbindungen der Formel (I) kann mit Hilfe von literaturbekannten Methoden in zwei Schritten erfolgen.

Im ersten Syntheseschritt können Verbindungen der Formel (III) nach verschiedenen Methoden zu Carbonsäureamiden der Formel (II) umgesetzt werden. Für R' = Alkyl kann diese Umsetzung ohne Aktivierung erfolgen (vgl. B. M. Trost und I. Fleming in Comprehensive Organic Synthesis, Ed. Pergamon, 1991, Vol. 6). Alternativ sind in der Literatur Aktivierungsmethoden durch die Bildung eines Aluminiumamids bekannt (siehe T. Ooi und K. Marouka in Science of Synthesis, Ed. Georg Thieme, 2003, Vol. 7, 225-246). Diese Aluminiumamide können beispielweise aus den Aminen oder deren Salzen durch Umsetzung mit Trimethylaluminium oder deren luft-stabilem Addukt mit 1,4-Diazobicyclo[2.2.3]oktan (DABCO) hergestellt werden (vgl. S. Woodward in Tet. Lett. 2006, 47, 5767-5769).

Alternativ können die Aminothiazole der Formel (III) mit R' = Alkyl in zwei Stufen zu den Amiden der Formeln (II) umgesetzt werden: zuerst Verseifung zu den Carboxylaten, zum Beispiel durch Umsetzung mit einer anorganischen Base (bevorzugt Natron- und Kalilaugen), gegebenenfalls in einem inerten organischen Lösungsmittel, gegebenenfalls lassen sich durch Ansäuern mit einer verdünnten Säure (z.B. wässrige Salzsäure) die Carbonsäuren der Formeln (III) mit R' = Wasserstoff herstellen und isolieren; anschließende Amidierungsreaktion mit den gewünschten Aminen führt zu den Verbindungen der Formel (II). Für den Amidierungsschritt sind zahlreiche Reaktionsbedingungen beschrieben worden, z.B. G. Benz in Comprehensive Organic Synthesis, 1st Ed., Pergamon Press, Oxford, 1991, Vol. 6, S. 381-417; P.D. Bailey et al. in Comprehensive Organic Functional Group Transformation, 1st Ed., Elsevier Science Ltd., Oxford, 1995, Vol. 5, S. 257-308 und R.C. Larock in Comprehensive Organic Transformations, 2nd Ed., Wiley-VCH, New York, Weinheim, 1999, S. 1929-1994. Einige dieser Reaktionen verlaufen über intermediäre Carbonsäurechloride, die isoliert oder in-situ erzeugt eingesetzt werden können.

Die Amidierungsreaktionen erfolgen gegebenenfalls in Gegenwart eines Kondensationsmittels, gegebenenfalls in Gegenwart eines Säureakzeptors und gegebenenfalls in Gegenwart eines Lösungsmittels.

Als Kondensationsmittel kommen alle üblicherweise für derartige Amidierungsreaktionen verwendbaren Kondensationsmittel infrage. Beispielhaft genannt seien Säurehalogenidbildner wie Phosgen, Phosphortrichlorid, Oxalylchlorid oder Thionylchlorid; Carbodiimide, wie *N,N'-*Dicyclohexylcarbodiimid (DCC) und 1-(3-Dimethylaminopropyl)-3-ethyl-carbodiimid (EDCI), oder andere übliche Kondensationsmittel, wie Phosphorpentoxid, Polyphosphorsäure, *N,N'-*Carbonyldiimidazol, 2-Chlorpyridin 1-Methoiodid (Mukaiyamas Reagenz), 2-Ethoxy-N-ethoxycarbonyl-1,2-dihydrochinolin (EEDQ), Triphenylphosphin/Tetrachlorkohlenstoff, Bromtripyrrolidino-phosphonium-hexafluorphosphat (BROP), O-(*1H*-Benzotriazol-1-yloxy)tris(dimethylamino)phosphonium-hexafluorphosphat (BOP), *N,N,N',N'-*Bis(tetramethylen)cloruronium-tetrafluorborat, O-(*1H*-Benzotriazol-1-yl)-*N,N,N',N'-*tetramethyluronium-hexafluorphosphat (HBTU), O-(*1H*-Benzotriazol-1-yl)-*N,N,N',N'-*bis(tetramethylen)uronium-hexafluorphosphat, O-(*1H*-Benzotriazol-1-yl)*-N,N,N',N'-*tetramethyluronium-tetrafluorborat (TBTU), O-(*1H*-Benzotriazol-1-yl)-*N,N,N',N'-*bis(tetramethylen)uronium-tetrafluorborat, O-(7-Azabenzotriazol-1-yl)-*N,N,N',N'*-tetramethyluronium-hexafluorphosphat (HATU), 1-Hydroxybenzotriazol (HOBt) und 4-(4,6-Dimethoxy-1,3,5-triazin-2-yl)-4-methylmorpholiniumsalz (DMT.MM), meistens als Chlorid verfügbar. Diese Reagenzien können separat oder gegebenenfalls in Kombination eingesetzt werden.

Als Säureakzeptor kommen alle üblichen anorganischen oder organischen Basen infrage, beispielsweise Triethylamin, Diisopropylethylamin, *N*-Methylmorpholin oder *N,N*-Dimethylaminopyridin. Das erfindungsgemäße Verfahren A wird gegebenenfalls in Gegenwart eines geeigneten Reaktionshilfstoffes wie beispielsweise *N,N*-Dimethylformamid oder *N,N*-Dimethylaminopyridin durchgeführt. Als Lösungs- oder Verdünnungsmittel kommen alle interten organischen Lösungsmittel in Betracht, beispielsweise aliphatische oder aromatische Kohlenwasserstoffe (wie Petrolether, Toluol), halogenierte Kohlenwasserstoffe (wie Chlortoluol, Dichlormethan, Chloroform, 1,2-Dichlorethan), Ether (wie Diethylether, Dioxan, Tetrahydrofuran, 1,2-Dimethoxyethan), Ester (wie Essigsäureethylester- oder - methylester), Nitrokohlenwasserstoffe (wie Nitromethan, Nitroethan, Nitrobenzol), Nitrile (wie Acetonitril, Benzonitril), Amide (wie *N,N*-Dimethylformamid, *N,N*-Dimethylacetamide, *N-*Methylformanilid, *N*-Methylpyrrolidon, Hexamethylphosphorsäuretriamid) sowie Dimethylsulfoxid oder Wasser oder Gemische der genannten Lösungsmittel.

Es können auch gemischte Anhydride zur Darstellung von Verbindungen der Formel (III) verwendet werden (vgl. J. Am. Chem. Soc. 1967, 5012). Bei diesem Verfahren können verschiedene Chlorameisensäureester zum Einsatz kommen, wie z.B. Chlorameisensäureisobutylester und Chlorameisensäureisopropylester. Ebenfalls können dafür Diethylacetylchlorid, Trimethylacetylchlorid und ähnliche verwendet werden.

In einem zweiten Syntheseschritt können die Carbonsäureamide der Formel (II) zu den Verbindungen der Formel (I) cyclisiert werden.

Für Q = C-R³, worin R³ für H oder Alkyl steht, kann die Cyclisierung von Carbonsäurenamiden der Formel (II) mit einem Orthoester, wie Orthoameisensäuretriethylester oder Orthoessigsäuretriethylester, gegebenenfalls in Gegenwart eines Lösungs- und Verdünnungsmittel (beispielsweise in Gegenwart von Alkoholen wie Ethanol, aber auch in Gegenwart von *N,N*-Dimethylformamid oder *N,N-*Dimethylacetamid), gegebenenfalls in Gegenwart einer organischen Säure (zum Beispiel *para-*Toluolsulfonsäure oder Essigsäure) oder anorganischen Säure (zum Beispiel Salzsäure oder Schwefelsäure) in katalytischen oder stöchiometrischen Mengen oder im Überschuss durchgeführt werden. Die genannten Säuren können auch anstelle des Lösungs- oder Verdünnungsmittels eingesetzt werden. Für R³ = H finden sich Beispiele für solche Reaktionen mit Orthoameisensäuretriethylester in Archiv der Pharmazie 2000, 333(8), 261-266 (zur Herstellung von Chinazolinonen), J. Het. Chem. 1990, 27(7), 1953-1956 (idem.), WO2010/54398 (zur Herstellung von Pyrazinopyrimidinonen). Für R³ = Methyl siehe zum Beispiel WO2010/100189 (zur Herstellung von Chinazolinonen).

Für Q = C-R³, worin R³ für Alkyl oder Halogenalkyl steht, kann die Herstellung der Thiazolopyrimidinone der Formeln (I) auch durch Umsetzung der Carbonsäurenamide der Formel (II) mit entsprechenden Carbonsäurehalogeniden oder Carbonsäurenanhydriden nach literaturbekannten Methoden erfolgen, wie zum Beispiel in WO2009/143049 für R³ = Methyl und in WO 2008/039489 für R³ = Trifluormethyl beschrieben.

Für Q = N können die Thiazolopyrimidinone der Formeln (I) durch Azodiazotierung der Carbonsäureamide der Formeln (II) nach literaturbekannten Methoden hergestellt werden. Beispielsweise werden Verbindungen der Formeln (II) bei 0 bis 5 °C mit einer Nitritquelle, wie Natriumnitrit oder Isobutylnitrit versetzt, typischerweise in Wasser, Alkohol oder einem polaren, inerten Lösungsmittel und in Gegenwart einer organischen oder anorganischen Säure. Beispiele für Reaktionsbedingungen finden sich in WO 2004/242572 oder in J. Amer. Chem. Soc. Perkin Trans. 1, 1980, 633-638.

Die Verbindungen der Formel (II) sind auch Gegenstand der Erfindung.

### Allgemeine Verfahren für die Oxidation von Thioethern zu Sulfoxiden und Sulfonen

Verbindungen der Formel (I), in welchen W für SO steht (Sulfoxide) oder W für SO₂ steht (Sulfone) lassen sich durch Oxidation nach literaturbekannten Verfahren aus Verbindungen der Formel (I), in welchen W für S (Thioethern) herstellen, beispielsweise durch ein Oxidationsmittel in einem geeigneten Lösungs- bzw. Verdünnungsmittel. Als Oxidationsmittel eignen sich zum Beispiel verdünnte Salpetersäure, Wasserstoffperoxid, Oxone® und Peroxycarbonsäuren, wie etwa *meta-*Chlorperbenzoesäure. Als Lösungsmittel bzw. Verdünnungsmittel eignen sich inerte organische Lösungsmittel, typischerweise Acetonitril und halogenierte Lösungsmittel wie Dichlormethan, Chloroform oder Dichlorethan, sowie Wasser und Alkohole wie Methanol für die Reaktion mit Oxone®. Möglich ist auch die Einführung geeigneter Aniline R¹-NH₂, Halogenide R¹-Hal oder Boronsäuren R¹-B(OH)₂, in welchen W für SO oder SO₂ steht, nach Verfahren A-1 oder A-2. Diese lassen sich aus den entsprechenden Vorstufen, in denen W für S steht, nach literaturbekannten Verfahren oxidieren, wie zum Beispiel in WO 2013/092350 beschrieben.

Zur Erzeugung enantiomeren angereicherten Sulfoxide eignen sich eine Vielfalt von Methoden, wie von A.R. Maguire in ARKIVOC, 2011(i), 1-110, beschrieben: metall-katalysierte asymmetrische Oxidationen von Thioethern, zum Beispiel mit Titanium oder Vanadium als meistbenutzten Katalysatorquellen, in Form von Ti(OⁱPr₄) oderVO(acac)₂, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nicht-metall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, beispielsweise durch präparative Trennung mittels einer chiralen HPLC.

Die im Verfahren A benötigten Aminothiazole der Formeln (III) können beispielweise nach den Verfahren B hergestellt werden. Im Falle von nicht kommerziell verfügbaren Heteroaryl-thioamiden als Ausgangsstoffe für Verfahren B können diese bekanntermaßen nach den Verfahren C1 oder C2 synthetisiert werden:

### Verfahren C1

Heteroaryl-thioamide der Formel (VII) können in einem Schritt nach im Prinzip bekannten Methoden aus den entsprechenden kommerziell erhältlichen Heteroaryl-carbonsäureamiden der Formel (VIII) und geeigneten Schwefeldonatoren hergestellt werden. So wird in WO 2013/104415 durch Petterson et al. eine Methode zur Transformierung von Carbonylgruppen in Thiocarbonylgruppen beschrieben, indem die entsprechenden Ausgangsverbindungen bei einer Temperatur zwischen 50 °C und 100 °C mit einer geeigneten Schwefelverbindung, z.B. Diphosphorpentasulfid-Dipyridinkomplex, umgesetzt werden, gegebenenfalls in einem inerten organischen Lösungsmittel (z.B. Acetonitril).

### Verfahren C2

Heteroaryl-thioamide der Formel (VII) können alternativ zu Verfahren B1 auch in einem Schritt nach im Prinzip bekannten Methoden aus den entsprechenden kommerziell erhältlichen Heteroaryl-nitrilen der Formel (IX) und geeigneten Schwefeldonatoren hergestellt werden. Zum Beispiel nach Mahammed et al. (Journal of Sulfur Chemistry, 28(3), 233-237; 2007) durch Umsetzung mit Thioessigsäure unter Präsenz von Calciumhydrid bei einer Temperatur zwischen 50 °C und 100°C in lösungsmittelfreier Umgebung. Alternativ ist z. B. auch eine mikrowellenunterstützte Synthese beschrieben (Synlett, (14), 2615-2617; 2004), indem die entsprechenden Nitrile bei einer Temperatur zwischen 50 °C und 100 °C mit Ammoniumsulfid in einem inerten organischen Lösungsmittel (z.B. Alkoholen) umgesetzt werden.

### Verfahren B

Beispiele für die Umsetzung von Thioamiden zu Thiazolderivaten des Typs (VI) sind in WO 2013/092711 A1 beschrieben (vgl. auch Bioorganic & Medicinal Chemistry Letters (2010), 20(9), 2828-2831). Die kommerziell erhältlichen oder nach Verfahren C1 oder C2 herzustellenden Thioamide (VII) werden zunächst in einem geeigneten inerten Lösungsmittel (z.B. Dioxan oder Toluol) unter Rückflusstemperatur, ggf. in Anwesenheit einer Base (z. B. Pyridin) mit Dimethyl-2-chloromalonat oder Dimethyl-2-bromomalonat zur gewünschten Verbindung (VI) umgesetzt.

Anschließend gelingt (z. B. nach Bioorganic & Medicinal Chemistry Letters (2010), 20(9), 2828-2831) durch Umsetzung von (VI) mit Trifluormethansulfonsäureanhyrid in Anwesenheit einer geeigneten Base (z. B. Triethylamin, Natriumcarbonat, Pyridin oder Lutidin) in einem inerten Lösungsmittel (z. B. Dichlormethan) bei einer Temperatur zwischen -20 °C bis Raumtemperatur die Herstellung der Verbindung (V).

Verbindungen des Typs (V) sind bekannterweise (z.B. nach WO 2003/101985 A1) transformierbar in Verbindungen des Typs (IV) durch Erhitzen mit Benzylamin in inerten Lösungsmitteln (z. B. Dioxan) bei Temperaturen zwischen 50 bis 100 °C.

Die Abspaltung der Benzylaminfunktion zur Darstellung der Zielverbindungen des Typs (III) kann nach im Prinzip bekannten Verfahren (z.B. WO 2003/066629 A2) durch Erhitzen in geeigneten Säuren (z. B. Trifluoressigsäure oder Schwefelsäure) bei Temperaturen zwischen 20 und 80 °C erfolgen. Ggf. dabei gebildete Säureamide werden durch Erhitzen mit Kaliumcarbonat in einem Wasser-Methanolgemisch zur Zielverbindung (III) hydrolysiert.

Die so hergestellten Thiazol-Derivate der Formeln (VI) bis (III) können getrennt werden, zum Beispiel mittels chromatographischer Trennung über Kieselgel oder RP(C-18) oder durch Verrühren oder Umkristallisieren unter Verwendung geeigneter Lösungsmittel.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Die Offenbarung betrifft auch Verfahren zur Bekämpfung von tierischen Schädlingen, bei dem man Verbindungen der Formel (I) auf tierische Schädlinge und/oder ihren Lebensraum einwirken lässt.

Bevorzugt wird die Bekämpfung der tierischen Schädlinge in der Land- und Forstwirtschaft und im Materialschutz durchgeführt. Hierunter ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Offenbarung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder Chloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,
weitere Wirkstoffe wie beispielsweise Afidopyropen, Azadirachtin, Benclothiaz, Benzoximate, Bifenazate, Bromopropylate, Chinomethionat, Cryolite,

Dicofol, Diflovidazin, Fluensulfone, Flometoquin, Flufenerim, Flufenoxystrobin, Flufiprole, Fluopyram, Flupyradifurone, Fufenozide, Heptafluthrin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis von Bacillus firmus (I-1582, BioNeem, Votivo), sowie folgende Verbindungen:3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{5-[3-Chlor-5-(trifluormethyl)phenyl]-5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazin-carboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethyl-hydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid.
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2- {2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1), (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb,, (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3-methoxy-4-(prop-2-yn-1-yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)imino][6-(difluormethoxy)-2,3-difluorphenyl]methyl} -2-phenylacetamid, (15.85) N- {(Z)-[(Cyclopropylmethoxy)imino] [6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)methylene]amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl}-N-ethyl-N-methylimidoformamid, (15.155) N'- {5-Brom-6-[(cis-4-isopropylcyclohexyl)oxy]-2-methylpyridin-3-yl} -N-ethyl-N-methylimidoformamid, (15.156) N'- {5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
Bacillus amyloliquefaciens, Stamm FZB42 (DSM 231179), oder Bacillus cereus, insbesondere B. cereus Stamm CNCM I-1562 oder Bacillus firmus, Stamm I-1582 (Accession number CNCM I-1582) oder Bacillus pumilus, insbesondere Stamm GB34 (Accession No. ATCC 700814) und Stamm QST2808 (Accession No. NRRL B-30087), oder Bacillus subtilis, insbesondere Stamm GB03 (Accession No. ATCC SD-1397), oder Bacillus subtilis Stamm QST713 (Accession No. NRRL B-21661) oder Bacillus subtilis Stamm OST 30002 (Accession No. NRRL B-50421) Bacillus thuringiensis, insbesondere B. thuringiensis subspecies israelensis (serotype H-14), Stamm AM65-52 (Accession No. ATCC 1276), oder B. thuringiensis subsp. aizawai, insbesondere Stamm ABTS-1857 (SD-1372), oder B. thuringiensis subsp. kurstaki Stamm HD-1, oder B. thuringiensis subsp. tenebrionis Stamm NB 176 (SD-5428), Pasteuria penetrans, Pasteuria spp. (Rotylenchulus reniformis nematode)-PR3 (Accession Number ATCC SD-5834), Streptomyces microflavus Stamm AQ6121 (= QRD 31.013, NRRL B-50550), Streptomyces galbus Stamm AQ 6047 (Acession Number NRRL 30232).

Beispiele für Pilze und Hefen, die als biologische Schädlingsbekämpfungsmittel eingesetzt werden bzw. verwendet werden können, sind:
Beauveria bassiana, insbesondere Stamm ATCC 74040, Coniothyrium minitans, insbesondere Stamm CON/M/91-8 (Accession No. DSM-9660), Lecanicillium spp., insbesondere Stamm HRO LEC 12, Lecanicillium lecanii, (ehemals bekannt als Verticillium lecanii), insbesondere Stamm KV01, Metarhizium anisopliae, insbesondere Stamm F52 (DSM3884/ ATCC 90448), Metschnikowia fructicola, insbesondere Stamm NRRL Y-30752, Paecilomyces fumosoroseus (neu: Isaria fumosorosea), insbesondere Stamm IFPC 200613, oder Stamm Apopka 97 (Accesion No. ATCC 20874), Paecilomyces lilacinus, insbesondere P. lilacinus Stamm 251 (AGAL 89/030550), Talaromyces flavus, insbesondere Stamm V117b, Trichoderma atroviride, insbesondere Stamm SC1 (Accession Number CBS 122089), Trichoderma harzianum, insbesondere T. harzianum rifai T39. (Accession Number CNCM I-952).

Beispiele für Viren, die als biologische Schädlingsbekämpfungsmittel eingesetzt werden bzw. verwendet werden können, sind:
Adoxophyes orana (Apfelschalenwickler) Granulosevirus (GV), Cydia pomonella (Apfelwickler) Granulosevirus (GV), Helicoverpa armigera (Baumwollkapselwurm) Nuklear Polyhedrosis Virus (NPV), Spodoptera exigua (Zuckerrübeneule) mNPV, Spodoptera frugiperda (Heerwurm) mNPV, Spodoptera littoralis (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
Agrobacterium spp., Azorhizobium caulinodans, Azospirillum spp., Azotobacter spp., Bradyrhizobium spp., Burkholderia spp., insbesondere Burkholderia cepacia (ehemals bekannt als Pseudomonas cepacia), Gigaspora spp., oder Gigaspora monosporum, Glomus spp., Laccaria spp., Lactobacillus buchneri, Paraglomus spp., Pisolithus tinctorus, Pseudomonas spp., Rhizobium spp., insbesondere Rhizobium trifolii, Rhizopogon spp., Scleroderma spp., Suillus spp., Streptomyces spp..

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lager-raum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung eignet sich daher insbesondere auch für ein Verfahren zum Schutz von Saatgut und keimenden Pflanzen vor dem Befall von Schädlingen, indem das Saatgut mit einer der Verbindungen der Formel (I) behandelt wird. Das Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Stemostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung. Die Produkte wurden mittels 1H-NMR Spektroskopie und/oder LC/MS (Liquid Chromatography Mass Spectrometry) charakterisiert.

Die Bestimmung der logP Werte erfolgte gemäß OECD Guideline 117 (EC Directive 92/69/EEC) durch HPLC (High Performance Liquid Chromatography) an reversed-phase (RP) Säulen (C18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität 1); δ₂ (Intensität 2);........; δᵢ (Intensität i);......; δₙ (Intensität n)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D6 und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

### Synthese von Verbindungen der Formel (I) nach Verfahren A

### Herstellbeispiel 1: 6-Isopropyl-2-(pyridin-3-yl)[1,3]thiazolo[4,5-d]pyrimidin-7(6H)-on (1)

### Schritt 1: 4-Amino-N-isopropyl-2-(pyridin-3-yl)-1,3-thiazol-5-carboxamid (II-1)

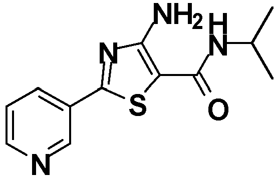

Unter Argon wurden zu 356 mg (6,02 mmol) Isopropylamin in 1,2-Dichlorethan 3 ml einer 2M Trimethylaluminium-Lösung (6,02 mmol) in Toluol langsam getropft und 30 min bei Raumtemperatur gerührt. Anschließend wurden 500 mg (2,00 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 100 ml) ausgetragen. Das Gemisch wurde drei Mal mit Dichlormethan extrahiert, die vereinigten organischen Phasen über Natriumsulfat getrocknet, filtriert und eingeengt. Der Rückstand wurde mit etwas Acetonitril zu einer Suspension verrührt. Der unlösliche Teil wurde abgesaugt und unter Vakuum getrocknet. Man erhielt 340 mg (99% Reinheit, 63,9% d. Th.) der Titelverbindung (II-1).
**1H-NMR(400,0 MHz, d₆-DMSO):** δ = 9,046(2,3); 9,042(2,4); 8,704(1,8); 8,700(2,0); 8,692(2,0); 8,688(1,9); 8,218(1,0); 8,214(1,4); 8,208(1,0); 8,198(1,2); 8,194(1,5); 8,193(1,5); 8,188(1,1); 7,639(1,3); 7,620(1,3); 7,581(1,4); 7,579(1,4); 7,569(1,4); 7,567(1,4); 7,561(1,4); 7,559(1,3); 7,549(1,3); 7,547(1,3); 6,982(4,0); 4,077(0,7); 4,061(1,0); 4,042(1,0); 4,025(0,7); 3,904(0,9); 3,338(73,4); 2,526(0,6); 2,513(11,6); 2,509(23,1); 2,504(30,0); 2,500(21,6); 2,495(10,5); 1,143(16,0); 1,126(15,8); 0,000(7,2);-0,008(0,3)

### Schritt 2: 6-Isopropyl-2-(pyridin-3-yl)[1,3]thiazolo[4,5-d]pyrimidin-7(6H)-on (1)

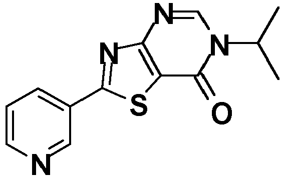

300 mg (1,14 mmol) 4-Amino-N-isopropyl-2-(pyridin-3-yl)-1,3-thiazol-5-carboxamid (II-1) wurden in *N,N*-Dimethylacetamid vorgelegt. 98 mg (0,57 mmol) *p*-Toluolsulfonsäure und 339 mg (2,29 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde im Vakuum eingeengt, der Rückstand mit Acetonitril verrührt und der Feststoff abfiltriert. Das Filtrat wurde anschließend eingeengt und mittels MPLC über eine RP(C-18) Säule chromatographisch gereinigt. Man erhielt 25 mg (96% Reinheit, 7,6% d. Th.) der Titelverbindung (1).
**1H-NMR (400,0 MHz, d₆-DMSO):** δ = 9,307(2,2); 9,302(2,2); 8,814(1,6); 8,811(1,7); 8,802(1,7); 8,799(1,7); 8,734(6,2); 8,516(1,0); 8,512(1,3); 8,506(1,0); 8,496(1,1); 8,491(1,4); 8,486(1,0); 8,144(3,9); 7,659(1,3); 7,647(1,3); 7,641(1,3); 7,639(1,3); 7,628(1,2); 7,627(1,2); 5,054(0,4); 5,037(1,1); 5,019(1,5); 5,002(1,1); 4,985(0,4); 3,337(1,4); 2,526(0,8); 2,513(17,3); 2,508(34,7); 2,504(45,2); 2,499(32,4); 2,495(15,5); 1,483(16,0); 1,466(15,8); 1,142(0,4); 1,126(0,4); 0,000(8,5)

### Herstellbeispiel 2: 6-Isobutyl-2-(pyridin-3-yl)[1,3]thiazolo[4,5-d]pyrimidin-7(6H)-on (3)

### Schritt 1: 4-Amino-N-isobutyl-2-(pyridin-3-yl)-1,3-thiazol-5-carboxamid ((II-3))

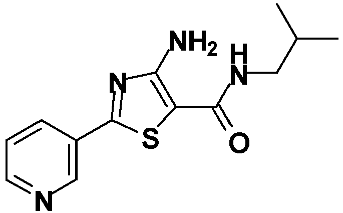

1,75 g (7,02 mmol) Ethyl-4-amino-2-(pyridin-3-yl)-1,3-thiazol-5-carboxylat (III-1) wurden in Ethanol vorgelegt und mit 1,97 g Kaliumhydroxid (35,10 mmol), gelöst in Ethanol, versetzt. Das Reaktionsgemisch wurde über Nacht bei 50 °C gerührt und anschließend zur Trockne eingeengt. Der Rückstand wurde in wenig Wasser angeschlemmt und das Produkt mit 10%iger wässriger Zitronensäurelösung ausgefällt. Der unlösliche Anteil wurde abgesaugt und gut unter Vakuum getrocknet. Die so erhaltene 4-Amino-2-(pyridin-3-yl)-1,3-thiazol-5-carbonsäure (1,32 g, 100% Reinheit, 85,0% d. Th.) wurde direkt weiter umgesetzt.
**1H-NMR(400,0 MHz, d₆-DMSO):** δ = 9,112(1,2); 9,108(1,4); 9,099(0,7); 9,086(15,3); 9,085(15,4); 9,080(16,0); 9,018(0,3); 9,012(0,4); 8,730(1,2); 8,727(1,2); 8,719(1,4); 8,715(1,4); 8,701(13,6); 8,697(14,4); 8,689(14,5); 8,685(14,2); 8,607(0,4); 8,603(0,4); 8,595(0,4); 8,297(0,7); 8,292(0,9); 8,287(0,8); 8,277(0,9); 8,271(1,2); 8,262(8,1); 8,258(10,9); 8,253(8,0); 8,242(8,7); 8,238(11,2); 8,233(8,1); 7,580(1,0); 7,566(1,5); 7,560(11,6); 7,558(11,0); 7,548(11,5); 7,546(10,9); 7,540(10,5); 7,538(9,8); 7,528(10,0); 7,526(9,3); 7,506(0,4); 7,494(0,4); 7,486(0,4); 7,473(0,4); 7,133(1,1); 6,917(1,6); 6,028(1,3); 4,271(0,9); 4,253(2,8); 4,235(2,8); 4,218(0,9); 3,578(0,4); 3,545(0,5); 3,466(1,1); 3,448(1,5); 3,431(1,8); 3,324(25,6); 3,120(0,5); 3,092(0,4); 2,951(0,3); 2,675(2,3); 2,671(3,1); 2,667(2,3); 2,555(5,6); 2,541(2,3); 2,524(9,2); 2,517(30,9); 2,511(156,3); 2,506(313,9); 2,502(413,1); 2,497(295,7); 2,493(141,3); 2,481(18,0); 2,443(5,2); 2,338(1,1); 2,333(2,2); 2,329(3,0); 2,324(2,2); 2,290(0,4); 1,909(0,8); 1,295(3,0); 1,277(6,4); 1,260(3,0); 1,073(0,8); 1,055(1,5); 1,038(0,7); 0,146(1,3); 0,008(11,0); 0,000(302,5); -0,009(11,0); -0,150(1,3)

100 mg (0,45 mmol) dieser 4-Amino-2-(pyridin-3-yl)-1,3-thiazol-5-carbonsäure wurden anschließend in Toluol vorgelegt. 376 mg (3,16 mmol) Thionylchlorid wurden zugegeben und das Reaktionsgemisch 3 Stunden unter Rückfluß erhitzt. Nach dem Abkühlen wurde der Ansatz im Vakuum eingedampft und im Hochvakuum getrocknet. Das so erhaltene Säurechlorid wurde in Acetonitril aufgenommen und zu einer Lösung aus 88 mg (0,68 mmol) N,N-Diisopropylethylamin und 36,36 mg (0,50 mmol) Isobutylamin in Acetonitril gegeben. Die Reaktionsmischung wurde anschließen über Nacht bei Raumtemperatur gerührt. Nach Abdampfen des Lösungsmittel wird der Rückstand in Ethylacetat und gesättigter Natriumchloridlösung aufgenommen. Die organische Phases wurde abgetrennt, mit Natriumsulfat getrocknet und eingedampft. Man erhielt so 71 mg (84% Reinheit, 47,7% d. Th.) der Titelverbindung (II-3), die direkt in Schritt 2 weiter zu (3) umgesetzt wurden.
**LC-MS: Masse gefunden** [m/z] = 276,10

### Schritt 2: 6-Isobutyl-2-(pyridin-3-yl)[1,3]thiazolo[4,5-d]pyrimidin-7(6H)-on (3)

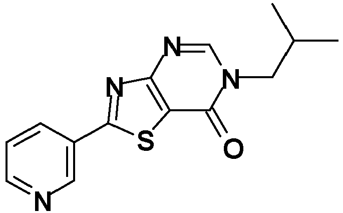

71 mg (0,22 mmol; 84%ig) 4-Amino-N-isobutyl-2-(pyridin-3-yl)-1,3-thiazol-5-carboxamid (II-3) wurden in 266 mg (3,05 mmol) *N,N-*Dimethylacetamid vorgelegt. 2 mg (0,013 mmol) *p-*Toluolsulfonsäure und 57 mg (0,39 mmol) Orthoameisensäuretriethylester wurden dazu gegeben und das Reaktionsgemisch für 10 min in einer CEM Discover Mikrowelle bei 200 Watt auf 120 °C erhitzt. Das abgekühlte Reaktionsgemisch wurde eingeengt und chromatographisch gereinigt. Man erhielt 24 mg (90% Reinheit, 34,3% d. Th.) der Titelverbindung (3).
**1H-NMR(400,0 MHz, d₆-DMSO):** δ = 9,302(2,1); 9,297(2,1); 9,049(0,4); 9,044(0,4); 8,815(1,8); 8,811(1,9); 8,803(1,8); 8,799(1,8); 8,703(0,3); 8,699(0,4); 8,691(0,4); 8,687(0,4); 8,634(6,2); 8,510(1,1); 8,506(1,4); 8,500(1,1); 8,490(1,1); 8,486(1,4); 8,484(1,4); 8,480(1,1); 7,662(1,3); 7,660(1,4); 7,650(1,3); 7,648(1,3); 7,642(1,3); 7,640(1,3); 7,630(1,2); 7,628(1,3); 6,974(0,7); 3,895(4,7); 3,876(4,7); 3,323(24,1); 3,022(0,4); 3,005(0,6); 2,990(0,4); 2,512(10,6); 2,508(21,2); 2,503(27,9); 2,499(20,3); 2,495(10,0); 2,162(0,4); 2,145(0,8); 2,128(1,0); 2,111(0,9); 2,093(0,4); 0,916(16,0); 0,899(15,5); 0,873(3,2); 0,856(3,1); 0,008(0,3); 0,000(8,4); -0,008(0,3)

### Herstellbeispiel 3: 2-(Pyridin-3-yl)-6-(2,2,2-trifluorethyl)[1,3]thiazolo[4,5-d]pyrimidin-7(6H)-on (5)

### Schritt 1: 4-Amino-2-(pyridin-3-yl)-N-(2,2,2-trifluorethyl)-1,3-thiazol-5-carboxamid (II-5)

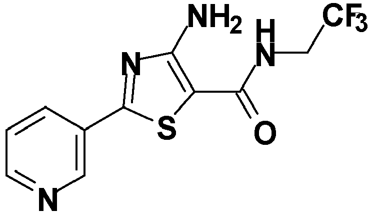

Unter Argon wurden zu 596 mg (6,02 mmol) 2,2,2-Trifluorethylamin in 1,2-Dichlorethan 3 ml einer 2M Trimethylaluminium-Lösung (6,02 mmol) in Toluol langsam getropft und 30 min bei Raumtemperatur gerührt. Anschließend wurden 500 mg (2,0 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 100 ml) ausgetragen. Das Gemisch wurde drei Mal mit Methylenchlorid ausgeschüttelt, die organische Phase abgetrennt, über Natriumsulfat getrocknet, filtriert und eingeengt. Der Rückstand wurde mit etwas Acetonitril zu einer Suspension verrührt. Der unlösliche Teil wurde abgesaugt und unter Vakuum getrocknet. Man erhielt 299 mg (91% Reinheit, 44,8% d. Th.) der Titelverbindung (II-5).
**1H-NMR(400,0 MHz, d₆-DMSO):** δ = 9,114(0,8); 9,110(0,8); 9,074(9,3); 9,070(9,3); 8,728(7,9); 8,724(7,8); 8,716(8,3); 8,712(7,5); 8,476(3,0); 8,461(6,3); 8,446(3,0); 8,318(0,4); 8,298(0,4); 8,293(0,5); 8,288(0,4); 8,277(0,4); 8,272(0,5); 8,268(0,5); 8,249(4,1); 8,245(5,6); 8,240(4,0); 8,229(4,5); 8,225(5,8); 8,224(5,8); 8,220(4,1); 7,594(5,4); 7,593(5,3); 7,582(5,6); 7,580(5,7); 7,574(5,3); 7,573(5,1); 7,562(5,2); 7,560(5,2); 7,548(0,5); 7,164(16,0); 5,759(1,8); 4,272(0,5); 4,254(1,5); 4,236(1,5); 4,218(0,5); 4,040(1,7); 4,016(5,6); 4,000(5,7); 3,992(6,1); 3,976(5,5); 3,968(2,4); 3,952(1,9); 3,905(2,0); 3,332(49,0); 2,678(0,5); 2,673(0,6); 2,669(0,4); 2,509(72,6); 2,504(92,8); 2,500(67,2); 2,335(0,5); 2,331(0,6); 2,327(0,4); 1,296(1,6); 1,279(3,2); 1,261(1,5); 0,008(2,8); 0,000(58,8); -0,008(2,6)

### Schritt 2: 2-(Pyridin-3-yl)-6-(2,2,2-trifluorethyl)[1,3]thiazolo[4,5-d]pyrimidin-7(6H)-on (5)

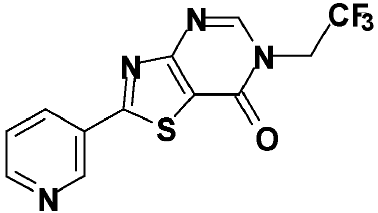

240 mg (0,79 mmol) 4-Amino-2-(pyridin-3-yl)-N-(2,2,2-trifluorethyl)-1,3-thiazol-5-carboxamid (II-5) wurden in 3 ml *N,N-*Dimethylacetamid vorgelegt. 68 mg (0,40 mmol) *p*-Toluolsulfonsäure und 235 mg (1,59 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde im Vakuum eingeengt, der Rückstand mit Acetonitril verrührt und der Feststoff abfiltriert. Dieser wurde anschließend mittels MPLC über eine RP(C-18) Säule mit gereinigt. Man erhielt 69 mg (92% Reinheit, 25,6% d. Th.) der Titelverbindung (5).
**1H-NMR(400,0 MHz, d₆-DMSO):** δ = 9,320(8,2); 9,315(8,2); 9,302(0,4); 8,829(6,4); 8,825(6,8); 8,817(6,9); 8,813(6,6); 8,698(16,0); 8,530(4,0); 8,526(5,1); 8,520(3,9); 8,510(4,3); 8,506(5,3); 8,505(5,3); 8,500(4,0); 8,318(0,4); 7,672(4,8); 7,671(4,8); 7,660(4,8); 7,659(4,7); 7,652(4,8); 7,651(4,6); 7,640(4,5); 7,639(4,4); 5,143(0,3); 5,121(0,4); 5,089(3,5); 5,067(11,4); 5,044(11,9); 5,021(4,0); 3,363(0,6); 3,336(289,1); 2,720(1,5); 2,678(0,6); 2,674(0,8); 2,669(0,6); 2,527(2,1); 2,513(48,7); 2,509(96,7); 2,505(125,4); 2,500(89,6); 2,496(42,7); 2,336(0,6); 2,331(0,8); 2,327(0,6); 2,077(0,4); 0,008(1,2); 0,000(30,0); -0,008(1,1)

### Herstellbeispiel 4: 6-(3-Methoxyphenyl)-2-(pyridin-3-yl)[1,3]thiazolo[4,5-d]pyrimidin-7(6H)-on (10)

### Schritt 1: 4-Amino-N-(3-methoxyphenyl)-2-(pyridin-3-yl)-1,3-thiazol-5-carboxamid (II-10)

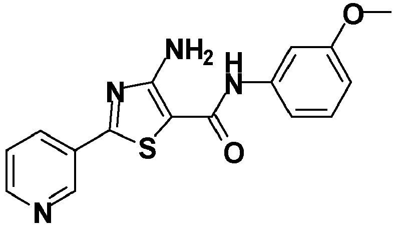

Unter Argon wurden zu 445 mg (3,61 mmol) 3-Methoxy-anilin in 1,2-Dichlorethan 1,8 ml einer 2M Trimethylaluminium-Lösung (3,61 mmol) in Toluol langsam getropft und 1 h bei Raumtemperatur gerührt. Anschließend wurden 300 mg (1,2 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 120 ml) ausgetragen. Das Gemisch wurde drei Mal mit Methylenchlorid extrahiert, die vereinigten organischen Phasen über Natriumsulfat getrocknet, filtriert und eingeengt. Der Rückstand wurde mit etwas Acetonitril zu einer Suspension verrührt. Der unlösliche Teil wurde abgesaugt und unter Vakuum getrocknet. Man erhielt 120 mg (96% Reinheit, 29,3% d. Th.) der Titelverbindung (II-10).
**1H-NMR(400,0 MHz, d₆-DMSO):** δ = 9,618(2,9); 9,113(0,4); 9,107(0,5); 9,099(2,3); 9,093(2,3); 8,736(1,5); 8,732(1,7); 8,724(1,7); 8,720(1,7); 8,271(1,0); 8,266(1,5); 8,261(0,9); 8,251(0,8); 8,246(1,4); 8,241(0,9); 7,607(1,3); 7,595(1,3); 7,587(1,3); 7,575(1,4); 7,361(1,4); 7,355(2,5); 7,350(1,7); 7,303(1,0); 7,282(1,7); 7,238(3,5); 7,232(3,1); 7,211(2,7); 7,191(1,2); 6,658(1,2); 6,652(1,2); 6,638(1,1); 6,631(1,1); 4,253(0,7); 4,235(0,7); 3,747(16,0); 3,330(15,3); 2,525(0,6); 2,507(25,7); 2,503(34,3); 2,499(26,3); 2,076(0,7); 1,295(0,8); 1,278(1,5); 1,260(0,7); 0,008(1,5); 0,007(1,5); 0,000(44,0); -0,008(1,9)

### Schritt 2: 6-(3-Methoxyphenyl)-2-(pyridin-3-yl)[1,3]thiazolo[4,5-d]pyrimidin-7(6H)-on (10)

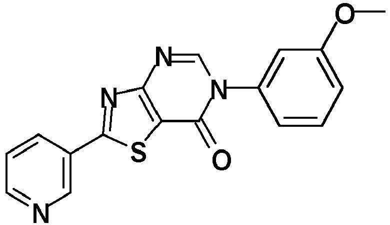

100 mg (0,29 mmol) 4-Amino-N-(3-methoxyphenyl)-2-(pyridin-3-yl)-1,3-thiazol-5-carboxamid (II-10) wurden in *N,N-*Dimethylacetamid vorgelegt. 25 mg (0,15 mmol) *p*-Toluolsulfonsäure und 87 mg (0,59 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde mit Wasser versetzt und mit Dichlormethan mehrfach extrahiert. Die vereinigten organischen Phasen wurden mit Natriumsulfat getrocknet, filtriert und eingedampft. Der Rückstand wurde anschließend per MPLC säulenchromatographisch gereinigt. Man erhielt 20 mg (100% Reinheit, 20,2% d. Th.) der Titelverbindung (10).
**1H-NMR(400,0 MHz, d₆-DMSO):** δ = 9,345(0,5); 9,340(0,5); 8,833(0,4); 8,829(0,4); 8,821(0,4); 8,817(0,4); 8,620(1,4); 7,495(0,6); 7,475(0,4); 7,217(0,3); 7,211(0,6); 7,206(0,4); 7,155(0,4); 7,134(0,5); 3,818(3,8); 3,328(15,7); 2,944(16,0); 2,784(13,5); 2,507(16,3); 2,502(21,3); 2,498(16,0); 1,957(14,1); 0,008(1,0); 0,000(24,1); -0,008(1,1)

### Herstellbeispiel 5: 6-{2-Fluor-4-methyl-5-[(2,2,2-trifluorethyl)sulfinyl]phenyl}-2-(pyridin-3-yl)[1,3]thiazolo[4,5-d]pyrimidin-7(6H)-on (22)

### Schritt 1: 4-Amino-N-{2-fluor-4-methyl-5-[(2,2,2-trifhiorethyl)sulfanyl]phenyl}-2-(pyridin-3-yl)-1,3-thiazol-5-carboxamid (II-21)

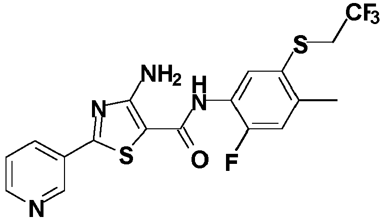

Unter Argon wurden zu 720 mg (3,01 mmol) 2-Fluor-4-methyl-5-[(2,2,2-trifluorethyl)sulfanyl]anilin in 1,2-Dichlorethan 1,5 ml einer 2M Trimethylaluminium-Lösung (3,01 mmol) in Toluol langsam getropft und 30 min bei Raumtemperatur gerührt. Anschließend wurden 250 mg (1,00 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 200 ml) ausgetragen. Das Gemisch wurde drei Mal mit Methylenchlorid extrahiert, die vereinigten organischen Phasen über Natriumsulfat getrocknet, filtriert und eingeengt. Der Rückstand wurde mit etwas Acetonitril zu einer Suspension verrührt. Der unlösliche Teil wurde abgesaugt und unter Vakuum getrocknet. Man erhielt 144 mg (92% Reinheit, 29,9% d. Th.) der Titelverbindung (II-21).
**1H-NMR(601,6 MHz, d₆-DMSO):** δ = 9,566(4,6); 9,110(0,4); 9,100(3,4); 9,096(3,4); 8,737(2,4); 8,734(2,6); 8,729(2,8); 8,726(2,8); 8,270(1,3); 8,267(1,9); 8,264(1,3); 8,257(1,3); 8,254(2,0); 8,251(1,3); 7,696(2,8); 7,683(2,8); 7,601(1,7); 7,594(1,7); 7,588(1,7); 7,580(1,6); 7,268(2,5); 7,250(2,5); 7,209(5,5); 4,250(0,6); 4,238(0,6); 3,891(1,1); 3,874(3,4); 3,857(3,5); 3,840(1,2); 3,343(61,4); 2,616(0,4); 2,507(51,6); 2,504(70,6); 2,501(53,2); 2,406(16,0); 1,290(0,6); 1,278(1,2); 1,266(0,6); 0,005(1,5); 0,000(41,7)

### Schritt 2: 6-{2-Fluor-4-methyl-5-[(2,2,2-trifluorethyl)sulfanyl]phenyl}-2-(pyridin-3-yl)[1,3]thiazolo[4,5-d]pyrimidin-7(6H)-on (21)

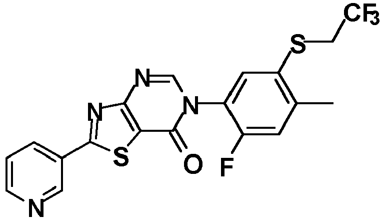

120 mg (0,27 mmol) 4-Amino-N-{2-fluor-4-methyl-5-[(2,2,2-trifluorethyl)sulfanyl]phenyl}-2-(pyridin-3-yl)-1,3-thiazol-5-carboxamid (II-21) wurden in *N,N-*Dimethylacetamid vorgelegt. 23 mg (0,14 mmol) *p*-Toluolsulfonsäure und 80 mg (0,54 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde im Vakuum eingeengt, der Rückstand mit Acetonitril verrührt und das Produkt als Feststoff abfiltriert. Das Filtrat wurde anschließend eingeengt und mittels MPLC über eine RP(C-18) Säule chromatographisch gereinigt. Man erhielt in Summe 43 mg (98% Reinheit, 34,3% d. Th.) der Titelverbindung (21).
**1H-NMR(400,0 MHz, d₆-DMSO):** δ = 9,350(3,0); 9,346(3,1); 9,344(3,0); 8,839(2,4); 8,836(2,6); 8,827(2,6); 8,824(2,6); 8,679(8,7); 8,561(1,4); 8,557(1,8); 8,551(1,4); 8,541(1,5); 8,537(1,9); 8,535(1,9); 8,531(1,5); 7,939(3,1); 7,921(3,1); 7,684(1,8); 7,683(1,7); 7,672(1,7); 7,671(1,7); 7,664(1,8); 7,663(1,7); 7,652(1,7); 7,651(1,7); 7,518(2,7); 7,491(2,6); 4,062(1,2); 4,037(3,9); 4,011(4,0); 3,985(1,4); 3,330(57,6); 2,945(1,0); 2,785(0,7); 2,672(0,4); 2,526(1,0); 2,512(19,8); 2,508(40,6); 2,503(53,8); 2,499(39,4); 2,494(19,5); 2,471(16,0); 2,330(0,3); 2,076(10,9); 1,958(0,8); 0,008(1,0); 0,000(30,1);-0,009(1,2)

### Schritt 3 - Hauptprodukt: 6-{2-Fluor-4-methyl-5-[(2,2,2-trifluorethyl)sulfinyl]phenyl}-2-(pyridin-3-yl)[1,3]thiazolo[4,5-d]pyrimidin-7(6H)-on (22)

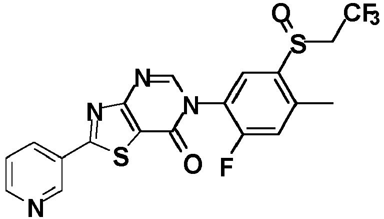

Zu einer Lösung von 32,0 mg (0,07 mmol) 6-{2-Fluor-4-methyl-5-[(2,2,2-trifluorethyl)sulfanyl]phenyl}-2-(pyridin-3-yl)[1,3]thiazolo[4,5-d]pyrimidin-7(6H)-on (21) in 3 ml Dichlormethan wurden bei 0 °C 19,18 mg (70%ig, 0,08 mmol) meta-Chlorperbenzoesäure gegeben. Das Reaktionsgemisch wurde 2 Stunden bei Raumtemperatur gerührt und dann mit gesättigter Natriumcarbonat-Lösung versetzt. Nach 20 Minuten wurden die Phasen getrennt, die wässrige Phase noch zweimal mit Dichlormethan extrahiert und die vereinigten organischen Phasen mit Natriumsulfat getrocknet. Nach Verdampfung des Lösungsmittels wurde der Rückstand anschließend mittels MPLC über einer RP(C-18) Säule chromatographisch gereinigt. Man erhielt 10 mg (95% Reinheit, 28,1% d. Th.) der Titelverbindung (22).
**1H-NMR(400,0 MHz, d₆-DMSO):** δ = 9,761(0,5); 9,353(6,6); 9,347(6,9); 9,102(0,4); 8,836(5,2); 8,828(4,9); 8,824(5,2); 8,738(0,5); 8,723(15,6); 8,557(4,0); 8,553(2,9); 8,543(2,8); 8,537(4,3); 8,533(3,1); 8,315(2,5); 8,195(6,5); 8,176(6,5); 8,039(0,4); 7,951(2,4); 7,685(3,8); 7,673(3,8); 7,665(4,0); 7,653(3,8); 7,635(5,0); 7,608(5,1); 7,366(0,4); 7,238(0,6); 5,755(1,0); 4,293(0,9); 4,271(1,2); 4,263(1,3); 4,233(1,1); 4,182(0,4); 4,124(1,0); 4,100(1,2); 3,505(1,6); 3,322(748,9); 2,944(1,9); 2,891(16,0); 2,784(1,6); 2,731(14,3); 2,716(0,5); 2,670(6,0); 2,666(4,8); 2,505(695,9); 2,501(960,0); 2,497(767,5); 2,395(0,8); 2,373(2,4); 2,328(6,2); 2,324(5,1); 2,283(0,4); 1,957(1,9); 1,303(0,3); 1,284(0,6); 1,233(2,0); 0,854(0,4); 0,146(3,0); 0,008(21,8); 0,000(638,0); -0,078(0,4); -0,150(3,2)

### Schritt 3 - Nebenprodukt: 6-{2-Fluor-4-methyl-5-[(2,2,2-trifluorethyl)sulfinyl]phenyl}-2-(1-oxidopyridin-3-yl)[1,3]thiazolo[4,5-d]pyrimidin-7(6H)-on (23)

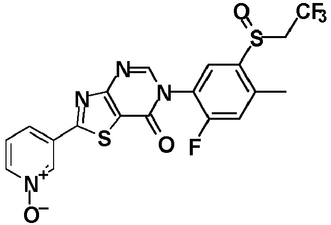

Bei der Herstellung der Verbindung (23) nach "Schritt 3 - Hauptprodukt" wurde gleichzeitig die Verbindung (23) als Nebenprodukt isoliert. Man erhielt 11 mg (94% Reinheit, 30,5% d. Th.) der Titelverbindung (23).
**1H-NMR(400,0 MHz, d₆-DMSO):** δ = 8,928(9,2); 8,734(16,0); 8,463(5,2); 8,446(5,3); 8,315(0,9); 8,196(7,0); 8,177(7,1); 8,082(5,1); 8,061(5,7); 7,668(4,5); 7,649(5,9); 7,633(8,7); 7,608(5,6); 7,573(0,4); 7,251(0,4); 4,265(1,6); 4,236(1,3); 4,117(1,2); 4,106(1,2); 4,092(1,4); 3,322(182,5); 2,671(2,1); 2,502(339,3); 2,498(298,4); 2,402(0,7); 2,373(1,6); 2,329(2,4); 0,146(1,6); 0,000(312,8); -0,083(0,4); - 0,150(1,7)

### Herstellbeispiel 6: 2-(Pyridin-3-yl)-6-[3-(trifluormethyl)benzyl][1,3]thiazolo[4,5-d]pyrimidin-7(6H)-on (28)

### Schritt 1: 4-Amino-2-(pyridin-3-yl)-N-[3-(trifluormethyl)benzyl]-1,3-thiazol-5-carboxamid (11-28)

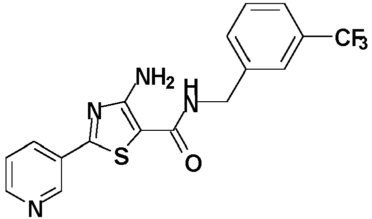

Unter Argon wurden zu 843 mg (4,81 mmol) 3-(Trifluormethyl)-benzylamin in 20 ml 1,2-Dichlorethan 2,4 ml einer 2M Trimethylaluminium-Lösung (4,81 mmol) in Toluol langsam getropft und 30 min bei Raumtemperatur gerührt. Anschließend wurden 400 mg (1,61 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 120 ml) ausgetragen. Das Gemisch wurde drei Mal mit Dichlormethan extrahiert, die vereinigten organischen Phasen über Natriumsulfat getrocknet, filtriert und eingeengt. Der Rückstand wurde mit etwas n-Pentan zu einer Suspension verrührt. Der unlösliche Teil wurde abgesaugt und unter Vakuum getrocknet. Man erhielt 425 mg (100% Reinheit, 70,0% d. Th.) der Titelverbindung (II-28).
**1H-NMR(400,0 MHz, d₆-DMSO):** δ = 9,063(9,1); 9,058(9,1); 8,714(6,6); 8,710(7,3); 8,702(7,1); 8,698(7,3); 8,543(2,9); 8,528(6,3); 8,514(3,0); 8,317(0,6); 8,237(3,6); 8,232(5,3); 8,227(3,9); 8,217(4,0); 8,212(5,8); 8,207(4,1); 7,656(9,5); 7,633(3,5); 7,615(8,3); 7,609(10,3); 7,591(9,1); 7,583(6,9); 7,572(9,3); 7,563(5,7); 7,552(5,8); 7,161(0,4); 7,099(0,5); 7,050(16,0); 6,941(0,4); 5,757(3,2); 4,483(12,6); 4,469(12,6); 3,903(1,3); 3,800(0,5); 3,327(183,9); 2,676(1,2); 2,672(1,8); 2,667(1,3); 2,525(4,0); 2,511(99,6); 2,507(202,4); 2,503(270,1); 2,498(201,5); 2,333(1,3); 2,329(1,8); 2,325(1,4); 1,989(0,6); 0,146(1,4); 0,008(9,9); 0,000(288,4); -0,008(11,9); -0,150(1,4)

### Schritt 2: 2-(Pyridin-3-yl)-6-[3-(trifluormethyl)benzyl][1,3]thiazolo[4,5-d]pyrimidin-7(6H)-on (28)

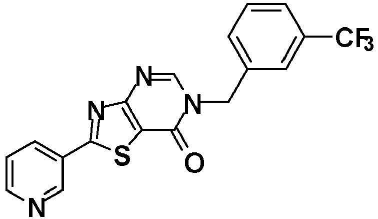

110 mg (0,29 mmol) 4-Amino-2-(pyridin-3-yl)-N-[3-(trifluormethyl)benzyl]-1,3-thiazol-5-carboxamid (II-28) wurden in N,N-Dimethylacetamid vorgelegt. 25 mg (0,14 mmol) p-Toluolsulfonsäure und 129 mg (0,87 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde mit gesättigter Natriumhydrogencarbonat-Lösung versetzt und mit Dichlormethan mehrfach extrahiert. Die vereinigten organischen Phasen wurden mit Magnesiumsulfat getrocknet, filtriert und eingedampft. Der Rückstand wurde anschließend mittels MPLC mit Laufmittel Cyclohexan/Ethylacetat gereinigt. Das Produkt wurde nach Verdampfen des Lösungsmittels mit Pentan/Ethylacetat verrieben und sonifiziert. Die so erhaltenen Kristalle wurden abgesaugt. Man erhielt 38 mg (98,3% Reinheit, 33,1% d. Th.) der Titelverbindung (28).
**1H-NMR(600,1 MHz, CD3CN):** δ = 9,2734(3,7); 9,2728(3,7); 9,270(3,8); 8,751(2,8); 8,748(2,9); 8,743(2,9); 8,740(2,8); 8,469(9,8); 8,403(1,8); 8,400(2,4); 8,399(2,3); 8,396(1,7); 8,389(1,9); 8,387(2,4); 8,386(2,3); 8,383(1,7); 7,744(3,9); 7,662(2,1); 7,650(3,1); 7,641(2,5); 7,576(2,2); 7,563(3,2); 7,550(1,3); 7,534(2,3); 7,533(2,2); 7,526(2,3); 7,525(2,2); 7,521(2,3); 7,520(2,1); 7,513(2,2); 7,512(2,0); 5,293(16,0); 2,957(13,9); 2,827(11,1); 2,154(11,6); 1,972(12,0); 1,967(0,5); 1,959(0,6); 1,955(0,8); 1,951(5,6); 1,947(10,0); 1,943(14,5); 1,938(9,7); 1,934(4,8); 0,000(6,8)

### Herstellbeispiel 7: 2-(Pyridin-3-yl)-6-(pyridin-2-ylmethyl)[1,3]thiazolo[4,5-d]pyrimidin-7(6H)-on (34)

### Schritt 1: 4-Amino-2-(pyridin-3-yl)-N-(pyridin-2-ylmethyl)-1,3-thiazol-5-carboxamid (11-34)

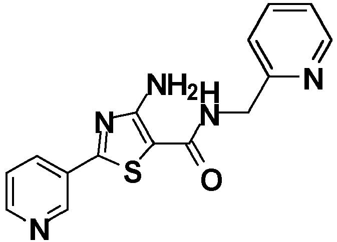

1 g (4,0 mmol) Ethyl-4-amino-2-(pyridin-3-yl)-1,3-thiazol-5-carboxylat (III-1) wurden in Ethanol vorgelegt und mit 2,25 g Kaliumhydroxid (40 mmol), gelöst in Ethanol, versetzt. Das Reaktionsgemisch wurde für 4 h bei 50 °C gerührt und anschließend zur Trockne eingeengt. Der Rückstand wurde in wenig Wasser angeschlemmt und das Produkt mit 10%iger wässriger Zitronensäurelösung ausgefällt. Der unlösliche Anteil wurde abgesaugt und gut unter Vakuum getrocknet. Die so erhaltene 4-Amino-2-(pyridin-3-yl)-1,3-thiazol-5-carbonsäure (583 mg, 100% Reinheit, 65,7% d. Th.) wurde direkt weiter umgesetzt.
**LC-MS: Masse gefunden** [m/z] = 221,03

148 mg (0,67 mmol) 4-Amino-2-(pyridin-3-yl)-1,3-thiazol-5-carbonsäure und 237 mg (0,74 mmol) O-(*1H*-Benzotriazol-1-yl)-*N,N,N',N'*-tetramethyluronium-tetrafluorborat (TBTU) wurden in DMF vorgelegt und 10 Minuten gerührt. Anschließend wurden 73 mg (0,67 mmol) 1-(Pyridin-2-yl)-methanamin sowie 130 mg (1 mmol) N,N-Diisopropylethylamin, gelöst in Dimethylformamid, zugegeben. Die Reaktionsmischung wurde bei Raumtemperatur über Nacht gerührt und danach im Vakuum eingedampft. Die weitere Reinigung des Rückstands erfolgte dann säulenchromatographisch mittels MPLC. Man erhielt 192 mg (43,0% Reinheit, 39,5% d. Th.) der Titelverbindung (II-34).
**LC-MS: Masse gefunden** [m/z] = 311,08

### Schritt 2: 2-(Pyridin-3-yl)-6-(pyridin-2-ylmethyl)[1,3]thiazolo[4,5-d]pyrimidin-7(6H)-on (34)

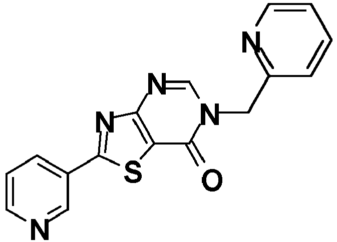

96 mg (0,31 mmol) 4-Amino-2-(pyridin-3-yl)-N-(pyridin-2-ylmethyl)-1,3-thiazol-5-carboxamid (II-34) wurden in 1,1 g *N,N-*Dimethylacetamid vorgelegt. 2,4 mg (0,015 mmol) *p*-Toluolsulfonsäure und 69 mg (0,46 mmol) Orthoameisensäuretriethylester wurden dazu gegeben und das Reaktionsgemisch für 10 min in einer CEM Discover Mikrowelle bei 200 Watt auf 120 °C erhitzt. Das abgekühlte Reaktionsgemisch wurde mit Wasser versetzt und mit Ethylacetat mehrfach extrahiert. Die vereinigten organischen Phasen wurden mit Natriummsulfat getrocknet, filtriert und eingedampft. Die weitere Reinigung des Rückstands erfolgte dann mittels MPLC mit Laufmittel Dichlormethan/Methanol (10:1) im Gradienten. Man erhielt 9,5 mg (100,0% Reinheit, 9,6% d. Th.) der Titelverbindung (34).
**1H NMR (400,0 MHz, d₆-DMSO):** δ = 9,324 (8,2); 9,320 (8,0); 8,828 (5,9); 8,819 (5,7); 8,652 (16,0); 8,533 (4,1); 8,528 (5,4); 8,523 (3,9); 8,513 (4,3); 8,508 (5,6); 8,503 (3,8); 7,673 (4,7); 7,661 (4,8); 7,653 (4,6); 7,641 (4,2); 6,830 (1,0); 6,818 (1,8); 6,805 (0,9); 6,700 (2,0); 6,688 (3,9); 6,676 (2,0); 6,570 (1,0); 6,558 (2,0); 6,546 (1,0); 4,914 (5,2); 4,875 (10,9); 4,837 (5,5); 3,905 (7,6); 3,506 (0,4); 3,338 (626,8); 3,225 (1,1); 3,062 (0,9); 2,677 (2,1); 2,672 (2,7); 2,668 (2,1); 2,508 (339,6); 2,503 (425,1); 2,499 (319,3); 2,334 (1,9); 2,330 (2,5); 2,325 (1,9); 1,298 (0,4); 1,258 (0,5); 1,233 (0,7); 1,145 (0,7); 0,000 (37,7)

### Herstellbeispiel 8: 6-[(2-Methyl-1,3-thiazol-4-yl)methyl]-2-(pyridin-3-yl)[1,3]thiazolo[4,5-d]pyrimidin-7(6H)-on (38)

### Schritt 1: 4-Amino-N-[(2-methyl-1,3-thiazol-4-yl)methyl]-2-(pyridin-3-yl)-1,3-thiazol-5-carboxamid (11-38)

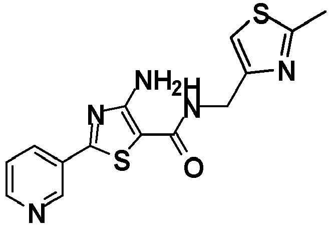

Unter Argon wurden zu 193 mg (1,5 mmol) 1-(2-Methyl-1,3-thiazol-4-yl)methanamin in 12 ml 1,2-Dichlorethan 0,75 ml einer 2M Trimethylaluminium-Lösung (1,5 mmol) in Toluol langsam getropft und 30 min bei Raumtemperatur gerührt. Anschließend wurden 250 mg (1,0 mmol) Ethyl-4-amino-2-(pyridin-3-yl)-1,3-thiazol-5-carboxylat (III-1) portionsweise dazu gegeben und das Reaktionsgemisch im Anschluss über Nacht unter Rückfluss erhitzt. Nach dem Abkühlen wurde das Reaktionsgemisch mit Argon vorsichtig auf eine 10%ige Kalium-Natrium-Tartrat-Lösung (ca. 120 ml) gedrückt. Das Gemisch wurde drei Mal mit Dichlormethan ausgeschüttelt, die organische Phase abgetrennt, über Natriumsulfat getrocknet, filtriert und eingeengt. Der Rückstand wurde mit etwas n-Pentan/Ethylacetat zu einer Suspension verrührt. Der unlösliche Teil wurde abgesaugt und anschließend mit Acetonitril verrührt. Der abfiltrierte Feststoff wurde danach unter Vakuum getrocknet. Man erhielt 71 mg (96,1% Reinheit, 20,5% d. Th.) der Titelverbindung.
**1H-NMR (400,0 MHz, d₆-DMSO):** δ = 9,059 (2,1); 9,054 (2,0); 8,711 (1,5); 8,708 (1,6); 8,699 (1,6); 8,695 (1,6); 8,426 (0,7); 8,412 (1,4); 8,398 (0,7); 8,231 (0,8); 8,226 (1,2); 8,221 (0,9); 8,211 (0,9); 8,206 (1,3); 8,201 (0,9); 7,581 (1,2); 7,569 (1,2); 7,561 (1,1); 7,549 (1,1); 7,166 (3,7); 7,035 (3,4); 4,433 (2,9); 4,419 (2,9); 3,904 (1,0); 3,330 (28,0); 2,629 (16,0); 2,507 (25,7); 2,503 (33,6); 2,499 (25,3); 1,278 (0,5); 0,008 (1,1); 0,000 (26,2); -0,008 (1,2)

### Schritt 2: 6-[(2-Methyl-1,3-thiazol-4-yl)methyl]-2-(pyridin-3-yl)[1,3]thiazolo[4,5-d]pyrimidin-7(6H)-on (38)

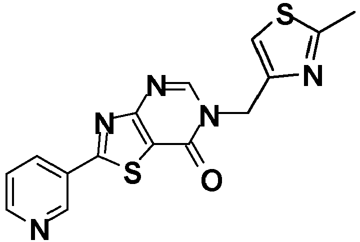

55 mg (0,17 mmol) 4-Amino-N-[(2-methyl-1,3-thiazol-4-yl)methyl]-2-(pyridin-3-yl)-1,3-thiazol-5-carboxamid (II-38) wurden in 0,5 ml *N,N-*Dimethylacetamid vorgelegt. 14 mg (0,08 mmol) *p-*Toluolsulfonsäure und 49 mg (0,33 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde mit Wasser versetzt und mit Dichlormethan mehrfach extrahiert. Die vereinigten organischen Phasen wurden mit gesättigter Natriumchloridlösung gewaschen, mit Magnesiumsulfat getrocknet, filtriert und eingedampft. Der Rückstand wurde nach Verdampfen des Lösungsmittels mit Acetonitril/Ethylacetat verrieben und die so erhaltenen Kristalle abgesaugt. Man erhielt 11 mg (80,0% Reinheit, 15,5% d. Th.) der Titelverbindung (38).
**1H-NMR(400,0 MHz, d₆-DMSO):** δ = 9,305(2,2); 9,301(2,3); 8,815(1,6); 8,812(1,9); 8,798(6,3); 8,511(1,3); 8,506(1,0); 8,495(1,0); 8,490(1,4); 8,486(1,1); 7,661(1,3); 7,648(1,3); 7,641(1,3); 7,628(1,2); 7,454(4,1); 7,277(0,5); 5,759(0,4); 5,308(7,1); 4,489(0,4); 4,476(0,4); 3,332(61,6); 2,945(0,9); 2,784(0,8); 2,672(0,9); 2,655(1,3); 2,637(2,1); 2,611(16,0); 2,507(95,2); 2,503(124,7); 2,499(102,9); 2,446(0,4); 2,330(0,8); 2,288(0,7); 1,958(0,8); 1,313(0,5); 0,146(0,4); 0,000(71,2); -0,150(0,4)

### Herstellbeispiel 13: 6-(Cyclopropylmethyl)-2-(pyridin-3-yl)[1,3]thiazolo[4,5-d]pyrimidin-7(6H)-on (39)

### Schritt 1: 4-Amino-N-(cyclopropylmethyl)-2-(pyridin-3-yl)-1,3-thiazol-5-carboxamid (II-39)

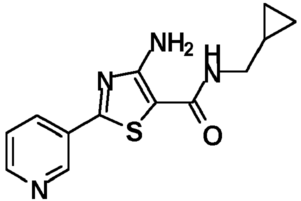

Zu 213 mg (3,00 mmol) 1-Cyclopropylmethanamin in 15 ml 1,2-Dichlorethan wurden unter Argon 1,5 ml einer 2M Trimethylaluminium-Lösung (3,01 mmol) in Toluol unter Eiskühlung langsam zugetropft und 60 min bei Raumtemperatur gerührt. Anschließend wurden 373 mg (1,50 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 100 ml) ausgetragen. Das Gemisch wurde drei Mal mit Methylenchlorid extrahiert, die vereinigten organischen Phasen über Natriumsulfat getrocknet, filtriert und eingeengt. Der Rückstand wurde mit etwas Acetonitril zu einer Suspension verrührt. Der unlösliche Teil wurde abgesaugt und unter Vakuum getrocknet. Man erhielt 300 mg (100% Reinheit, 73,0% d. Th.) der Titelverbindung (II-39).
**1H-NMR(400,0 MHz, d₆-DMSO):** δ = 9,056(9,2); 9,052(9,7); 8,710(6,7); 8,707(7,1); 8,699(7,2); 8,695(7,1); 8,229(3,8); 8,226(5,4); 8,220(3,9); 8,210(4,1); 8,205(5,7); 8,200(4,0); 8,025(3,0); 8,012(5,9); 7,998(3,1); 7,579(4,9); 7,567(5,0); 7,559(5,0); 7,547(4,6); 7,027(16,0); 3,454(4,0); 3,437(9,3); 3,422(9,5); 3,404(4,3); 3,312(106,1); 2,671(1,1); 2,573(1,2); 2,556(2,1); 2,545(3,8); 2,527(7,5); 2,506(132,9); 2,502(168,0); 2,498(133,8); 2,470(2,8); 2,452(1,3); 2,329(1,1); 0,000(11,5)

### Schritt 2: 6-(Cyclopropylmethyl)-2-(pyridin-3-yl)[1,3]thiazolo[4,5-d]pyrimidin-7(6H)-on (39)

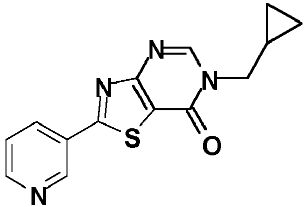

100 mg (0,37 mmol) 4-Amino-N-(cyclopropylmethyl)-2-(pyridin-3-yl)-1,3-thiazol-5-carboxamid (II-39) wurden in 1 ml *N,N-*Dimethylacetamid vorgelegt. 31 mg (0,18 mmol) *p*-Toluolsulfonsäure und 162 mg (1,09 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde im Vakuum eingeengt und der Rückstand mittels MPLC chromatographisch gereinigt. Man erhielt in Summe 2 mg (100% Reinheit, 2,2% d. Th.) der Titelverbindung (39).
**1H-NMR(601,6 MHz, CDCl₃):** δ = 9,341(5,1); 9,338(5,0); 8,781(3,5); 8,775(3,6); 8,426(2,9); 8,423(3,9); 8,420(2,8); 8,413(3,1); 8,410(4,0); 8,407(2,8); 8,299(16,0); 7,484(3,2); 7,476(3,3); 7,471(3,3); 7,463(3,1); 7,432(0,6); 7,261(86,9); 7,084(0,5); 5,299(2,5); 3,958(15,5); 3,946(15,6); 3,278(0,4); 3,269(0,5); 3,266(0,4); 3,257(0,4); 2,232(0,4); 2,220(0,6); 2,207(0,4); 1,637(0,4); 1,558(13,5); 1,445(0,4); 1,368(0,5); 1,360(0,9); 1,355(1,3); 1,352(0,9); 1,347(2,2); 1,342(2,1); 1,339(1,8); 1,334(3,5); 1,329(2,0); 1,326(2,4); 1,321(2,7); 1,313(2,0); 1,309(1,8); 1,301(1,4); 1,255(7,8); 0,892(1,0); 0,881(1,8); 0,869(1,1); 0,856(0,4); 0,841(0,4); 0,715(2,2); 0,707(6,7); 0,705(7,1); 0,697(3,5); 0,694(7,0); 0,692(6,7); 0,684(2,5); 0,577(0,4); 0,575(0,4); 0,564(0,4); 0,562(0,4); 0,491(2,4); 0,483(8,8); 0,473(8,4); 0,465(2,0); 0,276(0,4); 0,267(0,5); 0,097(0,4); 0,005(2,9); 0,000(86,6); -0,006(3,2);-0,100(0,4)

### Herstellbeispiel 14: N,N-Dimethyl-3-[7-oxo-2-(pyridin-3-yl)[1,3]thiazolo[4,5-d]pyrimidin-6(7H)-yl]benzolsulfonamid (42)

### Schritt 1: 4-Amino-N-[3-(dimethylsulfamoyl)phenyl]-2-(pyridin-3-yl)-1,3-thiazol-5-carboxamid (II-42)

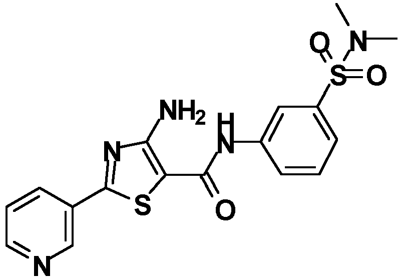

Zu 3,6 g (18,1 mmol) 3-Amino-N,N-dimethylbenzolsulfonamid in 200 ml 1,2-Dichlorethan wurden unter Argon 9,0 ml einer 2M Trimethylaluminium-Lösung (18,1 mmol) in Toluol unter Eiskühlung langsam zugetropft und 60 min bei Raumtemperatur gerührt. Anschließend wurden 1,5 g (6,02 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 250 ml) ausgetragen. Das Gemisch wurde drei Mal mit Methylenchlorid extrahiert, die vereinigten organischen Phasen über Natriumsulfat getrocknet, filtriert und eingeengt. Der Rückstand wurde mit etwas Acetonitril zu einer Suspension verrührt. Der unlösliche Teil wurde mit n-Pentan gewaschen, abgesaugt und unter Vakuum getrocknet. Der Feststoff wurde anschließend mittels MPLC mit Laufmittel Cyclohexan/Ethylacetat gereinigt. Man erhielt 340 mg (100% Reinheit, 14,0% d. Th.) der Titelverbindung (II-42).
**1H-NMR(400,0 MHz, d₆-DMSO):** δ = 9,997(1,7); 9,107(1,2); 9,102(1,3); 8,745(0,8); 8,742(0,9); 8,734(0,9); 8,731(0,9); 8,279(0,4); 8,275(0,7); 8,271(0,5); 8,259(0,5); 8,255(0,8); 8,251(0,5); 8,169(1,5); 8,062(0,6); 8,040(0,7); 7,614(1,2); 7,603(0,7); 7,594(1,8); 7,583(0,7); 7,574(0,8); 7,424(0,9); 7,404(0,7); 7,322(1,9); 3,320(8,0); 2,638(16,0); 2,507(14,9); 2,503(19,6); 2,499(15,3); 1,989(0,9); 1,176(0,5); 0,000(0,4)

### Schritt 2: N,N-Dimethyl-3-[7-oxo-2-(pyridin-3-yl)[1,3]thiazolo[4,5-d]pyrimidin-6(7H)-yl]benzolsulfonamid (42)

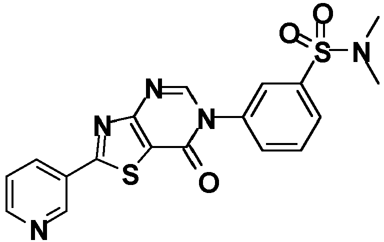

320 mg (0,79 mmol) 4-Amino-N-[3-(dimethylsulfamoyl)phenyl]-2-(pyridin-3-yl)-1,3-thiazol-5-carboxamid (II-42) wurden in *N,N-*Dimethylacetamid vorgelegt. 68 mg (0,40 mmol) *p-*Toluolsulfonsäure und 260 mg (1,75 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde im Vakuum eingeengt, der Rückstand mit Acetonitril verrührt und das Produkt als Feststoff abfiltriert. Der Feststoff wurde anschließend auf einer Tonplatte abgepresst und mittels MPLC mit Laufmittel n-Hexan/Ethylacetat gereinigt. Man erhielt 320 mg (100% Reinheit, 97,6% d. Th.) der Titelverbindung (42).
**1H-NMR(400,0 MHz, d₆-DMSO):** δ = 9,350(1,3); 9,345(1,3); 8,835(0,9); 8,831(1,0); 8,823(1,0); 8,819(1,0); 8,714(3,3); 8,560(0,5); 8,556(0,7); 8,551(0,5); 8,540(0,6); 8,535(0,8); 8,531(0,5); 8,055(1,6); 7,967(0,6); 7,962(0,4); 7,948(1,0); 7,944(0,7); 7,929(0,5); 7,925(0,4); 7,909(1,4); 7,894(1,3); 7,875(1,1); 7,856(0,4); 7,680(0,7); 7,668(0,7); 7,660(0,7); 7,648(0,7); 3,320(7,9); 2,681(16,0); 2,507(13,0); 2,502(16,4); 2,498(12,5); 0,000(16,1)

### Herstellbeispiel 15: 6-{2,4-Dimethyl-5-[(2,2,2-trifluorethyl)sulfinyl]phenyl}-2-(pyridin-3-yl)[1,3]thiazolo[4,5-d]pyrimidin-7(6H)-on (50)

### Schritt 1: 4-Amino-N-{2,4-dimethyl-5-[(2,2,2-trifluorethyl)sulfanyl]phenyl}-2-(pyridin-3-yl)-1,3-thiazol-5-carboxamid (II-49)

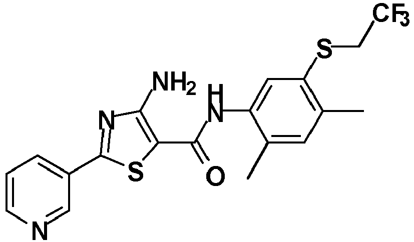

Zu 708 mg (3,01 mmol) 2,4-Dimethyl-5-[(2,2,2-trifluorethyl)sulfanyl]anilin in 1,2-Dichlorethan wurden unter Argon 1,5 ml einer 2M Trimethylaluminium-Lösung (3,01 mmol) in Toluol unter Eiskühlung langsam zugetropft und 30 min bei Raumtemperatur gerührt. Anschließend wurden 250 mg (1,00 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 120 ml) ausgetragen. Das Gemisch wurde dreimal mit Methylenchlorid extrahiert, die vereinigten organischen Phasen über Natriumsulfat getrocknet, filtriert und eingeengt. Der Rückstand wurde mit etwas Acetonitril zu einer Suspension verrührt. Der unlösliche Teil wurde abgesaugt und unter Vakuum getrocknet. Man erhielt 158 mg (100% Reinheit, 35,9% d. Th.) der Titelverbindung (II-49).
**1H-NMR(400,0 MHz, d₆-DMSO):** δ = 9,328(4,1); 9,086(3,2); 9,081(3,1); 8,729(2,3); 8,726(2,4); 8,717(2,4); 8,714(2,4); 8,259(1,3); 8,254(1,8); 8,249(1,3); 8,239(1,4); 8,234(1,9); 8,229(1,3); 7,598(1,7); 7,586(1,7); 7,579(1,7); 7,566(1,6); 7,468(5,9); 7,174(5,0); 7,133(5,2); 3,878(1,2); 3,852(3,8); 3,826(4,0); 3,800(1,4); 3,330(19,9); 2,507(38,9); 2,503(48,8); 2,499(36,6); 2,365(14,9); 2,330(0,4); 2,325(0,3); 2,163(16,0); 0,000(40,2)

### Schritt 2: 6-{2,4-Dimethyl-5-[(2,2,2-trifluorethyl)sulfanyl]phenyl}-2-(pyridin-3-yl)[1,3]thiazolo[4,5-d]pyrimidin-7(6H)-on (49)

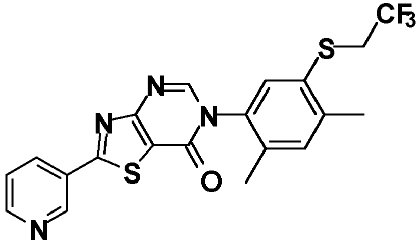

600 mg (1,37 mmol) 4-Amino-N-{2,4-dimethyl-5-[(2,2,2-trifluorethyl)sulfanyl]phenyl}-2-(pyridin-3-yl)-1,3-thiazol-5-carboxamid (II-49) wurden in 15 ml (90,2 mmol) Orthoameisensäuretriethylester vorgelegt. 118 mg (0,68 mmol) *p*-Toluolsulfonsäure wurden dazu gegeben und das Reaktionsgemisch für 2 h in einer CEM Discover Mikrowelle auf 170 °C erhitzt. Das abgekühlte Reaktionsgemisch wurde im Vakuum eingeengt und mittels MPLC mit Laufmittel Cyclohexan/Ethylacetat chromatographisch gereinigt. Man erhielt in Summe 200 mg (95,3% Reinheit, 31,1% d. Th.) der Titelverbindung (49).
**1H-NMR(400,0 MHz, d₆-DMSO):** δ = 9,347(2,9); 9,342(3,0); 8,838(2,3); 8,834(2,4); 8,826(2,4); 8,822(2,5); 8,558(1,3); 8,554(1,9); 8,548(1,4); ,536(10,2); 8,528(1,5); 8,317(0,8); 7,924(0,4); 7,683(1,9); 7,675(6,4); 7,665(2,0); 7,651(1,5); 7,360(4,7); 6,025(1,0); 4,055(0,6); 4,038(2,6); 4,020(2,4); 4,014(3,6); 4,002(0,9); 3,988(3,6); 3,962(1,3); 3,855(0,8); 3,837(0,8); 3,392(0,3); 3,374(0,5); 3,332(421,8); 2,676(1,7); 2,671(2,3); 2,667(1,7); 2,525(5,6); 2,511(131,6); 2,507(269,9); 2,502(357,0); 2,498(264,5); 2,494(133,5); 2,407(15,0); 2,365(0,4); 2,334(1,8); 2,329(2,4); 2,325(1,8); 2,078(16,0); 1,989(7,6); 1,890(0,3); 1,398(1,9); 1,235(0,6); 1,193(2,1); 1,175(4,1); 1,157(2,1); 1,100(0,8); 1,083(1,5); 1,065(0,8); 0,146(0,5); 0,008(3,7); 0,000(116,2); -0,008(4,9); -0,150(0,5)

### Schritt 3: 6-{2,4-Dimethyl-5-[(2,2,2-trifluorethyl)sulfinyl]phenyl}-2-(pyridin-3-yl)[1,3]thiazolo[4,5-d]pyrimidin-7(6H)-on (50)

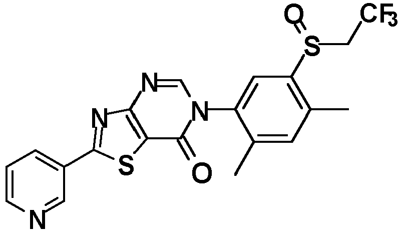

Zu einer Lösung von 250,0 mg (0,56 mmol) 6-{2,4-Dimethyl-5-[(2,2,2-trifluorethyl)sulfanyl]phenyl}-2-(pyridin-3-yl)[1,3]thiazolo[4,5-d]pyrimidin-7(6H)-on (49) in 10 ml Dichlormethan wurden bei 0 °C 127 mg (77%ig, 0,57 mmol) meta-Chlorperbenzoesäure gegeben. Das Reaktionsgemisch wurde 1 Stunde bei Raumtemperatur gerührt und dann mit wässriger Natriumhydrogensulfit-Lösung versetzt. Die abgetrennte organische Phase wurde anschließend mit gesättigter Natriumcarbonatlösung gewaschen und mit Natriumsulfat getrocknet. Nach Verdampfung des Lösungsmittels wurde der Rückstand mittels MPLC mit Laufmittel Cyclohexan/Ethylacetat chromatographisch gereinigt. Man erhielt 154 mg (99,0% Reinheit, 58,88% d. Th.) der Titelverbindung (50).
**1H-NMR(400,0 MHz, d₆-DMSO):** δ = 9,351(4,6); 9,346(4,6); 8,839(3,4); 8,835(3,7); 8,827(3,6); 8,823(3,7); 8,591(14,3); 8,561(1,9); 8,556(2,7); 8,551(1,9); 8,541(2,0); 8,536(2,8); 8,531(1,9); 8,317(1,3); 7,937(6,3); 7,928(4,2); 7,685(2,6); 7,673(2,6); 7,667(2,5); 7,653(2,4); 7,496(3,0); 7,470(4,4); 4,323(0,5); 4,295(0,6); 4,286(0,7); 4,259(0,7); 4,178(0,6); 4,171(0,6); 4,151(1,8); 4,144(1,8); 4,124(1,8); 4,117(1,8); 4,097(0,7); 4,090(0,6); 4,055(0,4); 4,037(1,0); 4,020(1,0); 4,002(0,4); 3,977(0,7); 3,951(0,8); 3,940(0,7); 3,924(0,3); 3,914(0,7); 3,372(0,9); 3,332(833,0); 2,676(2,9); 2,671(3,9); 2,667(2,9); 2,525(9,7); 2,507(449,3); 2,502(587,2); 2,498(437,0); 2,451(12,1); 2,446(16,0); 2,408(0,8); 2,353(0,6); 2,334(3,2); 2,329(4,0); 2,325(3,0); 2,279(0,4); 2,258(0,5); 2,188(15,9); 2,182(12,0); 1,989(4,2); 1,193(1,1); 1,175(2,3); 1,157(1,1); 0,146(0,8); 0,008(6,5); 0,000(190,8); -0,008(8,2); -0,150(0,9)

### Herstellbeispiel 16: 2-({4-Fluor-2-methyl-5-[7-oxo-2-(pyridin-3-yl)[1,3]thiazolo[4,5-d]pyrimidin-6(7H)-yl]phenyl}sulfinyl)-N,N-dimethylacetamid (55)

### Schritt 1: 2-[(5-Amino-4-fluor-2-methylphenyl)sulfanyl]-N,N-dimethylacetamid

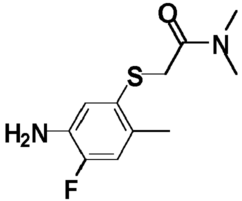

1,50 g (9,54 mmol) 5-Amino-4-fluor-2-methylbenzolthiol, 1,28 g (10,50 mmol) 2-Chlor-N,N-dimethylacetamid und 6,22 g (19,08 mmol) Cäsiumcarbonat wurden zusammen mit 100 ml trockenem Aceton versetzt und über Nacht unter Rückfluss gerührt. Der abgekühlte Reaktionsansatz wurde in eine Mischung aus Ethylacetat und Natriumcarbonat gegeben und die organische Phase abgetrennt. Die wässrige Phase wurde noch mehrfach mit Ethylacetat extrahiert, die vereinigten organischen Phasen über Magnesiumsulfat getrocknet, filtriert und eingedampft. Man erhielt 2,10 g (90,0% Reinheit, 81,75% d. Th.) der Titelverbindung 2-[(5-Amino-4-fluor-2-methylphenyl)sulfanyl]-N,N-dimethylacetamid.
**1H-NMR(601,6 MHz, CDCl₃):** δ = 7,264(2,5); 6,995(2,3); 6,980(2,3); 6,836(1,7); 6,816(1,7); 4,079(0,4); 3,605(10,1); 3,098(0,6); 3,034(0,4); 3,025(16,0); 2,991(0,6); 2,968(0,4); 2,951(14,4); 2,313(13,0); 2,170(1,4); 0,000(2,4)

### Schritt 2: 4-Amino-N-(5-{[2-(dimethylamino)-2-oxoethyl]sulfanyl}-2-fluor-4-methylphenyl)-2-(pyridin-3-yl)-1,3-thiazol-5-carboxamid (II-54)

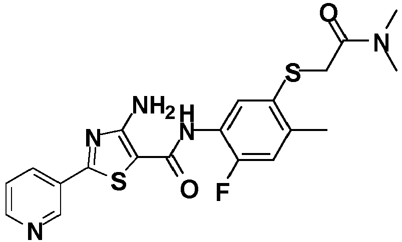

Zu 2,10 g (7,88 mmol) 2-[(5-Amino-4-fluor-2-methylphenyl)sulfanyl]-N,N-dimethylacetamid (siehe Herstellbeispiel 16, Schritt 1) in 150 ml 1,2-Dichlorethan wurden unter Argon 3,94 ml einer 2M Trimethylaluminium-Lösung (7,88 mmol) in Toluol unter Eiskühlung langsam zugetropft und 60 min bei Raumtemperatur gerührt. Anschließend wurden 655 mg (2,63 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 200 ml) ausgetragen. Das Gemisch wurde drei Mal mit Methylenchlorid extrahiert, die vereinigten organischen Phasen über Magnesiumsulfat getrocknet, filtriert und eingeengt. Der Rückstand wurde anschließend mittels MPLC mit Laufmittel Cyclohexan/Ethylacetat chromatographisch gereinigt. Man erhielt 370 mg (47,0% Reinheit, 14,86% d. Th.) der Titelverbindung (II-54), welche ohne weitere Aufreinigung direkt in Schritt 3 weiter umgesetzt wurde.
HPLC-MS (ESI positiv, m/z): [M+H] = 446.0

### Schritt 3: 2-({4-Fluor-2-methyl-5-[7-oxo-2-(pyridin-3-yl)[1,3]thiazolo[4,5-d]pyrimidin-6(7H)-yl]phenyl}sulfanyl)-N,N-dimethylacetamid (54)

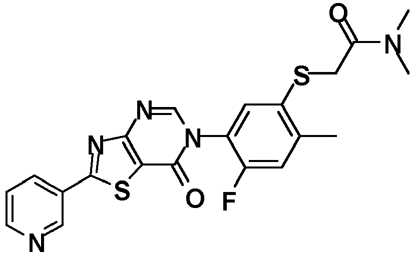

370 mg (47%ig, 0,39 mmol) 4-Amino-N-(5-{[2-(dimethylamino)-2-oxoethyl]sulfanyl}-2-fluor-4-methylphenyl)-2-(pyridin-3-yl)-1,3-thiazol-5-carboxamid (II-54) wurden in 1 ml *N,N*-Dimethylacetamid vorgelegt. 34 mg (0,20 mmol) *p*-Toluolsulfonsäure und 174 mg (1,17 mmol) Orthoameisensäuretriethylester wurden dazu gegeben und das Reaktionsgemisch für 1 h in einer CEM Discover Mikrowelle bei 200 Watt auf 130 °C erhitzt. Das abgekühlte Reaktionsgemisch wurde im Vakuum eingeengt, der Rückstand mit Acetonitril verrührt und das Produkt als Feststoff abfiltriert. Der Feststoff wurde anschließend auf einer Tonplatte abgepresst und mittels MPLC mit Laufmittel Acetonitril/Wasser gereinigt. Man erhielt 170 mg (100% Reinheit, 95,61% d. Th.) der Titelverbindung (54).
**1H-NMR(400,0 MHz, d₆-DMSO):** δ = 9,347(2,8); 9,342(2,6); 8,837(2,0); 8,825(2,0); 8,668(5,5); 8,552(1,6); 8,532(1,6); 8,141(0,9); 7,718(2,4); 7,700(2,4); 7,682(1,5); 7,669(1,5); 7,662(1,4); 7,649(1,3); 7,448(2,1); 7,422(2,1); 5,754(0,6); 4,011(8,8); 3,320(45,4); 3,027(16,0); 2,891(0,5); 2,832(14,9); 2,731(0,5); 2,670(0,6); 2,502(95,0); 2,406(12,3); 2,328(0,6); 0,000(4,0)

### Schritt 4: 2-({4-Fluor-2-methyl-5-[7-oxo-2-(pyridin-3-yl)[1,3]thiazolo[4,5-d]pyrimidin-6(7H)-yl]phenyl}sulfinyl)-N,N-dimethylacetamid (55)

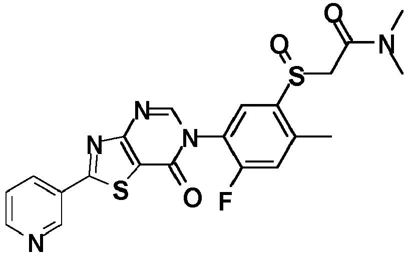

Zu einer Lösung von 170 mg (0,36 mmol) 2-({4-Fluor-2-methyl-5-[7-oxo-2-(pyridin-3-yl)[1,3]thiazolo[4,5-d]pyrimidin-6(7H)-yl]phenyl}sulfanyl)-N,N-dimethylacetamid (54) in 1 ml Dichlormethan wurden bei 0 °C 81 mg (77%ig, 0,36 mmol) meta-Chlorperbenzoesäure gegeben. Das Reaktionsgemisch wurde 1 Stunde bei Raumtemperatur gerührt und dann mit wässriger Natriumcarbonat-Lösung versetzt. Die abgetrennte wässrige Phase wurde noch mehrmals mit Dichlormethan extrahiert und die vereinigten organischen Phasen getrocknet, filtriert und eingedampft. Der Rückstand wurde anschließend mittels MPLC mit Laufmittel Cyclohexan/Ethylacetat chromatographisch gereinigt. Man erhielt 119 mg (100% Reinheit, 71,2% d. Th.) der Titelverbindung (55).
**1H-NMR(400,0 MHz, d₆-DMSO):** δ = 9,350(3,2); 9,345(3,1); 8,838(2,2); 8,835(2,3); 8,826(2,3); 8,823(2,3); 8,722(6,2); 8,556(1,8); 8,540(1,4); 8,535(1,9); 8,531(1,3); 8,139(4,6); 8,119(2,9); 7,685(1,7); 7,672(1,7); 7,665(1,7); 7,653(1,5); 7,568(2,4); 7,541(2,4); 4,185(1,3); 4,148(2,5); 4,086(1,2); 4,047(0,6); 3,340(160,4); 2,955(16,0); 2,863(15,8); 2,672(0,6); 2,507(78,3); 2,503(97,2); 2,473(15,1); 2,330(0,6); 0,000(3,2)

### Herstellbeispiel 17: 6-{2-Fluor-4-methyl-5-[(2,2,2-trifluorethyl)sulfinyl]phenyl}-5-methyl-2-(pyridin-3-yl)[1,3]thiazolo[4,5-d]pyrimidin-7(6H)-on (57)

### Schritt 1: 6-{2-Fluor-4-methyl-5-[(2,2,2-trifluorethyl)sulfanyl]phenyl}-5-methyl-2-(pyridin-3-yl)[1,3]thiazolo[4,5-d]pyrimidin-7(6H)-on (56)

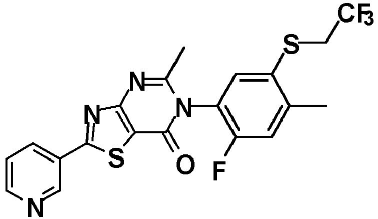

200 mg (0,45 mmol) 4-Amino-N-{2-fluor-4-methyl-5-[(2,2,2-trifluorethyl)sulfanyl]phenyl}-2-(pyridin-3-yl)-1,3-thiazol-5-carboxamid (II-21) wurden in 1 ml *N,N-*Dimethylacetamid vorgelegt. 39 mg (0,23 mmol) *p*-Toluolsulfonsäure und 220 mg (1,36 mmol) Orthoessigsäuresäuretriethylester wurden dazu gegeben und das Reaktionsgemisch für 30 min in einer CEM Discover Mikrowelle bei 200 Watt auf 130 °C erhitzt. Das abgekühlte Reaktionsgemisch wurde im Hochvakuum eingedampft. Der Rückstand wurde anschließend mittels MPLC mit Laufmittel Acetonitril/Wasser gereinigt. Man erhielt 30 mg (100% Reinheit, 14,2% d. Th.) der Titelverbindung (56).
**1H-NMR(400,0 MHz, d₆-DMSO):** δ = 9,333(2,4); 9,328(2,4); 8,834(1,8); 8,830(2,0); 8,822(1,9); 8,818(2,0); 8,535(1,1); 8,531(1,4); 8,526(1,1); 8,515(1,2); 8,510(1,5); 8,506(1,1); 7,939(2,4); 7,920(2,4); 7,680(1,4); 7,668(1,4); 7,661(1,4); 7,648(1,3); 7,541(2,1); 7,515(2,1); 4,085(0,5); 4,071(0,5); 4,060(0,6); 4,046(1,3); 4,037(0,6); 4,021(1,3); 4,012(1,3); 3,995(0,7); 3,986(1,3); 3,972(0,5); 3,960(0,5); 3,946(0,5); 3,329(49,4); 2,676(0,4); 2,672(0,5); 2,668(0,4); 2,525(1,3); 2,512(29,6); 2,507(59,8); 2,503(79,0); 2,498(58,4); 2,494(29,1); 2,469(13,3); 2,443(0,6); 2,417(0,6); 2,339(0,3); 2,334(0,5); 2,329(0,6); 2,325(0,5); 2,302(0,4); 2,281(16,0); 2,076(1,0); 0,008(1,9); 0,000(57,6); -0,009(2,3)

### Schritt 2: 6-{2-Fluor-4-methyl-5-[(2,2,2-trifluorethyl)sulfinyl]phenyl}-5-methyl-2-(pyridin-3-yl)[1,3]thiazolo[4,5-d]pyrimidin-7(6H)-on (57)

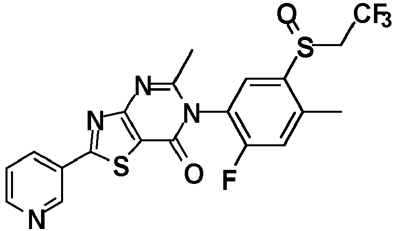

Zu einer Lösung von 25 mg (0,06 mmol) 6-{2-Fluor-4-methyl-5-[(2,2,2-trifluorethyl)sulfanyl]phenyl}-5-methyl-2-(pyridin-3-yl)[1,3]thiazolo[4,5-d]pyrimidin-7(6H)-on (56) in 1 ml Dichlormethan wurden bei 0 °C 12 mg (77%ig, 0,06 mmol) meta-Chlorperbenzoesäure gegeben. Das Reaktionsgemisch wurde 1 Stunde bei Raumtemperatur gerührt und dann mit wässriger Natriumcarbonat-Lösung versetzt. Die abgetrennte wässrige Phase wurde noch mehrmals mit Dichlormethan extrahiert und die vereinigten organischen Phasen getrocknet, filtriert und eingedampft. Man erhielt 14 mg (88,0% Reinheit, 47,7% d. Th.) der Titelverbindung (57).
**1H-NMR(601,6 MHz, CDCl₃):** δ = 9,363(2,0); 8,807(1,5); 8,469(0,8); 8,466(1,1); 8,463(0,8); 8,452(1,9); 8,441(0,9); 8,438(1,1); 8,435(0,8); 8,065(1,7); 8,029(1,8); 8,017(1,8); 8,003(1,8); 7,991(1,8); 7,979(1,0); 7,966(1,1); 7,556(0,7); 7,554(0,7); 7,542(0,9); 7,541(0,9); 7,526(0,9); 7,519(1,3); 7,513(1,7); 7,507(1,3); 7,500(0,9); 7,415(1,0); 7,402(1,7); 7,389(0,8); 7,304(1,8); 7,302(1,9); 7,288(1,9); 7,286(1,8); 7,272(7,2); 5,302(6,3); 3,617(0,5); 3,609(0,4); 3,601(0,6); 3,593(1,0); 3,576(1,1); 3,563(1,1); 3,555(0,3); 3,547(3,0); 3,539(0,6); 3,530(3,1); 3,523(0,8); 3,514(1,2); 3,506(0,7); 2,590(0,5); 2,517(16,0); 2,501(0,6); 2,493(0,4); 2,484(0,3); 2,418(0,7); 2,408(12,8); 2,395(0,6); 2,386(12,7); 2,378(1,1); 2,369(0,4); 2,356(0,5); 2,352(0,4); 2,344(0,4); 2,332(0,4); 1,335(0,5); 1,329(0,3); 1,306(0,6); 1,295(0,9); 1,286(1,3); 1,283(1,3); 1,271(1,3); 1,255(6,2); 1,232(0,7); 1,221(0,5); 1,212(0,5); 0,891(0,8); 0,880(1,4); 0,868(1,0); 0,856(0,5); 0,843(0,9); 0,841(0,9); 0,834(0,9); 0,005(0,5); 0,000(14,7); -0,006(0,7)

### Herstellbeispiel 18: 3-{2-Fluor-4-methyl-5-[(2,2,2-trifluorethyl)sulfinyl]phenyl}-6-(pyridin-3-yl)[1,3]thiazolo[4,5-d][1,2,3]triazin-4(3H)-on (59)

### Schritt 2: 3-{2-Fluor-4-methyl-5-[(2,2,2-trifluorethyl)sulfanyl]phenyl}-6-(pyridin-3-yl)[1,3]thiazolo[4,5-d][1,2,3]triazin-4(3H)-on (58)

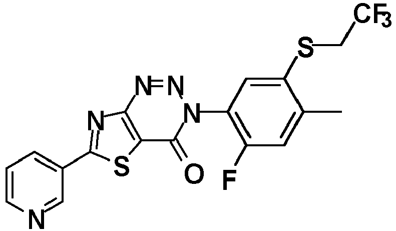

160 mg (0,36 mmol) 4-Amino-N-{2-fluor-4-methyl-5-[(2,2,2-trifluorethyl)sulfanyl]phenyl}-2-(pyridin-3-yl)-1,3-thiazol-5-carboxamid (II-21) wurden in 4 ml konzentrierter Salzsäure vorgelegt. Zu dieser Mischung wurden dann 212 mg (3,07 mmol) Natriumnitrit, gelöst in 4 ml Wasser, zugetropft und der Ansatz 4 h bei 70 °C gerührt. Anschließend wurde der abgekühlte Ansatz in gesättigte Natriumcarbonatlösung gegeben und mehrfach mit Dichlormethan extrahiert. Die vereinigten organischen Phasen wurden über Magnesiumsulfat getrocknet, filtriert und eingedampft. Man erhielt 112 mg (90,0% Reinheit, 61,5% d. Th.) der Titelverbindung (58).
1H-NMR(400,0 MHz, DMSO): δ = 9,430(10,3); 9,425(10,4); 8,891(8,1); 8,887(8,8); 8,879(8,5); 8,875(8,7); 8,651(4,7); 8,646(6,4); 8,641(4,7); 8,631(5,1); 8,625(6,5); 8,621(4,7); 8,317(0,4); 8,260(1,0); 8,242(1,1); 7,992(9,9); 7,973(9,9); 7,729(6,1); 7,717(6,0); 7,709(6,0); 7,697(6,2); 7,673(0,9); 7,583(8,6); 7,556(8,5); 5,758(16,0); 4,361(0,7); 4,351(0,4); 4,344(0,4); 4,324(0,4); 4,086(0,4); 4,059(0,4); 4,049(0,4); 4,018(4,1); 3,993(12,7); 3,967(13,1); 3,941(4,5); 3,331(218,6); 2,677(1,1); 2,672(1,5); 2,668(1,1); 2,512(92,5); 2,508(154,3); 2,503(197,3); 2,499(145,6); 2,495(73,3); 2,374(0,4); 2,353(0,4); 2,334(1,0); 2,330(1,3); 2,326(1,0); 1,343(0,4); 1,326(0,8); 1,308(0,4); 0,146(0,4); 0,008(3,0); 0,000(84,9); -0,008(3,4); -0,150(0,4)

### Schritt 2: 3-{2-Fluor-4-methyl-5-[(2,2,2-trifluorethyl)sulfinyl]phenyl}-6-(pyridin-3-yl)[1,3]thiazolo [4,5-d][1,2,3]triazin-4(3H)-on (59)

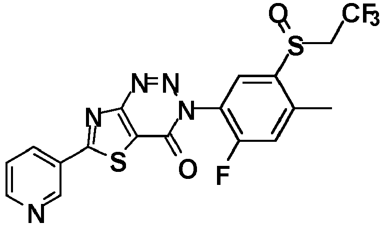

Zu einer Lösung von 96 mg (90%ig, 0,19 mmol) 3-{2-Fluor-4-methyl-5-[(2,2,2-trifluorethyl)sulfanyl]phenyl}-6-(pyridin-3-yl)[1,3]thiazolo[4,5-d][1,2,3]triazin-4(3H)-on (58) in 1 ml Dichlormethan wurden bei 0 °C 44 mg (77%ig, 0,19 mmol) meta-Chlorperbenzoesäure gegeben. Das Reaktionsgemisch wurde 40 Minuten bei Raumtemperatur gerührt und dann mit wässriger Natriumcarbonat-Lösung versetzt. Die abgetrennte wässrige Phase wurde noch mehrmals mit Dichlormethan extrahiert und die vereinigten organischen Phasen getrocknet, filtriert und eingedampft. Der Rückstand wurde anschließend mittels MPLC mit Laufmittel Acetonitril/Wasser gereinigt. Man erhielt 30 mg (100% Reinheit, 33,5% d. Th.) der Titelverbindung (59).
1H-NMR(400,0 MHz, DMSO): δ = 9,432(5,9); 9,428(6,0); 8,891(4,5); 8,887(4,8); 8,879(4,8); 8,875(4,8); 8,652(2,6); 8,648(3,5); 8,642(2,6); 8,632(2,8); 8,626(3,7); 8,622(2,7); 8,260(5,9); 8,241(6,0); 7,729(3,3); 7,717(3,3); 7,709(3,5); 7,699(7,6); 7,673(4,8); 5,758(16,0); 4,415(0,5); 4,388(1,7); 4,378(0,8); 4,360(2,0); 4,351(2,3); 4,333(0,8); 4,323(2,3); 4,296(0,7); 4,112(0,6); 4,085(2,0); 4,075(0,7); 4,058(2,3); 4,048(1,9); 4,031(0,9); 4,021(1,8); 3,994(0,6); 3,331(101,6); 2,676(0,7); 2,672(1,0); 2,668(0,8); 2,507(117,2); 2,503(134,4); 2,498(99,6); 2,334(0,6); 2,330(0,9); 2,325(0,7); 1,233(0,7); 0,008(1,1); 0,000(27,5)

### Herstellbeispiel 19: 2-(Pyridin-3-yl)-6-{4-[(trifluormethyl)sulfinyl]phenyl}[1,3]thiazolo[4,5-d]pyrimidin-7(6H)-on (63)

### Schritt 1: 4-Amino-2-(pyridin-3-yl)-N-{4-[(trifluormethyl)sulfanyl]phenyl}-1,3-thiazol-5-carboxamid (II-62)

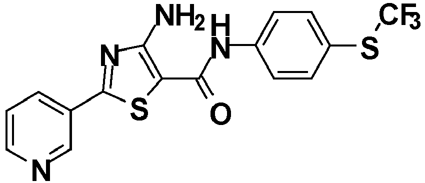

Zu 1,16 g (6,0 mmol) 4-[(Trifluormethyl)sulfanyl]anilin in 50 ml 1,2-Dichlorethan wurden unter Argon 3,0 ml einer 2M Trimethylaluminium-Lösung (6,0 mmol) in Toluol unter Eiskühlung langsam zugetropft und 1 Stunde bei Raumtemperatur gerührt. Anschließend wurden 500 mg (2,0 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 120 ml) ausgetragen. Das Gemisch wurde drei Mal mit Methylenchlorid extrahiert, die vereinigten organischen Phasen über Magnesiumsulfat getrocknet, filtriert und eingeengt. Der Rückstand wurde mit etwas Acetonitril zu einer Suspension verrührt. Der unlösliche Teil wurde abgesaugt und anschließend mittels MPLC mit Laufmittel Acetonitril/Wasser gereinigt. Man erhielt 130 mg (99,0% Reinheit, 16,2% d. Th.) der Titelverbindung (II-62).
**1H-NMR(400,0 MHz, d₆-DMSO):** δ = 9,958(9,7); 9,780(0,6); 9,109(7,3); 9,104(7,2); 9,103(7,2); 9,096(0,8); 8,746(5,4); 8,742(6,0); 8,734(6,2); 8,730(6,1); 8,283(3,0); 8,278(4,1); 8,273(3,4); 8,263(3,6); 8,257(4,6); 8,253(3,5); 7,889(1,4); 7,883(12,5); 7,878(4,6); 7,866(4,6); 7,861(16,0); 7,854(2,0); 7,730(0,9); 7,725(0,3); 7,713(0,3); 7,707(1,1); 7,679(12,9); 7,657(10,8); 7,615(3,8); 7,613(4,1); 7,601(4,0); 7,595(3,9); 7,593(4,0); 7,583(3,6); 7,581(3,9); 7,384(1,2); 7,379(0,5); 7,361(1,4); 7,337(10,7); 7,267(0,8);3,329(92,9);2,677(0,8);2,672(1,1);2,668(0,8);2,525(2,6);2,512(58,6);2,508(119, 3);2,503(158,4);2,499(116,9);2,494(58,1);2,334(0,7);2,330(1,0);2,325(0,8);2,321(0,4);2,076(1,4);0,146 (0,6);0,008(4,8);0,000(137,9);-0,009(5,2);-0,150(0,6)

### Schritt 2: 2-(Pyridin-3-yl)-6-{4-[(trifluormethyl)sulfanyl]phenyl}[1,3]thiazolo[4,5-d]pyrimidin-7(6H)-on (62)

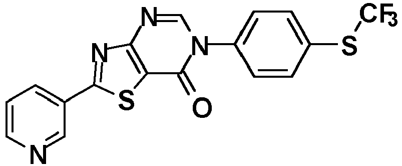

65 mg (0,16 mmol) 4-Amino-2-(pyridin-3-yl)-N-{4-[(trifluormethyl)sulfanyl]phenyl}-1,3-thiazol-5-carboxamid (II-62) wurden in 5 ml absolutem Ethanol vorgelegt. 14 mg (0,08 mmol) *p-*Toluolsulfonsäure sowie 72 mg (0,49 mmol) Orthoameisensäuretriethylester wurden dazu gegeben und das Reaktionsgemisch für 15 min in einer CEM Discover Mikrowelle auf 80 °C erhitzt. Das abgekühlte Reaktionsgemisch wurde im Vakuum eingeengt und in 3 ml Dichlormethan aufgenommen. Die Lösung wurde mit 2 ml gesättigter Natriumcarbonatlösung gewaschen und die abgetrennte organische Phase getrocknet, filtriert und eingedampft. Der Rückstand wurde anschließend mittels MPLC chromatographisch gereinigt. Man erhielt in Summe 20 mg (86,0% Reinheit, 26,1% d. Th.) der Titelverbindung (62).
**1H-NMR(400,0 MHz, d₆-DMSO):** δ = 9,954(1,2); 9,347(5,1); 9,343(5,1); 9,341(5,2); 9,106(0,9); 9,100(0,9); 8,835(3,9); 8,831(4,4); 8,823(4,0); 8,819(4,4); 8,744(0,7); 8,740(0,8); 8,732(0,8); 8,728(0,8); 8,701(16,0); 8,636(1,4); 8,558(2,1); 8,552(3,1); 8,548(2,5); 8,538(2,3); 8,532(3,3); 8,528(2,5); 8,280(0,4); 8,275(0,5); 8,271(0,4); 8,260(0,4); 8,255(0,6); 8,251(0,4); 7,969(7,3); 7,948(9,3); 7,881(1,5); 7,859(2,0); 7,806(1,5); 7,799(11,4); 7,795(3,7); 7,783(3,0); 7,778(9,2); 7,772(1,0); 7,688(0,6); 7,679(4,0); 7,667(4,3); 7,660(3,6); 7,647(4,4); 7,625(0,4); 7,612(0,5); 7,600(0,5); 7,592(0,5); 7,579(0,5); 7,333(1,3); 3,324(74,8); 2,676(0,7); 2,671(0,9); 2,667(0,7); 2,524(2,5); 2,511(45,7); 2,507(92,5); 2,502(124,5); 2,498(94,2); 2,493(47,7); 2,333(0,5); 2,329(0,8); 2,325(0,6); 1,298(0,5); 1,259(0,7); 1,233(1,0); 0,000(1,1)

### Schritt 3: 2-(Pyridin-3-yl)-6-{4-[(trifluormethyl)sulfinyl]phenyl}[1,3]thiazolo[4,5-d]pyrimidin-7(6H)-on (63)

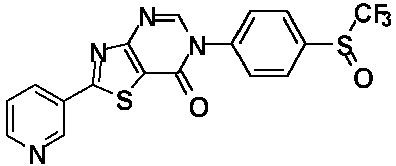

Zu einer Lösung von 35,0 mg (0,09 mmol) 2-(Pyridin-3-yl)-6-{4-[(trifluormethyl)sulfanyl]phenyl}[1,3]thiazolo[4,5-d]pyrimidin-7(6H)-on (62) in 1 ml Dichlormethan wurden bei 0 °C 20 mg (77%ig, 0,09 mmol) meta-Chlorperbenzoesäure gegeben. Das Reaktionsgemisch wurde 90 Minuten bei Raumtemperatur gerührt und dann mit gesättigter Natriumcarbonatlösung versetzt. Die wässrige Phase wurde noch dreimal mit Dichlormethan extrahiert und die vereinigten organischen Phasen getrocknet und filtriert. Nach Verdampfung des Lösungsmittels wurde der Rückstand anschließend mit Laufmittel Dichlormethan/Methanol (30:1) chromatographisch gereinigt. Man erhielt 10 mg (95,0% Reinheit, 26,1% d. Th.) der Titelverbindung (63).
**1H-NMR(400,0 MHz, d₆-DMSO):** δ = 9,344(0,4); 8,925(9,0); 8,748(0,7); 8,714(16,0); 8,649(1,1); 8,542(0,3); 8,462(5,6); 8,446(5,9); 8,317(1,5); 8,137(0,4); 8,117(0,7); 8,081(5,4); 8,061(5,8); 7,970(10,5); 7,950(13,1); 7,793(14,1); 7,772(11,5); 7,689(0,8); 7,666(5,7); 7,647(6,7); 7,629(4,4); 5,757(7,6); 3,507(0,3); 3,487(0,3); 3,447(0,4); 3,439(0,4); 3,409(0,5); 3,326(536,7); 3,245(0,3); 2,840(0,4); 2,820(0,4); 2,805(0,4); 2,765(0,4); 2,671(8,5); 2,622(1,1); 2,618(1,1); 2,502(1215,9); 2,329(7,9); 2,184(0,3); 1,355(0,6); 1,299(1,8); 1,259(2,9); 1,235(7,6); 1,166(0,5); 1,149(1,1); 1,066(0,4); 0,854(1,6); 0,843(1,0); 0,834(1,1); 0,814(0,7); 0,784(0,5); 0,146(5,7); 0,083(0,5); 0,000(1101,9); - 0,150(5,9)

### Herstellbeispiel 20: 2-(Pyridin-3-yl)-6-{2-[(2,2,2-trifluorethyl)sulfinyl]phenyl}[1,3]thiazolo[4,5-d]pyrimidin-7(6H)-on (69)

### Schritt 1: 4-Amino-2-(pyridin-3-yl)-N-{2-[(2,2,2-trifluorethyl)sulfanyl]phenyl}-1,3-thiazol-5-carboxamid (11-68)

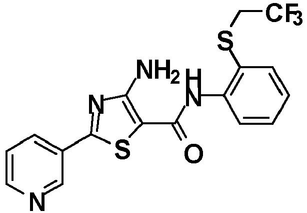

Zu 1,50 g (7,22 mmol) 2-[(2,2,2-Trifluorethyl)sulfinyl]anilin in 50 ml 1,2-Dichlorethan wurden unter Argon 3,6 ml einer 2M Trimethylaluminium-Lösung (7,22 mmol) in Toluol unter Eiskühlung langsam zugetropft und 1 Stunde bei Raumtemperatur gerührt. Anschließend wurden 600 mg (2,41 mmol) Ethyl-4-amino-2-(pyridin-3-yl)-1,3-thiazol-5-carboxylat (III-1) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 120 ml) ausgetragen. Das Gemisch wurde drei Mal mit Methylenchlorid extrahiert, die vereinigten organischen Phasen über Magnesiumsulfat getrocknet, filtriert und eingeengt. Der Rückstand wurde mit etwas Acetonitril zu einer Suspension verrührt. Der unlösliche Teil wurde abgesaugt und anschließend mittels MPLC mit Laufmittel Acetonitril/Wasser gereinigt. Man erhielt 240 mg (96,0% Reinheit, 23,3% d. Th.) der Titelverbindung (II-68).
**1H-NMR(400,0 MHz, d₆-DMSO):** δ = 9,458(10,4); 9,104(6,7); 9,099(6,7); 8,738(5,3); 8,734(5,7); 8,726(5,8); 8,722(5,6); 8,316(0,4); 8,279(3,0); 8,275(4,2); 8,269(3,1); 8,259(3,3); 8,254(4,3); 8,249(3,1); 7,661(4,8); 7,644(5,2); 7,641(5,2); 7,605(3,9); 7,603(3,9); 7,593(3,9); 7,591(3,9); 7,585(3,9); 7,583(3,8); 7,573(3,7); 7,571(3,6); 7,536(4,8); 7,519(5,5); 7,517(5,7); 7,365(2,4); 7,362(2,5); 7,346(5,0); 7,343(5,0); 7,327(3,3); 7,324(3,1); 7,287(3,6); 7,283(3,6); 7,267(4,9); 7,264(4,9); 7,249(2,2); 7,245(2,1); 7,176(12,1); 4,253(0,6); 4,235(0,6); 3,944(2,8); 3,918(8,8); 3,892(9,1); 3,866(3,1); 3,330(242,9); 2,676(0,9); 2,672(1,2); 2,667(0,9); 2,525(2,8); 2,507(127,7); 2,503(166,7); 2,498(124,1); 2,334(0,9); 2,329(1,2); 2,325(0,9); 2,075(16,0); 1,295(0,6); 1,278(1,3); 1,260(0,6); 1,233(0,7); 0,008(0,4); 0,000(10,3); -0,008(0,4)

### Schritt 2: 2-(Pyridin-3-yl)-6-{2-[(2,2,2-trifluorethyl)sulfanyl]phenyl}[1,3]thiazolo[4,5-d]pyrimidin-7(6H)-on (68)

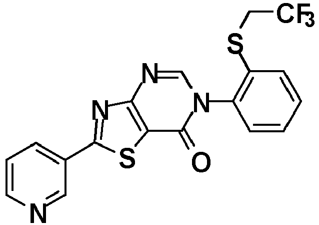

225 mg (0,53 mmol) 4-Amino-2-(pyridin-3-yl)-N-{2-[(2,2,2-trifluorethyl)sulfanyl]phenyl}-1,3-thiazol-5-carboxamid (II-68) und 45 mg (0,26 mmol) *p*-Toluolsulfonsäure wurden zusammen mit 5 ml (30,1 mmol) Orthoameisensäuretriethylester versetzt und das Reaktionsgemisch für 60 min in einer CEM Discover Mikrowelle auf 170 °C erhitzt. Das abgekühlte Reaktionsgemisch wurde im Vakuum eingeengt und anschließend mittels MPLC mit Laufmittel Cyclohexan/Ethylacetat chromatographisch gereinigt. Man erhielt in Summe 95 mg (99,0% Reinheit, 42,5% d. Th.) der Titelverbindung (68).
**1H-NMR(400,0 MHz, d₆-DMSO):** δ = 9,348(4,6); 9,344(4,6); 8,841(3,7); 8,837(4,0); 8,829(4,0); 8,825(4,0); 8,558(2,5); 8,551(16,0); 8,539(2,6); 8,533(3,3); 8,529(2,4); 7,918(3,2); 7,915(3,1); 7,899(4,0); 7,688(2,9); 7,686(2,8); 7,676(2,9); 7,674(2,9); 7,667(5,2); 7,663(4,0); 7,654(4,6); 7,648(3,9); 7,645(5,9); 7,639(5,5); 7,635(3,0); 7,620(3,2); 7,616(2,2); 7,598(3,4); 7,595(3,5); 7,579(3,1); 7,576(3,5); 7,561(1,3); 7,557(1,2); 5,757(3,6); 4,056(0,6); 4,038(1,0); 4,031(1,1); 4,020(1,3); 4,017(1,0); 4,006(1,3); 3,992(2,6); 3,980(0,7); 3,967(3,0); 3,944(2,9); 3,930(0,6); 3,918(2,8); 3,905(1,2); 3,892(1,0); 3,878(1,2); 3,852(0,4); 3,329(31,2); 2,677(0,4); 2,672(0,5); 2,668(0,4); 2,526(1,3); 2,512(26,5); 2,508(53,3); 2,503(70,5); 2,499(52,7); 2,495(27,0); 2,335(0,3); 2,330(0,5); 2,325(0,4); 1,990(3,7); 1,397(1,3); 1,299(0,8); 1,259(1,1); 1,250(0,4); 1,234(1,6); 1,193(1,2); 1,176(2,1); 1,158(1,1); 0,146(0,6); 0,008(4,9); 0,000(120,6); -0,008(6,0); -0,150(0,6)

### Schritt 3: 2-(Pyridin-3-yl)-6-{2-[(2,2,2-trifluorethyl)sulfinyl]phenyl}[1,3]thiazolo[4,5-d]pyrimidin-7(6H)-on (69)

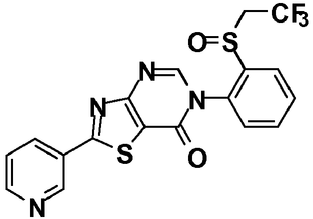

Zu einer Lösung von 80,0 mg (0,18 mmol) 2-(Pyridin-3-yl)-6-{2-[(2,2,2-trifluorethyl)sulfanyl]phenyl}[1,3]thiazolo[4,5-d]pyrimidin-7(6H)-on (68) in 1 ml Dichlormethan wurden bei 0 °C 42 mg (77%ig, 0,19 mmol) meta-Chlorperbenzoesäure gegeben. Das Reaktionsgemisch wurde 30 Minuten bei Raumtemperatur gerührt und dann mit gesättigter Natriumcarbonatlösung versetzt. Die wässrige Phase wurde noch dreimal mit Dichlormethan extrahiert und die vereinigten organischen Phasen getrocknet und filtriert. Nach Verdampfung des Lösungsmittels wurde der Rückstand anschließend mittels MPLC mit Laufmittel Cyclohexan/Ethylacetat chromatographisch gereinigt. Man erhielt 10 mg (90,0% Reinheit, 11,3% d. Th.) der Titelverbindung (69).
**1H-NMR(601,6 MHz, CDCl₃):** δ = 9,372(12,3); 8,822(9,3); 8,817(9,2); 8,465(7,5); 8,453(8,0); 8,307(16,0); 8,291(8,4); 8,278(8,2); 8,192(1,6); 8,154(0,9); 7,937(0,8); 7,910(4,3); 7,898(8,3); 7,885(5,1); 7,837(5,4); 7,825(9,3); 7,813(6,0); 7,583(0,8); 7,525(6,1); 7,516(7,8); 7,513(7,9); 7,505(6,3); 7,451(9,5); 7,439(8,9); 7,263(66,7); 7,086(0,4); 5,301(4,1); 4,129(1,1); 4,112(3,4); 4,094(4,4); 4,072(3,5); 4,055(1,2); 3,882(0,3); 3,859(0,5); 3,841(0,4); 3,642(0,4); 3,625(0,5); 3,597(1,4); 3,580(3,6); 3,564(4,4); 3,558(4,0); 3,541(3,2); 3,525(1,1); 3,490(0,5); 1,577(178,8); 1,426(0,4); 1,371(1,1); 1,333(2,4); 1,285(4,4); 1,256(10,6); 1,104(0,5); 0,881(1,6); 0,842(1,6); 0,070(0,6); 0,000(47,7)

### Herstellbeispiel 21: 6-{2-Fluor-4-methyl-5-[(2,2,2-trifluorethyl)sulfinyl]phenyl}-2-(5-fluorpyridin-3-yl)[1,3]thiazolo[4,5-d]pyrimidin-7(6H)-on (74)

### Schritt 1: 4-Amino-N-{2-fluor-4-methyl-5-[(2,2,2-trifluorethyl)sulfanyl]phenyl}-2-(5-fluorpyridin-3-yl)-1,3-thiazol-5-carboxamid (II-73)

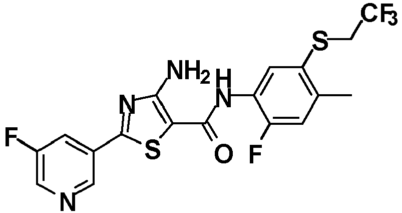

Zu 1,34 g (5,61 mmol) 2-Fluor-4-methyl-5-[(2,2,2-trifluorethyl)sulfanyl]anilin in 50 ml 1,2-Dichlorethan wurden unter Argon 2,8 ml einer 2M Trimethylaluminium-Lösung (5,61 mmol) in Toluol unter Eiskühlung langsam zugetropft und 1 Stunde bei Raumtemperatur gerührt. Anschließend wurden 500 mg (1,87 mmol) Ethyl-4-amino-2-(5-fluorpyridin-3-yl)-1,3-thiazol-5-carboxylat (III-2) dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 120 ml) ausgetragen. Das Gemisch wurde drei Mal mit Methylenchlorid extrahiert, die vereinigten organischen Phasen über Magnesiumsulfat getrocknet, filtriert und eingeengt. Der Rückstand wurde mit etwas Acetonitril zu einer Suspension verrührt. Der unlösliche Teil wurde abgesaugt, mit n-Pentan gewaschen und getrocknet. Man erhielt 330 mg (98,0% Reinheit, 37,6% d. Th.) der Titelverbindung (II-73).
**1H-NMR(400,0 MHz, d₆-DMSO):** δ = 9,613(4,5); 8,971(2,3); 8,968(4,0); 8,770(3,9); 8,763(4,1); 8,155(1,2); 8,150(1,6); 8,144(1,2); 8,132(1,3); 8,127(1,7); 8,120(1,2); 7,705(3,1); 7,686(3,1); 7,273(2,8); 7,245(2,8); 7,208(5,1); 4,259(0,3); 4,241(0,3); 3,903(2,7); 3,875(4,0); 3,849(4,2); 3,823(1,4); 3,329(26,3); 2,672(0,4); 2,668(0,3); 2,525(1,0); 2,512(24,8); 2,507(49,6); 2,503(65,4); 2,499(49,2); 2,407(16,0); 2,334(0,3); 2,330(0,4); 2,325(0,3); 1,297(0,4); 1,279(0,7); 1,262(0,3); 0,008(1,0); 0,000(27,4); -0,008(1,2)

### Schritt 2: 6-{2-Fluor-4-methyl-5-[(2,2,2-trifluorethyl)sulfanyl]phenyl}-2-(5-fluorpyridin-3-yl)[1,3]thiazolo[4,5-d]pyrimidin-7(6H)-on (73)

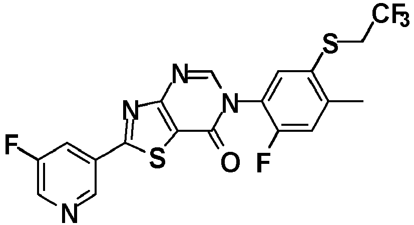

200 mg (Reinheit 98%ig, 0,43 mmol) 4-Amino-N-{2-fluor-4-methyl-5-[(2,2,2-trifluorethyl)sulfanyl]phenyl}-2-(5-fluorpyridin-3-yl)-1,3-thiazol-5-carboxamid (II-73) wurden in 1 ml *N,N*-Dimethylacetamid vorgelegt. 37 mg (0,21 mmol) *p*-Toluolsulfonsäure und 189 mg (1,28 mmol) Orthoameisensäuretriethylester wurden dazu gegeben und das Reaktionsgemisch für 30 Minuten in einer CEM Discover Mikrowelle bei 200 Watt auf 130 °C erhitzt. Das abgekühlte Reaktionsgemisch wurde im Hochvakuum eingeengt und mittels MPLC mit Laufmittel Acetonitril/Wasser gereinigt. Man erhielt 50 mg (97,0% Reinheit, 24,2% d. Th.) der Titelverbindung (73).
**1H-NMR(400,0 MHz, d₆-DMSO):** δ = 9,243(3,5); 8,875(3,5); 8,868(3,6); 8,693(7,7); 8,535(1,2); 8,530(1,7); 8,524(1,2); 8,512(1,2); 8,507(1,7); 8,501(1,1); 7,940(3,1); 7,922(3,1); 7,521(2,7); 7,494(2,7); 4,061(1,2); 4,035(3,8); 4,009(3,9); 3,984(1,4); 3,329(53,8); 2,676(0,3); 2,672(0,5); 2,668(0,3); 2,507(49,1); 2,503(64,3); 2,499(49,1); 2,471(16,0); 2,414(0,5); 2,334(0,3); 2,330(0,4); 2,325(0,3); 2,076(14,3); 1,233(0,9); 0,000(0,4)

### Schritt 3: 6-{2-Fluor-4-methyl-5-[(2,2,2-trifluorethyl)sulfinyl]phenyl}-2-(5-fluorpyridin-3-yl)[1,3]thiazolo[4,5-d]pyrimidin-7(6H)-on (74)

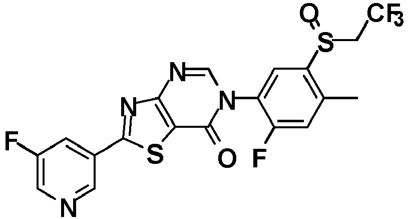

Zu einer Lösung von 96,0 mg (0,19 mmol) 6-{2-Fluor-4-methyl-5-[(2,2,2-trifluorethyl)sulfanyl]phenyl}-2-(5-fluorpyridin-3-yl)[1,3]thiazolo[4,5-d]pyrimidin-7(6H)-on (73) in 1 ml Dichlormethan wurden bei 0 °C 42 mg (77%ig, 0,19 mmol) meta-Chlorperbenzoesäure gegeben. Das Reaktionsgemisch wurde 1 Stunde bei Raumtemperatur gerührt und dann mit gesättigter Natriumcarbonatlösung versetzt. Die wässrige Phase wurde noch dreimal mit Dichlormethan extrahiert und die vereinigten organischen Phasen getrocknet und filtriert. Nach Verdampfung des Lösungsmittels wurde der Rückstand anschließend mittels MPLC mit Laufmittel Cyclohexan/Ethylacetat chromatographisch gereinigt. Man erhielt 65 mg (94,0% Reinheit, 68,4% d. Th.) der Titelverbindung (74).
**1H-NMR(400,0 MHz, d₆-DMSO):** δ = 9,249(4,3); 9,246(7,4); 9,242(4,4); 8,876(7,8); 8,870(8,1); 8,738(16,0); 8,538(2,5); 8,533(3,1); 8,531(3,1); 8,527(2,4); 8,515(2,6); 8,510(3,3); 8,503(2,4); 8,447(0,5); 8,317(0,7); 8,200(6,2); 8,182(6,2); 7,639(4,3); 7,613(4,3); 5,758(2,5); 4,299(0,6); 4,266(1,0); 4,241(0,8); 4,147(0,4); 4,126(0,7); 4,099(0,8); 4,038(0,5); 4,020(0,4); 3,568(3,3); 3,329(336,9); 2,676(1,4); 2,671(2,0); 2,667(1,5); 2,525(5,0); 2,520(7,9); 2,511(110,1); 2,507(225,1); 2,502(308,8); 2,498(233,4); 2,494(111,7); 2,377(1,3); 2,334(1,5); 2,329(2,0); 2,325(1,5); 1,989(1,2); 1,234(0,4); 1,207(0,4); 1,193(0,6); 1,189(0,7); 1,175(0,8); 1,157(0,3); 0,000(0,9)

### Herstellbeispiel 22: 6-{2,4-Dimethyl-5-[(2,2,2-trifluorethyl)sulfinyl]phenyl}-2-(pyrimidin-5-yl)[1,3]thiazolo[4,5-d]pyrimidin-7(6H)-on (77)

### Schritt 1: 4-Amino-N-{2,4-dimethyl-5-[(2,2,2-trifluorethyl)sulfanyl]phenyl}-2-(pyrimidin-5-yl)-1,3-thiazol-5-carboxamid (11-76)

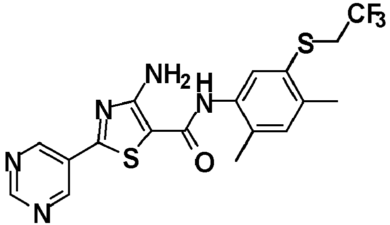

Zu 987 mg (4,20 mmol) 2,4-Dimethyl-5-[(2,2,2-trifluorethyl)sulfanyl]anilin in 70 ml 1,2-Dichlorethan wurden unter Argon 2,1 ml einer 2M Trimethylaluminium-Lösung (4,20 mmol) in Toluol unter Eiskühlung langsam zugetropft und 1 Stunde bei Raumtemperatur gerührt. Anschließend wurden 350 mg (1,40 mmol) Ethyl-4-amino-2-(pyrimidin-5-yl)-1,3-thiazol-5-carboxylat (III-3)dazu gegeben und das Reaktionsgemisch im Anschluss über Nacht unter Rückfluss erhitzt. Nach dem Abkühlen wurde das Reaktionsgemisch vorsichtig in eine 10%ige Kalium-Natrium-Tartrat-Lösung (ca. 120 ml) ausgetragen. Das Gemisch wurde drei Mal mit Methylenchlorid extrahiert, die vereinigten organischen Phasen über Magnesiumsulfat getrocknet, filtriert und eingeengt. Der Rückstand wurde mit etwas Acetonitril zu einer Suspension verrührt. Der unlösliche Teil wurde abgesaugt, mit n-Pentan gewaschen und getrocknet. Man erhielt 215 mg (90,0% Reinheit, 31,5% d. Th.) der Titelverbindung (II-76).
**1H-NMR(400,0 MHz, d₆-DMSO):** δ = 9,410(4,5); 9,327(6,4); 9,285(1,2); 9,247(11,7); 7,465(5,7); 7,176(7,8); 7,170(7,4); 6,814(0,5); 6,793(0,6); 5,754(0,7); 4,743(0,5); 4,264(0,5); 4,246(0,5); 3,875(1,3); 3,849(4,0); 3,823(4,1); 3,798(1,5); 3,691(0,5); 3,666(0,5); 3,320(121,1); 2,671(1,0); 2,502(151,6); 2,418(0,3); 2,367(15,3); 2,329(1,4); 2,205(1,9); 2,162(16,0); 2,074(0,8); 2,000(1,8); 1,300(0,5); 1,282(1,1); 1,265(0,6); 0,147(0,4); 0,000(72,2); -0,149(0,4)

### Schritt 2: 6-{2,4-Dimethyl-5-[(2,2,2-trifluorethyl)sulfanyl]phenyl}-2-(pyrimidin-5-yl)[1,3]thiazolo[4,5-d]pyrimidin-7(6H)-on (76)

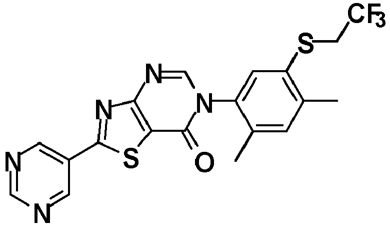

100 mg (0,23 mmol) 4-Amino-N-{2,4-dimethyl-5-[(2,2,2-trifluorethyl)sulfanyl]phenyl}-2-(pyrimidin-5-yl)-1,3-thiazol-5-carboxamid (II-76) und 20 mg (0,11 mmol) *p*-Toluolsulfonsäure wurden zusammen mit 5 ml (30,1 mmol) Orthoameisensäuretriethylester versetzt und das Reaktionsgemisch für 40 min in einer CEM Discover Mikrowelle auf 150 °C erhitzt. Das abgekühlte Reaktionsgemisch wurde im Vakuum eingeengt und anschließend mittels MPLC chromatographisch gereinigt. Man erhielt 36 mg (94,0% Reinheit, 33,1% d. Th.) der Titelverbindung (76).
**1H-NMR(400,0 MHz, d6-DMSO): δ** = 9,538(16,0); 9,429(7,5); 8,559(8,5); 7,680(4,9); 7,362(3,9); 5,754(1,8); 4,034(1,0); 4,008(2,9); 3,982(3,1); 3,956(1,1); 3,318(38,5); 2,671(0,4); 2,524(1,0); 2,511(23,9); 2,506(47,7); 2,502(62,3); 2,497(45,4); 2,493(22,1); 2,411(12,3); 2,368(0,6); 2,329(0,4); 2,170(0,5); 2,080(13,3); 1,352(1,1); 1,336(0,4); 1,259(0,4); 1,250(0,6); 1,229(1,2); 0,000(3,8)

### Schritt 3: 6-{2,4-Dimethyl-5-[(2,2,2-trifluorethyl)sulfinyl]phenyl}-2-(pyrimidin-5-yl)[1,3]thiazolo[4,5-d]pyrimidin-7(6H)-on (77)

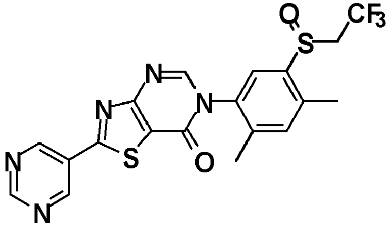

Zu einer Lösung von 70,0 mg (0,15 mmol) 6-{2,4-Dimethyl-5-[(2,2,2-trifluorethyl)sulfanyl]phenyl}-2-(pyrimidin-5-yl)[1,3]thiazolo[4,5-d]pyrimidin-7(6H)-on (76) in 3 ml Dichlormethan wurden bei 0 °C 33 mg (77%ig, 0,15 mmol) meta-Chlorperbenzoesäure gegeben. Das Reaktionsgemisch wurde noch 1 Stunde in der Kälte und anschließend über Nacht bei Raumtemperatur gerührt. Der Ansatz wurde dann nacheinander mit gesättigter Natriumhydrogensulfitlösung, Natriumhydrogencarbonatlösung und Natriumchloridlösung gewaschen, getrocknet und eingeengt. Der Rückstand wurde anschließend mittels MPLC chromatographisch gereinigt. Man erhielt 20 mg (97,0% Reinheit, 28,7% d. Th.) der Titelverbindung (77).
**1H-NMR(400,0 MHz, d₆-DMSO):** δ = 9,542(16,0); 9,429(7,8); 8,612(8,8); 7,949(4,1); 7,939(2,8); 7,497(2,0); 7,471(3,0); 5,754(0,9); 4,321(0,4); 4,293(0,5); 4,284(0,5); 4,257(0,5); 4,175(0,4); 4,166(0,4); 4,148(1,1); 4,139(1,1); 4,121(1,1); 4,112(1,2); 4,094(0,4); 4,085(0,4); 3,969(0,5); 3,942(0,5); 3,932(0,4); 3,905(0,5); 3,317(54,5); 2,675(0,5); 2,671(0,7); 2,666(0,5); 2,506(79,6); 2,502(106,3); 2,497(82,4); 2,452(8,0); 2,447(10,7); 2,338(0,5); 2,333(0,6); 2,328(0,7); 2,324(0,6); 2,258(0,4); 2,188(10,5); 2,182(8,0); 1,234(0,7); 0,008(0,5); 0,000(13,1)

### Synthese von Aminothiazolen der Formel (III) nach Verfahren B

### Herstellbeispiel 9: Ethyl-4-amino-2-(pyridin-3-yl)-1,3-thiazol-5-carboxylat (III-1)

### Schritt 1: Ethyl-4-hydroxy-2-(pyridin-3-yl)-1,3-thiazol-5-carboxylat (VI-1)

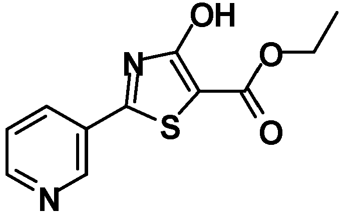

32,5 g (235,2 mmol) Pyridin-3-carbothioamid wurden in 200 ml Ethanol vorgelegt und 58,5 g (244,6 mmol) Diethyl-brommalonat hinzugegeben. Die Mischung wurde über Nacht unter Rückfluss erhitzt, anschließend abgekühlt und filtriert. Der Filterrückstand wurde gut mit Ethanol gewaschen und im Vakuum getrocknet. Man erhielt 26,0 g (83,0% Reinheit, 36,7% d. Th.) der Titelverbindung (VI-1).
**1H-NMR (399,9 MHz, d₆-DMSO):** δ = 9,150 (2,8); 9,145 (2,8); 8,771 (1,9); 8,768 (2,0); 8,759 (2,0); 8,756 (2,0); 8,399 (1,1); 8,395 (1,7); 8,390 (1,3); 8,379 (1,3); 8,375 (1,8); 7,670 (1,6); 7,657 (1,7); 7,649 (1,7); 7,637 (1,5); 7,249 (13,6); 7,121 (14,1); 6,993 (13,7); 4,695 (0,3); 4,673 (0,4); 4,609 (0,4); 4,597 (0,5); 4,584 (0,5); 4,578 (0,5); 4,559 (0,5); 4,507 (0,6); 4,498 (0,6); 4,488 (0,6); 4,475 (0,6); 4,459 (0,6); 4,441 (0,7); 4,435 (0,7); 4,404 (0,7); 4,397 (0,7); 4,387 (0,8); 4,369 (0,8); 4,351 (0,7); 4,335 (0,7); 4,294 (3,1); 4,276 (8,4); 4,259 (8,5); 4,241 (3,1); 4,111 (0,4); 4,089 (0,3); 4,071 (0,3); 2,533 (0,5); 2,504 (7,3); 2,500 (14,4); 2,495 (20,1); 2,490 (14,7); 2,486 (7,6); 1,306 (7,9); 1,288 (16,0); 1,270 (7,7); 1,064 (0,5); 0,000 (2,3)

### Schritt 2: Ethyl-2-(pyridin-3-yl)-4-{[(trifluormethyl)sulfonyl]oxy}-1,3-thiazol-5-carboxylat (V-1)

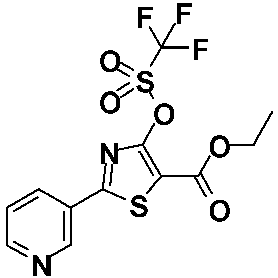

500 mg (2 mmol) trockenes Ethyl-4-hydroxy-2-(pyridin-3-yl)-1,3-thiazol-5-carboxylat (VI-1) wurden zusammen mit 414 g (3 mmol) Kaliumcarbonat in Dichlormethan unter Argon bei Raumtemperatur vorgelegt. 620 mg (2,2 mmol) Trifluormethansulfonsäureanhydrid wurden innerhalb von 10 Minuten zugetropft und die Mischung über Nacht bei Raumtemperatur gerührt. Der Ansatz wurde anschließend mit gesättigter Natriumhydrogencarbonat-Lösung gewaschen, die organische Phase über wenig Celite filtriert, mit Dichlormethan nachgewaschen und das Filtrat über Natriumsulfat getrocknet. Die Aufreinigung des nach Eindampfen erhaltenen Rückstands erfolgte dann mittels MPLC im Laufmittel Cyclohexan/Ethylacetat im Gradienten. Man erhielt 220 mg (100,0% Reinheit, 28,7% d. Th.) der Titelverbindung (V-1).
**1H-NMR(399,9 MHz, d₆-DMSO):** δ = 3,115 (16,0); 2,498 (0,9); 2,493 (1,8); 2,489 (2,5); 2,484 (1,8); 2,479 (0,9); 1,341 (0,5)

### Schritt 3: Ethyl-4-(benzylamino)-2-(pyridin-3-yl)-1,3-thiazol-5-carboxylat (IV-1)

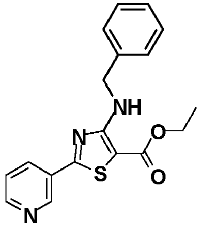

20,0 g (52,0 mmol) Ethyl-2-(pyridin-3-yl)-4-{[(trifluormethyl)sulfonyl]oxy}-1,3-thiazol-5-carboxylat (V-1) und 11,2 g (104,1 mmol) Benzylamin wurden in 100 ml Dioxan über Nacht unter Rückfluss gerührt. Nach Abkühlung und Eindampfen der Reaktionsmischung wurde der ölige Rückstand mit Methanol versetzt, der ausgefallene gelbe Feststoff abgesaugt und mit Pentan gewaschen. Überschüssiges Benzylamin im Rohprodukt wurde anschließend durch Waschen mit 1 N Salzsäure entfernt. Das Rohprodukt wurde danach mit Wasser gewaschen, mit Pentan verrührt, abgesaugt und im Vakuum getrocknet. Man erhielt 13,5 g (100% Reinheit, 76,4% d. Th.) der Titelverbindung (IV-1).
**1H-NMR(400,0 MHz, d₆-DMSO):** δ = 9,153(4,6); 9,148(4,9); 8,745(3,2); 8,742(3,3); 8,733(3,3); 8,730(3,1); 8,374(1,6); 8,370(2,5); 8,366(1,7); 8,354(1,9); 8,350(2,7); 7,618(3,8); 7,605(3,4); 7,597(2,9); 7,585(2,4); 7,413(4,4); 7,394(7,2); 7,347(4,0); 7,329(7,1); 7,309(3,6); 7,251(2,2); 7,233(3,1); 7,215(1,1); 4,780(4,8); 4,767(4,8); 4,285(2,3); 4,267(7,3); 4,249(7,4); 4,232(2,5); 4,039(0,4); 4,021(0,4); 3,913(2,0); 3,775(0,7); 3,760(0,7); 3,744(0,7); 2,509(41,8); 2,505(53,2); 2,501(40,7); 2,332(0,4); 1,990(1,1); 1,297(7,8); 1,280(16,0); 1,262(7,6); 1,176(0,6); 0,000(6,0)

### Schritt 4: Ethyl-4-amino-2-(pyridin-3-yl)-1,3-thiazol-5-carboxylat (III-1)

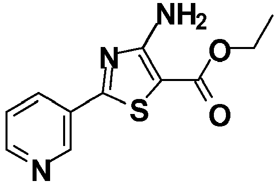

40 ml konzentrierte Schwefelsäure wurden vorgelegt und 8,5 g (25,0 mmol) Ethyl-4-(benzylamino)-2-(pyridin-3-yl)-1,3-thiazol-5-carboxylat (IV-1) langsam bei 20 °C zugegeben. Die Reaktionsmischung wurde 3 h bei Raumtemperatur gerührt, danach auf 500 ml Eiswasser gegeben und vorsichtig mit 30%iger Kaliumhydroxidlösung auf pH10 eingestellt. Die Mischung wurde anschließend mit Ethylacetat extrahiert, die organischen Phasen getrocknet und eingedampft. Man erhielt 6,0 g (100% Reinheit, 96,1% d. Th.) der Titelverbindung (III-1).
**1H-NMR(400,0 MHz, d₆-DMSO):** δ = 9,113(4,3); 9,109(4,3); 9,107(4,3); 8,730(3,0); 8,727(3,4); 8,718(3,3); 8,715(3,4); 8,296(1,7); 8,291(2,6); 8,286(1,9); 8,276(1,8); 8,271(2,7); 8,266(2,0); 7,578(2,4); 7,566(2,5); 7,558(2,4); 7,546(2,3); 7,139(3,5); 4,271(2,4); 4,253(7,5); 4,236(7,6); 4,218(2,5); 3,333(25,6); 2,504(43,0); 2,500(34,6); 1,990(0,5); 1,296(7,8); 1,278(16,0); 1,261(7,7); 0,000(0,6)

### Herstellbeispiel 23: Ethyl-4-amino-2-(5-fluorpyridin-3-yl)-1,3-thiazol-5-carboxylat (III-2)

### Schritt 1: Ethyl-2-(5-fluorpyridin-3-yl)-4-hydroxy-1,3-thiazol-5-carboxylat (VI-2)

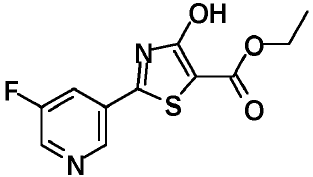

38,6 g (247 mmol) 5-Fluorpyridin-3-carbothioamid wurden in 250 ml Ethanol vorgelegt und anschließend 60 ml Pyridin und 177,3 g (741 mmol) Diethyl-brommalonat hinzugegeben. Die Mischung wurde über Nacht unter Rückfluss erhitzt, anschließend abgekühlt und filtriert. Der Filterrückstand wurde gut mit Ethanol gewaschen und im Vakuum getrocknet. Man erhielt 37,0 g (94,9% Reinheit, 53,0% d. Th.) der Titelverbindung (VI-2).
**1H-NMR (400,0 MHz, d₆-DMSO):** δ = 9,013(4,9); 8,765(4,9); 8,758(5,0); 8,227(1,6); 8,221(2,1); 8,216(1,6); 8,204(1,6); 8,198(2,1); 8,193(1,5); 4,275(2,3); 4,258(7,3); 4,240(7,4); 4,222(2,4); 3,324(2,6); 2,508(32,1); 2,504(41,6); 2,500(31,7); 1,294(7,8); 1,276(16,0); 1,259(7,6); 0,000(3,7)

### Schritt 2: Ethyl-2-(5-fluorpyridin-3-yl)-4-{[(trifluormethyl)sulfonyl]oxy}-1,3-thiazol-5-carboxylat (V-2)

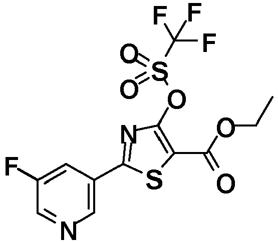

37,0 g (138 mmol) Ethyl-2-(5-fluorpyridin-3-yl)-4-hydroxy-1,3-thiazol-5-carboxylat (VI-2) wurden unter Argon in 300 ml Dichlormethan vorgelegt, 45 ml Pyridin zugegeben und der Ansatz auf 0 °C abgekühlt. Anschließend wurden 54,5 g (193 mmol) Trifluormethansulfonsäureanhydrid langsam zugetropft und die Mischung über Nacht bei Raumtemperatur gerührt. Der Ansatz wurde filtriert, der Filterrückstand mit Dichlormethan gewaschen und das Filtrat eingedampft. Der erhaltene Rückstand wurde mit 400 ml Methyl-tert-butyl-ether (MTBE) für 30 min auf 55 °C erwärmt und warm dekantiert. Dieser Vorgang wurde mit jeweils weiteren 300 ml MTBE wiederholt. Die vereinigten MTBE-Fraktionen wurden eingedampft und der erhaltene Rückstand im Vakuum getrocknet. Man erhielt 55,8 g (98,6% Reinheit, 99,6% d. Th.) der Titelverbindung (V-2).
**1H-NMR(400,0 MHz, d₆-DMSO): δ** = **9**,083(2,7); 9,079(4,5); 9,076(2,7); 8,849(4,5); 8,842(4,5); 8,364(1,5); 8,359(1,9); 8,357(1,9); 8,353(1,5); 8,341(1,6); 8,336(2,0); 8,334(1,8); 8,329(1,4); 4,427(2,2); 4,409(7,2); 4,392(7,3); 4,374(2,3); 3,569(13,3); 3,344(8,3); 2,672(0,3); 2,526(0,8); 2,512(19,0); 2,508(38,3); 2,503(50,2); 2,499(37,2); 2,494(18,6); 2,330(0,3); 1,355(7,6); 1,337(16,0); 1,319(7,4); 0,000(3,2)

### Schritt 3: Ethyl-4-(benzylamino)-2-(5-fluorpyridin-3-yl)-1,3-thiazol-5-carboxylat (IV-2)

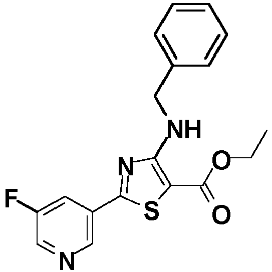

55,8 g (139 mmol) Ethyl-2-(5-fluorpyridin-3-yl)-4-{[(trifluormethyl)sulfonyl]oxy}-1,3-thiazol-5-carboxylat (V-2) und 29,7 g (277 mmol) Benzylamin wurden in 300 ml Dioxan über Nacht unter Rückfluss gerührt. Nach Abkühlung und Eindampfen der Reaktionsmischung wurde der ölige Rückstand mit Methanol versetzt, der ausfallende gelbe Feststoff abgesaugt und mit Pentan gewaschen. Überschüssiges Benzylamin im Rohprodukt wurde anschließend durch Waschen mit 1 N Salzsäure entfernt. Das Rohprodukt wurde danach mit Wasser gewaschen, mit Pentan verrührt, abgesaugt und im Vakuum getrocknet. Man erhielt 42,6 g (100% Reinheit, 85,9% d. Th.) der Titelverbindung (IV-2).
**1H-NMR(400,0 MHz, d₆-DMSO):** δ = 9,015(2,9); 9,011(4,9); 8,746(4,6); 8,740(4,7); 8,249(1,5); 8,244(1,9); 8,242(1,9); 8,238(1,5); 8,225(1,6); 8,220(2,0); 8,214(1,5); 7,628(1,1); 7,612(2,3); 7,596(1,2); 7,444(0,4); 7,411(4,0); 7,393(6,2); 7,344(3,6); 7,340(1,3); 7,326(6,6); 7,307(3,3); 7,249(2,0); 7,231(2,8); 7,213(1,0); 4,782(5,7); 4,766(5,7); 4,289(2,2); 4,271(7,1); 4,253(7,3); 4,236(2,3); 4,029(0,5); 3,568(0,4); 3,318(32,2); 2,676(0,4); 2,671(0,6); 2,667(0,4); 2,524(1,1); 2,511(33,4); 2,507(65,6); 2,502(85,2); 2,498(63,3); 2,494(31,8); 2,333(0,4); 2,329(0,6); 2,324(0,4); 1,298(7,6); 1,280(16,0); 1,262(7,5); 0,146(0,4); 0,008(3,4); 0,000(85,6); -0,008(3,8); -0,019(0,4); -0,150(0,4)

### Schritt 4: Ethyl-4-amino-2-(5-fluorpyridin-3-yl)-1,3-thiazol-5-carboxylat (III-2)

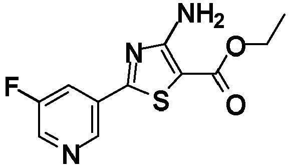

3 ml konzentrierte Schwefelsäure wurden vorgelegt und 500 mg (1,39 mmol) Ethyl-4-(benzylamino)-2-(5-fluorpyridin-3-yl)-1,3-thiazol-5-carboxylat (IV-2) langsam bei 20 °C zugegeben. Die Reaktionsmischung wurde über Nacht bei Raumtemperatur gerührt, danach auf 10 ml Eiswasser gegeben und vorsichtig mit 30%iger Kaliumhydroxidlösung auf pH10 eingestellt. Die Mischung wurde anschließend mit Ethylacetat extrahiert, die organischen Phasen über Magnesiumsulfat getrocknet und eingedampft. Der Rückstand wurde mit n-Pentan verrührt, filtriert und getrocknet. Man erhielt 200 mg (100% Reinheit, 53,5% d. Th.) der Titelverbindung (III-2).
**1H-NMR(400,0 MHz, d6-DMSO):** δ = 8,994(6,0); 8,757(5,4); 8,750(5,8); 8,193(2,8); 8,170(2,8); 7,143(4,8); 4,278(2,6); 4,260(7,7); 4,242(7,8); 4,225(2,7); 3,319(18,0); 2,673(0,3); 2,504(52,6); 2,331(0,3); 1,298(8,1); 1,280(16,0); 1,263(7,9); 0,000(11,1)

Weitere Verbindungen der Formel (I) sind in der folgenden Tabelle aufgeführt.

**Tabelle 1**

| Verbindungen der Formel (I) | | | | | |
|---|---|---|---|---|---|
| 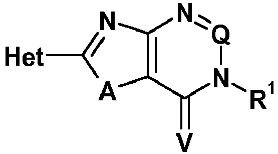 | | | | | |
| **Verbindungs-Nr.** | **Het** | **A** | **Q** | **V** | **R¹** |
| 1 | 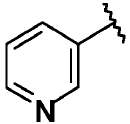 | S | CH | O | 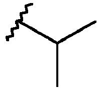 |
| 2 | 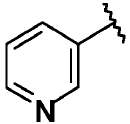 | S | CH | O | 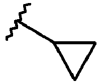 |
| 3 | 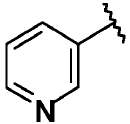 | S | CH | O | 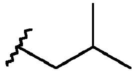 |
| 4 | 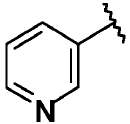 | S | CH | O | 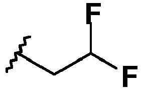 |
| 5 | 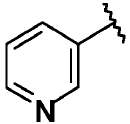 | S | CH | O | 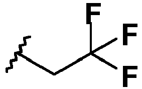 |
| 6 | 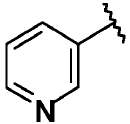 | S | CH | O | 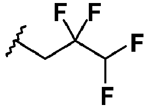 |
| 7 | 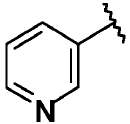 | S | CH | O | 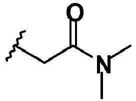 |
| 8 | 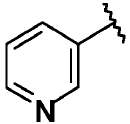 | S | CH | O | 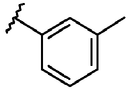 |

| **Verbindungs-Nr.** | **Het** | **A** | **Q** | **V** | **R¹** |
|---|---|---|---|---|---|
| 9 | 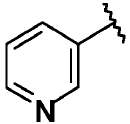 | S | CH | O | 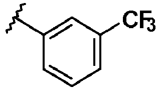 |
| 10 | 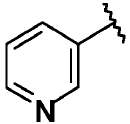 | S | CH | O | 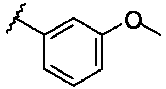 |
| 11 | 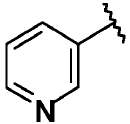 | S | CH | O | 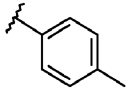 |
| 12 | 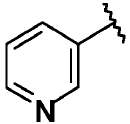 | S | CH | O | 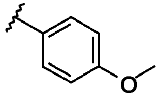 |
| 13 | 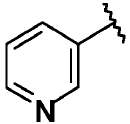 | S | CH | O | 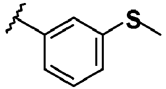 |
| 14 | 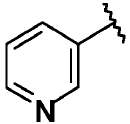 | S | CH | O | 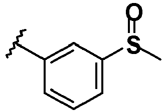 |
| 15 | 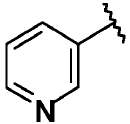 | S | CH | O | 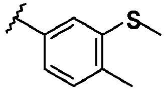 |
| 16 | 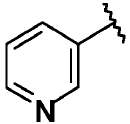 | S | CH | O | 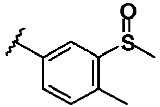 |
| 17 | 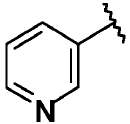 | S | CH | O | 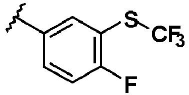 |
| 18 | 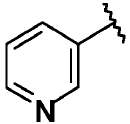 | S | CH | O | 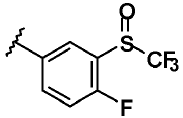 |
| 19 | 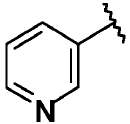 | S | CH | O | 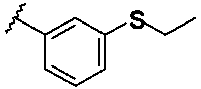 |
| 20 | 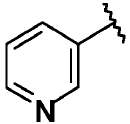 | S | CH | O | 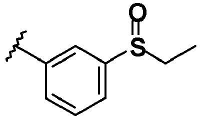 |
| 21 | 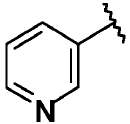 | S | CH | O | 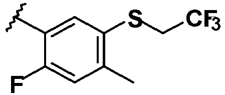 |
| 22 | 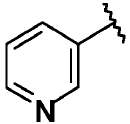 | S | CH | O | 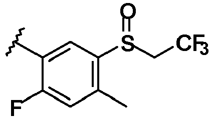 |
| 23 | 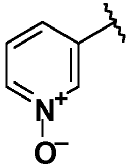 | S | CH | O | 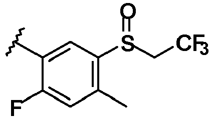 |
| 24 | 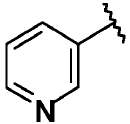 | S | CH | O | 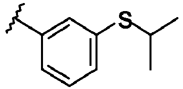 |
| 25 | 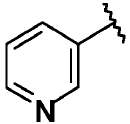 | S | CH | O | 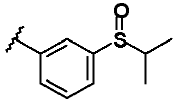 |
| 26 | 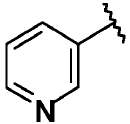 | S | CH | O | 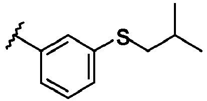 |
| 27 | 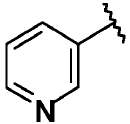 | S | CH | O | 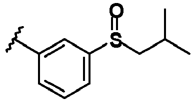 |
| 28 | 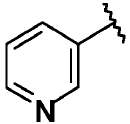 | S | CH | O | 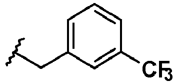 |
| 32 | 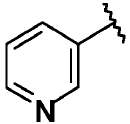 | S | CH | O | 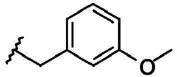 |
| 34 | 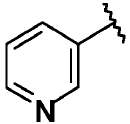 | S | CH | O | 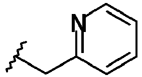 |
| 35 | 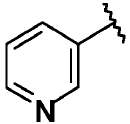 | S | CH | O | 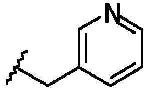 |
| 36 | 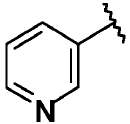 | S | CH | O | 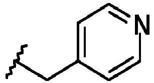 |
| 37 | 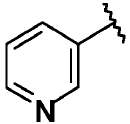 | S | CH | O | 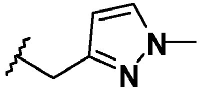 |
| 38 | 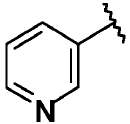 | S | CH | O | 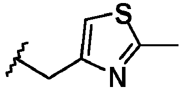 |
| 39 | 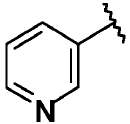 | S | CH | O | 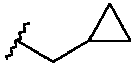 |
| 40 | 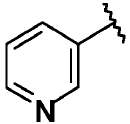 | S | CH | O | 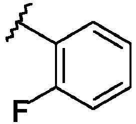 |
| 41 | 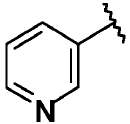 | S | CH | O | 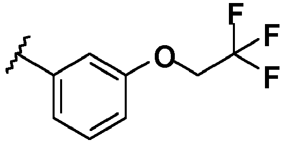 |
| 42 | 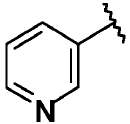 | S | CH | O | 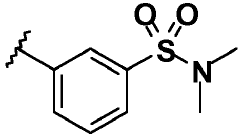 |
| 43 | 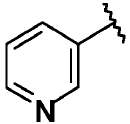 | S | CH | O | 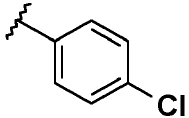 |
| 44 | 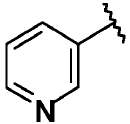 | S | CH | O | 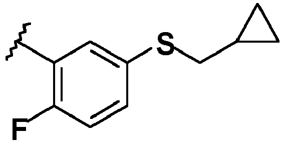 |
| 45 | 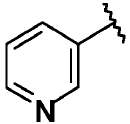 | S | CH | O | 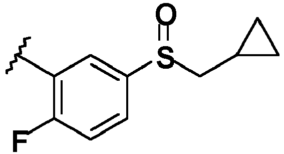 |
| 46 | 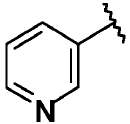 | S | CH | O | 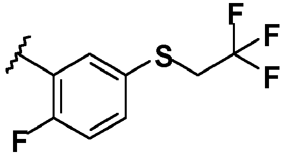 |
| 47 | 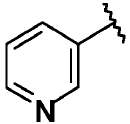 | S | CH | O | 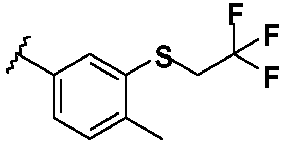 |
| 48 | 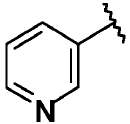 | S | CH | O | 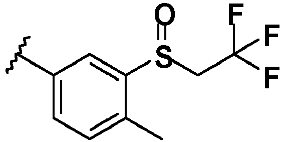 |
| 49 | 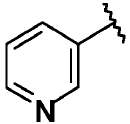 | S | CH | O | 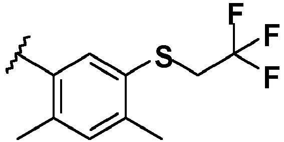 |
| 50 | 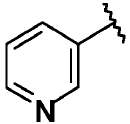 | S | CH | O | 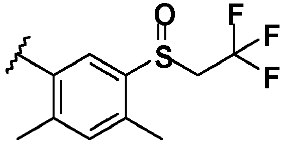 |
| 51 | 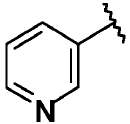 | S | CH | O | 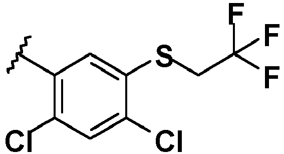 |
| 52 | 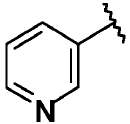 | S | CH | O | 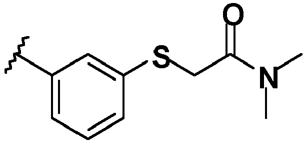 |
| 53 | 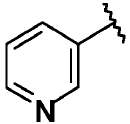 | S | CH | O | 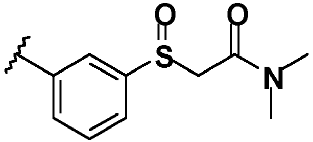 |
| 54 | 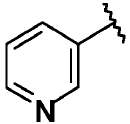 | S | CH | O | 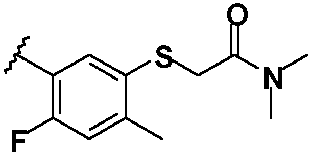 |
| 55 | 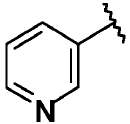 | S | CH | O | 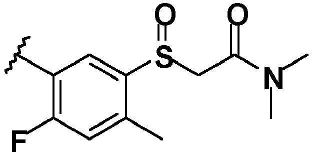 |
| 56 | 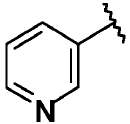 | S | C-CH3 | O | 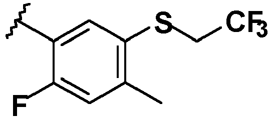 |
| 57 | 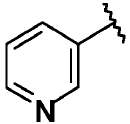 | S | C-CH3 | O | 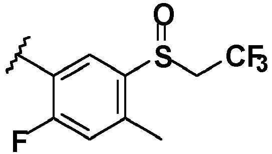 |
| 58 | 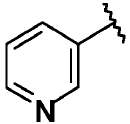 | S | N | O | 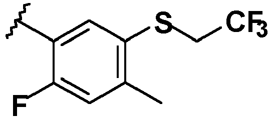 |
| 59 | 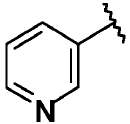 | S | N | O | 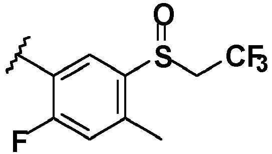 |
| 60 | 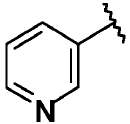 | S | CH | O | 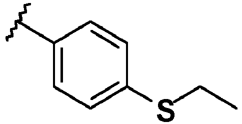 |
| 61 | 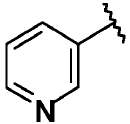 | S | CH | O | 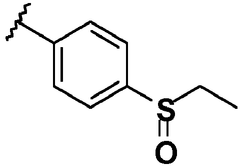 |
| 62 | 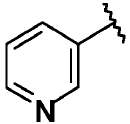 | S | CH | O | 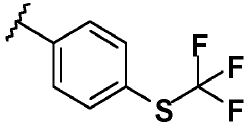 |
| 63 | 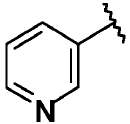 | S | CH | O | 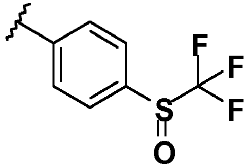 |
| 64 | 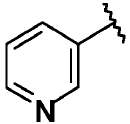 | S | CH | O | 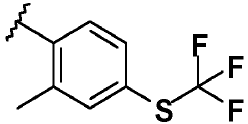 |
| 65 | 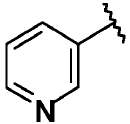 | S | CH | O | 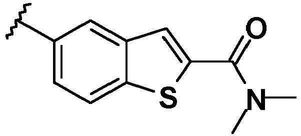 |
| 66 | 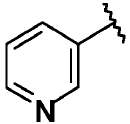 | S | CH | O | 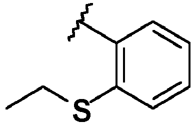 |
| 67 | 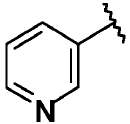 | S | CH | O | 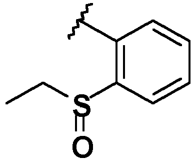 |
| 68 | 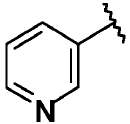 | S | CH | O | 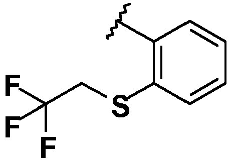 |
| 69 | 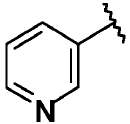 | S | CH | O | 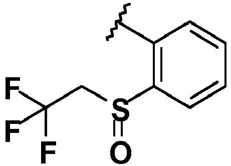 |
| 70 | 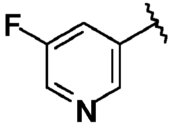 | S | CH | O | 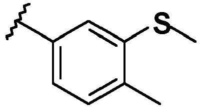 |
| 71 | 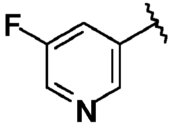 | S | CH | O | 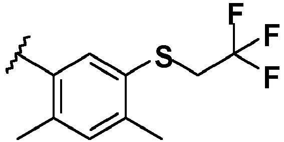 |
| 72 | 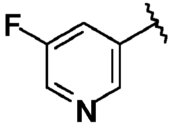 | S | CH | O | 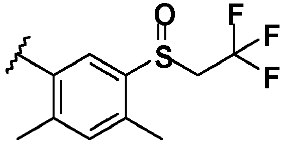 |
| 73 | 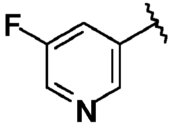 | S | CH | O | 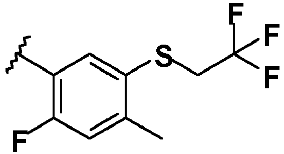 |
| 74 | 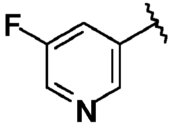 | S | CH | O | 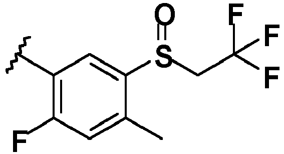 |
| 75 | 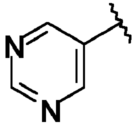 | S | CH | O | 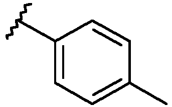 |
| 76 | 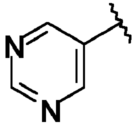 | S | CH | O | 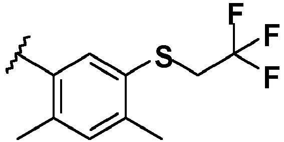 |
| 77 | 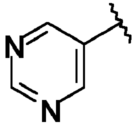 | S | CH | O | 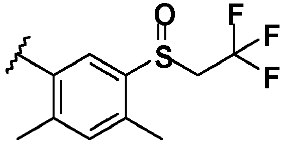 |
| 78 | 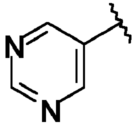 | S | CH | O | 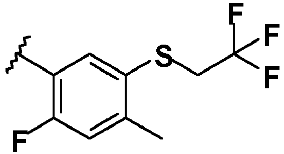 |
| 79 | 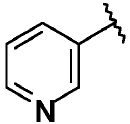 | S | CH | O | 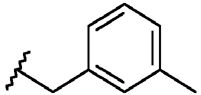 |
| 80 | 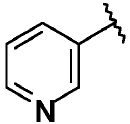 | S | C-CH3 | O | 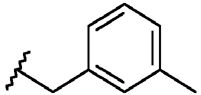 |
| 81 | 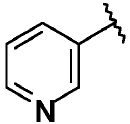 | S | CH | O | 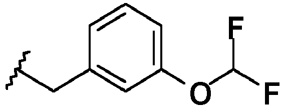 |
| 82 | 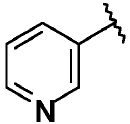 | S | CH | O | 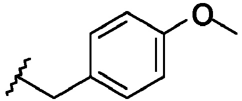 |
| 83 | 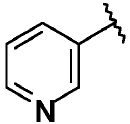 | S | CH | O | 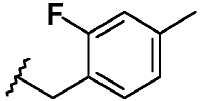 |
| 84 | 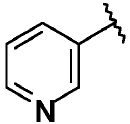 | S | CH | O | 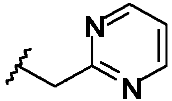 |
| 85 | 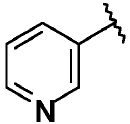 | S | CH | O | 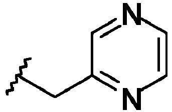 |
| 86 | 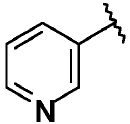 | S | CH | O | 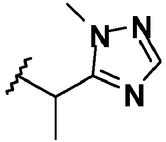 |

**Tabelle 2**

| Analytische Daten zu den angegebenen Verbindungen | | | |
|---|---|---|---|
| **Bsp.-Nr.** | **logP[a]** | **logP[b]** | **1H-NMR [σ (ppm)] bzw. LC-MS [m/z]** |
| 1 | 1,35 | 1,43 | 1H-NMR(400,0 MHz, d₆-DMSO): δ= 9,307(2,2); 9,302(2,2); 8,814(1,6); 8,811(1,7); 8,802(1,7); 8,799(1,7); 8,734(6,2); 8,516(1,0); 8,512(1,3); 8,506(1,0); 8,496(1,1); 8,491(1,4); 8,486(1,0); 8,144(3,9); 7,659(1,3); 7,647(1,3); 7,641(1,3); 7,639(1,3); 7,628(1,2); 7,627(1,2); 5,054(0,4); 5,037(1,1); 5,019(1,5); 5,002(1,1); 4,985(0,4); 3,337(1,4); 2,526(0,8); 2,513(17,3); 2,508(34,7); 2,504(45,2); 2,499(32,4); 2,495(15,5); 1,483(16,0); 1,466(15,8); 1,142(0,4); 1,126(0,4); 0,000(8,5) |

| **Bsp.-Nr.** | **logP[a]** | **logP[b]** | **1H-NMR [σ (ppm)] bzw. LC-MS [m/z]** |
|---|---|---|---|
| 2 | | 1,05 | **LC-MS: Masse gefunden** [m/z] = 270,06 |
| 3 | 1,70 | | 1H-NMR(400,0 MHz, d₆-DMSO): δ= 9,302(2,1); 9,297(2,1); 9,049(0,4); 9,044(0,4); 8,815(1,8); 8,811(1,9); 8,803(1,8); 8,799(1,8); 8,703(0,3); 8,699(0,4); 8,691(0,4); 8,687(0,4); 8,634(6,2); 8,510(1,1); 8,506(1,4); 8,500(1,1); 8,490(1,1); 8,486(1,4); 8,484(1,4); 8,480(1,1); 7,662(1,3); 7,660(1,4); 7,650(1,3); 7,648(1,3); 7,642(1,3); 7,640(1,3); 7,630(1,2); 7,628(1,3); 6,974(0,7); 3,895(4,7); 3,876(4,7); 3,323(24,1); 3,022(0,4); 3,005(0,6); 2,990(0,4); 2,512(10,6); 2,508(21,2); 2,503(27,9); 2,499(20,3); 2,495(10,0); 2,162(0,4); 2,145(0,8); 2,128(1,0); 2,111(0,9); 2,093(0,4); 0,916(16,0); 0,899(15,5); 0,873(3,2); 0,856(3,1); 0,008(0,3); 0,000(8,4); -0,008(0,3) |
| 4 | 1,19 | | 1H-NMR(400,0 MHz, d₆-DMSO): δ= 9,317(7,3); 9,313(7,0); 9,064(1,2); 9,060(1,2); 8,824(5,6); 8,820(6,0); 8,812(5,9); 8,808(5,8); 8,719(0,9); 8,716(1,0); 8,707(1,0); 8,704(0,9); 8,642(16,0); 8,527(3,3); 8,522(4,3); 8,517(3,2); 8,507(3,5); 8,502(4,6); 8,497(3,3); 8,317(0,5); 8,238(0,7); 8,233(1,1); 8,229(0,7); 8,218(1,3); 8,214(1,0); 8,208(0,8); 8,149(2,5); 7,666(4,2); 7,654(4,2); 7,646(4,1); 7,636(3,8); 7,634(3,9); 7,586(0,7); 7,574(0,7); 7,566(0,6); 7,554(0,6); 7,101(2,0); 6,573(1,0); 6,564(2,0); 6,555(0,9); 6,436(2,0); 6,427(4,2); 6,418(1,9); 6,298(1,0); 6,289(2,1); 6,280(1,0); 6,077(0,6); 5,937(0,3); 4,626(3,1); 4,617(3,4); 4,589(6,9); 4,580(6,7); 4,551(3,5); 4,542(3,3); 3,622(0,4); 3,611(0,3); 3,597(0,6); 3,583(0,7); 3,573(0,6); 3,559(0,3); 3,545(0,4); 3,334(26,6); 2,677(0,6); 2,673(0,8); 2,669(0,6); 2,526(1,7); 2,513(45,8); 2,508(92,2); 2,504(120,6); 2,500(86,8); 2,495(41,6); 2,335(0,6); 2,331(0,8); 2,326(0,6); 2,288(0,5); 0,008(0,6); 0,000(15,7);-0,009(0,6) |
| 5 | 1,54 | | 1H-NMR(400,0 MHz, d₆-DMSO): δ= 9,320(8,2); 9,315(8,2); 9,302(0,4); 8,829(6,4); 8,825(6,8); 8,817(6,9); 8,813(6,6); 8,698(16,0); 8,530(4,0); 8,526(5,1); 8,520(3,9); 8,510(4,3); 8,506(5,3); 8,505(5,3); 8,500(4,0); 8,318(0,4); 7,672(4,8); 7,671(4,8); 7,660(4,8); 7,659(4,7); 7,652(4,8); 7,651(4,6); 7,640(4,5); 7,639(4,4); 5,143(0,3); 5,121(0,4); 5,089(3,5); 5,067(11,4); 5,044(11,9); 5,021(4,0); 3,363(0,6); 3,336(289,1); 2,720(1,5); 2,678(0,6); 2,674(0,8); 2,669(0,6); 2,527(2,1); 2,513(48,7); 2,509(96,7); 2,505(125,4); 2,500(89,6); 2,496(42,7); 2,336(0,6); 2,331(0,8); 2,327(0,6); 2,077(0,4); 0,008(1,2); 0,000(30,0); -0,008(1,1) |
| 6 | 1,57 | | 1H-NMR(400,0 MHz, d₆-DMSO): δ= 9,313(4,1); 9,308 (4,1); 8,818 2,9); 8,809 (2,9); 8,806 (3,0); 8,789 (13,6); 8,521 (2,0); 8,517 (2,8); 8,511 (2,1); 8,501 (2,3); 8,496 (3,3); 8,491 (4,6); 8,477 (3,1); 7,841 (1,8); 7,837 (1,8); 7,822 (3,6); 7,818 (3,7); 7,803 (2,1); 7,798 (2,1); 7,666 (2,5); 7,654 (2,5); 7,646 (2,4); 7,634 (2,3); 7,478 (4,2); 7,458 (3,8); 7,333 (2,2); 7,321 (2,3); 7,316 (2,3); 7,304 (2,0); 5,399 (16,0); 3,904 (5,6); 3,508 (0,3); 3,477 (0,4); 3,455 (0,5); 3,354 (684,8); 3,268 (0,4); 3,175 (0,6); 3,162 (0,6); 2,678 (0,9); 2,673 (1,3); 2,669 (1,0); 2,526 (3,9); 2,513 (80,9); 2,509 (159,9); 2,504 (207,8); 2,500 (153,1); 2,496 (77,4); 2,33 (0,9); 2,331 (1,2); 2,327 (0,9); 1,234 0,5); 0,008 0,000 (16,1); -0,008 (0,6) |
| 7 | 0,65 | | 1H-NMR(400,0 MHz, d₆-DMSO): δ= 9,317(2,0); 9,312(2,1); 8,821(1,7); 8,817(1,8); 8,809(1,8); 8,805(1,7); 8,525(1,0); 8,521(1,4); 8,516(1,1); 8,501(7,3); 7,667(1,3); 7,655(1,3); 7,647(1,3); 7,635(1,2); 5,010(7,5); 3,320(48,4); 3,297(0,4); 3,104(16,0); 2,884(14,6); 2,670(0,4); 2,540(0,3); 2,506(47,9); 2,502(61,3); 2,497(44,5); 2,328(0,4); 0,008(0,8); 0,000(16,9); -0,008(0,8) |
| 8 | 1,94 | 1,94 | 1H-NMR(400,0 MHz, d₆-DMSO): δ= 9,342(3,0); 9,337(2,9); 8,832(2,2); 8,828(2,5); 8,820(2,4); 8,816(2,5); 8,612(9,7); 8,553(1,3); 8,548(1,7); 8,543(1,4); 8,533(1,4); 8,527(1,8); 8,523(1,4); 7,678(1,8); 7,666(1,7); 7,658(1,7); 7,646(1,6); 7,497(1,3); 7,477(2,7); 7,459(2,4); 7,398(3,1); 7,381(4,4); 7,362(3,0); 5,758(0,5); 3,327(46,7); 2,676(0,4); 2,671(0,6); 2,667(0,4); 2,525(1,4); 2,520(2,2); 2,511(31,8); 2,507(65,7); 2,502(87,7); 2,498(64,6); 2,493(31,9); 2,402(16,0); 2,333(0,4); 2,329(0,6); 2,325(0,4); 2,309(0,5); 1,299(0,5); 1,259(0,7); 1,233(0,9); 1,183(0,3); 0,146(0,5); 0,008(3,7); 0,000(106,2); 0,009(3,7); -0,150(0,5) |
| 9 | 2,26 | 2,23 | 1H-NMR(400,0 MHz, d₆-DMSO): δ= 9,352(4,9); 9,346(4,8); 8,836(3,7); 8,833(4,1); 8,824(4,0); 8,821(4,1); 8,707(16,0); 8,563(2,1); 8,558(2,8); 8,553(2,2); 8,543(2,3); 8,537(3,1); 8,533(2,2); 8,089(5,0); 7,953(3,6); 7,933(6,0); 7,868(3,1); 7,848(3,3); 7,829(1,4); 7,681(2,8); 7,669(2,8); 7,661(2,7); 7,650(2,5); 7,649(2,7); 4,038(0,5); 4,020(0,5); 3,328(60,0); 2,944(5,2); 2,784(4,1); 2,676(0,4); 2,672(0,5); 2,667(0,4); 2,525(1,3); 2,512(28,1); 2,507(57,8); 2,503(77,3); 2,498(57,0); 2,494(28,2); 2,334(0,4); 2,329(0,5); 2,325(0,4); 1,989(2,2); 1,957(4,3); 1,193(0,9); 1,175(1,3); 1,157(0,6); 0,008(2,3); 0,000(68,3); -0,009(2,6) |
| 10 | 1,73 | 1,76 | 1H-NMR(400,0 MHz, d₆-DMSO): δ= 9,345 5(0,5); 9,340(0,5); 8,833(0,4); 8,829(0,4); 8,821(0,4); 8,817(0,4); 8,620(1,4); 7,495(0,6); 7,475(0,4); 7,217(0,3); 7,211(0,6); 7,206(0,4); 7,155(0,4); 7,134(0,5); 3,818(3,8); 3,328(15,7); 2,944(16,0); 2,784(13,5); 2,507(16,3); 2,502(21,3); 2,498(16,0); 1,957(14,1); 0,008(1,0); 0,000(24,1); -0,008(1,1) |
| 11 | 1,92 | 1,96 | 1H-NMR(400,0 MHz, d₆-DMSO): δ= 9,341(3,2); 9,336(3,1); 8,831(2,4); 8,827(2,6); 8,819(2,5); 8,815(2,6); 8,598(10,7); 8,552(1,4); 8,547(1,9); 8,542(1,4); 8,532(1,5); 8,526(2,0); 8,522(1,4); 8,317(0,4); 7,677(1,9); 7,665(1,8); 7,657(1,8); 7,645(1,8); 7,462(4,1); 7,442(8,2); 7,399(6,4); 7,378(3,3); 5,758(0,4); 3,325(150,9); 2,675(1,1); 2,671(1,5); 2,666(1,2); 2,524(3,5); 2,510(82,2); 2,506(170,6); 2,502(229,7); 2,497(169,6); 2,493(83,7); 2,408(16,0); 2,333(1,1); 2,328(1,5); 2,324(1,1); 1,298(0,7); 1,258(1,0); 1,234(1,3); 0,000(2,4) |
| 12 | 1,73 | 1,76 | 1H-NMR(40 0,0 MHz, d₆-DMSO); δ= 8,59(0,4); 3,841(1,0); 3,328(7,1); 2,945(16,0); 2,784(13,9); 2,506(6,9); 2,502(8,5); 2,498(6,5); 1,958(14,6); 0,000(5,1) |
| 13 | 2,09 | 2,08 | 1H-NMR(400,0 MHz, d₆-DMSO): δ= 9,344(5,3); 9,340(5,4); 8,829(4,0); 8,820(4,1); 8,631(16,0); 8,554(2,6); 8,550(3,8); 8,545(2,8); 8,534(2,9); 8,529(4,0); 8,525(2,8); 8,150(2,0); 7,678(3,4); 7,666(3,5); 7,658(3,5); 7,646(3,3); 7,534(2,8); 7,515(7,2); 7,495(13,4); 7,459(0,3); 7,440(5,4); 7,420(3,2); 7,350(4,5); 7,329(3,5); 3,324(14,3); 2,859(0,4); 2,768(0,4); 2,671(0,9); 2,554(1,1); 2,529(47,8); 2,506(102,3); 2,502(131,0); 2,498(102,0); 2,476(2,9); 2,329(0,9); 1,295(0,3); 1,278(0,6); 1,187(0,4); 0,000(38,9) |
| 14 | 1,04 | 1,09 | 1H-NMR(400,0 MHz, d₆-DMSO): δ= 9,351(2,4); 9,346(2,2); 8,835(1,7); 8,832(1,8); 8,823(1,8); 8,820(1,8); 8,721(1,4); 8,707(6,9); 8,561(1,1); 8,556(1,4); 8,551(1,0); 8,541(1,2); 8,536(1,5); 8,531(1,0); 8,213(0,5); 8,208(0,3); 8,152(0,6); 7,939(1,5); 7,935(2,7); 7,907(0,4); 7,888(0,5); 7,879(0,7); 7,875(1,2); 7,871(0,8); 7,860(1,2); 7,856(2,2); 7,852(1,3); 7,828(1,2); 7,808(2,5); 7,789(1,8); 7,786(1,7); 7,782(2,4); 7,777(1,5); 7,767(0,6); 7,762(0,8); 7,758(0,4); 7,682(1,4); 7,670(1,3); 7,662(1,4); 7,650(1,3); 5,756(0,7); 3,323(10,5); 3,314(5,1); 2,847(0,7); 2,837(16,0); 2,671(0,4); 2,511(20,1); 2,507(40,7); 2,502(54,5); 2,498(41,2); 2,494(21,4); 2,329(0,3); 0,146(0,4); 0,008(3,0); 0,000(74,0); 0,008(4,1); -0,15 0(0,3) |
| 15 | 2,42 | 2,35 | 1H-NMR(601,6 MHz, d₆-DMSO): δ= 9,357(2,9); 9,354(2,9); 9,353(2,8); 8,842(2,5); 8,839(2,6); 8,834(2,6); 8,831(2,6); 8,636(10,4); 8,564(1,4); 8,561(1,9); 8,560(1,7); 8,557(1,4); 8,551(1,5); 8,548(1,8); 8,547(1,9); 8,544(1,5); 7,686(1,7); 7,685(1,7); 7,678(1,7); 7,677(1,7); 7,672(1,7); 7,671(1,7); 7,665(1,7); 7,663(1,7); 7,403(3,7); 7,400(3,9); 7,393(2,6); 7,380(2,9); 7,266(2,3); 7,263(2,2); 7,253(1,9); 7,249(1,9); 3,342(244,4); 2,955(1,3); 2,794(1,0); 2,627(0,7); 2,625(1,0); 2,622(0,7); 2,534(1,7); 2,531(2,2); 2,528(2,4); 2,519(52,0); 2,516(108,9); 2,513(148,0); 2,510(110,3); 2,507(54,0); 2,500(25,7); 2,400(0,7); 2,397(1,0); 2,394(0,7); 2,327(16,0); 2,297(0,4); 1,968(1,1) |
| 16 | 1,25 | 1,26 | 1H-NMR(400,0 MHz, d₆-DMSO): δ= 9,348(2,8); 9,343(2,8); 8,831(2,2); 8,819(2,3); 8,675(6,0); 8,554(1,7); 8,534(1,8); 7,960(3,2); 7,956(3,2); 7,679(1,7); 7,667(2,9); 7,660(2,0); 7,647(3,3); 7,541(2,8); 7,521(2,1); 3,326(55,5); 2,944(0,5); 2,773(16,0); 2,690(0,5); 2,671(0,5); 2,502(76,7); 2,436(13,1); 2,328(0,5); 2,301(0,4); 1,957(0,4); 1,234(0,6); 0,000(0,5) |
| 17 | 2,70 | 2,66 | 1H-NMR(400,0 MHz, d₆-DMSO): δ= 9,353(5,0); 9,347(5,0); 8,837(3,7); 8,833(4,2); 8,825(4,0); 8,821(4,2); 8,659(16,0); 8,563(2,1); 8,558(2,9); 8,554(2,2); 8,543(2,2); 8,538(3,1); 8,534(2,3); 8,443(0,5); 8,317(0,6); 8,187(2,6); 8,181(2,8); 8,172(2,7); 8,165(2,8); 7,993(1,6); 7,986(1,6); 7,982(1,9); 7,975(1,8); 7,971(2,1); 7,964(2,1); 7,960(2,1); 7,953(1,9); 7,773(3,5); 7,751(6,1); 7,729(2,9); 7,682(2,9); 7,669(2,8); 7,662(2,8); 7,649(2,8); 5,758(0,5); 3,326(262,3); 2,675(2,1); 2,671(3,0); 2,666(2,3); 2,524(7,8); 2,519(11,5); 2,511(161,4); 2,506(336,0); 2,502(452,0); 2,497(336,0); 2,493(167,4); 2,333(2,1); 2,329(3,0); 2,324(2,2); 1,298(0,6); 1,259(0,8); 1,234(1,2); 1,203(0,4); 1,185(0,8); 1,167(0,4); 1,148(0,3); 0,146(2,7); 0,025(0,6); 0,008(19,5); 0,000(584,9); -0,009(21,7); - 0,150(2,7) |
| 18 | 2,01 | 1,95 | 1H-NMR(400,0 MHz, d₆-DMSO): δ= 9,347(0,3); 8,927(7,1); 8,670(16,0); 8,659(1,1); 8,461(3,7); 8,459(3,6); 8,445(3,9); 8,443(3,8); 8,317(0,5); 8,182(3,0); 8,175(3,3); 8,166(3,2); 8,160(3,1); 8,083(3,7); 8,063(4,1); 7,987(1,8); 7,980(1,9); 7,976(2,1); 7,969(2,0); 7,965(2,4); 7,958(2,4); 7,954(2,4); 7,947(1,9); 7,773(3,7); 7,751(6,6); 7,730(3,1); 7,665(3,7); 7,648(4,1); 7,645(4,1); 7,628(3,2); 5,758(0,4); 3,325(270,0); 2,675(2,0); 2,671(2,7); 2,667(2,1); 2,574(0,4); 2,506(313,6); 2,502(411,0); 2,498(314,6); 2,333(1,9); 2,329(2,7); 2,324(2,0); 1,297(0,3); 1,259(0,4); 1,235(1,5); 1,196(0,4); 1,178(0,7); 1,160(0,4); 0,146(1,4); 0,008(11,9); 0,000(302,6); -0,150(1,5) |
| 19 | 2,39 | 2,43 | 1H-NMR(400,0 MHz, d₆-DMSO): δ= 9,343(3,7); 9,338(3,7); 8,832(2,7); 8,828(3,0); 8,820(2,9); 8,816(3,1); 8,633(11,2); 8,554(1,5); 8,549(2,2); 8,544(1,7); 8,534(1,7); 8,528(2,3); 8,524(1,7); 8,316(0,7); 7,677(2,1); 7,665(2,1); 7,657(2,1); 7,645(2,0); 7,545(2,7); 7,540(5,9); 7,519(4,3); 7,500(3,6); 7,468(3,5); 7,448(1,7); 7,373(2,6); 7,357(1,6); 7,353(2,0); 3,324(30,1); 3,083(2,3); 3,065(7,5); 3,046(7,6); 3,028(2,5); 2,945(1,3); 2,785(1,1); 2,671(0,4); 2,525(1,0); 2,507(46,5); 2,502(62,8); 2,498(47,8); 2,329(0,4); 1,957(1,1); 1,302(7,8); 1,284(16,0); 1,266(7,5); 0,008(2,3); 0,000(63,4); -0,008(3,2) |
| 20 | 1,24 | 1,26 | 1H-NMR(400,0 MHz, d₆-DMSO): δ= 9,356(4,7); 9,351(4,7); 8,841(3,4); 8,838(3,6); 8,829(3,6); 8,826(3,6); 8,735(0,5); 8,712(12,7); 8,566(2,0); 8,562(2,8); 8,557(2,0); 8,546(2,2); 8,541(2,9); 8,537(2,0); 8,164(2,1); 7,889(5,6); 7,823(5,6); 7,821(5,6); 7,814(4,9); 7,809(7,5); 7,797(1,3); 7,787(3,5); 7,782(2,9); 7,778(2,2); 7,772(2,6); 7,764(1,0); 7,759(0,9); 7,687(2,6); 7,675(2,6); 7,667(2,6); 7,655(2,5); 3,407(0,5); 3,388(0,7); 3,332(3,2); 3,172(0,5); 3,154(1,8); 3,136(2,2); 3,120(2,5); 3,102(2,2); 3,084(0,7); 2,901(0,6); 2,883(2,2); 2,864(2,5); 2,849(2,1); 2,830(1,8); 2,812(0,5); 2,679(0,4); 2,674(0,3); 2,514(50,7); 2,510(65,6); 2,506(49,5); 2,341(0,3); 2,337(0,4); 1,169(0,6); 1,106(7,5); 1,088(16,0); 1,070(7,3); 1,058(0,8) |
| 21 | 2,95 | 2,91 | 1H-NMR(400,0 MHz, d₆-DMSO): δ= 9,350(3,0); 9,346(3,1); 9,344(3,0); 8,839(2,4); 8,836(2,6); 8,827(2,6); 8,824(2,6); 8,679(8,7); 8,561(1,4); 8,557(1,8); 8,551(1,4); 8,541(1,5); 8,537(1,9); 8,535(1,9); 8,531(1,5); 7,939(3,1); 7,921(3,1); 7,684(1,8); 7,683(1,7); 7,672(1,7); 7,671(1,7); 7,664(1,8); 7,663(1,7); 7,652(1,7); 7,651(1,7); 7,518(2,7); 7,491(2,6); 4,062(1,2); 4,037(3,9); 4,011(4,0); 3,985(1,4); 3,330(57,6); 2,945(1,0); 2,785(0,7); 2,672(0,4); 2,526(1,0); 2,512(19,8); 2,508(40,6); 2,503(53,8); 2,499(39,4); 2,494(19,5); 2,471(16,0); 2,330(0,3); 2,076(10,9); 1,958(0,8); 0,008(1,0); 0,000(30,1); -0,009(1,2) |
| 22 | 2,01 | 1,98 | 1H-NMR(400,0 MHz, d₆-DMSO): δ= 9,761(0,5); 9,353(6,6); 9,347(6,9); 9,102(0,4); 8,836(5,2); 8,828(4,9); 8,824(5,2); 8,738(0,5); 8,723(15,6); 8,557(4,0); 8,553(2,9); 8,543(2,8); 8,537(4,3); 8,533(3,1); 8,315(2,5); 8,195(6,5); 8,176(6,5); 8,039(0,4); 7,951(2,4); 7,685(3,8); 7,673(3,8); 7,665(4,0); 7,653(3,8); 7,635(5,0); 7,608(5,1); 7,366(0,4); 7,238(0,6); 5,755(1,0); 4,293(0,9); 4,271(1,2); 4,263(1,3); 4,233(1,1); 4,182(0,4); 4,124(1,0); 4,100(1,2); 3,505(1,6); 3,322(748,9); 2,944(1,9); 2,891(16,0); 2,784(1,6); 2,731(14,3); 2,716(0,5); 2,670(6,0); 2,666(4,8); 2,505(695,9); 2,501(960,0); 2,497(767,5); 2,395(0,8); 2,373(2,4); 2,328(6,2); 2,324(5,1); 2,283(0,4); 1,957(1,9); 1,303(0,3); 1,284(0,6); 1,233(2,0); 0,854(0,4); 0,146(3,0); 0,008(21,8); 0,000(638,0); -0,078(0,4);-0,150(3,2) |
| 23 | 1,49 | 1,45 | 1H-NMR(400,0 MHz, d₆-DMSO): δ= 8,928(9,2); 8,734(16,0); 8,463(5,2); 8,446(5,3); 8,315(0,9); 8,196(7,0); 8,177(7,1); 8,082(5,1); 8,061(5,7); 7,668(4,5); 7,649(5,9); 7,633(8,7); 7,608(5,6); 7,573(0,4); 7,251(0,4); 4,265(1,6); 4,236(1,3); 4,117(1,2); 4,106(1,2); 4,092(1,4); 3,322(182,5); 2,671(2,1); 2,502(339,3); 2,498(298,4); 2,402(0,7); 2,373(1,6); 2,329(2,4); 0,146(1,6); 0,00(312,8);-0,083(0,4); 0,150(1,7) |
| 24 | 2,76 | 2,70 | 1H-NMR(400,0 MHz, d₆-DMSO); δ= 9,345(0,4); 9,340(0,4); 8,640(1,0); 7,597(0,5); 7,539(0,3); 7,520(0,4); 7,512(0,4); 3,327(6,0); 2,945(16,0); 2,785(13,5); 2,507(6,8); 2,503(9,0); 2,498(6,9); 1,989(0,6); 1,958(14,1); 1,305(2,7); 1,289(2,7); 0,000(8,3); -0,008(0,4) |
| 25 | 1,39 | 1,43 | 1H-NMR(400,0 MHz, d₆-DMSO): δ= 9,351(4,6); 9,347(4,5); 8,835(3,5); 8,832(3,8); 8,823(3,7); 8,820(3,8); 8,727(0,5); 8,701(15,5); 8,562(2,1); 8,558(2,7); 8,556(2,6); 8,552(2,1); 8,542(2,3); 8,538(2,7); 8,536(2,9); 8,532(2,1); 8,317(0,3); 7,858(3,3); 7,853(5,8); 7,828(0,7); 7,821(0,4); 7,810(2,1); 7,791(7,7); 7,788(7,6); 7,785(6,6); 7,783(5,9); 7,777(5,1); 7,772(3,1); 7,765(1,1); 7,759(1,4); 7,754(1,0); 7,682(2,6); 7,680(2,6); 7,670(2,5); 7,668(2,6); 7,662(2,5); 7,660(2,5); 7,650(2,4); 7,648(2,5); 5,757(16,0); 3,325(116,5); 3,085(0,8); 3,068(2,2); 3,051(3,1); 3,034(2,3); 3,016(0,9); 2,680(0,4); 2,676(0,9); 2,671(1,3); 2,667(0,9); 2,524(3,1); 2,511(71,5); 2,507(145,1); 2,502(193,0); 2,498(144,3); 2,493(72,7); 2,425(0,5); 2,333(1,0); 2,329(1,3); 2,324(1,0); 1,259(0,6); 1,241(15,6); 1,224(15,5); 1,205(1,6); 1,186(1,2); 1,172(0,6); 1,169(0,5); 0,996(15,2); 0,979(15,1); 0,965(0,8); 0,853(0,3); 0,000(1,5) |
| 26 | 3,18 | 3,08 | 1H-NMR(400,0 MHz, d₆-DMSO): δ= 9,345(1,9); 9,340(2,0); 8,833(1,4); 8,829(1,6); 8,821(1,5); 8,817(1,6); 8,625(6,3); 8,556(0,8); 8,551(1,2); 8,546(0,9); 8,536(0,9); 8,530(1,2); 8,526(0,9); 7,679(1,1); 7,667(1,1); 7,660(1,1); 7,646(1,0); 7,550(1,4); 7,546(2,6); 7,542(1,7); 7,525(0,7); 7,505(2,0); 7,486(2,0); 7,477(1,3); 7,473(2,2); 7,469(1,4); 7,457(0,6); 7,453(0,8); 7,364(1,0); 7,360(1,5); 7,355(1,0); 7,345(0,9); 7,340(1,1); 7,337(0,8); 3,329(56,9); 2,941(4,9); 2,924(5,0); 2,676(0,4); 2,671(0,5); 2,667(0,4); 2,524(1,7); 2,511(32,1); 2,507(63,8); 2,502(83,8); 2,498(62,6); 2,333(0,4); 2,329(0,5); 2,324(0,4); 1,895(0,4); 1,878(0,9); 1,862(1,1); 1,845(0,9); 1,828(0,5); 1,234(0,5); 1,020(16,0); 1;003(15,4); 0,000(8,1) |
| 27 | 1,73 | 1,68 | 1H-NMR(400,0 MHz, d₆-DMSO): δ= 9,351(5,0); 9,346(5,1); 8,835(3,7); 8,831(4,0); 8,823(4,0); 8,819(3,9); 8,724(0,3); 8,706(14,0); 8,561(2,1); 8,557(2,9); 8,552(2,2); 8,541(2,3); 8,536(3,1); 8,532(2,2); 7,924(6,3); 7,860(2,3); 7,857(1,6); 7,846(3,0); 7,842(5,0); 7,838(3,2); 7,824(2,8); 7,805(5,5); 7,786(4,3); 7,784(4,2); 7,779(5,4); 7,774(3,3); 7,764(1,3); 7,760(1,8); 7,755(1,0); 7,681(2,8); 7,669(2,8); 7,661(2,8); 7,649(2,6); 5,757(4,4); 3,332(67,3); 2,853(0,6); 2,820(4,9); 2,814(4,8); 2,800(8,5); 2,782(0,7); 2,766(0,3); 2,672(0,7); 2,507(77,9); 2,503(99,5); 2,498(76,3); 2,330(0,7); 2,325(0,5); 2,160(0,9); 2,143(1,5); 2,126(1,8); 2,108(1,5); 2,091(0,9); 2,075(0,3); 1,233(1,0); 1,192(0,4); 1,130(15,9); 1,113(15,7); 1,094(0,8); 1,034(15,8); 1,017(16,0); 1,004(1,2); 0,995(0,9); 0,977(0,3); 0,000(0,8) |
| 28 | 2,47 | 2,43 | 1H-NMR(600,1 MHz, CD3CN): δ= 9,2734(3,7); 9,2728(3,7); 9,270(3,8); 8,751(2,8); 8,748(2,9); 8,743(2,9); 8,740(2,8); 8,469(9,8); 8,403(1,8); 8,400(2,4); 8,399(2,3); 8,396(1,7); 8,389(1,9); 8,387(2,4); 8,386(2,3); 8,383(1,7); 7,744(3,9); 7,662(2,1); 7,650(3,1); 7,641(2,5); 7,576(2,2); 7,563(3,2); 7,550(1,3); 7,534(2,3); 7,533(2,2); 7,526(2,3); 7,525(2,2); 7,521(2,3); 7,520(2,1); 7,513(2,2); 7,512(2,0); 5,293(16,0); 2,957(13,9); 2,827(11,1); 2,154(11,6); 1,972(12,0); 1,967(0,5); 1,959(0,6); 1,955(0,8); 1,951(5,6); 1,947(10,0); 1,943(14,5); 1,938(9,7); 1,934(4,8); 0,000(6,8) |
| 32 | 1,91 | 1,92 | 1H-NMR(400,0 MHz, d₆-DMSO): δ= 9,303(2,1); 9,297(2,1); 8,859(5,1); 8,814(1,4); 8,810(1,7); 8,802(1,5); 8,798(1,6); 8,513(0,8); 8,508(1,2); 8,503(0,9); 8,493(0,9); 8,487(1,3); 8,483(0,9); 7,658(1,2); 7,646(1,2); 7,638(1,1); 7,626(1,1); 7,300(1,2); 7,280(2,2); 7,260(1,4); 6,987(2,0); 6,943(1,5); 6,924(1,3); 6,899(1,2); 6,894(1,1); 6,879(1,0); 6,873(1,0); 5,241(6,2); 3,744(16,0); 3,332(39,1); 2,945(0,5); 2,785(0,5); 2,673(0,4); 2,668(0,4); 2,508(50,3); 2,504(67,2); 2,499(52,6); 2,330(0,4); 1,958(0,5); 1,233(0,5); 0,008(1,6); 0,000(42,7) |
| 34 | 1,09 | | 1H-NMR(400,0 MHz, d₆-DMSO): δ= 9,324 (8,2); 9,320 (8,0); 8,828 (5,9); 8,819 (5,7); 8,652 (16,0); 8,533 (4,1); 8,528 (5,4); 8,523 (3,9); 8,513 (4,3); 8,508 (5,6); 8,503 (3,8); 7,673 (4,7); 7,661 (4,8); 7,653 (4,6); 7,641 (4,2); 6,830 (1,0); 6,818 (1,8); 6,805 (0,9); 6,700 (2,0); 6,688 (3,9); 6,676 (2,0); 6,570 (1,0); 6,558 (2,0); 6,546 (1,0); 4,914 (5,2); 4,875 (10,9); 4,837 (5,5); 3,905 (7,6); 3,506 (0,4); 3,338 (626,8); 3,225 (1,1); 3,062 (0,9); 2,677 (2,1); 2,672 (2,7); 2,668 (2,1); 2,508 (339,6); 2,503 (425,1); 2,499 (319,3); 2,334 (1,9); 2,330 (2,5); 2,325 (1,9); 1,298 (0,4); 1,258 (0,5); 1,233 (0,7); 1,145 (0,7); 0,000 (37,7) |
| 35 | | 0,72 | **LC-MS: Masse gefunden** [m/z] = 321,07 |
| 36 | 0,55 | | 1H-NMR(400,0 MHz, d₆-DMSO): δ= 9,319 (4,3); 9,314 (4,3); 8,846 (14,5); 8,822 (3,1); 8,813 (3,3); 8,554 (4,4); 8,542 (4,4); 8,527 (2,8); 8,522 (3,4); 8,517 (2,5); 8,507 (2,8); 8,501 (3,4); 8,497 (2,4); 8,489 (0,5); 8,364 (0,4); 8,357 (0,4); 7,669 (2,7); 7,657 (2,8); 7,649 (2,9); 7,637 (2,6); 7,331 (6,3); 7,317 (6,1); 5,316 (16,0); 3,905 (6,2); 3,508 (0,4); 3,469 (0,4); 3,349 (770,9); 2,678 (1,1); 2,673 (1,5); 2,669 (1,1); 2,526 (4,9); 2,513 (93,8); 2,508 (184,1); 2,504 (238,5); 2,500 (175,4); 2,495 (88,2); 2,335 (1,1); 2,331 (1,4); 2,326 (1,1); 1,234 (0,6); 0,008 (0,7); 0,000 (21,0); -0,008 (0,8) |
| 37 | 0,97 | 1,08 | 1H-NMR(400,0 MHz, d₆-DMSO): δ= 9,295(1,6); 9,290(1,6); 8,809(6,3); 8,797(1,4); 8,793(1,4); 8,746(0,3); 8,743(0,3); 8,503(0,8); 8,498(1,1); 8,493(0,9); 8,483(0,9); 8,478(1,2); 8,473(0,9); 8,350(0,3); 7,772(3,5); 7,652(1,1); 7,640(1,1); 7,632(1,1); 7,622(1,3); 7,603(0,3); 7,595(0,4); 7,583(0,3); 7,563(0,6); 7,554(0,3); 7,506(3,6); 7,480(0,5); 7,460(0,5); 7,363(0,8); 7,325(0,5); 7,118(0,4); 7,008(0,3); 5,757(1,6); 5,097(6,4); 4,268(0,7); 4,254(0,7); 4,205(0,4); 4,191(0,4); 3,950(0,3); 3,788(4,5); 3,782(16,0); 3,750(0,6); 3,703(0,3); 3,394(0,4); 3,328(25,5); 3,264(0,5); 2,676(0,4); 2,672(0,5); 2,667(0,4); 2,542(0,4); 2,525(1,2); 2,512(29,4); 2,507(60,8); 2,503(81,9); 2,498(62,0); 2,494(32,1); 2,334(0,4); 2,330(0,6); 2,325(0,4); 2,287(1,3); 1,318(0,3); 1,298(1,0); 1,278(0,4); 1,259(1,3); 1,233(1,3); 1,212(0,4); 1,196(0,3); 0,008(2,4); 0,000(75,0); 0,008(3,4);-0,150(0,3) |
| 38 | 1,36 | 1,42 | 1H-NMR(400,0 MHz, d₆-DMSO): δ= 9,305(2,2); 9,301(2,3); 8,815(1,6); 8,812(1,9); 8,798(6,3); 8,511(1,3); 8,506(1,0); 8,495(1,0); 8,490(1,4); 8,486(1,1); 7,661(1,3); 7,648(1,3); 7,641(1,3); 7,628(1,2); 7,454(4,1); 7,277(0,5); 5,759(0,4); 5,308(7,1); 4,489(0,4); 4,476(0,4); 3,332(61,6); 2,945(0,9); 2,784(0,8); 2,672(0,9); 2,655(1,3); 2,637(2,1); 2,611(16,0); 2,507(95,2); 2,503(124,7); 2,499(102,9); 2,446(0,4); 2,330(0,8); 2,288(0,7); 1,958(0,8); 1,313(0,5); 0,146(0,4); 0,000(71,2);-0,150(0,4) |
| 39 | 1,53 | 1,52 | 1H-NMR(601,6 MHz, CDCl₃): δ= 9,341(5,1); 9,338(5,0); 8,781(3,5); 8,775(3,6); 8,426(2,9); 8,423(3,9); 8,420(2,8); 8,413(3,1); 8,410(4,0); 8,407(2,8); 8,299(16,0); 7,484(3,2); 7,476(3,3); 7,471(3,3); 7,463(3,1); 7,432(0,6); 7,261(86,9); 7,084(0,5); 5,299(2,5); 3,958(15,5); 3,946(15,6); 3,278(0,4); 3,269(0,5); 3,266(0,4); 3,257(0,4); 2,232(0,4); 2,220(0,6); 2,207(0,4); 1,637(0,4); 1,558(13,5); 1,445(0,4); 1,368(0,5); 1,360(0,9); 1,355(1,3); 1,352(0,9); 1,347(2,2); 1,342(2,1); 1,339(1,8); 1,334(3,5); 1,329(2,0); 1,326(2,4); 1,321(2,7); 1,313(2,0); 1,309(1,8); 1,301(1,4); 1,255(7,8); 0,892(1,0); 0,881(1,8); 0,869(1,1); 0,856(0,4); 0,841(0,4); 0,715(2,2); 0,707(6,7); 0,705(7,1); 0,697(3,5); 0,694(7,0); 0,692(6,7); 0,684(2,5); 0,577(0,4); 0,575(0,4); 0,564(0,4); 0,562(0,4); 0,491(2,4); 0,483(8,8); 0,473(8,4); 0,465(2,0); 0,276(0,4); 0,267(0,5); 0,097(0,4); 0,005(2,9); 0,000(86,6); 0,006(3,2); -0,100(0,4) |
| 40 | 1,70 | 1,69 | 1H-NMR(400,0 MHz, d6-DMSO): δ= 9,347(5,5); 9,343(5,8); 8,838(4,1); 8,835(4,6); 8,826(4,5); 8,823(4,6); 8,709(16,0); 8,692(0,3); 8,558(3,0) ;8,554(4,0); 8,553(3,9); 8,549(3,2); 8,538(3,3); 8,534(4,1); 8,533(4,3); 8,528(3,4); 8,319(0,3); 8,236(1,6); 7,749(2,1); 7,745(2,5); 7,730(4,4); 7,726(5,0); 7,711(2,6); 7,706(2,9); 7,683(4,8); 7,678(1,9); 7,670(4,7); 7,664(6,0); 7,650(5,7); 7,644(3,1); 7,639(2,1); 7,630(1,9); 7,626(1,7); 7,556(3,1); 7,554(3,4); 7,532(4,4); 7,528(3,8); 7,510(2,4); 7,507(2,4) ;7,481(0,3); 7,470(3,3); 7,468(3,3); 7,451(5,2); 7,449(5,3); 7,432(2,5); 7,429(2,5); 5,758(1,7); 4,354(0,3); 4,336(0,4); 4,252(0,6); 4,234(0,6); 3,364(1,0); 2,678(0,5); 2,673(0,7); 2,669(0,5); 2,526(1,6); 2,522(2,4); 2,513(34,4); 2,508(71,9); 2,504(96,4); 2,499(71,1); 2,495(35,5); 2,335(0,6); 2,331(0,8); 2,326(0,6); 2,287(0,4); 2,077(2,4); 1,342(0,5); 1,335(0,6); 1,318(1,0); 1,300(0,8); 1,296(1,0); 1,278(1,5); 1,260(0,9); 1,232(0,7); 0,146(0,9); 0,008(7,0); 0,000(192,4); -0,009(8,3); -0,150(0,9) |
| 41 | 2,33 | 2,34 | 1H-NMR(400,0 MHz, d6-DMSO): δ = 9,344(5,7); 9,339(6,0); 8,833(4,1); 8,829(4,6); 8,821(4,4); 8,817(4,6); 8,627(16,0); 8,554(2,3); 8,549(3,4); 8,544(2,5); 8,534(2,5); 8,529(3,6); 8,524(2,5); 7,678(3,2); 7,666(3,2); 7,658(3,2); 7,646(3,0); 7,576(3,2); 7,556(6,7); 7,535(4,2); 7,372(3,8); 7,366(6,9); 7,361(4,9); 7,283(4,3); 7,278(4,5); 7,275(4,6); 7,266(4,6); 7,261(3,9); 7,254(3,7); 7,248(3,1); 5,754(0,8); 4,878(2,5); 4,856(8,1); 4,834(8,5); 4,812(2,9); 4,738(0,4); 3,319(61,3); 2,676(0,6); 2,671(0,8); 2,667(0,7); 2,506(96,9); 2,502(128,4); 2,498(98,2); 2,333(0,6); 2,329(0,8); 2,324(0,6); 1,234(1,8); 1,187(0,5); 0,008(0,6); 0,000(16,4) |
| 42 | 1,63 | 1,72 | 1H-NMR(400,0 MHz, d6-DMSO): δ = 9,350(1,3); 9,345(1,3); 8,835(0,9); 8,831(1,0); 8,823(1,0); 8,819(1,0); 8,714(3,3); 8,560(0,5); 8,556(0,7); 8,551(0,5); 8,540(0,6); 8,535(0,8); 8,531(0,5); 8,055(1,6); 7,967(0,6); 7,962(0,4); 7,948(1,0); 7,944(0,7); 7,929(0,5); 7,925(0,4); 7,909(1,4); 7,894(1,3); 7,875(1,1); 7,856(0,4); 7,680(0,7); 7,668(0,7); 7,660(0,7); 7,648(0,7); 3,320(7,9); 2,681(16,0); 2,507(13,0); 2,502(16,4); 2,498(12,5); 0,000(16,1) |
| 43 | 2,05 | 2,06 | **1H-NMR(400,0 MHz, d6-DMSO):** δ = 9,343(2,6); 8,829(1,8); 8,820(1,9); 8,635(3,7); 8,549(1,5); 8,530(1,6); 7,666(6,6); 7,647(6,5); 7,625(2,2); 7,459(0,4); 7,412(0,5); 7,390(0,6); 7,364(0,7); 7,333(0,9); 7,267(0,4); 7,245(0,3); 7,181(1,3); 7,115(0,4); 3,318(83,2); 2,671(2,4); 2,501(395,6); 2,327(3,0); 2,286(1,2); 2,180(0,5); 1,988(0,4); 1,386(2,2); 1,335(12,4); 1,298(9,2); 1,249(16,0); 1,235(16,0); 1,188(3,6); 1,105(1,7); 0,852(4,1); 0,834(3,9); 0,146(0,4); 0,000(59,8); -0,151(0,4) |
| 44 | 2,91 | 3,00 | **1H-NMR(400,0 MHz, d6-DMSO):** δ = 9,345(7,4); 9,340(7,7); 8,836(5,3); 8,834(5,7); 8,825(5,5); 8,822(5,6); 8,700(16,0); 8,550(4,4); 8,534(3,2); 8,530(4,5); 7,753(4,1); 7,747(4,7); 7,736(4,3); 7,730(4,5); 7,681(4,1); 7,669(4,1); 7,661(4,1); 7,649(3,9); 7,601(1,9); 7,595(2,1); 7,589(2,3); 7,579(3,1); 7,573(3,1); 7,568(3,1); 7,562(2,6); 7,490(4,6); 7,467(6,6); 7,444(3,2); 3,312(33,5); 2,988(15,3); 2,970(15,6); 2,672(0,7); 2,502(104,5); 2,329(0,6); 2,074(2,1); 1,234(0,4); 1,085(0,6); 1,078(1,0); 1,066(1,9); 1,059(1,8); 1,047(2,9); 1,035(1,9); 1,029(2,1); 1,016(1,1); 1,010(0,7); 0,998(0,3); 0,567(2,3); 0,556(7,5); 0,552(8,2); 0,536(7,6); 0,532(7,5); 0,522(2,6); 0,282(2,7); 0,269(9,5); 0,256(9,5); 0,245(2,2); 0,000(8,6) |
| 45 | 1,63 | 1,66 | **1H-NMR(400,0 MHz, d6-DMSO):** δ = 9,354(9,8); 9,353(9,9); 9,349(9,7); 8,841(7,9); 8,837(8,0); 8,829(8,1); 8,825(7,7); 8,759(8,5); 8,753(16,0); 8,564(4,8); 8,560(6,0); 8,554(4,1); 8,544(5,1); 8,540(6,0); 8,538(5,7); 8,534(3,9); 8,369(1,6); 8,363(1,7); 8,352(1,6); 8,346(1,6); 8,318(1,2); 8,185(0,9); 8,179(0,9); 8,173(1,1); 8,167(1,1); 8,163(1,1); 8,157(1,1); 8,152(1,1); 8,146(0,9); 8,087(4,6); 8,082(5,1); 8,070(4,8); 8,065(4,8); 7,965(2,7); 7,960(2,7); 7,954(3,4); 7,948(3,1); 7,943(3,4); 7,938(3,3); 7,932(3,3); 7,927(2,7); 7,872(1,0); 7,869(1,3); 7,862(1,7); 7,851(0,7); 7,840(2,5); 7,817(1,4); 7,785(5,2); 7,762(7,2); 7,740(4,3); 7,687(6,0); 7,675(5,9); 7,667(5,8); 7,655(5,5); 7,589(0,4); 7,571(0,6); 5,758(8,2); 5,599(0,5); 4,153(0,4); 4,142(0,5); 4,129(0,4); 3,852(0,4); 3,601(0,5); 3,589(0,5); 3,577(0,4); 3,519(1,8); 3,506(0,8); 3,378(4,0); 3,360(4,6); 3,329(329,8); 3,279(0,4); 3,273(0,5); 2,977(0,4); 2,927(7,4); 2,909(7,4); 2,891(3,0); 2,844(0,4); 2,820(8,6); 2,748(0,5); 2,732(1,9); 2,676(2,3); 2,672(3,1); 2,667(2,4); 2,650(0,3); 2,621(0,4); 2,595(2,0); 2,584(0,8); 2,525(12,9); 2,511(184,2); 2,507(356,1); 2,503(459,1); 2,498(337,4); 2,494(171,5); 2,334(2,2); 2,329(2,9); 2,325(2,2); 2,087(6,4); 1,492(0,4); 1,474(0,5); 1,454(0,4); 1,291(0,4); 1,274(0,6); 1,259(0,9); 1,234(2,5); 1,187(0,4); 0,996(0,9); 0,984(1,8); 0,977(1,8); 0,965(2,6); 0,953(1,9); 0,946(2,0); 0,934(1,4); 0,921(0,9); 0,915(1,0); 0,902(1,1); 0,889(0,8); 0,883(0,9); 0,870(0,8); 0,862(1,0); 0,852(0,9); 0,844(1,8); 0,825(1,8); 0,806(0,7); 0,608(0,5); 0,585(2,5); 0,574(8,6); 0,565(4,7); 0,554(8,4); 0,545(2,8); 0,531(0,7); 0,522(1,0); 0,504(2,6); 0,484(2,4); 0,342(1,8); 0,336(1,9); 0,322(4,0); 0,311(3,7); 0,296(0,9); 0,284(0,9); 0,270(3,7); 0,258(4,0); 0,245(2,0); 0,238(1,7); 0,227(0,8); 0,146(5,9); 0,137(2,5); 0,053(0,3); 0,008(34,5); 0,000(766,5);-0,008(43,1); -0,064(0,6); -0,079(0,4); -0,099(0,3);-0,150(3,6) |
| 46 | 2,60 | 2,56 | **1H-NMR(400,0 MHz, d6-DMSO):** δ = 9,351(5,7); 9,347(5,7); 8,841(4,5); 8,837(5,0); 8,829(4,8); 8,825(4,9); 8,710(16,0); 8,563(2,6); 8,558(3,5); 8,553(2,7); 8,543(2,9); 8,537(3,6); 8,533(2,7); 8,317(1,0); 7,948(3,6); 7,942(4,0); 7,931(3,7); 7,925(3,8); 7,791(1,9); 7,785(2,0); 7,780(2,2); 7,774(2,1); 7,770(2,3); 7,763(2,4); 7,758(2,4); 7,752(2,0); 7,685(3,4); 7,672(3,3); 7,666(3,3); 7,654(3,1); 7,652(3,1); 7,571(3,9); 7,548(5,4); 7,525(3,2); 4,139(2,5); 4,114(7,7); 4,088(8,1); 4,062(2,7); 3,330(493,3); 3,288(0,4); 2,676(2,0); 2,671(2,7); 2,667(2,0); 2,525(7,4); 2,511(147,5); 2,507(299,9); 2,502(398,3); 2,498(297,0); 2,494(151,2); 2,457(1,0); 2,437(0,6); 2,334(2,0); 2,329(2,7); 2,325(2,0); 2,075(3,9); 0,146(0,9); 0,008(6,8); 0,000(200,5); -0,008(8,5); -0,150(0,9) |
| 47 | 2,73 | 2,75 | **1H-NMR(400,0 MHz, d6-DMSO):** δ = 10,481(0,3); 9,349(3,0); 9,344(3,0); 8,834(2,4); 8,830(2,6); 8,822(2,5); 8,818(2,6); 8,612(10,2); 8,559(1,4); 8,554(1,8); 8,549(1,4); 8,539(1,5); 8,534(2,0); 8,529(1,5); 8,435(0,6); 7,787(0,4); 7,769(3,7); 7,764(3,7); 7,679(1,8); 7,667(1,8); 7,659(1,8); 7,647(1,7); 7,472(2,0); 7,451(3,9); 7,415(2,9); 7,410(2,8); 7,395(1,5); 7,390(1,5); 5,757(0,9); 4,118(1,2); 4,092(3,8); 4,066(4,0); 4,040(1,4); 3,880(0,4); 3,865(0,4); 3,854(0,4); 3,848(0,4); 3,328(110,4); 2,676(0,7); 2,671(0,9); 2,667(0,7); 2,525(2,5); 2,511(52,6); 2,507(105,2); 2,502(138,7); 2,498(103,1); 2,493(51,6); 2,427(16,0); 2,358(1,6); 2,334(0,7); 2,329(1,0); 2,324(0,8); 1,233(0,5); 1,201(0,4); 1,184(0,9); 1,166(0,4); 0,146(0,9); 0,008(7,4); 0,000(188,5); -0,009(7,9); -0,150(0,9) |
| 48 | 1,86 | 1,85 | **1H-NMR(400,0 MHz, d6-DMSO):** δ = 9,350(3,3); 9,345(3,3); 8,834(2,3); 8,830(2,5); 8,822(2,4); 8,818(2,4); 8,689(8,7); 8,559(1,4); 8,555(1,9); 8,550(1,4); 8,539(1,5); 8,534(2,0); 8,530(1,4); 8,145(1,0); 8,046(4,0); 8,041(4,2); 7,895(0,7); 7,891(0,6); 7,878(0,4); 7,758(1,9); 7,753(1,8); 7,738(2,4); 7,733(2,4); 7,679(1,9); 7,668(2,0); 7,660(1,8); 7,648(1,7); 7,594(3,3); 7,573(2,6); 7,531(0,4); 4,272(0,9); 4,263(0,5); 4,253(0,7); 4,245(1,1); 4,235(1,8); 4,217(0,6); 4,208(1,3); 4,181(0,4); 4,123(0,4); 4,096(1,2); 4,087(0,4); 4,069(1,4); 4,059(1,0); 4,042(0,5); 4,032(0,9); 3,320(6,0); 2,996(0,5); 2,671(0,5); 2,667(0,4); 2,506(55,7); 2,502(71,4); 2,498(55,1); 2,477(16,0); 2,329(0,5); 1,296(0,5); 1,278(1,1); 1,260(0,5); 0,000(2,1) |
| 49 | 3,04 | 2,98 | **1H-NMR(400,0 MHz, d6-DMSO):** δ = 9,347(2,9); 9,342(3,0); 8,838(2,3); 8,834(2,4); 8,826(2,4); 8,822(2,5); 8,558(1,3); 8,554(1,9); 8,548(1,4); ,536(10,2); 8,528(1,5); 8,317(0,8); 7,924(0,4); 7,683(1,9); 7,675(6,4); 7,665(2,0); 7,651(1,5); 7,360(4,7); 6,025(1,0); 4,055(0,6); 4,038(2,6); 4,020(2,4); 4,014(3,6); 4,002(0,9); 3,988(3,6); 3,962(1,3); 3,855(0,8); 3,837(0,8); 3,392(0,3); 3,374(0,5); 3,332(421,8); 2,676(1,7); 2,671(2,3); 2,667(1,7); 2,525(5,6); 2,511(131,6); 2,507(269,9); 2,502(357,0); 2,498(264,5); 2,494(133,5); 2,407(15,0); 2,365(0,4); 2,334(1,8); 2,329(2,4); 2,325(1,8); 2,078(16,0); 1,989(7,6); 1,890(0,3); 1,398(1,9); 1,235(0,6); 1,193(2,1); 1,175(4,1); 1,157(2,1); 1,100(0,8); 1,083(1,5); 1,065(0,8); 0,146(0,5); 0,008(3,7); 0,000(116,2); -0,008(4,9); -0,150(0,5) |
| 50 | 2,06 | 2,01 | **1H-NMR(400,0 MHz, d6-DMSO):** δ = 9,351(4,6); 9,346(4,6); 8,839(3,4); 8,835(3,7); 8,827(3,6); 8,823(3,7); 8,591(14,3); 8,561(1,9); 8,556(2,7); 8,551(1,9); 8,541(2,0); 8,536(2,8); 8,531(1,9); 8,317(1,3); 7,937(6,3); 7,928(4,2); 7,685(2,6); 7,673(2,6); 7,667(2,5); 7,653(2,4); 7,496(3,0); 7,470(4,4); 4,323(0,5); 4,295(0,6); 4,286(0,7); 4,259(0,7); 4,178(0,6); 4,171(0,6); 4,151(1,8); 4,144(1,8); 4,124(1,8); 4,117(1,8); 4,097(0,7); 4,090(0,6); 4,055(0,4); 4,037(1,0); 4,020(1,0); 4,002(0,4); 3,977(0,7); 3,951(0,8); 3,940(0,7); 3,924(0,3); 3,914(0,7); 3,372(0,9); 3,332(833,0); 2,676(2,9); 2,671(3,9); 2,667(2,9); 2,525(9,7); 2,507(449,3); 2,502(587,2); 2,498(437,0); 2,451(12,1); 2,446(16,0); 2,408(0,8); 2,353(0,6); 2,334(3,2); 2,329(4,0); 2,325(3,0); 2,279(0,4); 2,258(0,5); 2,188(15,9); 2,182(12,0); 1,989(4,2); 1,193(1,1); 1,175(2,3); 1,157(1,1); 0,146(0,8); 0,008(6,5); 0,000(190,8); -0,008(8,2); -0,150(0,9) |
| 51 | 3,25 | 3,17 | **1H-NMR(400,0 MHz, d6-DMSO):** δ = 9,354(5,1); 9,348(5,2); 8,844(3,9); 8,840(4,4); 8,832(4,1); 8,828(4,4); 8,658(16,0); 8,565(2,1); 8,561(3,0); 8,556(2,3); 8,546(2,3); 8,540(3,2); 8,536(2,3); 8,138(10,9); 8,091(13,9); 8,003(0,3); 7,812(0,5); 7,687(2,9); 7,675(2,9); 7,667(2,9); 7,655(2,8); 5,757(3,1); 4,262(0,4); 4,248(0,9); 4,238(1,2); 4,223(2,7); 4,213(2,8); 4,198(2,9); 4,188(2,8); 4,173(1,3); 4,163(1,0); 4,149(0,4); 3,340(73,8); 2,676(0,9); 2,671(1,2); 2,667(0,9); 2,524(3,2); 2,511(67,2); 2,507(136,2); 2,502(181,2); 2,498(135,3); 2,493(68,1); 2,333(0,8); 2,329(1,2); 2,324(0,9); 2,287(0,6); 1,234(0,3); 0,000(7,9) |
| 52 | 1,57 | 1,55 | **1H-NMR(400,0 MHz, d6-DMSO):** δ = 9,342(2,1); 9,337(2,1); 8,831(1,5); 8,828(1,6); 8,819(1,6); 8,816(1,6); 8,620(5,9); 8,552(0,9); 8,547(1,3); 8,542(0,9); 8,532(1,0); 8,527(1,3); 8,522(0,9); 7,677(1,2); 7,664(1,2); 7,657(1,3); 7,645(1,2); 7,601(2,8); 7,543(0,5); 7,539(0,5); 7,525(4,0); 7,509(2,3); 7,489(0,8); 7,397(1,0); 7,392(1,6); 7,386(1,0); 7,380(0,9); 7,375(1,3); 7,370(0,8); 5,754(9,1); 4,101(8,1); 3,986(0,9); 3,977(0,6); 3,962(0,4); 3,320(15,8); 3,053(16,0); 3,044(2,3); 2,849(15,1); 2,507(32,7); 2,502(41,4); 2,498(30,9); 1,187(0,4); 0,000(3,5) |
| 53 | 0,91 | 1,08 | **1H-NMR(400,0 MHz, d6-DMSO):** δ = 9,349(2,0); 8,833(1,5); 8,823(1,5); 8,697(5,9); 8,556(1,4); 8,552(1,1); 8,541(1,1); 8,536(1,5); 8,140(1,0); 7,968(3,1); 7,904(0,8); 7,900(0,8); 7,893(1,7); 7,886(1,2); 7,882(1,3); 7,878(1,2); 7,820(0,4); 7,799(3,8); 7,788(3,6); 7,682(1,3); 7,669(1,3); 7,662(1,4); 7,649(1,2); 5,754(3,5); 4,219(0,7); 4,182(3,7); 4,166(3,5); 4,129(0,8); 4,100(0,4); 4,090(0,3); 3,318(8,9); 3,053(0,6); 2,934(16,0); 2,915(1,8); 2,858(14,1); 2,844(2,3); 2,671(0,7); 2,505(83,9); 2,502(106,8); 2,498(84,9); 2,328(0,7); 2,074(0,6); 0,000(3,2) |
| 54 | 1,90 | 1,91 | **1H-NMR(400,0 MHz, d6-DMSO):** δ = 9,347(2,8); 9,342(2,6); 8,837(2,0); 8,825(2,0); 8,668(5,5); 8,552(1,6); 8,532(1,6); 8,141(0,9); 7,718(2,4); 7,700(2,4); 7,682(1,5); 7,669(1,5); 7,662(1,4); 7,649(1,3); 7,448(2,1); 7,422(2,1); 5,754(0,6); 4,011(8,8); 3,320(45,4); 3,027(16,0); 2,891(0,5); 2,832(14,9); 2,731(0,5); 2,670(0,6); 2,502(95,0); 2,406(12,3); 2,328(0,6); 0,000(4,0) |
| 55 | 1,38 | 1,38 | **1H-NMR(400,0 MHz, d6-DMSO):** δ = 9,350(3,2); 9,345(3,1); 8,838(2,2); 8,835(2,3); 8,826(2,3); 8,823(2,3); 8,722(6,2); 8,556(1,8); 8,540(1,4); 8,535(1,9); 8,531(1,3); 8,139(4,6); 8,119(2,9); 7,685(1,7); 7,672(1,7); 7,665(1,7); 7,653(1,5); 7,568(2,4); 7,541(2,4); 4,185(1,3); 4,148(2,5); 4,086(1,2); 4,047(0,6); 3,340(160,4); 2,955(16,0); 2,863(15,8); 2,672(0,6); 2,507(78,3); 2,503(97,2); 2,473(15,1); 2,330(0,6); 0,000(3,2) |
| 56 | 3,25 | 3,00 | **1H-NMR(400,0 MHz, d6-DMSO):** δ = 9,333(2,4); 9,328(2,4); 8,834(1,8); 8,830(2,0); 8,822(1,9); 8,818(2,0); 8,535(1,1); 8,531(1,4); 8,526(1,1); 8,515(1,2); 8,510(1,5); 8,506(1,1); 7,939(2,4); 7,920(2,4); 7,680(1,4); 7,668(1,4); 7,661(1,4); 7,648(1,3); 7,541(2,1); 7,515(2,1); 4,085(0,5); 4,071(0,5); 4,060(0,6); 4,046(1,3); 4,037(0,6); 4,021(1,3); 4,012(1,3); 3,995(0,7); 3,986(1,3); 3,972(0,5); 3,960(0,5); 3,946(0,5); 3,329(49,4); 2,676(0,4); 2,672(0,5); 2,668(0,4); 2,525(1,3); 2,512(29,6); 2,507(59,8); 2,503(79,0); 2,498(58,4); 2,494(29,1); 2,469(13,3); 2,443(0,6) 2,417(0,6); 2,339(0,3); 2,334(0,5); 2,329(0,6); 2,325(0,5); 2,302(0,4); 2,281(16,0); 2,076(1,0); 0,008(1,9); 0,000(57,6); -0,009(2,3) |
| 57 | 2,25 | 2,05 | **1H-NMR(601,6 MHz, CDCl₃):** δ = 9,363(2,0); 8,807(1,5); 8,469(0,8); 8,466(1,1); 8,463(0,8); 8,452(1,9); 8,441(0,9); 8,438(1,1); 8,435(0,8); 8,065(1,7); 8,029(1,8); 8,017(1,8); 8,003(1,8); 7,991(1,8); 7,979(1,0); 7,966(1,1); 7,556(0,7); 7,554(0,7); 7,542(0,9); 7,541(0,9); 7,526(0,9); 7,519(1,3); 7,513(1,7); 7,507(1,3); 7,500(0,9); 7,415(1,0); 7,402(1,7); 7,389(0,8); 7,304(1,8); 7,302(1,9); 7,288(1,9); 7,286(1,8); 7,272(7,2); 5,302(6,3); 3,617(0,5); 3,609(0,4); 3,601(0,6); 3,593(1,0); 3,576(1,1); 3,563(1,1); 3,555(0,3); 3,547(3,0); 3,539(0,6); 3,530(3,1); 3,523(0,8); 3,514(1,2); 3,506(0,7); 2,590(0,5); 2,517(16,0); 2,501(0,6); 2,493(0,4); 2,484(0,3); 2,418(0,7); 2,408(12,8); 2,395(0,6); 2,386(12,7); 2,378(1,1); 2,369(0,4); 2,356(0,5); 2,352(0,4); 2,344(0,4); 2,332(0,4); 1,335(0,5); 1,329(0,3); 1,306(0,6); 1,295(0,9); 1,286(1,3); 1,283(1,3); 1,271(1,3); 1,255(6,2); 1,232(0,7); 1,221(0,5); 1,212(0,5); 0,891(0,8); 0,880(1,4); 0,868(1,0); 0,856(0,5); 0,843(0,9); 0,841(0,9); 0,834(0,9); 0,005(0,5); 0,000(14,7); -0,006(0,7) |
| 58 | 3,45 | 3,32 | 1H-NMR(400,0 MHz, DMSO): δ = 9,430(10,3); 9,425(10,4); 8,891(8,1); 8,887(8,8); 8,879(8,5); 8,875(8,7); 8,651(4,7); 8,646(6,4); 8,641(4,7); 8,631(5,1); 8,625(6,5); 8,621(4,7); 8,317(0,4); 8,260(1,0); 8,242(1,1); 7,992(9,9); 7,973(9,9); 7,729(6,1); 7,717(6,0); 7,709(6,0); 7,697(6,2); 7,673(0,9); 7,583(8,6); 7,556(8,5); 5,758(16,0); 4,361(0,7); 4,351(0,4); 4,344(0,4); 4,324(0,4); 4,086(0,4); 4,059(0,4); 4,049(0,4); 4,018(4,1); 3,993(12,7); 3,967(13,1); 3,941(4,5); 3,331(218,6); 2,677(1,1); 2,672(1,5); 2,668(1,1); 2,512(92,5); 2,508(154,3); 2,503(197,3); 2,499(145,6); 2,495(73,3); 2,374(0,4); 2,353(0,4); 2,334(1,0); 2,330(1,3); 2,326(1,0); 1,343(0,4); 1,326(0,8); 1,308(0,4); 0,146(0,4); 0,008(3,0); 0,000(84,9); -0,008(3,4);-0,150(0,4) |
| 59 | 2,40 | 2,35 | 1H-NMR(400,0 MHz, DMSO): δ = 9,432(5,9); 9,428(6,0); 8,891(4,5); 8,887(4,8); 8,879(4,8); 8,875(4,8); 8,652(2,6); 8,648(3,5); 8,642(2,6); 8,632(2,8); 8,626(3,7); 8,622(2,7); 8,260(5,9); 8,241(6,0); 7,729(3,3); 7,717(3,3); 7,709(3,5); 7,699(7,6); 7,673(4,8); 5,758(16,0); 4,415(0,5); 4,388(1,7); 4,378(0,8); 4,360(2,0); 4,351(2,3); 4,333(0,8); 4,323(2,3); 4,296(0,7); 4,112(0,6); 4,085(2,0); 4,075(0,7); 4,058(2,3); 4,048(1,9); 4,031(0,9); 4,021(1,8); 3,994(0,6); 3,331(101,6); 2,676(0,7); 2,672(1,0); 2,668(0,8); 2,507(117,2); 2,503(134,4); 2,498(99,6); 2,334(0,6); 2,330(0,9); 2,325(0,7); 1,233(0,7); 0,008(1,1); 0,000(27,5) |
| 60 | 2,41 | 2,45 | **1H-NMR(400,0 MHz, d6-DMSO):** δ = 9,339(7,0); 8,827(5,1); 8,818(4,9); 8,618(9,6); 8,546(4,0); 8,527(4,0); 8,312(0,4); 7,676(3,3); 7,661(3,9); 7,646(2,9); 7,529(5,3); 7,509(15,6); 7,494(14,9); 7,475(4,6); 3,643(0,5); 3,637(0,5); 3,606(0,5); 3,586(0,6); 3,331(671,5); 3,145(0,9); 3,109(3,6); 3,091(8,5); 3,073(8,4); 3,055(3,4); 3,009(0,5); 2,987(0,4); 2,974(0,3); 2,959(0,3); 2,937(0,3); 2,919(0,4); 2,897(0,4); 2,885(0,3); 2,803(0,4); 2,671(3,3); 2,502(411,9); 2,395(1,4); 2,330(3,0); 2,243(0,3); 2,073(0,6); 1,318(8,9); 1,300(16,0); 1,282(8,2); 1,235(0,6); 146(0,7); 0,000(103,8);-0,073(0,5); -0,141(0,3); -0,150(0,7) |
| 61 | 1,10 | 1,22 | **1H-NMR(400,0 MHz, d6-DMSO):** δ = 9,349(4,7); 9,344(4,8); 8,835(3,5); 8,831(3,6); 8,823(3,7); 8,819(3,5); 8,683(11,9); 8,558(2,0); 8,554(2,8); 8,548(1,9); 8,538(2,2); 8,533(2,8); 8,528(1,9); 8,313(0,4); 7,870(4,9); 7,849(12,5); 7,822(12,3); 7,801(4,7); 7,681(2,6); 7,669(2,6); 7,661(2,6); 7,649(2,4); 5,753(3,4); 3,317(42,6); 3,178(0,5); 3,159(1,7); 3,141(2,1); 3,125(2,5); 3,107(2,2); 3,089(0,7); 2,898(0,6); 2,879(2,1); 2,861(2,5); 2,845(2,1); 2,827(1,8); 2,809(0,5); 2,671(0,9); 2,506(103,2); 2,502(129,1); 2,498(96,5); 2,333(0,6); 2,329(0,8); 1,235(0,7); 1,113(7,6); 1,094(16,0); 1,076(7,2); 0,146(1,0); 0,033(0,4); 0,000(200,8); 0,150(1,0) |
| 62 | 2,71 | 2,69 | **1H-NMR(400,0 MHz, d6-DMSO):** δ = 9,954(1,2); 9,347(5,1); 9,343(5,1); 9,341(5,2); 9,106(0,9); 9,100(0,9); 8,835(3,9); 8,831(4,4); 8,823(4,0); 8,819(4,4); 8,744(0,7); 8,740(0,8); 8,732(0,8); 8,728(0,8); 8,701(16,0); 8,636(1,4); 8,558(2,1); 8,552(3,1); 8,548(2,5); 8,538(2,3); 8,532(3,3); 8,528(2,5); 8,280(0,4); 8,275(0,5); 8,271(0,4); 8,260(0,4); 8,255(0,6); 8,251(0,4); 7,969(7,3); 7,948(9,3); 7,881(1,5); 7,859(2,0); 7,806(1,5); 7,799(11,4); 7,795(3,7); 7,783(3,0); 7,778(9,2); 7,772(1,0); 7,688(0,6); 7,679(4,0); 7,667(4,3); 7,660(3,6); 7,647(4,4); 7,625(0,4); 7,612(0,5); 7,600(0,5); 7,592(0,5); 7,579(0,5); 7,333(1,3); 3,324(74,8); 2,676(0,7); 2,671(0,9); 2,667(0,7); 2,524(2,5); 2,511(45,7); 2,507(92,5); 2,502(124,5); 2,498(94,2); 2,493(47,7); 2,333(0,5); 2,329(0,8); 2,325(0,6); 1,298(0,5); 1,259(0,7); 1,233(1,0); 0,000(1,1) |
| 63 | 2,03 | 1,99 | **1H-NMR(400,0 MHz, d₆-DMSO):** δ = 9,344(0,4); 8,925(9,0); 8,748(0,7); 8,714(16,0); 8,649(1,1); 8,542(0,3); 8,462(5,6); 8,446(5,9); 8,317(1,5); 8,137(0,4); 8,117(0,7); 8,081(5,4); 8,061(5,8); 7,970(10,5); 7,950(13,1); 7,793(14,1); 7,772(11,5); 7,689(0,8); 7,666(5,7); 7,647(6,7); 7,629(4,4); 5,757(7,6); 3,507(0,3); 3,487(0,3); 3,447(0,4); 3,439(0,4); 3,409(0,5); 3,326(536,7); 3,245(0,3); 2,840(0,4); 2,820(0,4); 2,805(0,4); 2,765(0,4); 2,671(8,5); 2,622(1,1); 2,618(1,1); 2,502(1215,9); 2,329(7,9); 2,184(0,3); 1,355(0,6); 1,299(1,8); 1,259(2,9); 1,235(7,6); 1,166(0,5); 1,149(1,1); 1,066(0,4); 0,854(1,6); 0,843(1,0); 0,834(1,1); 0,814(0,7); 0,784(0,5); 0,146(5,7); 0,083(0,5); 0,000(1101,9); -0,150(5,9) |
| 64 | 2,91 | 2,89 | **1H-NMR(400,0 MHz, d6-DMSO):** δ = 9,343(2,9); 9,338(3,0); 8,837(2,1); 8,833(2,4); 8,825(2,3); 8,821(2,4); 8,623(8,0); 8,552(1,2); 8,548(1,7); 8,543(1,3); 8,532(1,3); 8,527(1,8); 8,523(1,3); 7,871(3,3); 7,793(1,4); 7,772(1,9); 7,769(1,9); 7,691(4,1); 7,683(1,9); 7,670(4,3); 7,663(1,8); 7,650(1,6); 5,751(1,5); 3,310(93,8); 2,675(0,6); 2,670(0,8); 2,666(0,6); 2,523(2,1); 2,506(94,0); 2,501(124,8); 2,497(95,6); 2,328(0,8); 2,323(0,6); 2,200(16,0); 1,235(0,4); 0,008(0,4); 0,000(12,5) |
| 65 | 1,70 | 1,70 | **1H-NMR(400,0 MHz, d6-DMSO):** δ = 10,654(0,5); 10,355(0,5); 9,356(2,4); 9,351(2,4); 9,213(0,4); 8,839(2,0); 8,835(2,0); 8,827(1,9); 8,823(1,7); 8,775(0,4); 8,718(7,1); 8,560(1,4); 8,557(1,3); 8,546(1,4); 8,542(1,6); 8,466(0,9); 8,383(0,5); 8,362(0,6); 8,337(1,0); 8,314(5,1); 8,241(2,4); 8,220(2,6); 8,143(2,9); 8,138(2,9); 8,110(0,4); 7,994(0,4); 7,973(0,4); 7,940(0,9); 7,910(5,0); 7,848(0,7); 7,837(0,5); 7,794(1,6); 7,715(0,6); 7,685(1,4); 7,673(1,7); 7,667(1,6); 7,651(2,4); 7,645(2,1); 7,629(2,0); 7,624(2,0); 7,593(0,5); 7,530(0,4); 7,520(0,6); 7,516(0,6); 7,494(0,6); 5,924(0,4); 5,754(1,3); 5,584(0,4); 4,637(0,4); 4,306(0,4); 4,273(0,5); 4,186(0,4); 4,151(0,4); 4,130(0,4); 4,096(0,4); 4,055(0,4); 4,039(0,5); 4,021(0,5); 3,986(0,5); 3,967(0,6); 3,941(0,4); 3,921(0,5); 3,905(0,6); 3,884(0,6); 3,868(0,7); 3,851(0,5); 3,838(0,5); 3,823(0,5); 3,765(0,5); 3,692(0,6); 3,637(0,6); 3,608(0,6); 3,585(0,7); 3,573(0,7); 3,544(0,7); 3,530(0,7); 3,509(0,9); 3,470(1,0); 3,452(1,0); 3,438(1,0); 3,398(1,3); 3,392(1,4); 3,378(1,8); 3,317(1231,0); 3,277(1,8); 3,265(2,1); 3,259(1,7); 3,254(1,7); 3,217(1,2); 3,189(1,1); 3,174(1,2); 3,147(1,3); 3,123(1,4); 3,095(2,1); 3,071(2,2); 3,041(1,8); 3,002(2,0); 2,979(1,8); 2,937(1,6); 2,916(0,8); 2,902(0,7); 2,889(0,8); 2,861(0,9); 2,830(0,9); 2,812(1,0); 2,775(1,2); 2,710(1,6); 2,694(2,0); 2,675(12,0); 2,670(16,0); 2,666(12,5); 2,561(4,8); 2,557(5,4); 2,523(51,5); 2,510(893,1); 2,506(1772,8); 2,501(2326,4); 2,497(1726,9); 2,332(9,4); 2,328(13,4); 2,324(9,8); 1,352(0,6); 1,336(0,7); 1,300(0,5); 1,235(6,1); 1,213(1,1); 1,194(1,3); 1,176(1,0); 1,158(0,7); 0,854(1,1); 0,837(0,7); 0,214(0,4); 0,196(0,4); 0,146(13,9); 0,121(0,7); 0,112(0,7); 0,104(0,8); 0,091(0,7); 0,082(1,1); 0,074(1,0); 0,061(1,2); 0,056(1,0); 0,008(120,2); 0,000(2914,5); 0,008(142,9); -0,052(1,8); -0,058(0,9); -0,063(0,7); 0,068(0,7); -0,150(13,4) |
| 66 | 2,20 | 2,21 | **1H-NMR400,0 MHz, d6-DMSO:** δ = 9,340(4,1); 9,334(4,3); 8,836(2,9); 8,833(3,2); = 9,340(4,1); 8,825(3,0); 8,821(3,2); 8,548(1,6); 8,544(2,5); 8,539(1,8); 8,525(12,5); 7,682(2,3); 7,670(2,3); 7,661(2,7); 7,656(2,6); 7,650(2,8); 7,638(3,8); 7,635(3,5); 7,599(2,2); 7,585(4,8); 7,566(4,6); 7,459(2,0); 7,456(2,1); 7,437(3,1); 7,421(1,2); 7,418(1,3); 4,039(0,5); 4,021(0,5); 3,321(22,4); 2,983(1,3); 2,979(1,6); 2,964(4,1); 2,961(4,4); 2,946(4,3); 2,943(4,4); 2,928(1,6); 2,671(0,3); 2,507(39,0); 2,503(51,4); 2,499(39,7); 2,330(0,3); 1,989(1,9); 1,187(7,9); 1,175(1,7); 1,168(16,0); 1,158(1,1); 1,150(7,6); 0,000(1,1) |
| 67 | 1,28 | 1,29 | **1H-NMR(400,0 MHz, d6-DMSO):** δ = 9,347(8,0); 9,342(8,3); 8,839(6,5); 8,832(13,6); 8,734(8,2); 8,553(4,6); 8,533(4,8); 8,315(0,3); 8,010(3,0); 7,991(3,8); 7,974(2,1); 7,955(2,9); 7,883(2,4); 7,864(5,3); 7,845(3,6); 7,834(1,8); 7,818(4,0); 7,816(4,1); 7,797(4,2); 7,781(1,6); 7,712(7,0); 7,688(6,1); 7,675(4,2); 7,667(4,2); 7,655(3,9); 4,356(0,5); 4,343(0,9); 4,331(0,5); 3,456(0,9); 3,443(0,9); 3,439(1,0); 3,426(0,9); 3,409(0,3); 3,319(49,2); 2,979(0,5); 2,961(1,6); 2,943(2,1); 2,927(2,4); 2,920(1,6); 2,908(2,3); 2,902(1,7); 2,886(2,0); 2,867(1,6); 2,849(0,5); 2,700(0,6); 2,690(0,8); 2,682(2,1); 2,672(3,4); 2,664(2,7); 2,654(2,7); 2,648(2,0); 2,638(2,3); 2,630(1,6); 2,620(1,8); 2,611(0,7); 2,602(0,7); 2,541 (1,1); 2,502(179,7); 2,329(1,0); 1,077(7,4); 1,059(16,0) ;1,040(8,1); 1,026(5,7); 1,007(11,5); 0,989(5,3); 0,146(1,0); 0,027(0,5); 0,000 (183,4); -0,150(1,0) |
| 68 | 2,34 | 2,29 | **1H-NMR(400,0 MHz, d₆-DMSO):** δ = 9,348(4,6); 9,344(4,6); 8,841(3,7); 8,837(4,0); 8,829(4,0); 8,825(4,0); 8,558(2,5); 8,551(16,0); 8,539(2,6); 8,533(3,3); 8,529(2,4); 7,918(3,2); 7,915(3,1); 7,899(4,0); 7,688(2,9); 7,686(2,8); 7,676(2,9); 7,674(2,9); 7,667(5,2); 7,663(4,0); 7,654(4,6); 7,648(3,9); 7,645(5,9); 7,639(5,5); 7,635(3,0); 7,620(3,2); 7,616(2,2); 7,598(3,4); 7,595(3,5); 7,579(3,1); 7,576(3,5); 7,561(1,3); 7,557(1,2); 5,757(3,6); 4,056(0,6); 4,038(1,0); 4,031(1,1); 4,020(1,3); 4,017(1,0); 4,006(1,3); 3,992(2,6); 3,980(0,7); 3,967(3,0); 3,944(2,9); 3,930(0,6); 3,918(2,8); 3,905(1,2); 3,892(1,0); 3,878(1,2); 3,852(0,4); 3,329(31,2); 2,677(0,4); 2,672(0,5); 2,668(0,4); 2,526(1,3); 2,512(26,5); 2,508(53,3); 2,503(70,5); 2,499(52,7); 2,495(27,0); 2,335(0,3); 2,330(0,5); 2,325(0,4); 1,990(3,7); 1,397(1,3); 1,299(0,8); 1,259(1,1); 1,250(0,4); 1,234(1,6); 1,193(1,2); 1,176(2,1); 1,158(1,1); 0,146(0,6); 0,008(4,9); 0,000(120,6); -0,008(6,0); -0,150 (0,6) |
| 69 | 1,74 | 1,75 | **1H-NMR(601,6 MHz, CDCl₃):** δ = 9,372(12,3); 8,822(9,3); 8,817(9,2); 8,465(7,5); 8,453(8,0); 8,307(16,0); 8,291(8,4); 8,278(8,2); 8,192(1,6); 8,154(0,9); 7,937(0,8); 7,910(4,3); 7,898(8,3); 7,885(5,1); 7,837(5,4); 7,825(9,3); 7,813(6,0); 7,583(0,8); 7,525(6,1); 7,516(7,8); 7,513(7,9); 7,505(6,3); 7,451(9,5); 7,439(8,9); 7,263(66,7); 7,086(0,4); 5,301(4,1); 4,129(1,1); 4,112(3,4); 4,094(4,4); 4,072(3,5); 4,055(1,2); 3,882(0,3); 3,859(0,5); 3,841(0,4); 3,642(0,4); 3,625(0,5); 3,597(1,4); 3,580(3,6); 3,564(4,4); 3,558(4,0); 3,541(3,2); 3,525(1,1); 3,490(0,5); 1,577(178,8); 1,426(0,4); 1,371(1,1); 1,333(2,4); 1,285(4,4); 1,256(10,6); 1,104(0,5); 0,881(1,6); 0,842(1,6); 0,070(0,6); 0,000(47,7) |
| 70 | 2,82 | 2,75 | **1H-NMR(400,0 MHz, d6-DMSO):** δ = 9,238(3,7); 8,867(3,7); 8,861(4,0); 8,636(9,5); 8,528(1,2); 8,522(1,6); 8,517(1,2); 8,505(1,3); 8,500(1,7); 8,494(1,2); 8,175(0,4); 7,395(3,9); 7,389(5,0); 7,366(3,3); 7,259(2,5); 7,254(2,4); 7,239(2,0); 7,234(1,9); 7,199(0,4); 5,758(0,4); 3,437(0,3); 3,329(65,1); 2,676(0,7); 2,671(1,0); 2,667(0,8); 2,662(0,5); 2,541(0,4); 2,525(2,4); 2,511(55,7); 2,507(114,5) 2,502(153,6) ; 2,498(117,2); 2,494(63,2); 2,489(35,6); 2,474(2,6); 2,424(0,4); 2,333(1,1); 2,329(1,5); 2,316(16,0); 2,287(0,4); 2,216(1,0); 1,434(0,6); 1,296(0,4); 1,234(1,3); 1,073(0,9); 1,055(1,8); 1,038(0,9); 0,70 0(0,7); 0,000(0 ,3) |
| 71 | 3,64 | 3,34 | **1H-NMR(400,0 MHz, d6-DMSO):** δ = 9,238(2,3); 9,234(3,8); 8,869(3,7); 8,862(3,9); 8,547(9,3); 8,524(1,2); 8,519(1,5); 8,517(1,5); 8,512(1,2); 8,500(1,3); 8,496(1,6); 8,489(1,1); 7,676(5,9); 7,360(4,8); 5,754(0,4); 4,038(0,8); 4,033(1,2); 4,021(0,8); 4,007(3,6); 3,981(3,7); 3,955(1,3); 3,319(43,7); 2,671(0,4); 2,525(1,1); 2,511(20,9); 2,507(41,2); 2,502(53,7); 2,498(39,5); 2,494(19,6); 2,410(14,9); 2,329(0,4); 2,080(16,0); 1,989(2,6); 1,398(0,9); 1,193(0,7); 1,176(1,4); 1,158(0,7); 0,008(0,8); 0,000(20,9); -0,008(0,8) |
| 72 | 2,35 | 2,29 | **1H-NMR(400,0 MHz, d6-DMSO):** δ = 9,241(5,8); 8,872(5,8); 8,866(6,0); 8,604(14,2); 8,532(1,9); 8,526(2,5); 8,521(1,8); 8,509(2,0); 8,504(2,6); 8,498(1,8); 7,944(6,4); 7,934(4,4); 7,496(3,1); 7,470(4,6); 5,757(13,7); 4,324(0,6); 4,297(0,7); 4,287(0,7); 4,260(0,7); 4,178(0,6); 4,168(0,5); 4,160(0,5); 4,150(1,7); 4,142(1,6); 4,133(0,5); 4,124(1,8); 4,115(1,8); 4,105(0,5); 4,097(0,6); 4,087(0,7); 3,970(0,7); 3,943(0,8); 3,933(0,6); 3,906(0,6); 3,328(81,6); 2,676(0,5); 2,672(0,7); 2,667(0,5); 2,507(83,1); 2,503(109,0); 2,498(81,5); 2,451(12,1); 2,446(15,8); 2,408(0,6); 2,333(0,6); 2,329(0,8); 2,325(0,6); 2,189(16,0); 2,183(11,7); 2,076(11,2); 0,000(1,0) |
| 73 | 3,36 | 3,25 | **1H-NMR(400,0 MHz, d6-DMSO):** δ = 9,243(3,5); 8,875(3,5);8,868(3,6);8,693(7,7);8,535(1,2); 8,530(1,7);8,524(1,2);8,512(1,2);8,507(1,7); 8,501(1,1);7,940(3,1);7,922(3,1);7,521(2,7); 7,494(2,7);4,061(1,2);4,035(3,8);4,009(3,9); 3,984(1,4);3,329(53,8);2,676(0,3);2,672(0,5); 2,668(0,3);2,507(49,1);2,503(64,3);2,499(49,1); 2,471(16,0);2,414(0,5);2,334(0,3);2,330(0,4); 2,325(0,3); 2,076(14,3); 1,233(0,9); 0,000(0,4) |
| 74 | 2,34 | 2,26 | **1H-NMR(400,0 MHz, d₆-DMSO):** δ = 9,249(4,3); 9,246(7,4); 9,242(4,4); 8,876(7,8); 8,870(8,1); 8,738(16,0); 8,538(2,5); 8,533(3,1); 8,531(3,1); 8,527(2,4); 8,515(2,6); 8,510(3,3); 8,503(2,4); 8,447(0,5); 8,317(0,7); 8,200(6,2); 8,182(6,2); 7,639(4,3); 7,613(4,3); 5,758(2,5); 4,299(0,6); 4,266(1,0); 4,241(0,8); 4,147(0,4); 4,126(0,7); 4,099(0,8); 4,038(0,5); 4,020(0,4); 3,568(3,3); 3,329(336,9); 2,676(1,4); 2,671(2,0); 2,667(1,5); 2,525(5,0); 2,520(7,9); 2,511(110,1); 2,507(225,1); 2,502(308,8); 2,498(233,4) 2,494(111,7); 2,377(1,3); 2,334(1,5); 2,329(2,0); 2,325(1,5); 1,989(1,2); 1,234(0,4); 1,207(0,4); 1,193(0,6); 1,189(0,7); 1,175(0,8); 1,,157(0,3); 0,000(0,9) |
| 75 | 1,80 | 1,72 | **1H-NMR(400,0 MHz, d6-DMSO):** δ = 9,532(12,8); 9,421(6,3); 9,396(0,9); 9,367(0,5); 8,619(6,6); 8,449(0,5); 7,792(0,7); 7,772(0,8); 7,525(0,5); 7,493(1,2); 7,466(4,3); 7,446(7,2); 7,401(6,7); 7,381(4,2); 7,252(0,3); 7,239(0,4); 7,183(0,6); 7,163(0,6); 7,116(0,3); 5,754(1,6); 4,095(0,3); 4,077(0,8); 4,060(0,8); 4,041(0,3); 3,865(0,3); 3,846(0,3); 3,318(48,1); 2,671(0,9); 2,566(0,8); 2,502(128,3); 2,424(4,2); 2,409(16,0); 2,328(1,3); 2,306(0,5); 2,284(2,0); 1,344(0,4); 1,326(0,4); 1,302(0,5); 1,285(0,6); 1,265(0,5); 1,248(0,5); 1,242(0,5); 1,225(0,6); 1,205(1,4); 1,187(2,1); 1,178(1,2); 1,169(1,3); 1,098(0,4); 0,146(0,6); 0,000(109,4); -0,150(0,6) |
| 76 | 2,88 | 2,77 | **1H-NMR(400,0 MHz, d6-DMSO):** δ = 9,538(16,0); 9,429(7,5); 8,559(8,5); 7,680(4,9); 7,362(3,9); 5,754(1,8); 4,034(1,0); 4,008(2,9); 3,982(3,1); 3,956(1,1); 3,318(38,5); 2,671(0,4); 2,524(1,0); 2,511(23,9); 2,506(47,7); 2,502(62,3); 2,497(45,4); 2,493(22,1); 2,411(12,3); 2,368(0,6); 2,329(0,4); 2,170(0,5); 2,080(13,3); 1,352(1,1); 1,336(0,4); 1,259(0,4); 1,250(0,6); 1,229(1,2); 0,000(3,8) |
| 77 | 1,84 | 1,82 | **1H-NMR(400,0 MHz, d6-DMSO):** δ = 9,542(16,0); 9,429(7,8); 8,612(8,8); 7,949(4,1); 7,939(2,8); 7,497(2,0); 7,471(3,0); 5,754(0,9); 4,321(0,4); 4,293(0,5); 4,284(0,5); 4,257(0,5); 4,175(0,4); 4,166(0,4); 4,148(1,1); 4,139(1,1); 4,121(1,1); 4,112(1,2); 4,094(0,4); 4,085(0,4); 3,969(0,5); 3,942(0,5); 3,932(0,4); 3,905(0,5); 3,317(54,5); 2,675(0,5); 2,671(0,7); 2,666(0,5); 2,506(79,6); 2,502(106,3); 2,497(82,4); 2,452(8,0); 2,447(10,7); 2,338(0,5); 2,333(0,6); 2,328(0,7); 2,324(0,6); 2,258(0,4); 2,188(10,5); 2,182(8,0); 1,234(0,7); 0,008(0,5); 0,000(13,1) |
| 78 | 2,73 | 2,65 | **1H-MR(400,0 MHz, d6-DMSO):** δ= 9,543(16,0); 9,448(1,1); 9,430(7,6); 9,399(0,4); 9,365(0,8); 8,700(6,9); 8,313(0,4); 7,943(2,5); 7,925(2,5); 7,520(2,1); 7,493(2,0); 6,896(0,8); 6,706(0,5); 5,753(0,7); 4,364(0,5); 4,346(0,6); 4,058(1,0); 4,032(3,1); 4,006(3,2); 3,981(1,2); 3,974(0,6); 3,317(53,6); 2,675(0,6); 2,671(0,8); 2,666(0,6); 2,538(0,6); 2,524(2,2); 2,519(3,5); 2,511(44,1); 2,506(91,2); 2,502(121,8); 2,497(89,5); 2,493(43,8); 2,473(12,9); 2,415(0,6); 2,333(0,6); 2,329(0,8); 2,324(0,6); 1,352(7,8); 1,336(2,3); 1,322(1,5); 1,315(0,6); 1,304(0,9); 1,298(1,5); 1,259(2,2); 1,250(3,5); 1,229(7,3); 1,180(0,4); 0,868(0,3); 0,851(0,8); 0,834(0,3); 0,008(0,4); 0,000(12,0); 0,008(0,4) |
| 79 | 2,20 | 2,17 | **1H-NMR(400,0 MHz, d6-DMSO):** δ = 9,302(2,4); 9,298(2,4); 9,296(2,3); 8,849(7,4); 8,813(1,9); 8,809(2,1); 8,801(2,1); 8,797(2,1); 8,511(1,1); 8,507(1,5); 8,506(1,4); 8,501(1,2); 8,491(1,2); 8,487(1,5); 8,485(1,6); 8,481(1,2); 7,658(1,4); 7,657(1,5); 7,646(1,4); 7,645(1,4); 7,638(1,4); 7,637(1,4); 7,626(1,4); 7,625(1,4); 7,271(1,0); 7,252(2,8); 7,233(2,2); 7,209(2,7); 7,184(2,0); 7,164(1,2); 7,134(1,7); 7,115(1,5); 7,030(0,5); 5,232(8,1); 4,363(0,3); 4,348(0,3); 3,329(63,9); 2,672(0,5); 2,668(0,3); 2,543(1,8); 2,526(0,9); 2,512(24,0); 2,508(49,4); 2,503(66,3); 2,499(49,7); 2,494(25,0); 2,334(0,3); 2,330(0,5); 2,325(0,3); 2,288(16,0); 0,000(0,4) |
| 80 | 2,39 | 2,42 | **1H-NMR(400,0 MHz, d6-DMSO):** δ = 9,307(2,7); 8,818(2,1); 8,808(2,0); 8,506(1,8); 8,486(1,8); 7,669(1,5); 7,657(1,7); 7,649(1,6); 7,637(1,4); 7,270(1,2); 7,251(2,7); 7,232(1,7); 7,124(2,2); 7,106(1,7); 7,048(3,5); 7,014(2,1); 6,995(1,8); 5,390(6,4); 3,329(45,5); 2,672(0,8); 2,564(16,0); 2,503(112,3); 2,330(0,9); 2,281(14,1); 1,398(0,4); 0,000(18,3) |
| 81 | 2,17 | 2,13 | **1H-NMR(400,0 MHz, d6-DMSO):** δ = 9,895(0,4); 9,305(5,1); 9,300(5,1); 9,276(0,3); 9,215(0,6); 8,956(0,7); 8,940(0,8); 8,871(12,3); 8,815(4,1); 8,811(4,4); 8,803(4,3); 8,799(4,1); 8,514(2,4); 8,509(3,3); 8,504(2,5); 8,494(2,6); 8,489(3,4); 8,484(2,4); 8,362(0,4); 8,150(0,5); 7,659(3,0); 7,647(3,1); 7,640(3,1); 7,627(2,8); 7,481(0,6); 7,461(0,9); 7,446(2,4); 7,438(1,3); 7,425(6,7); 7,406(3,8); 7,385(0,5); 7,363(0,3); 7,294(0,5); 7,266(4,8); 7,247(10,3); 7,238(9,9); 7,215(0,9); 7,202(0,7); 7,143(3,5); 7,138(3,6); 7,120(3,6); 7,099(0,9); 7,069(1,1); 7,053(3,7); 7,030(0,4); 5,286(16,0); 4,789(0,4); 4,770(0,4); 4,583(0,6); 4,570(0,6); 4,327(0,8); 4,311(0,7); 4,198(0,5); 4,181(0,5); 3,329(67,5); 2,945(1,8); 2,785(1,5); 2,672(1,0); 2,508(109,0); 2,504(135,0); 2,499(106,7); 2,330(1,0); 2,288(2,1); 2,076(1,3); 1,959(1,5); 1,630(0,5); 1,612(1,0); 1,594(0,5); 1,248(0,7); 1,231(1,3);1,213(0,6); 0,146(0,6); 0,000(110,1); 0,150(0,6) |
| 82 | 1,91 | 1,90 | **1H-NMR(400,0 MHz, d6-DMSO):** δ= 9,296(1,8); 9,291(1,7); 8,862(4,8); 8,809(1,3); 8,805(1,4); 8,797(1,4); 8,794(1,3); 8,505(0,9); 8,500(1,2); 8,495(0,8); 8,485(0,9); 8,480(1,2); 8,475(0,8); 7,653(1,1); 7,641(1,1); 7,633(1,1); 7,621(1,0); 7,391(3,4); 7,369(3,6); 6,940(0,8); 6,933(4,0); 6,911(3,6); 5,193(6,3); 3,750(0,9); 3,728(16,0); 3,331(54,2); 2,508(36,4); 2,503(46,4); 2,499(35,6); 2,330(0,3); 2,287(0,7); 1,397(0,6); 1,183(0,3) |
| 83 | 2,27 | 2,27 | **1H-NMR(400,0 MHz, d6-DMSO):** δ = 9,310(3,1); 9,306(3,1); 8,817(2,4); 8,808(2,4); 8,781(5,0); 8,525(1,9); 8,505(2,0); 7,673(1,6); 7,660(1,8); 7,653(1,8); 7,641(1,5); 7,481(1,4); 7,461(1,6); 7,243(1,3); 7,223(2,6); 7,203(1,5); 7,119(1,6); 7,099(1,6); 7,083(2,2); 7,054(2,1); 7,009(2,5); 6,990(2,1); 5,271(8,3); 4,109(1,6); 2,672(0,7); 2,503(100,4); 2,329(1,0); 2,295(16,0); 0,146(0,3); 0,000(64,6); -0,150(0,4) |
| 84 | 0,90 | | **1H-NMR(600,1 MHz, d6-DMSO):** δ = 9,317(4,7); 9,314(4,5); 9,313(4,4); 8,941(0,8); 8,927(0,7); 8,821(3,7); 8,819(3,9); 8,813(3,9); 8,810(3,9); 8,794(13,9); 8,788(14,8); 8,785(15,5); 8,521(2,1); 8,518(2,7); 8,517(2,7); 8,514(2,1); 8,508(2,2); 8,505(2,7); 8,504(2,9); 8,501(2,1); 7,664(2,6); 7,663(2,6); 7,656(2,6); 7,655(2,6); 7,651(2,6); 7,649(2,6); 7,643(2,5); 7,641(2,5); 7,468(2,9); 7,460(5,5); 7,452(2,8); 5,513(16,0); 4,987(0,4); 4,973(0,4); 4,966(0,4); 4,952(0,4); 3,325(57,1); 3,256(0,3); 3,085(2,2); 3,075(0,4); 2,788(2,2); 2,524(0,7); 2,521 (0,9); 2,518(1,0); 2,509(17,5); 2,506(35,7); 2,503(48,2); 2,500(35,3); 2,498(16,9); 2,076(1,3); 1,352(0,6); 1,232(1,0); 1,225(0,7); 0,000(0,8) |
| 85 | 0,90 | 1,00 | **1H-NMR(400,0 MHz, d6-DMSO):** δ = 9,306(5,5); 8,832(9,0); 8,817(14,8); 8,696(0,4); 8,685(0,4); 8,587(7,8); 8,571(7,3); 8,513(3,8); 8,493(3,8); 8,317(0,3); 7,663(3,2); 7,650(3,5); 7,643(3,5); 7,631(2,9); 5,758(1,0); 5,481(16,0); 3,327(92,7); 2,808(0,3); 2,785(0,3); 2,672(2,6); 2,502(293,6); 2,329(2,1); 2,288(0,5); 1,990(0,5); 1,397(1,9); 1,175(0,4); 1,150(0,4); 0,1,146(1,2); 0,000(186,4);-0,084(0,5); -0,150(1,1) |
| 86 | 1,01 | 1,07 | **1H-NMR(400,0 MHz, d6-DMSO):** δ = 9,307(1,7); 9,303(1,7); 8,817(1,4); 8,814(1,5); 8,805(1,4); 8,802(1,5); 8,701(5,3); 8,519(0,8); 8,514(1,0); 8,509(0,8); 8,498(0,9); 8,493(1,1); 8,489(0,8); 7,941(4,3); 7,661(1,0); 7,660(1,0); 7,647(1,0); 7,641(1,0); 7,640(1,0); 7,629(0,9); 7,627(0,9); 6,445(0,4); 6,427(1,4); 6,409(1,4); 6,392(0,4); 5,758(1,5); 3,895(16,0); 3,329(46,3); 2,672(0,4); 2,542(1,1); 2,525(1,0); 2,512(22,5); 2,507(45,6); 2,503(60,2); 2,498(44,0); 2,494(21,6); 2,330(0,4); 1,890(4,9); 1,872(4,9); 0,000(1,4) |

### Biologische Beispiele

**Boophilus microplus -Injektionstest**

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigte z.B. die folgende Verbindung der Herstellungsbeispiele eine Wirkung von 100 % bei einer Aufwandmenge von 20 µg/Tier: 57

**Meloidogyne incognita- Test**

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita)* und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigte z. B. die folgende Verbindung der Herstellungsbeispiele Wirkung von 90 % bei einer Aufwandmenge von 20 ppm: 63

**Myzus persicae - Sprühtest**

| | | |
|---|---|---|
| Lösungsmittel: | 78 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 100 % bei einer Aufwandmenge von 500 g/ha: 1, 2, 3, 4, 5, 7, 9, 15, 20, 23, 34, 35, 39, 40, 43, 45, 48, 49, 50, 51, 54, 57, 60, 61, 64, 66, 70, 77, 83, 84

Bei diesem Test zeigten z. B. die folgenden Verbindungen der Herstellungsbeispiele Wirkung von 90 % bei einer Aufwandmenge von 500 g/ha: 6, 8, 12, 13, 14, 16, 17, 18, 21, 22, 24, 25, 26, 27, 36, 37, 41, 46, 47, 52, 53, 55, 56, 58, 59, 62, 63, 67, 71, 72, 73, 74, 76, 78, 80, 81, 86

Bei diesem Test zeigten z. B. die folgenden Verbindungen der Herstellungsbeispiele Wirkung von 90 % bei einer Aufwandmenge von 100 g/ha: 19, 79

**Phaedon cochleariae - Sprühtest**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 100 % bei einer Aufwandmenge von 500 g/ha: 11, 37, 62

**Spodoptera frugiperda - Sprühtest**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigten z. B. die folgenden Verbindungen der Herstellungsbeispiele Wirkung von 100 % bei einer Aufwandmenge von 500 g/ha: 11, 37

**Tetranychus urticae - Sprühtest, OP-resistent**

| | | |
|---|---|---|
| Lösungsmittel: | 78,0 | GewichtsteileAceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 90 % bei einer Aufwandmenge von 500 g/ha: 11, 57

**Myzus persicae - Sprühtest**

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigte z. B. die folgende Verbindung der Herstellungsbeispiele Wirkung von 100 % bei einer Aufwandmenge von 4 ppm: 44

Bei diesem Test zeigte z. B. die folgende Verbindung der Herstellungsbeispiele Wirkung von 96 % bei einer Aufwandmenge von 4 ppm: 10

## Patentansprüche

1. Verbindungen der Formel (I) 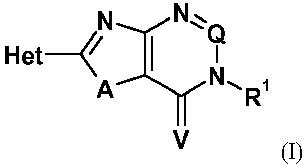 in welcher
Het für einen Rest aus der Reihe 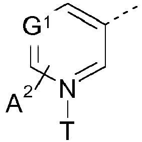 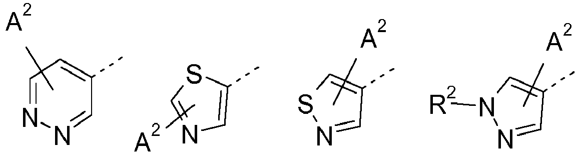 steht, worin die gestrichelte Linie die Bindung zum Kohlenstoffatom in der an Het gebundenen N=C-A-Gruppe darstellt und
G¹ für N oder C-A¹ steht,
A¹ für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
A² für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
T für Sauerstoff oder ein Elektronenpaar steht,
R² für Wasserstoff, Alkyl, Halogenalkyl oder gegebenenfalls substituiertes Cycloalkyl steht,
R¹ für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylsulfanylalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, Dialkylaminosulfanylalkyl, Dialkylaminosulfinylalkyl, Dialkylaminosulfonylalkyl, gegebenenfalls durch Halogen substituiertes Alkoxycarbonyl, gegebenenfalls durch Halogen substituiertes Alkoxycarbonylalkyl, gegebenenfalls durch Halogen substituiertes Alkinyloxy, gegebenenfalls durch Halogen substituiertes Alkinyloxycarbonyl, Dialkylaminocarbonyl, N-Alkyl-N-Cycloalkylaminocarbonyl, Dialkylaminocarbonylalkyl, N-Alkyl-N-Cycloalkylaminocarbonylalkyl, Heterocyclylcarbonylalkyl, Alkylsulfanyl, Halogenalkylsulfanyl, Alkylsulfinyl, Halogenalkylsulfinyl, Alkylsulfonyl, Halogenalkylsulfonyl, gegebenenfalls durch Halogen, Cyano, Nitro, Alkyl, Cycloalkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Nitro, Alkyl, Halogenalkyl, Cycloalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylcarbonyl, gegebenenfalls durch Halogen, Cyano, Nitro, Alkyl, Halogenalkyl, Cycloalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Hydroxy, Alkyl, Halogenalkyl, Cycloalkyl (welches gegebenenfalls substituiert ist), Alkoxy, Halogenalkoxy, Alkylthio, Halogenalkylthio, Alkylsulfinyl, Alkylsulfonyl, Halogenalkylsulfinyl, Halogenalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Hydroxy, Amino, Alkyl, Halogenalkyl, Cycloalkyl (welches gegebenenfalls substituiert ist) Alkoxy oder Halogenalkoxy substituiertes Aryl, gegebenenfalls durch Halogen, Cyano (auch im Alkylteil), Nitro, Hydroxy, Amino, Alkyl, Cycloalkyl (welches gegebenenfalls substituiert ist), Halogenalkyl, Alkoxy oder Halogenalkoxy substituiertes Arylalkyl, gegebenenfalls durch Halogen, Cyano, Nitro, Hydroxy, Alkyl, Halogenalkyl, Cycloalkyl (welches gegebenenfalls substituiert ist), Alkoxy, Halogenalkoxy, Alkylthio, Halogenalkylthio, Alkylsulfinyl, Alkylsulfonyl, Halogenalkylsulfinyl, Halogenalkylsulfonyl, Amino, Alkylamino, Dialkylamino, Alkylaminocarbonyl, Dialkylaminocarbonyl, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Hydroxy, Alkyl, Halogenalkyl, Cycloalkyl (welches gegebenenfalls substituiert ist), Alkoxy, Halogenalkoxy, Alkylthio, Halogenalkylthio, Alkylsulfinyl, Alkylsulfonyl, Halogenalkylsulfinyl, Halogenalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
R¹ auch für einen Rest aus der Reihe 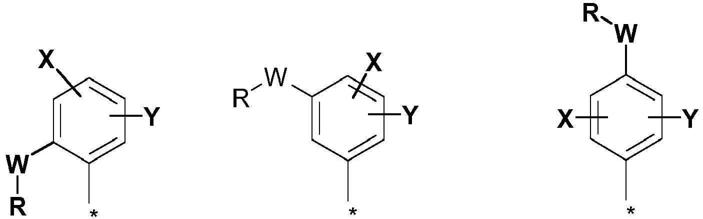 steht,
worin die Bindung zum Stickstoffatom in der C(=V)-N-Q-Gruppe in der Formel (I) mit dem Stern (*) markiert ist,
R für NR⁷R⁸ oder für einen jeweils gegebenenfalls substituierten Rest aus der Reihe Alkyl, Alkenyl, Alkinyl, Alkoxyalkyl, Alkyl-S(O)ₘ-alkyl, R⁷-CO-alkyl, NR⁷R⁸-CO-alkyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
W für einen Rest aus der Reihe O, S, SO und SO₂ steht,
X für einen Rest aus der Reihe Wasserstoff, Halogen, Cyano, Nitro, Alkyl, Halogenalkyl, Alkoxy, Halogenalkoxy und Cycloalkyl steht,
Y für einen Rest aus der Reihe Wasserstoff, Halogen, Alkyl, Cycloalkyl, Halogenalkyl, Alkoxy, Halogenalkoxy, Cyano und NR⁵R⁶ steht,
A für Sauerstoff oder Schwefel steht,
Q für Stickstoff oder C-R³ steht, worin
R³ für einen Rest aus der Reihe Wasserstoff, Alkyl, Cycloalkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
V für einen Rest aus der Reihe Sauerstoff, Schwefel und NR⁴ steht,
R⁴ für einen Rest aus der Reihe Wasserstoff, Cyano, Alkyl, Halogenalkyl und Cycloalkyl steht,
R⁵ für einen Rest aus der Reihe Wasserstoff, Alkyl, Cycloalkyl und Halogenalkyl steht,
R⁶ für einen Rest aus der Reihe Wasserstoff, Alkyl, Cycloalkyl und Halogenalkyl steht,
oder
R⁵ und R⁶ gemeinsam mit dem Stickstoff, an dem sie gebunden sind, für einen gegebenenfalls substituierten und gegebenenfalls ein oder mehrere weitere Heteroatome enthaltenden gesättigten oder ungesättigten 3- bis 6-gliedrigen Ring stehen,
R⁷ für Wasserstoff, Hydroxy oder einen jeweils gegebenenfalls substituierten Rest aus der Reihe Alkyl, Alkoxy, Alkoxyalkyl, Alkyl-S(O)ₘ-alkyl, Alkylcarbonyl, Alkoxycarbonyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
R⁸ für Wasserstoff, ein Metallion, ein gegebenenfalls substituiertes Ammoniumion oder einen jeweils gegebenenfalls substituierten Rest aus der Reihe Alkyl, Alkoxy, Alkoxyalkyl, Alkyl-S(O)ₘ-alkyl steht und
m für eine Zahl aus der Reihe 0, 1 und 2 steht.

2. Verbindungen der Formel (I) gemäß Anspruch 1, worin
Het für einen Rest aus der Reihe 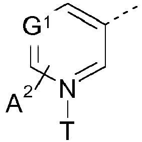 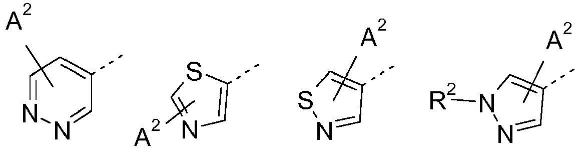 steht , worin die gestrichelte Linie die Bindung zum Kohlenstoffatom in der an Het gebundenen N=C-A-Gruppe darstellt,
G¹ für N oder C-A¹ steht,
A¹ für einen Rest aus der Reihe Wasserstoff, Halogen, C₁-C₆-Alkyl und C₁-C₄-Halogenalkyl steht,
A² für einen Rest aus der Reihe Wasserstoff, Halogen, C₁-C₆-Alkyl und C₁-C₄-Halogenalkyl steht,
T für Sauerstoff oder ein Elektronenpaar steht,
R² für einen Rest aus der Reihe Wasserstoff, C₁-C₆-Alkyl, C₁-C₆-Halogenalkyl und gegebenenfalls einfach bis dreifach durch Halogen, Cyano, Nitro, C₁-C₃-Alkyl, C₁-C₃-Alkoxy und einfach durch C₃-C₆-Cycloalkyl substituiertes C₃-C₆-Cycloalkyl steht,
R¹ für einen Rest aus der Reihe Wasserstoff, C₁-C₆-Alkyl, C₂-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylsulfanyl-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, Di-(C₁-C₆-alkyl)-aminosulfanyl-C₁-C₆-alkyl, Di-(C₁-C₆)-alkylanonosulfinyl-C₁-C₆-alkyl, Di-(C₁-C₆-alkyl)-aminosulfonyl-C₁-C₆-alkyl, gegebenenfalls durch Halogen substituiertes C₁-C₆-Alkoxycarbonyl, gegebenenfalls durch Halogen substituiertes C₁-C₆-Alkoxycarbonyl-C₁-C₆-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₆-alkyl)-aminocarbonyl, N-C₁-C₆-Alkyl-N-C₃-C₆-cycloalkylaminocarbonyl, Di-(C₁-C₆-alkyl)-aminocarbonyl-C₁-C₆-alkyl, N-C₁-C₆-Alkyl-N-C₃-C₆-cycloalkylaminocarbonyl-C₁-C₆-alkyl, Heterocyclylcarbonyl-C₁-C₆-alkyl, C₁-C₆-Alkylsulfanyl, C₁-C₆-Halogenalkylsulfanyl, C₁-C₆-Alkylsulfinyl, C₁-C₆-Halogenalkylsulfinyl, C₁-C₆-Alkylsulfonyl, C₁-C₆-Halogenalkylsulfonyl, gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, gegebenenfalls durch Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, gegebenenfalls durch Halogen, Cyano (auch im C₁-C₆-Alkylteil von Heterocyclyl-C₁-C₆-alkyl), Nitro, Hydroxy, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Alkylsulfonyl, C₁-C₆-Halogenalkylsulfinyl, C₁-C₆-Halogenalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Heterocyclyl-C₁-C₆-alkyl, gegebenenfalls durch Halogen, Cyano, Nitro, Hydroxy, Amino, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy oder C₁-C₆-Halogenalkoxy substituiertes Aryl, gegebenenfalls durch Halogen, Cyano (auch im C₁-C₆-Alkylteil von Aryl-C₁-C₆-alkyl), Nitro, Hydroxy, Amino, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy oder C₁-C₆-Halogenalkoxy substituiertes Aryl-C₁-C₆-alkyl, gegebenenfalls durch Halogen, Cyano, Nitro, Hydroxy, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Alkylsulfonyl, C₁-C₆-Halogenalkylsulfinyl, C₁-C₆-Halogenalkylsulfonyl, Amino, C₁-C₆-Alkylamino, Di-(C₁-C₆-alkyl)-amino, C₁-C₆-Alkylcarbonylamino, C₁-C₆-Alkylaminocarbonyl, Di-(C₁-C₆-alkyl)-aminocarbonyl, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl, gegebenenfalls durch Halogen, Cyano (auch im C₁-C₆-Alkylteil von Hetaryl-C₁-C₆-alkyl), Nitro, Hydroxy, C₁-C₆-Alkyl, C₁-C₆-Halogenalkyl, C₃-C₆-Cycloalkyl (welches gegebenenfalls durch Halogen, Cyano, C₁-C₆-Alkyl und C₃-C₆-Cycloalkyl substituiert ist), C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Alkylsulfonyl, C₁-C₆-Halogenalkylsulfinyl, C₁-C₆-Halogenalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl-C₁-C₆-alkyl steht,
R¹ auch für einen Rest aus der Reihe 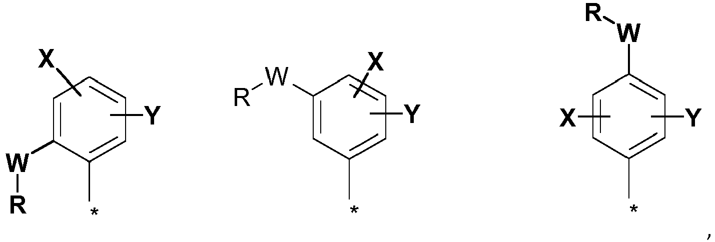 steht , worin die Bindung zum Stickstoffatom in der C(=V)-N-Q-Gruppe in der Formel (I) mit dem Stern (*) markiert ist,
R für NR⁷R⁸ oder für jeweils gegebenenfalls durch Halogen, Sauerstoff (führt zu C=O) oder Cyano substituiertes C₁-C₆-Alkyl, C₃-C₆-Alkenyl, C₃-C₆-Alkinyl, C₁-C₆-Alkoxy-C₁-C₄-alkyl, C₁-C₆-Alkyl-S(O)ₘ-C₁-C₄-alkyl, für R⁷-CO-C₁-C₄-alkyl, für NR⁷R⁸-CO-C₁-C₄-alkyl, für gegebenenfalls einfach oder zweifach durch Sauerstoff (führt zu C=O), C₁-C₄-Alkyl, C₃-C₈-Cycloalkyl, C₁-C₄-Alkoxy und C₁-C₄-Halogenalkyl substituiertes C₃-C₆-Cycloalkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkenyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₃-C₆-Cycloalkyl, C₁-C₄-Halogenalkyl, C₁-C₄-Alkoxy oder C₁-C₄-Halogenalkoxy substituiertes Phenyl, Phenyl-C₁-C₄-alkyl, Hetaryl und Hetaryl-C₁-C₄-alkyl steht,
W für einen Rest aus der Reihe O, S, SO und SO₂ steht,
X für einen Rest aus der Reihe Wasserstoff, Halogen, Cyano, Nitro, C₁-C₆-Alkyl, C₁-C₆-Halogenalkyl, C₃-C₆-Cycloalkyl, C₁-C₆-Alkoxy und C₁-C₆-Halogenalkoxy steht,
Y für aus der Reihe Wasserstoff, Halogen, C₁-C₆-Alkyl, C₃-C₆-Cycloalkyl, C₁-C₆-Halogenalkyl, C₁-C₆-Alkoxy, C₁-C₆-Halogenalkoxy, Cyano und NR⁵R⁶ steht,
A für Schwefel steht,
Q für Stickstoff oder C-R³ steht,
R³ für einen Rest aus der Reihe Wasserstoff, C₁-C₆-Alkyl, C₁-C₆-Halogenalkyl, C₃-C₆-Cycloalkyl, OH, C₁-C₆-Alkoxy, C₁-C₆-Halogenalkoxy, SH, C₁-C₆-Alkylsulfanyl, C₁-C₆-Alkylsulfinyl, C₁-C₆-Alkylsulfonyl, NH₂, C₁-C₆-Alkylamino und Di-(C₁-C₆-alkyl)-amino steht,
V für einen Rest aus der Reihe Sauerstoff, Schwefel und NR⁴ steht,
R⁴ für einen Rest aus der Reihe Wasserstoff, Cyano, C₁-C₆-Alkyl, C₂-C₆-Halogenalkyl und C₃-C₆-Cycloalkyl steht,
R⁵ für einen Rest aus der Reihe Wasserstoff, C₁-C₆-Alkyl, C₃-C₆₋Cycloalkyl und C₂-C₆-Halogenalkyl steht,
R⁶ für einen Rest aus der Reihe Wasserstoff, C₁-C₆-Alkyl, C₃-C₆₋Cycloalkyl und C₂-C₆-Halogenalkyl steht,
R⁵ und R⁶ auch gemeinsam mit dem Stickstoffatom, an das sie gebunden sind, für einen gegebenenfalls durch Halogen, Cyano, C₁-C₄-Alkyl, C₁-C₄-Halogenalkyl, C₁-C₄-Alkoxy, C₁-C₄-Halogenalkoxy substituierten gesättigten bis dreifach ungesättigten 3- bis 6-gliedrigen Ring stehen können,
R⁷ für einen Rest aus der Reihe Wasserstoff, Hydroxy, jeweils gegebenenfalls einfach oder mehrfach durch Halogen oder einfach oder zweifach durch Cyano substituiertes C₁-C₆-Alkyl, C₁-C₆-Alkoxy, C₁-C₆-Alkoxy-C₁-C₄-alkyl, C₁-C₆-Alkyl-S(O)ₘ-C₁-C₄-alkyl, C₁-C₆-Alkylcarbonyl, C₁-C₆-Alkoxycarbonyl, C₃-C₆-Cycloalkyl, C₃-C₆-Cycloalkenyl, C₃-C₆-Cycloalkyl-C₁-C₃-alkyl, C₃-C₆-Cycloalkenyl-C₁-C₃-alkyl, Heterocyclyl, Heterocyclyl-C₁-C₃-alkyl und jeweils gegebenenfalls einfach bis vierfach durch C₁-C₄-Alkyl, C₁-C₄-Halogenalkyl, C₁-C₄-Alkoxy, C₁-C₄-Halogenalkoxy, C₃-C₆-Cycloalkyl, Halogen oder Cyano substituiertes Phenyl, Phenyl-C₁-C₃-alkyl, Hetaryl und Hetaryl-C₁-C₃-alkyl steht,
R⁸ für Wasserstoff, ein Alkali- oder Erdalkalimetallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₄-alkyl und C₁-C₄-Alkyl-S(O)ₘ-C₁-C₄-alkyl steht,
m für eine Zahl aus der Reihe 0, 1 und 2 steht und worin
Alkaliion ausgewählt ist aus der Reihe Lithium, Natrium, Kalium, Rubidium, Cäsium,
Erdalkaliion ausgewählt ist aus der Reihe Beryllium, Magnesium, Calcium, Strontium, Barium,
Halogen ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl, Phenanthrenyl,
Hetaryl (gleichbedeutend mit Heteroaryl, auch als Teil einer größeren Einheit, wie beispielsweise Hetarylalkyl) ausgewählt ist aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl und
Heterocyclyl ein gesättigter 4-, 5- oder 6-Ring ist, der 1 oder 2 Stickstoffatome und/oder ein Sauerstoffatom und/oder ein Schwefelatom enthält.

3. Verbindungen der Formel (I) gemäß Anspruch 1, worin
Het für einen Rest aus der Reihe 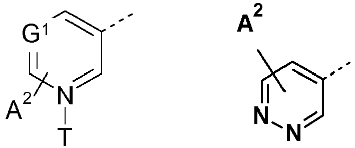 steht, worin die gestrichelte Linie die Bindung zum Kohlenstoffatom in der an Het gebundenen N=C-A-Gruppe darstellt,
G¹ für N oder C-A¹ steht,
A¹ für einen Rest aus der Reihe Wasserstoff, Halogen, C₁-C₄-Alkyl und C₁-C₄-Halogenalkyl steht,
A² für einen Rest aus der Reihe Wasserstoff, Halogen, C₁-C₄-Alkyl und C₁-C₄-Halogenalkyl steht,
T für Sauerstoff oder ein Elektronenpaar steht,
R¹ für einen Rest aus der Reihe Wasserstoff, C₁-C₄-Alkyl, C₂-C₄-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylsulfanyl-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, Di-(C₁-C₄-alkyl)-aminosulfanyl-C₁-C₄-alkyl, Di-(C₁-C₄-alkyl)-aminosulfinyl-C₁-C₄-alkyl, Di-(C₁-C₄-alkyl)-aminosulfonyl-C₁-C₄-alkyl, gegebenenfalls durch Halogen substituiertes C₁-C₄-Alkoxycarbonyl, gegebenenfalls durch Halogen substituiertes C₁-C₄-Alkoxycarbonyl-C₁-C₄-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₄-alkyl)-aminocarbonyl, N-C₁-C₄-Alkyl-N-C₃-C₆-cycloalkylaminocarbonyl, Di-(C₁-C₄-alkyl)-aminocarbonyl-C₁-C₄-alkyl, N-C₁-C₄-Alkyl-N-C₃-C₆-cycloalkylaminocarbonyl-C₁-C₄-alkyl, Heterocyclylcarbonyl-C₁-C₄-alkyl, C₁-C₄-Alkylsulfanyl, C₁-C₄-Halogenalkylsulfanyl, C₁-C₄-Alkylsulfinyl, C₁-C₄-Halogenalkylsulfinyl, C₁-C₄-Alkylsulfonyl, C₁-C₄-Halogenalkylsulfonyl, gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, gegebenenfalls durch Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkoxycarbonyl, C₁-C₄-Halogenalkoxycarbonyl, Pyridyl, Pyrimidyl, Pyrazanyl, Pyridazinyl, Thiazolyl, Isothiazolyl, Thiadiazolyl, Oxazolyl, Isoxazolyl, Oxadiazolyl, Pyrazolyl, Triazinyl oder Triazolyl (wobei die genannten Hetaryle gegebenenfalls selbst durch C₁-C₄-Alkyl oder Halogen substituiert sind) substituiertes C₃-C₆-Cycloalkyl-C₁-C₄-alkyl, gegebenenfalls durch Halogen, Cyano (auch im C₁-C₄-Alkylteil von Heterocyclyl-C₁-C₄-alkyl), Nitro, Hydroxy, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Heterocyclyl-C₁-C₄-alkyl, gegebenenfalls durch Halogen, Cyano, Nitro, Hydroxy, Amino, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy oder C₁-C₄-Halogenalkoxy substituiertes Aryl, gegebenenfalls durch Halogen, Cyano (auch im C₁-C₄-Alkylteil von Aryl-C₁-C₄-alkyl), Nitro, Hydroxy, Amino, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy oder C₁-C₄-Halogenalkoxy substituiertes Aryl-C₁-C₄-alkyl, gegebenenfalls durch Halogen, Cyano, Nitro, Hydroxy, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkylaminocarbonyl, Di-(C₁-C₄-alkyl)-aminocarbonyl, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl, gegebenenfalls durch Halogen, Cyano (auch im C₁-C₄-Alkylteil von Hetaryl-C₁-C₄-alkyl), Nitro, Hydroxy, C₁-C₄-Alkyl, C₁-C₄-Halogenalkyl, C₃-C₆-Cycloalkyl (welches gegebenenfalls durch Halogen, Cyano, C₁-C₄-Alkyl und C₃-C₆-Cycloalkyl substituiert ist), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl steht,
R¹ auch für einen Rest aus der Reihe 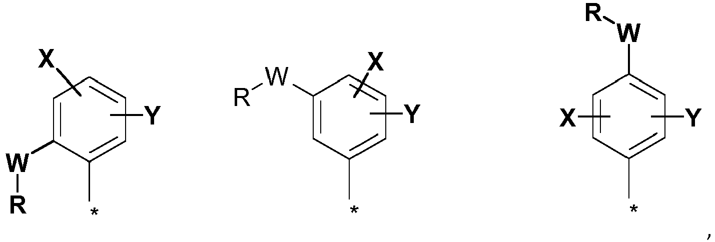 steht , worin die Bindung zum Stickstoffatom in der C(=V)-N-Q-Gruppe in der Formel (I) mit dem Stern (*) markiert ist,
R für NR⁷R⁸ oder für jeweils gegebenenfalls einfach bis siebenfach durch Halogen, einfach oder zweifach durch Sauerstoff (führt zu C=O) oder einfach oder zweifach durch Cyano substituiertes C₁-C₆-Alkyl, C₃-C₆-Alkenyl, C₃-C₆-Alkinyl, C₁-C₄-Alkoxy-C₁-C₃-alkyl, C₁-C₄-Alkyl-S(O)ₘ-C₁-C₃-alkyl, für R⁷-CO-C₁-C₂-alkyl, für NR⁷R⁸-CO-C₁-C₂-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₈-Cycloalkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes C₃-C₆-Cycloalkenyl-C₁-C₄-alkyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₃-C₆-Cycloalkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₁-C₄-Halogenalkyl, C₃-C₆-Cycloalkyl, C₁-C₄-Alkoxy oder C₁-C₄-Halogenalkoxy substituiertes Phenyl, Phenyl-C₁-C₄-alkyl, Hetaryl, und Hetaryl-C₁-C₄-alkyl steht,
W für einen Rest aus der Reihe S, SO und SO₂ steht,
X für einen Rest aus der Reihe Wasserstoff, Fluor, Chlor, Brom, Jod, Cyano, Nitro, C₁-C₄-Alkyl, C₁-C₄-Halogenalkyl, C₃-C₆-Cycloalkyl, C₁-C₄-Alkoxy und C₁-C₄-Halogenalkoxy steht,
Y für einen Rest aus der Reihe Wasserstoff, Fluor, Chlor, Brom, Jod, Cyano, C₁-C₄-Alkyl, C₁-C₄-Halogenalkyl, C₃-C₆-Cycloalkyl, C₁-C₄-Alkoxy und C₁-C₄-Halogenalkoxy steht,
A für Schwefel steht,
Q für Stickstoff oder C-R³ steht,
R³ für einen Rest aus der Reihe Wasserstoff, C₁-C₄-Alkyl, C₁-C₄-Halogenalkyl, C₃-C₆-Cycloalkyl, OH, C₁-C₄-Alkoxy, C₁-C₄-Halogenalkoxy, SH, C₁-C₄-Alkylsulfanyl, C₁-C₄-Alkylsulfinyl, C₁-C₄-Alkylsulfonyl, NH₂, C₁-C₄-Alkylamino und Di-(C₁-C₄-alkyl)-amino steht,
V für Sauerstoff steht,
R⁷ für einen Rest aus der Reihe Wasserstoff, Hydroxy, jeweils gegebenenfalls einfach oder mehrfach durch Halogen oder einfach oder zweifach durch Cyano substituiertes C₁-C₄-Alkyl, C₁-C₄-Alkoxy, C₁-C₄-Alkoxy-C₁-C₄-alkyl, C₁-C₄-Alkyl-S(O)ₘ-C₁-C₃-alkyl, C₁-C₄-Alkylcarbonyl, C₁-C₄-Alkoxycarbonyl, C₃-C₆-Cycloalkyl, C₃-C₆-Cycloalkyl-C₁-C₃-alkyl, Heterocyclyl, Heterocyclyl-C₁-C₃-alkyl und jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor Chlor, Brom oder Cyano substituiertes Phenyl, Benzyl, Pyridyl, Pyrimidyl, Thiazolyl, Oxazolyl, Pyrazolyl, Thienyl, Furanyl, Pyridinylmethyl und Thiazolylmethyl steht,
R⁸ für Wasserstoff, für ein Alkali- oder Erdalkalimetallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl steht,
m für eine Zahl aus der Reihe 0, 1 und 2 steht, und worin
Alkaliion ausgewählt ist aus der Reihe Lithium, Natrium, Kalium, Rubidium, Cäsium,
Erdalkaliion ausgewählt ist aus der Reihe Beryllium, Magnesium, Calcium, Strontium, Barium,
Halogen ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl und Phenanthrenyl,
Hetaryl (gleichbedeutend mit Heteroaryl, auch als Teil einer größeren Einheit, wie beispielsweise Hetarylalkyl) ausgewählt ist aus der Reihe Pyrazolyl, Imidazolyl, 1,2,3-Triazolyl, 1,2,4-Triazolyl, Oxazolyl, Isoxazolyl, Thiazolyl, Isothiazolyl, Pyridyl, Pyrimidinyl, Pyridazinyl, Pyrazinyl, 1,2,3-Triazinyl, 1,2,4-Triazinyl, 1,3,5-Triazinyl, Benzothienyl und
Heterocyclyl ausgewählt ist aus der Reihe Azetidinyl, Azolidinyl, Azinanyl, Oxetanyl, Oxolanyl, Oxanyl, Dioxanyl, Thiethanyl, Thiolanyl, Thianyl, Tetrahydrofuryl, Piperazinyl, Morpholinyl.

4. Verbindungen der Formel (I) gemäß Anspruch 1, worin
Het für einen Rest aus der Reihe 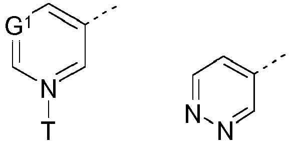 steht, worin die gestrichelte Linie die Bindung zum Kohlenstoffatom in der an Het gebundenen N=C-A-Gruppe darstellt,
G¹ für N oder C-A¹ steht,
A¹ für einen Rest aus der Reihe Wasserstoff, Fluor, Chlor steht,
T für Sauerstoff oder ein Elektronenpaar steht,
R¹ für einen Rest aus der Reihe Methyl, Ethyl, Isopropyl, n-Propyl, n-Butyl, iso-Butyl, sec-Butyl, *tert-Butyl,* 2,2,2-Trifluorethyl, 2,2-Difluorethyl, 2,2-Difluor-n-propyl, Methylsulfanyl-methyl, Methylsulfanylethyl, Methylsulfanyl-n-propyl, Trifluormethylsulfonylmethyl, Ethylsulfonylmethyl, 2,2,2-Trifluorethylsulfonylmethyl, 2,2-Difluorethylsulfonylmethyl, Isopropylsulfanylmethyl, Methylsulfmylmethyl, Trifluormethylsulfmylmethyl, Ethylsulfinylmethyl, 2,2,2-Trifluorethylsulfmylmethyl, 2,2-Difluorethylsulfinylmethyl, Isopropylsulfinylmethyl, Methylsulfonylmethyl, Trifluormethylsulfonylmethyl, Ethylsulfonylmethyl, 2,2,2-Trifluorethylsulfonylmethyl, 2,2-Difluorethylsulfonylmethyl, Isopropylsulfonylmethyl, Methoxycarbonyl, Ethoxycarbonyl, Methoxycarbonylmethyl, Ethoxycarbonylmethyl, Methoxycarbonylethyl, Ethoxycarbonylethyl, Dimethylaminocarbonyl, Diethylaminocarbonyl, *N-*Ethyl-*N*-methylaminocarbonyl, *N*-Isopropyl-*N*-methylaminocarbonyl, Dimethylaminocarbonylmethyl, Diethylaminocarbonylmethyl, *N*-Ethyl-*N*-methylaminocarbonylmethyl, *N-*Isopropyl-*N*-methylaminocarbonylmethyl, Dimethylaminocarbonylethyl, Diethylaminocarbonylethyl, *N*-Ethyl-*N*-methylaminocarbonylethyl, *N*-Isopropyl-*N*-methylaminocarbonylethyl, *N*-Cyclopropyl-*N*-methylaminocarbonylmethyl, *N*-Cyclopropyl-*N*-methylaminocarbonylethyl, Methylsulfanyl, Trifluormethylsulfanyl, Ethylsulfanyl, 2,2,2-Trifluorethylsulfanyl, 2,2-Difluorethylsulfanyl, Isopropylsulfanyl, Methylsulfinyl, Trifluormethylsulfinyl, Ethylsulfinyl, 2,2,2-Trifluorethylsulfinyl, 2,2-Difluorethylsulfinyl, Isopropylsulfinyl, Methylsulfonyl, Trifluormethylsulfonyl, Ethylsulfonyl, 2,2,2-Trifluorethylsulfonyl, 2,2-Difluorethylsulfonyl, Isopropylsulfonyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, 2-Cyclopropylcyclopropyl, Cyclobutyl, Cyclopentyl, Cyclohexyl, 4-Trifluormethylcyclohexyl, Cyclopropylmethyl, Cyclopropylethyl, Cyclobutylmethyl, *N*-Cyclopropyl-*N-*methylaminocarbonyl, Morpholin-4-ylcarbonylmethyl, Piperazin-1-ylcarbonylmethyl, 4-Methylpiperazin-1-ylcarbonylmethyl, jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Heterocyclylmethyl und Heterocyclylethyl, jeweils durch Cyclopropyl substituiertes Heterocyclylmethyl und Heterocyclylethyl, wobei der Cyclopropylrest gegebenenfalls einfach oder zweifach durch Methyl, Fluor, Chlor, Cyano oder einfach durch Cyclopropyl substituiert ist, gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Dimethylaminocarbonyl, substituiertes Hetaryl, durch Cyclopropyl substituiertes Hetaryl, wobei der Cyclopropylrest gegebenenfalls einfach oder zweifach durch Methyl, Fluor, Chlor, Cyano oder einfach durch Cyclopropyl substituiert ist, gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Trifluorethoxy substituiertes Aryl, durch Cyclopropyl substituiertes Aryl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Arylmethyl und Arylethyl, jeweils durch Cyclopropyl substituiertes Arylmethyl und Arylethyl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl, jeweils durch Cyclopropyl substituiertes Hetarylmethyl und Hetarylethyl steht, wobei der Cyclopropylrest gegebenenfalls einfach oder zweifach durch Methyl, Fluor, Chlor, Cyano oder einfach durch Cyclopropyl substituiert ist,
R¹ auch für einen Rest aus der Reihe 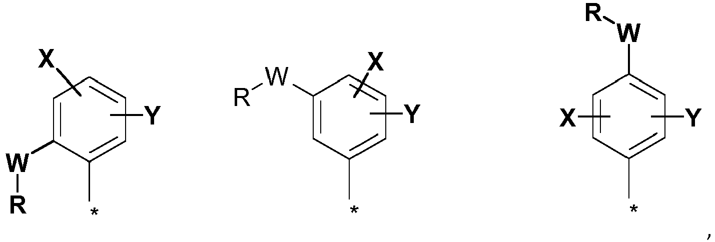 steht, worin die Bindung zum Stickstoffatom in der C(=V)-N-Q-Gruppe in der Formel (I) mit dem Stern (*) markiert ist,
R für NR⁷R⁸ oder für einen jeweils gegebenenfalls einfach, zweifach, dreifach, vierfach oder fünffach durch Fluor, Chlor oder einfach oder zweifach durch Cyano substituierten Rest aus der Reihe C₁-C₄-Alkyl, C₃-C₄-Alkenyl, C₃-C₄-Alkinyl, C₁-C₂-Alkoxy-C₁-C₂-alkyl und C₁-C₂-Alkyl-S(O)ₘ-C₁-C₂-alkyl, für R⁷-CO-C₁-C₂-alkyl, für NR⁷R⁸-CO-C₁-C₂-alkyl, für gegebenenfalls einfach oder zweifach durch C₁-C₂-Alkyl, C₁-C₂-Alkoxy oder C₁-C₂-Halogenalkyl oder durch ein Sauerstoffatom (führt zu C=O) substituiertes C₃-C₆-Cycloalkyl, für gegebenenfalls einfach oder zweifach durch C₁-C₂-Alkyl, C₁-C₂-Alkoxy oder C₁-C₂-Halogenalkyl oder durch ein Sauerstoffatom (führt zu C=O) substituiertes C₃-C₆-Cycloalkenyl, für gegebenenfalls einfach bis zweifach durch C₁-C₂-Alkyl, C₁-C₂-Alkoxy oder C₁-C₂-Halogenalkyl substituiertes C₃-C₆-Cycloalkyl-C₁-C₂-alkyl, für gegebenenfalls einfach oder zweifach durch C₁-C₂-Alkyl, C₁-C₂-Alkoxy oder C₁-C₂-Halogenalkyl substituiertes C₃-C₆-Cycloalkenyl-C₁-C₂-alkyl, für gegebenenfalls einfach oder zweifach durch C₁-C₂-Alkyl, C₁-C₂-Alkoxy oder C₁-C₂-Halogenalkyl substituiertes Heterocyclyl, für gegebenenfalls einfach oder zweifach durch C₁-C₂-Alkyl, C₁-C₂-Alkoxy oder C₁-C₂-Halogenalkyl substituiertes Heterocyclyl-C₁-C₂-alkyl oder für jeweils gegebenenfalls einfach oder zweifach durch Fluor, Chlor, Brom, Cyano, Methyl, Ethyl, Difluormethyl, Trifluormethyl, Methoxy, Ethoxy, Difluormethoxy oder Trifluormethoxy substituiertes Phenyl, Benzyl, Pyridyl, Pyrimidyl, Thiazolyl, Oxazolyl, Pyrazolyl, Thienyl, Furanyl, Pyridinylmethyl oder Thiazolylmethyl steht,
W für einen Rest aus der Reihe S, SO und SO₂ steht,
X für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy steht,
Y für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy steht,
A für Schwefel steht,
Q für Stickstoff oder C-R³ steht,
R³ für Wasserstoff steht,
V für Sauerstoff steht,
R⁷ für einen Rest aus der Reihe Wasserstoff, Hydroxy, für jeweils gegebenenfalls einfach, zweifach, dreifach, vierfach oder fünffach durch Fluor, Chlor oder einfach oder zweifach durch Cyano substituiertes C₁-C₄-Alkyl, C₁-C₄-Alkoxy, C₁-C₄-Alkoxy-C₁-C₄-alkyl, C₁-C₄-Alkyl-S(O)ₘ-C₁-C₂-alkyl, C₁-C₄-Alkylcarbonyl, C₁-C₄-Alkoxycarbonyl, C₃-C₆-Cycloalkyl, C₃-C₆-Cycloalkyl-C₁-C₃-alkyl, Heterocyclyl und Heterocyclyl-C₁-C₃-alkyl und jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor, Chlor, Brom oder Cyano substituiertes Phenyl, Benzyl, Pyridyl, Pyrimidyl, Thiazolyl, Oxazolyl, Pyrazolyl, Thienyl, Furanyl, Pyridinylmethyl und Thiazolylmethyl steht,
R⁸ für Wasserstoff, ein Alkali- oder Erdalkaliion, für ein gegebenenfalls einfach bis vierfach durch C₁-C₄-Alkyl substituiertes Ammoniumion oder für einen jeweils gegebenenfalls einfach, zweifach, dreifach, vierfach oder fünffach durch Fluor, Chlor oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl steht,
m für eine Zahl aus der Reihe 0, 1 und 2 und worin
Alkaliion für ein Ion aus der Reihe Lithium, Natrium, Kalium, Rubidium, Cäsium steht,
Erdalkaliion für ein Ion aus der Reihe Beryllium, Magnesium, Calcium, Strontium und Barium steht,
Halogen für Fluor, Chlor, Brom und Iod steht,
Heterocyclyl für einen Rest aus der Reihe Oxetanyl, Thiethanyl, Tetrahydrofuryl und Morpholinyl steht,
Aryl für Phenyl steht und
Hetaryl (gleichbedeutend mit Heteroaryl, auch als Teil einer größeren Einheit, wie beispielsweise Hetarylalkyl) für einen Rest aus der Reihe Pyridyl, Pyrimidyl, Pyrazinyl, Pyridazinyl, Thiazolyl, Pyrazolyl und Benzothienyl steht.

5. Verbindungen der Formel (I) gemäß Anspruch 1, worin
Het für einen Rest aus der Reihe 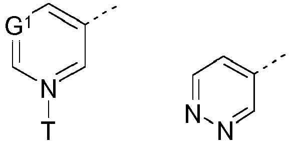 steht, worin die gestrichelte Linie die Bindung zum Kohlenstoffatom in der an Het gebundenen N=C-A-Gruppe darstellt,
G¹ für N oder C-A¹ steht,
A¹ für einen Rest aus der Reihe Wasserstoff, Fluor, Chlor steht,
T für Sauerstoff oder ein Elektronenpaar steht,
R¹ für einen Rest aus der Reihe Methyl, Ethyl, Isopropyl, n-Propyl, n-Butyl, iso-Butyl, sec-Butyl, *tert-*Butyl*,* 2,2,2-Trifluorethyl, 2,2-Difluorethyl, 2,2-Difluor-n-propyl, Methylsulfanyl-methyl, Methylsulfanylethyl, Methylsulfanyl-n-propyl, Trifluormethylsulfonylmethyl, Ethylsulfonylmethyl, 2,2,2-Trifluorethylsulfonylmethyl, 2,2-Difluorethylsulfonylmethyl, Isopropylsulfanylmethyl, Methylsulfmylmethyl, Trifluormethylsulfinylmethyl, Ethylsulfinylmethyl, 2,2,2-Trifluorethylsulfinylmethyl, 2,2-Difluorethylsulfinylmethyl, Isopropylsulfinylmethyl, Methylsulfonylmethyl, Trifluormethylsulfonylmethyl, Ethylsulfonylmethyl, 2,2,2-Trifluorethylsulfonylmethyl, 2,2-Difluorethylsulfonylmethyl, Isopropylsulfonylmethyl, Methoxycarbonyl, Ethoxycarbonyl, Methoxycarbonylmethyl, Ethoxycarbonylmethyl, Methoxycarbonylethyl, Ethoxycarbonylethyl, Dimethylaminocarbonyl, Diethylaminocarbonyl, *N-*Ethyl-*N*-methylaminocarbonyl, *N*-Isopropyl-*N*-methylaminocarbonyl, Dimethylaminocarbonylmethyl, Diethylaminocarbonylmethyl, *N*-Ethyl-*N*-methylaminocarbonylmethyl, *N*-Isopropyl-*N-*methylaminocarbonylmethyl, Dimethylaminocarbonylethyl, Diethylaminocarbonylethyl, *N*-Ethyl-*N*-methylaminocarbonylethyl, *N*-Isopropyl-*N*-methylaminocarbonylethyl, *N*-Cyclopropyl-*N-*methylaminocarbonylmethyl, *N*-Cyclopropyl-*N*-methylaminocarbonylethyl, Methylsulfanyl, Trifluormethylsulfanyl, Ethylsulfanyl, 2,2,2-Trifluorethylsulfanyl, 2,2-Difluorethylsulfanyl, Isopropylsulfanyl, Methylsulfinyl, Trifluormethylsulfinyl, Ethylsulfinyl, 2,2,2-Trifluorethylsulfinyl, 2,2-Difluorethylsulfinyl, Isopropylsulfinyl, Methylsulfonyl, Trifluormethylsulfonyl, Ethylsulfonyl, 2,2,2-Trifluorethylsulfonyl, 2,2-Difluorethylsulfonyl, Isopropylsulfonyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, 2-Cyclopropylcyclopropyl, Cyclobutyl, Cyclopentyl, Cyclohexyl, 4-Trifluormethylcyclohexyl, Cyclopropylmethyl, Cyclopropylethyl, Cyclobutylmethyl *N*-Cyclopropyl-*N-*methylaminocarbonyl, Morpholin-4-ylcarbonylmethyl, Piperazin-l-ylcarbonylmethyl, 4-Methylpiperazin-1-ylcarbonylmethyl,
jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Heterocyclylmethyl und Heterocyclylethyl, jeweils durch Cyclopropyl substituiertes Heterocyclylmethyl und Heterocyclylethyl, wobei der Cyclopropylrest gegebenenfalls einfach oder zweifach durch Methyl, Fluor, Chlor, Cyano oder einfach durch Cyclopropyl substituiert ist, gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Dimethylaminocarbonyl substituiertes Hetaryl, durch Cyclopropyl substituiertes Hetaryl, wobei der Cyclopropylrest gegebenenfalls einfach oder zweifach durch Methyl, Fluor, Chlor, Cyano oder einfach durch Cyclopropyl substituiert ist, gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy, Trifluorethoxy substituiertes Aryl, durch Cyclopropyl substituiertes Aryl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach, zweifach oder dreifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Arylmethyl und Arylethyl, jeweils durch Cyclopropyl substituiertes Arylmethyl und Arylethyl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl, jeweils durch Cyclopropyl substituiertes Hetarylmethyl und Hetarylethyl steht, wobei der Cyclopropylrest gegebenenfalls einfach oder zweifach durch Methyl, Fluor, Chlor, Cyano oder einfach durch Cyclopropyl substituiert ist,
R¹ auch für den Rest der Formel 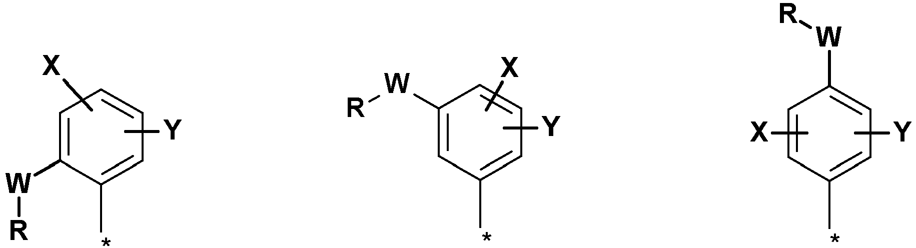 steht, worin die Bindung zum Stickstoffatom in der C(=V)-N-Q-Gruppe in der Formel (I) mit dem Stern (*) markiert ist
R für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Methyl, Ethyl, Methoxy, Ethoxy oder Trifluormethyl substituiertes C₃-C₆-Cycloalkyl, für gegebenenfalls einfach durch Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethlyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl steht,
W für einen Rest aus der Reihe S, SO und SO₂ steht,
X für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy steht,
Y für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy steht,
A für Schwefel steht,
Q für Stickstoff oder C-R³ steht,
R³ für Wasserstoff oder Methyl steht,
V für Sauerstoff steht und worin
Alkaliion für ein Ion aus der Reihe Lithium, Natrium, Kalium, Rubidium, Cäsium steht,
Erdalkaliion für ein Ion aus der Reihe Beryllium, Magnesium, Calcium, Strontium und Barium steht,
Halogen für Fluor, Chlor, Brom und Iod steht,
Heterocyclyl für einen Rest aus der Reihe Oxetanyl, Thiethanyl, Tetrahydrofuryl und Morpholinyl steht,
Aryl für Phenyl steht und
Hetaryl (gleichbedeutend mit Heteroaryl, auch als Teil einer größeren Einheit, wie beispielsweise Hetarylalkyl) für einen Rest aus der Reihe Pyridyl, Pyrimidyl, Pyrazinyl, Pyridazinyl, Thiazolyl, Pyrazolyl und Benzothienyl steht.

6. Verbindungen der Formel (I) gemäß Anspruch 1, in welcher
Het für 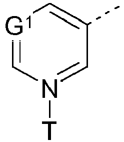 steht, worin die gestrichelte Linie die Bindung zum Kohlenstoffatom in der an Het gebundenen N=C-A-Gruppe darstellt,
G¹ für C-A¹ steht,
A¹ für Wasserstoff steht,
T für Sauerstoff oder ein Elektronenpaar steht,
R¹ für
Methyl, Ethyl, Isopropyl, n-Propyl, n-Butyl, iso-Butyl, sec-Butyl, *tert-*Butyl*,* 2,2,2-Trifluorethyl, 2,2-Difluorethyl, 2,2-Difluor-n-propyl, Dimethylaminocarbonyl, Diethylaminocarbonyl, *N*-Ethyl-*N*-methylaminocarbonyl, *N*-Isopropyl-*N*-methylaminocarbonyl, Dimethylaminocarbonylmethyl, Diethylaminocarbonylmethyl, *N*-Ethyl-*N*-methylaminocarbonylmethyl, *N*-Isopropyl-*N-*methylaminocarbonylmethyl, Dimethylaminocarbonylethyl, Diethylaminocarbonylethyl, *N*-Ethyl-*N*-methylaminocarbonylethyl, *N*-Isopropyl-*N*-methylaminocarbonylethyl, *N*-Cyclopropyl-*N-*methylaminocarbonylmethyl, *N*-Cyclopropyl-*N*-methylaminocarbonylethyl, Cyclopropyl, Cyclobutyl, Cyclopentyl, Cyclohexyl, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl steht,
R¹ auch für 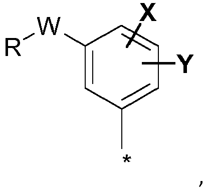 steht, worin die Bindung zum Stickstoffatom in der C(=V)-N-Q-Gruppe in der Formel (I) mit dem Stern (*) markiert ist.
R für gegebenenfalls einfach bis dreifach durch Fluor oder Chlor substituiertes C₁-C₄-Alkyl steht,
W für einen Rest aus der Reihe S und SO steht,
X für einen Rest aus der Reihe Wasserstoff, Fluor, Chlor, Brom, Methyl, Ethyl, Difluormethyl und Trifluormethyl steht,
Y für einen Rest aus der Reihe Wasserstoff, Fluor, Chlor, Brom, Jod, Methyl und Ethyl steht,
A für Schwefel steht,
Q für Stickstoff oder C-R³ steht,
R³ für Wasserstoff steht,
V für Sauerstoff steht und worin
Halogen für Fluor, Chlor, Brom und Iod steht und
Hetaryl für einen Rest aus der Reihe Pyridyl, Pyrimidinyl, Thiazolyl, Pyrazolyl und Benzothienyl steht.

7. Verbindungen der Formel (I) gemäß Anspruch 1, in welcher
Het für 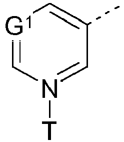 steht, worin die gestrichelte Linie die Bindung zum Kohlenstoffatom in der an Het gebundenen N=C-A-Gruppe darstellt,
G¹ für C-A¹ oder N steht,
A¹ für Wasserstoff oder Fluor steht,
T für Sauerstoff oder ein Elektronenpaar steht,
R¹ für Methyl, Ethyl, Isopropyl, n-Propyl, n-Butyl, iso-Butyl, sec-Butyl, *tert-*Butyl*,* 2,2,2-Trifluorethyl, 2,2-Difluorethyl, 2,2-Difluor-n-propyl, Dimethylaminocarbonyl, Diethylaminocarbonyl, *N*-Ethyl-*N*-methylaminocarbonyl, *N*-Isopropyl-*N*-methylaminocarbonyl, Dimethylaminocarbonylmethyl, Diethylaminocarbonylmethyl, *N*-Ethyl-*N-*methylaminocarbonylmethyl, *N*-Isopropyl-*N*-methylaminocarbonylmethyl, Dimethylaminocarbonylethyl, Diethylaminocarbonylethyl, *N*-Ethyl-*N*-methylaminocarbonylethyl, *N*-Isopropyl-*N*-methylaminocarbonylethyl, *N*-Cyclopropyl-*N*-methylaminocarbonylmethyl, *N-*Cyclopropyl-*N*-methylaminocarbonylethyl, Cyclopropyl, Cyclobutyl, Cyclopentyl, Cyclohexyl, Cyclopropylmethyl, gegebenenfalls durch Dimethylaminocarbonyl substituiertes Benzothienyl, gegebenenfalls durch Halogen, Methyl oder Trifluorethoxy substituiertes Phenyl, gegebenenfalls durch Methyl, Methoxy, Difluormethoxy oder Halogen substituiertes Phenylmethyl, jeweils gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy, Difluormethoxy substituiertes Hetarylmethyl und Hetarylethyl steht,
R¹ auch für 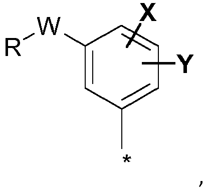 steht, worin die Bindung zum Stickstoffatom in der C(=V)-N-Q-Gruppe in der Formel (I) mit dem Stern (*) markiert ist,
R für gegebenenfalls einfach bis dreifach durch Fluor oder Chlor substituiertes C₁-C₄-Alkyl steht,
W für einen Rest aus der Reihe S,SO und SO₂ steht,
X für einen Rest aus der Reihe Wasserstoff, Fluor, Chlor, Brom, Methyl, Ethyl, Difluormethyl und Trifluormethyl steht,
Y für einen Rest aus der Reihe Wasserstoff, Fluor, Chlor, Brom, Jod, Methyl und Ethyl steht,
A für Schwefel steht,
Q für Stickstoff oder C-R³ steht,
R³ für Wasserstoff oder Methyl steht,
V für Sauerstoff steht und worin
Halogen für Fluor, Chlor, Brom und Iod steht und
Hetaryl für einen Rest aus der Reihe Pyridyl, Pyrimidinyl, Thiazolyl, Pyrazolyl und Benzothienyl steht.

8. Verbindungen der Formel (I) gemäß Ansprüchen 1 bis 3, in welchen Het für den Rest der Formel 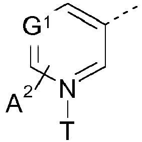 steht.

9. Verbindungen der Formel (I) gemäß Ansprüchen 1 bis 8, worin T für ein Elektronenpaar steht.

10. Verbindungen der Formel (I) gemäß Ansprüchen 1 bis 7, in welchen Het für Pyridin-3-yl steht.

11. Verbindungen der Formel (I) gemäß Ansprüchen 1 bis 7, in welchen Het für 5-Fluor-pyridin-3-yl steht.

12. Verbindungen der Formel (I) gemäß Ansprüchen 1 bis 5, in welchen R¹ für den Rest 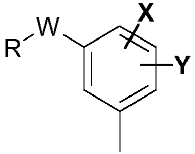 steht.

13. Mittel, **gekennzeichnet durch** einen Gehalt von mindestens einer Verbindung der Formel (I) gemäß einem der Ansprüche 1 bis 12 und üblichen Streckmitteln und/oder oberflächenaktiven Substanzen.

14. Nicht-therapeutische Verwendung von Verbindungen der Formel (I) gemäß einem der Ansprüche 1 bis 12 oder von Mitteln gemäß Anspruch 13 zur Bekämpfung von Schädlingen.

15. Verbindungen der Formel (II) 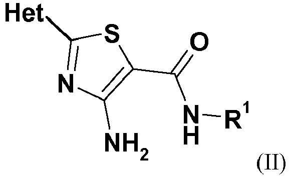 worin Het und R¹ die im Anspruch 1 angegebenen Bedeutungen haben.

## Claims

1. Compounds of the formula (I) 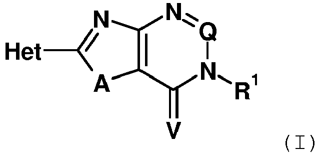 in which
Het represents a radical from the group consisting of 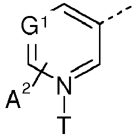 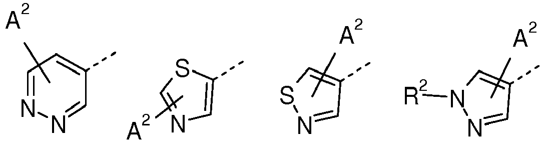 where the dotted line represents the bond to the carbon atom in the N=C-A group attached to Het and
G¹ represents N or C-A¹,
A¹ represents hydrogen, halogen, cyano, nitro, alkyl, haloalkyl, alkoxy, haloalkoxy or in each case optionally substituted cycloalkyl or cycloalkenyl,
A² represents hydrogen, halogen, cyano, nitro, alkyl, haloalkyl, alkoxy, haloalkoxy or in each case optionally substituted cycloalkyl or cycloalkenyl,
T represents oxygen or an electron pair,
R² represents hydrogen, alkyl, haloalkyl or optionally substituted cycloalkyl,
R¹ represents a radical from the group consisting of hydrogen, alkyl, haloalkyl, cyanoalkyl, alkenyl, haloalkenyl, alkynyl, haloalkynyl, alkoxy, haloalkoxy, optionally halogen-substituted alkoxyalkyl, optionally halogen-substituted bis(alkoxy)alkyl, optionally halogen-substituted alkylsulphanylalkyl, optionally halogen-substituted alkylcarbonylalkyl, optionally halogen-substituted alkylsulphinylalkyl, optionally halogen-substituted alkylsulphonylalkyl, dialkylaminosulphanylalkyl, dialkylaminosulphinylalkyl, dialkylaminosulphonylalkyl, optionally halogen-substituted alkoxycarbonyl, optionally halogen-substituted alkoxycarbonylalkyl, optionally halogen-substituted alkynyloxy, optionally halogen-substituted alkynyloxycarbonyl, dialkylaminocarbonyl, N-alkylN-cycloalkylaminocarbonyl, dialkylaminocarbonylalkyl, N-alkyl-N-cycloalkylaminocarbonylalkyl, heterocyclylcarbonylalkyl, alkylsulphanyl, haloalkylsulphanyl, alkylsulphinyl, haloalkylsulphinyl, alkylsulphonyl, haloalkylsulphonyl, optionally halogen-, cyano-, nitro-, alkyl-, cycloalkyl-, haloalkyl-, alkoxy-, haloalkoxy-, alkoxycarbonyl-, haloalkoxycarbonyl- or hetaryl- (which for its part may optionally be alkyl- or halogen-substituted) substituted cycloalkyl, optionally halogen-, cyano-, nitro-, alkyl-, haloalkyl-, cycloalkyl-, alkoxy-, haloalkoxy-, alkoxycarbonyl-, haloalkoxycarbonyl- or hetaryl- (which for its part may optionally be alkyl- or halogen-substituted) substituted cycloalkylcarbonyl, optionally halogen-, cyano-, nitro-, alkyl-, haloalkyl-, cycloalkyl-, alkoxy-, haloalkoxy-, alkoxycarbonyl-, haloalkoxycarbonyl- or hetaryl- (which for its part may optionally be alkyl- or halogen-substituted) substituted cycloalkylalkyl, optionally substituted heterocyclyl, optionally halogen-, cyano- (also in the alkyl part), nitro-, hydroxy-, alkyl-, haloalkyl-, cycloalkyl- (which is optionally substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl- or aminocarbonyl-substituted heterocyclylalkyl, optionally halogen-, cyano-, nitro-, hydroxy-, amino-, alkyl-, haloalkyl-, cycloalkyl- (which is optionally substituted) alkoxy- or haloalkoxy-substituted aryl, optionally halogen-, cyano- (also in the alkyl part), nitro-, hydroxy-, amino-, alkyl-, cycloalkyl- (which is optionally substituted), haloalkyl-, alkoxy- or haloalkoxy-substituted arylalkyl, optionally halogen-, cyano-, nitro-, hydroxy-, alkyl-, haloalkyl-, cycloalkyl-(which is optionally substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylaminocarbonyl-, dialkylaminocarbonyl-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl- or aminocarbonyl-substituted hetaryl, optionally halogen-, cyano- (also in the alkyl part), nitro-, hydroxy-, alkyl-, haloalkyl-, cycloalkyl- (which is optionally substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl- or aminocarbonyl-substituted hetarylalkyl,
R¹ also represents a radical from the group consisting of 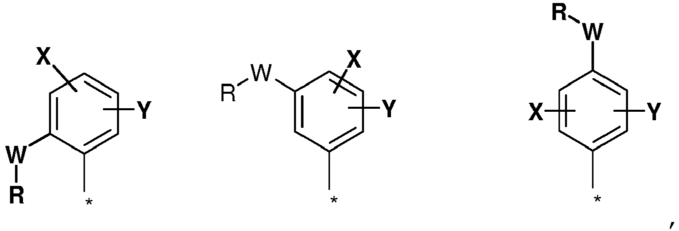 in which the bond to the nitrogen atom in the C(=V)-N-Q group in formula (I) is marked by the asterisk (*),
R represents NR⁷R⁸, or represents an in each case optionally substituted radical from the group consisting of alkyl, alkenyl, alkynyl, alkoxyalkyl, alkyl-S(O)ₘ-alkyl, R⁷-CO-alkyl, NR⁷R⁸-CO-alkyl, cycloalkyl, cycloalkenyl, cycloalkylalkyl, cycloalkenylalkyl, heterocyclyl, heterocyclylalkyl, phenyl, phenylalkyl, hetaryl and hetarylalkyl,
W represents a radical from the group consisting of O, S, SO and SO₂,
X represents a radical from the group consisting of hydrogen, halogen, cyano, nitro, alkyl, haloalkyl, alkoxy, haloalkoxy and cycloalkyl,
Y represents a radical from the group consisting of hydrogen, halogen, alkyl, cycloalkyl, haloalkyl, alkoxy, haloalkoxy, cyano and NR⁵R⁶,
A represents oxygen or sulphur,
Q represents nitrogen or C-R³ in which
R³ represents a radical from the group consisting of hydrogen, alkyl, cycloalkyl, haloalkyl, OH, alkoxy, haloalkoxy, SH, alkylsulphanyl, alkylsulphinyl, alkylsulphonyl, NH₂, alkylamino and dialkylamino,
V represents a radical from the group consisting of oxygen, sulphur and NR⁴,
R⁴ represents a radical from the group consisting of hydrogen, cyano, alkyl, haloalkyl and cycloalkyl,
R⁵ represents a radical from the group consisting of hydrogen, alkyl, cycloalkyl and haloalkyl,
R⁶ represents a radical from the group consisting of hydrogen, alkyl, cycloalkyl and haloalkyl,
or
R⁵ and R⁶ together with the nitrogen to which they are bonded represent an optionally substituted saturated or unsaturated 3- to 6-membered ring which optionally contains one or more further heteroatoms,
R⁷ represents hydrogen, hydroxy or an in each case optionally substituted radical from the group consisting of alkyl, alkoxy, alkoxyalkyl, alkyl-S(O)ₘ-alkyl, alkylcarbonyl, alkoxycarbonyl, cycloalkyl, cycloalkenyl, cycloalkylalkyl, cycloalkenylalkyl, heterocyclyl, heterocyclylalkyl, phenyl, phenylalkyl, hetaryl and hetarylalkyl,
R⁸ represents hydrogen, a metal ion, an optionally substituted ammonium ion or an in each case optionally substituted radical from the group consisting of alkyl, alkoxy, alkoxyalkyl, alkyl-S(O)ₘ- alkyl and
m represents a number from the group consisting of 0, 1 and 2.

2. Compounds of the formula (I) according to Claim 1, in which
Het represents a radical from the group consisting of 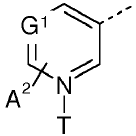 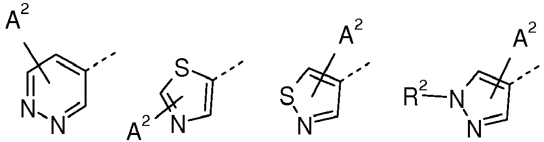 where the dotted line represents the bond to the carbon atom in the N=C-A group attached to Het and
G¹ represents N or C-A¹,
A¹ represents a radical from the group consisting of hydrogen, halogen, C₁-C₆-alkyl and C₁-C₄-haloalkyl,
A² represents a radical from the group consisting of hydrogen, halogen, C₁-C₆-alkyl and C₁-C₄-haloalkyl,
T represents oxygen or an electron pair,
R² represents a radical from the group consisting of hydrogen, C₁-C₆-alkyl, C₁-C₆-haloalkyl and C₃-C₆-cycloalkyl which is optionally mono- to trisubstituted by halogen, cyano, nitro, C₁-C₃-alkyl, C₁-C₃-alkoxy and monosubstituted by C₃-C₆-cycloalkyl,
R¹ represents a radical from the group consisting of hydrogen, C₁-C₆-alkyl, C₂-C₆-haloalkyl, cyano-C₁-C₆-alkyl, C₂-C₄-alkenyl, C₂-C₄-haloalkenyl, C₂-C₄-alkynyl, C₂-C₄-haloalkynyl, C₁-C₆-alkoxy, C₁-C₆-haloalkoxy, optionally halogen-substituted C₁-C₆-alkoxy-C₁-C₆-alkyl, optionally halogen-substituted bis (C₁-C₆-alkoxy)-C₁-C₆-alkyl, optionally halogen-substituted C₁-C₆-alkylsulphanyl-C₁-C₆-alkyl, optionally halogen-substituted C₁-C₄-alkylcarbonyl-C₁-C₄-alkyl, optionally halogen-substituted C₁-C₆-alkylsulphinyl-C₁-C₆-alkyl, optionally halogen-substituted C₁-C₆-alkylsulphonyl-C₁-C₆-alkyl, di-(C₁-C₆-alkyl)-aminosulphanyl-C₁-C₆-alkyl, di-(C₁-C₆)-alkylaminosulphinyl-C₁-C₆-alkyl, di-(C₁-C₆-alkyl)-aminosulphonyl-C₁-C₆-alkyl, optionally halogen-substituted C₁-C₆-alkoxycarbonyl, optionally halogen-substituted C₁-C₆-alkoxycarbonyl-C₁-C₆-alkyl, optionally halogen-substituted C₂-C₄-alkynyloxy, optionally halogen-substituted C₂-C₄-alkynyloxycarbonyl, di-(C₁-C₆-alkyl)-aminocarbonyl, N-C₁-C₆-alkyl-N-C₃-C₆-cycloalkylaminocarbonyl, di-(C₁-C₆-alkyl)-aminocarbonyl-C₁-C₆-alkyl, N-C₁-C₆-alkyl-N-C₃-C₆-cycloalkylaminocarbonyl-C₁-C₆-alkyl, heterocyclylcarbonyl-C₁-C₆-alkyl, C₁-C₆-alkylsulphanyl, C₁-C₆-haloalkylsulphanyl, C₁-C₆-alkylsulphinyl, C₁-C₆-haloalkylsulphinyl, C₁-C₆-alkylsulphonyl, C₁-C₆-haloalkylsulphonyl, optionally halogen-, cyano-, nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkoxycarbonyl-, C₁-C₆-haloalkoxycarbonyl- or hetaryl- (which for its part is optionally C₁-C₆-alkyl- or halogen-substituted) substituted C₃-C₆-cycloalkyl, optionally halogen-, cyano-, nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkoxycarbonyl-, C₁-C₆-haloalkoxycarbonyl- or hetaryl- (which for its part is optionally C₁-C₆-alkyl- or halogen-substituted) substituted C₃-C₆-cycloalkylcarbonyl, optionally halogen-, cyano-, nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkoxycarbonyl-, C₁-C₆-haloalkoxycarbonyl- or hetaryl- (which for its part is optionally C₁-C₆-alkyl- or halogen-substituted) substituted C₃-C₆-cycloalkyl-C₁-C₆-alkyl, optionally halogen-, cyano-(also in the C₁-C₆-alkyl part of heterocyclyl-C₁-C₆-alkyl), nitro-, hydroxy-, C₁-C₆-alkyl-, C₁-C₆-haloalkyl-, C₃-C₆-cycloalkyl- (which is optionally substituted by halogen, cyano, C₁-C₆-alkyl and C₃-C₆-cycloalkyl), C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-alkylsulphonyl-, C₁-C₆-haloalkylsulphinyl-, C₁-C₆-haloalkylsulphonyl-, amino-, C₁-C₆-alkylamino-, di-(C₁-C₆-alkyl)-amino-, C₁-C₆-alkylcarbonylamino-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted heterocyclyl-C₁-C₆-alkyl, optionally halogen-, cyano-, nitro-, hydroxy-, amino-, C₁-C₆-alkyl-, C₁-C₆-haloalkyl-, C₃-C₆-cycloalkyl- (which is optionally substituted by halogen, cyano, C₁-C₆-alkyl and C₃-C₆-cycloalkyl), C₁-C₆-alkoxy- or C₁-C₆-haloalkoxy-substituted aryl, optionally halogen-, cyano- (also in the C₁-C₆-alkyl part of aryl-C₁-C₆-alkyl), nitro-, hydroxy-, amino-, C₁-C₆-alkyl-, C₁-C₆-haloalkyl-, C₃-C₆-cycloalkyl- (which is optionally substituted by halogen, cyano, C₁-C₆-alkyl and C₃-C₆-cycloalkyl), C₁-C₆-alkoxy- or C₁-C₆-haloalkoxy-substituted aryl-C₁-C₆-alkyl, optionally halogen-, cyano-, nitro-, hydroxy-, C₁-C₆-alkyl-, C₁-C₆-haloalkyl-, C₃-C₆-cycloalkyl- (which is optionally substituted by halogen, cyano, C₁-C₆-alkyl and C₃-C₆-cycloalkyl), C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-alkylsulphonyl-, C₁-C₆-haloalkylsulphinyl-, C₁-C₆-haloalkylsulphonyl-, amino-, C₁-C₆-alkylamino-, di-(C₁-C₆-alkyl)-amino-, C₁-C₆-alkylcarbonylamino-, C₁-C₆-alkylaminocarbonyl-, di-(C₁-C₆-alkyl)-aminocarbonyl-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted hetaryl, optionally halogen-, cyano- (also in the C₁-C₆-alkyl part of hetaryl-C₁-C₆-alkyl), nitro-, hydroxy-, C₁-C₆-alkyl-, C₁-C₆-haloalkyl-, C₃-C₆-cycloalkyl- (which is optionally substituted by halogen, cyano, C₁-C₆-alkyl and C₃-C₆-cycloalkyl), C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-alkylsulphonyl-, C₁-C₆-haloalkylsulphinyl-, C₁-C₆-haloalkylsulphonyl-, amino-, C₁-C₆-alkylamino-, di-(C₁-C₆-alkyl)-amino-, C₁-C₆-alkylcarbonylamino-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted hetaryl-C₁-C₆-alkyl,
R¹ also represents a radical from the group consisting of 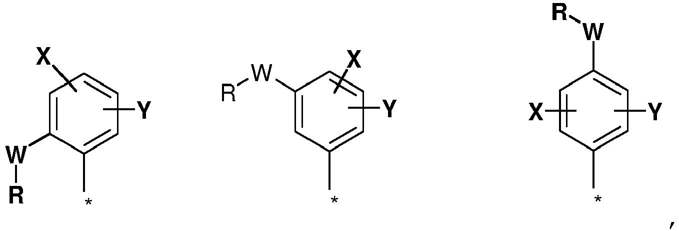 in which the bond to the nitrogen atom in the C(=V)-N-Q group in formula (I) is marked by the asterisk (*),
R represents NR⁷R⁸ or represents in each case optionally halogen-, oxygen- (leads to C=O) or cyano-substituted C₁-C₆-alkyl, C₃-C₆-alkenyl, C₃-C₆-alkynyl, C₁-C₆-alkoxy-C₁-C₄-alkyl, C₁-C₆-alkyl-S(O)ₘ-C₁-C₄-alkyl, represents R⁷-CO-C₁-C₄-alkyl, represents NR⁷R⁸-CO-C₁-C₄-alkyl, represents C₃-C₆-cycloalkyl which is optionally mono- or disubstituted by oxygen (leads to C=O), C₁-C₄-alkyl, C₃-C₈-cycloalkyl, C₁-C₄-alkoxy and C₁-C₄-haloalkyl, represents C₃-C₈-cycloalkenyl which is optionally mono- or disubstituted by oxygen (leads to C=O), C₁-C₄-alkyl, C₃-C₆-cycloalkyl, C₁-C₄-alkoxy and C₁-C₄-haloalkyl, represents C₃-C₆-cycloalkyl-C₁-C₄-alkyl which is optionally mono- or disubstituted by oxygen (leads to C=O), C₁-C₄-alkyl, C₃-C₆-cycloalkyl, C₁-C₄-alkoxy and C₁-C₄-haloalkyl, represents C₃-C₆-cycloalkenyl-C₁-C₄-alkyl which is optionally mono- or disubstituted by oxygen (leads to C=O), C₁-C₄-alkyl, C₃-C₆-cycloalkyl, C₁-C₄-alkoxy and C₁-C₄-haloalkyl, represents heterocyclyl which is optionally mono- or disubstituted by oxygen (leads to C=O), C₁-C₄-alkyl, C₃-C₆-cycloalkyl, C₁-C₄-alkoxy and C₁-C₄-haloalkyl, represents heterocyclyl-C₁-C₄-alkyl which is optionally mono- or disubstituted by oxygen (leads to C=O), C₁-C₄-alkyl, C₃-C₆-cycloalkyl, C₁-C₄-alkoxy and C₁-C₄-haloalkyl or represents phenyl, phenyl-C₁-C₄-alkyl, hetaryl or hetaryl-C₁-C₄-alkyl, each of which is optionally mono- to trisubstituted by halogen, cyano, C₁-C₄-alkyl, C₃-C₆-cycloalkyl, C₁-C₄-haloalkyl, C₁-C₄-alkoxy or C₁-C₄-haloalkoxy,
W represents a radical from the group consisting of O, S, SO and SO₂,
X represents a radical from the group consisting of hydrogen, halogen, cyano, nitro, C₁-C₆-alkyl, C₁-C₆-haloalkyl, C₃-C₆-cycloalkyl, C₁-C₆-alkoxy and C₁-C₆-haloalkoxy,
Y represents a radical from the group consisting of hydrogen, halogen, C₁-C₆-alkyl, C₃-C₆-cycloalkyl, C₁-C₆-haloalkyl, C₁-C₆-alkoxy, C₁-C₆-haloalkoxy, cyano and NR⁵R⁶,
A represents sulphur,
Q represents nitrogen or C-R³,
R³ represents a radical from the group consisting of hydrogen, C₁-C₆-alkyl, C₁-C₆-haloalkyl, C₃-C₆-cycloalkyl, OH, C₁-C₆-alkoxy, C₁-C₆-haloalkoxy, SH, C₁-C₆-alkylsulphanyl, C₁-C₆-alkylsulphinyl, C₁-C₆-alkylsulphonyl, NH₂, C₁-C₆-alkylamino and di-(C₁-C₆-alkyl)-amino,
V represents a radical from the group consisting of oxygen, sulphur and NR⁴,
R⁴ represents a radical from the group consisting of hydrogen, cyano, C₁-C₆-alkyl, C₂-C₆-haloalkyl and C₃-C₆-cycloalkyl,
R⁵ represents a radical from the group consisting of hydrogen, C₁-C₆-alkyl, C₃-C₆-cycloalkyl and C₂-C₆-haloalkyl,
R⁶ represents a radical from the group consisting of hydrogen, C₁-C₆-alkyl, C₃-C₆-cycloalkyl and C₂-C₆-haloalkyl,
R⁵ and R⁶ can also together with the nitrogen atom to which they are bonded represent a saturated to triunsaturated 3- to 6-membered ring which is optionally substituted by halogen, cyano, C₁-C₄-alkyl, C₁-C₄-haloalkyl, C₁-C₄-alkoxy, C₁-C₄-haloalkoxy,
R⁷ represents a radical from the group consisting of hydrogen, hydroxy, and C₁-C₆-alkyl, C₁-C₆-alkoxy, C₁-C₆-alkoxy-C₁-C₄-alkyl, C₁-C₆-alkyl-S(O)ₘ-C₁-C₄-alkyl, C₁-C₆-alkylcarbonyl, C₁-C₆-alkoxycarbonyl, C₃-C₆-cycloalkyl, C₃-C₆-cycloalkenyl, C₃-C₆-cycloalkyl-C₁-C₃-alkyl, C₃-C₆-cycloalkenyl-C₁-C₃-alkyl, heterocyclyl, heterocyclyl-C₁-C₃-alkyl, each of which is optionally mono- or polysubstituted by halogen or mono- or disubstituted by cyano, and represents phenyl, phenyl-C₁-C₃-alkyl, hetaryl and hetaryl-C₁-C₃-alkyl, each of which is optionally mono- to tetrasubstituted by C₁-C₄-alkyl, C₁-C₄-haloalkyl, C₁-C₄-alkoxy, C₁-C₄-haloalkoxy, C₃-C₆-cycloalkyl, halogen or cyano,
R⁸ represents hydrogen, an alkali or alkaline earth metal ion, or represents an ammonium ion which is optionally mono- to tetrasubstituted by C₁-C₄-alkyl, or represents a radical from the group consisting of C₁-C₄-alkyl, C₁-C₄-alkoxy, C₁-C₄-alkoxy-C₁-C₄-alkyl and C₁-C₄-alkyl-S(O)ₘ-C₁-C₄-alkyl, each of which is optionally mono- or polysubstituted by halogen or mono- or disubstituted by cyano,
m represents a number from the group consisting of 0, 1 and 2, and where
alkali metal ion is selected from the group consisting of lithium, sodium, potassium, rubidium, caesium,
alkaline earth metal ion is selected from the group consisting of beryllium, magnesium, calcium, strontium, barium,
halogen is selected from the group consisting of fluorine, chlorine, bromine and iodine,
aryl (including as part of a larger unit, for example arylalkyl) is selected from the group consisting of phenyl, naphthyl, anthryl, phenanthrenyl,
hetaryl (synonymous with heteroaryl, including as part of a larger unit, for example hetarylalkyl) is selected from the group consisting of furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzoisofuryl, benzothienyl, benzoisothienyl, indolyl, isoindolyl, indazolyl, benzothiazolyl, benzoisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl, and
heterocyclyl represents a saturated 4-, 5- or 6-membered ring which contains 1 or 2 nitrogen atoms and/or one oxygen atom and/or one sulphur atom.

3. Compounds of the formula (I) according to Claim 1, in which
Het represents a radical from the group consisting of 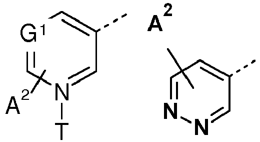 where the dotted line represents the bond to the carbon atom in the N=C-A group attached to Het,
G¹ represents N or C-A¹,
A¹ represents a radical from the group consisting of hydrogen, halogen, C₁-C₄-alkyl and C₁-C₄-haloalkyl,
A² represents a radical from the group consisting of hydrogen, halogen, C₁-C₄-alkyl and C₁-C₄-haloalkyl,
T represents oxygen or an electron pair,
R¹ represents a radical from the group consisting of hydrogen, C₁-C₄-alkyl, C₂-C₄-haloalkyl, cyano-C₁-C₄-alkyl, C₂-C₄-alkenyl, C₂-C₄-haloalkenyl, C₂-C₄-alkynyl, C₂-C₄-haloalkynyl, C₁-C₄-alkoxy, C₁-C₄-haloalkoxy, optionally halogen-substituted C₁-C₂-alkoxy-C₁-C₄-alkyl, optionally halogen-substituted bis(C₁-C₂-alkoxy)-C₁-C₄-alkyl, optionally halogen-substituted C₁-C₄-alkylsulphanyl-C₁-C₄-alkyl, optionally halogen-substituted C₁-C₄-alkylcarbonyl-C₁-C₄-alkyl, optionally halogen-substituted C₁-C₄-alkylsulphinyl-C₁-C₄-alkyl, optionally halogen-substituted C₁-C₄-alkylsulphonyl-C₁-C₄-alkyl, di-(C₁-C₄-alkyl)-aminosulphanyl-C₁-C₄-alkyl, di-(C₁-C₄-alkyl)-aminosulphinyl-C₁-C₄-alkyl, di-(C₁-C₄-alkyl)-aminosulphonyl-C₁-C₄-alkyl, optionally halogen-substituted C₁-C₄-alkoxycarbonyl, optionally halogen-substituted C₁-C₄-alkoxycarbonyl-C₁-C₄-alkyl, optionally halogen-substituted C₂-C₄-alkynyloxy, optionally halogen-substituted C₂-C₄-alkynyloxycarbonyl, di-(C₁-C₄-alkyl)-aminocarbonyl, N-C₁-C₄-alkyl-N-C₃-C₆-cycloalkylaminocarbonyl, di-(C₁-C₄-alkyl)-aminocarbonyl-C₁-C₄-alkyl, N-C₁-C₄-alkyl-N-C₃-C₆-cycloalkylaminocarbonyl-C₁-C₄-alkyl, heterocyclylcarbonyl-C₁-C₄-alkyl, C₁-C₄-alkylsulphanyl, C₁-C₄-haloalkylsulphanyl, C₁-C₄-alkylsulphinyl, C₁-C₄-haloalkylsulphinyl, C₁-C₄-alkylsulphonyl, C₁-C₄-haloalkylsulphonyl, optionally halogen-, cyano-, nitro-, C₁-C₄-alkyl-, C₁-C₄-haloalkyl-, C₃-C₆-cycloalkyl- C₁-C₄-alkoxy-, C₁-C₄-haloalkoxy-, C₁-C₄-alkoxycarbonyl-, C₁-C₄-haloalkoxycarbonyl- or pyridyl- (which for its part is optionally substituted by C₁-C₄-alkyl or halogen) substituted C₃-C₆-cycloalkyl, optionally halogen-, cyano-, nitro-, C₁-C₄-alkyl-, C₁-C₄-haloalkyl-, C₃-C₆-cycloalkyl- C₁-C₄-alkoxy-, C₁-C₄-haloalkoxy-, C₁-C₄-alkoxycarbonyl-, C₁-C₄-haloalkoxycarbonyl- or pyridyl- (which for its part is optionally substituted by C₁-C₄-alkyl or halogen) substituted C₃-C₆-cycloalkylcarbonyl, optionally halogen-, cyano-, nitro-, C₁-C₄-alkyl-, C₁-C₄-haloalkyl-, C₃-C₆-cycloalkyl- C₁-C₄-alkoxy-, C₁-C₄-haloalkoxy-, C₁-C₄-alkoxycarbonyl-, C₁-C₄-haloalkoxycarbonyl-, pyridyl-, pyrimidyl-, pyrazanyl-, pyridazinyl-, thiazolyl-, isothiazolyl-, thiadiazolyl-, oxazolyl-, isoxazolyl-, oxadiazolyl-, pyrazolyl-, triazinyl- or triazolyl- (where the hetaryls mentioned for their part are optionally substituted by C₁-C₄-alkyl or halogen) substituted C₃-C₆-cycloalkyl-C₁-C₄-alkyl, optionally halogen-, cyano- (also in the C₁-C₄-alkyl part of heterocyclyl-C₁-C₄-alkyl), nitro-, hydroxy-, C₁-C₄-alkyl-, C₁-C₄-haloalkyl-, C₃-C₆-cycloalkyl- (which is optionally substituted by halogen, cyano, C₁-C₄-alkyl and C₃-C₆-cycloalkyl), C₁-C₄-alkoxy-, C₁-C₄-haloalkoxy-, C₁-C₄-alkylthio-, C₁-C₄-haloalkyl-thio-, C₁-C₄-alkylsulphinyl-, C₁-C₄-alkylsulphonyl-, C₁-C₄-haloalkylsulphinyl-, C₁-C₄-haloalkylsulphonyl-, amino,- C₁-C₄-alkylamino-, di-(C₁-C₄-alkyl)-amino-, C₁-C₄-alkylcarbonylamino-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted heterocyclyl-C₁-C₄-alkyl, optionally halogen-, cyano-, nitro-, hydroxy-, amino-, C₁-C₄-alkyl-, C₁-C₄-haloalkyl-, C₃-C₆-cycloalkyl- (which is optionally substituted by halogen, cyano, C₁-C₄-alkyl and C₃-C₆-cycloalkyl), C₁-C₄-alkoxy- or C₁-C₄-haloalkoxy-substituted aryl, optionally halogen-, cyano- (also in the C₁-C₄-alkyl part of aryl-C₁-C₄-alkyl), nitro-, hydroxy-, amino-, C₁-C₄-alkyl-, C₁-C₄-haloalkyl-, C₃-C₆-cycloalkyl- (which is optionally substituted by halogen, cyano, C₁-C₄-alkyl and C₃-C₆-cycloalkyl), C₁-C₄-alkoxy- or C₁-C₄-haloalkoxy-substituted aryl-C₁-C₄-alkyl, optionally halogen-, cyano-, nitro-, hydroxy-, C₁-C₄-alkyl-, C₁-C₄-haloalkyl-, C₃-C₆-cycloalkyl- (which is optionally substituted by halogen, cyano, C₁-C₄-alkyl and C₃-C₆-cycloalkyl), C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-alkylsulphonyl-, C₁-C₄-haloalkylsulphinyl-, C₁-C₄-haloalkylsulphonyl-, amino-, C₁-C₄-alkylamino-, di-(C₁-C₄-alkyl)-amino-, C₁-C₄-alkylcarbonylamino-, C₁-C₄-alkylaminocarbonyl-, di-(C₁-C₄-alkyl)-aminocarbonyl-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted hetaryl, optionally halogen-, cyano- (also in the C₁-C₄-alkyl part of hetaryl-C₁-C₄-alkyl), nitro-, hydroxy-, C₁-C₄-alkyl-, C₁-C₄-haloalkyl-, C₃-C₆-cycloalkyl- (which is optionally substituted by halogen, cyano, C₁-C₄-alkyl and C₃-C₆-cycloalkyl), C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-alkylsulphonyl-, C₁-C₄-haloalkylsulphinyl-, C₁-C₄-haloalkylsulphonyl-, amino-, C₁-C₄-alkylamino-, di-(C₁-C₄-alkyl)-amino-, C₁-C₄-alkylcarbonylamino-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted hetaryl-C₁-C₄-alkyl,
R¹ also represents a radical from the group consisting of 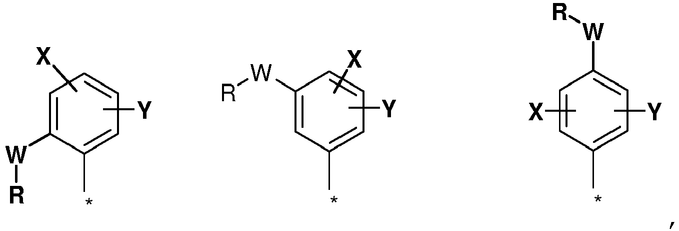 in which the bond to the nitrogen atom in the C(=V)-N-Q group in formula (I) is marked by the asterisk (*),
R represents NR⁷R⁸ or represents C₁-C₆-alkyl, C₃-C₆-alkenyl, C₃-C₆-alkynyl, C₁-C₄-alkoxy-C₁-C₃-alkyl, C₁-C₄-alkyl-S(O)ₘ-C₁-C₃-alkyl, each of which is optionally mono- to heptasubstituted by halogen, mono- or disubstituted by oxygen (leads to C=O) or mono- or disubstituted by cyano, represents R⁷-CO-C₁-C₂-alkyl, represents NR⁷R⁸-CO-C₁-C₂-alkyl, represents C₃-C₈-cycloalkyl which is optionally mono- or disubstituted by oxygen (leads to C=O), C₁-C₄-alkyl, C₃-C₆-cycloalkyl, C₁-C₄-alkoxy or C₁-C₄-haloalkyl, represents C₃-C₈-cycloalkenyl which is optionally mono- or disubstituted by oxygen (leads to C=O), C₁-C₄-alkyl, C₃-C₆-cycloalkyl, C₁-C₄-alkoxy or C₁-C₄-haloalkyl, represents C₃-C₆-cycloalkyl-C₁-C₄-alkyl which is optionally mono- or disubstituted by oxygen (leads to C=O), C₁-C₄-alkyl, C₃-C₆-cycloalkyl, C₁-C₄-alkoxy or C₁-C₄-haloalkyl, represents C₃-C₆-cycloalkenyl-C₁-C₄-alkyl which is optionally mono- or disubstituted by oxygen (leads to C=O), C₁-C₄-alkyl, C₃-C₆-cycloalkyl, C₁-C₄-alkoxy or C₁-C₄-haloalkyl, represents heterocyclyl which is optionally mono- or disubstituted by oxygen (leads to C=O), C₁-C₄-alkyl, C₃-C₆-cycloalkyl, C₁-C₄-alkoxy or C₁-C₄-haloalkyl, represents heterocyclyl-C₁-C₄-alkyl which is optionally mono- or disubstituted by oxygen (leads to C=O), C₁-C₄-alkyl, C₃-C₆-cycloalkyl, C₁-C₄-alkoxy or C₁-C₄-haloalkyl or represents phenyl, phenyl-C₁-C₄-alkyl, hetaryl or hetaryl-C₁-C₄-alkyl, each of which is optionally mono- to trisubstituted by halogen, cyano, C₁-C₄-alkyl, C₁-C₄-haloalkyl, C₃-C₆-cycloalkyl, C₁-C₄-alkoxy or C₁-C₄-haloalkoxy,
W represents a radical from the group consisting of S, SO and SO₂,
X represents a radical from the group consisting of hydrogen, fluorine, chlorine, bromine, iodine, cyano, nitro, C₁-C₄-alkyl, C₁-C₄-haloalkyl, C₃-C₆-cycloalkyl, C₁-C₄-alkoxy and C₁-C₄-haloalkoxy,
Y represents a radical from the group consisting of hydrogen, fluorine, chlorine, bromine, iodine, cyano, C₁-C₄-alkyl, C₁-C₄-haloalkyl, C₃-C₆-cycloalkyl, C₁-C₄-alkoxy and C₁-C₄-haloalkoxy,
A represents sulphur,
Q represents nitrogen or C-R³,
R³ represents a radical from the group consisting of hydrogen, C₁-C₄-alkyl, C₁-C₄-haloalkyl, C₃-C₆-cycloalkyl, OH, C₁-C₄-alkoxy, C₁-C₄-haloalkoxy, SH, C₁-C₄-alkylsulphanyl, C₁-C₄-alkylsulphinyl, C₁-C₄-alkylsulphonyl, NH₂, C₁-C₄-alkylamino and di-(C₁-C₄-alkyl)-amino,
V represents oxygen,
R⁷ represents a radical from the group consisting of hydrogen, hydroxy, or C₁-C₄-alkyl, C₁-C₄-alkoxy, C₁-C₄-alkoxy-C₁-C₄-alkyl, C₁-C₄-alkyl-S(O)ₘ-C₁-C₃-alkyl, C₁-C₄-alkylcarbonyl, C₁-C₄-alkoxycarbonyl, C₃-C₆-cycloalkyl, C₃-C₆-cycloalkyl-C₁-C₃-alkyl, heterocyclyl and heterocyclyl-C₁-C₃-alkyl, each of which is optionally mono- or polysubstituted by halogen or mono- or disubstituted by cyano, or represents phenyl, benzyl, pyridyl, pyrimidyl, thiazolyl, oxazolyl, pyrazolyl, thienyl, furanyl, pyridinylmethyl or thiazolylmethyl, each of which is optionally mono- to trisubstituted by C₁-C₄-alkyl, C₁-C₃-haloalkyl, C₁-C₃-alkoxy, C₁-C₃-haloalkoxy, cyclopropyl, fluorine, chlorine, bromine or cyano,
R⁸ represents hydrogen, represents an alkali or alkaline earth metal ion, or represents an ammonium ion which is optionally mono- to tetrasubstituted by C₁-C₄-alkyl, or represents a radical from the group consisting of C₁-C₄-alkyl, C₁-C₄-alkoxy, C₁-C₄-alkoxy-C₁-C₂-alkyl and C₁-C₄-alkyl-S(O)ₘ-C₁-C₂-alkyl, each of which is optionally mono- or polysubstituted by halogen or mono- or disubstituted by cyano,
m represents a number from the group consisting of 0, 1 and 2, and where
alkali metal ion is selected from the group consisting of lithium, sodium, potassium, rubidium, caesium,
alkaline earth metal ion is selected from the group consisting of beryllium, magnesium, calcium, strontium, barium,
halogen is selected from the group consisting of fluorine, chlorine, bromine and iodine,
aryl (including as part of a larger unit, for example arylalkyl) is selected from the group consisting of phenyl, naphthyl, anthryl and phenanthrenyl,
hetaryl (synonymous with heteroaryl, also as part of a larger unit such as, for example, hetarylalkyl) is selected from the group consisting of pyrazolyl, imidazolyl, 1,2,3-triazolyl, 1,2,4-triazolyl, oxazolyl, isoxazolyl, thiazolyl, isothiazolyl, pyridyl, pyrimidinyl, pyridazinyl, pyrazinyl, 1,2,3-triazinyl, 1,2,4-triazinyl, 1,3,5-triazinyl, benzothienyl, and
heterocyclyl is selected from the group consisting of azetidinyl, azolidinyl, azinanyl, oxetanyl, oxolanyl, oxanyl, dioxanyl, thiethanyl, thiolanyl, thianyl, tetrahydrofuryl, piperazinyl, morpholinyl.

4. Compounds of the formula (I) according to Claim 1, in which
Het is a radical from the group of 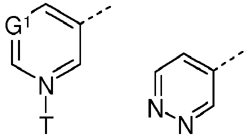 where the dotted line represents the bond to the carbon atom in the N=C-A group attached to Het,
G¹ represents N or C-A¹,
A¹ represents a radical from the group consisting of hydrogen, fluorine and chlorine,
T represents oxygen or an electron pair,
R¹ represents a radical from the group consisting of methyl, ethyl, isopropyl, n-propyl, n-butyl, isobutyl, sec-butyl, tert-butyl, 2,2,2-trifluoroethyl, 2,2-difluoroethyl, 2,2-difluoro-n-propyl, methylsulphanylmethyl, methylsulphanylethyl, methylsulphanyl-n-propyl, trifluoromethylsulphonylmethyl, ethylsulphonylmethyl, 2,2,2-trifluoroethyl-sulphonylmethyl, 2,2-difluoroethylsulphonylmethyl, isopropylsulphanylmethyl, methylsulphinylmethyl, trifluoromethylsulphinylmethyl, ethylsulphinyl-methyl, 2,2,2-trifluoroethylsulphinylmethyl, 2,2-difluoroethylsulphinylmethyl, isopropylsulphinyl-methyl, methylsulphonylmethyl, trifluoromethyl-sulphonylmethyl, ethylsulphonylmethyl, 2,2,2-trifluoroethylsulphonylmethyl, 2,2-difluoroethyl-sulphonylmethyl, isopropylsulphonylmethyl, methoxycarbonyl, ethoxycarbonyl, methoxycarbonyl-methyl, ethoxycarbonylmethyl, methoxycarbonylethyl, ethoxycarbonylethyl, dimethylaminocarbonyl, diethylaminocarbonyl, *N*-ethyl-*N-*methylaminocarbonyl, *N*-isopropyl-*N-*methylaminocarbonyl, dimethylaminocarbonylmethyl, diethylaminocarbonylmethyl, *N*-ethyl-*N-*methylaminocarbonylmethyl, *N*-isopropyl-*N-*methylaminocarbonylmethyl, dimethylaminocarbonyl-ethyl, diethylaminocarbonylethyl, *N*-ethyl-*N-*methylaminocarbonylethyl, *N*-isopropyl-*N-*methylaminocarbonylethyl, *N*-cyclopropyl-*N-*methylaminocarbonylmethyl, *N*-cyclopropyl-*N-*methylaminocarbonylethyl, methylsulphanyl, trifluoromethylsulphanyl, ethylsulphanyl, 2,2,2-trifluoroethylsulphanyl, 2,2-difluoroethylsulphanyl, isopropylsulphanyl, methylsulphinyl, trifluoromethylsulphinyl, ethylsulphinyl, 2,2,2-trifluoroethylsulphinyl, 2,2-difluoroethylsulphinyl, isopropylsulphinyl, methylsulphonyl, trifluoromethylsulphonyl, ethylsulphonyl, 2,2,2-trifluoroethylsulphonyl, 2,2-difluoroethylsulphonyl, isopropylsulphonyl, cyclopropyl, 1-cyanocyclopropyl, 1-chlorocyclopropyl, 1-fluorocyclopropyl, 2-cyanocyclopropyl, 2-chlorocyclopropyl, 2-fluorocyclopropyl, 2,2,3,3-tetrafluorocyclopropyl, 2-cyclopropylcyclopropyl, cyclobutyl, cyclopentyl, cyclohexyl, 4-trifluoromethylcyclohexyl, cyclopropylmethyl, cyclopropylethyl, cyclobutylmethyl, *N*-cyclopropyl-*N-*methylaminocarbonyl, morpholin-4-ylcarbonylmethyl, piperazin-1-ylcarbonylmethyl, 4-methylpiperazin-1-ylcarbonylmethyl, of heterocyclylmethyl and heterocyclylethyl, each of which is optionally mono-, di- or trisubstituted by identical or different substitutents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy and difluoromethoxy, of in each case cyclopropyl-substituted heterocyclylmethyl and heterocyclylethyl, where the cyclopropyl radical is optionally mono- or disubstituted by methyl, fluorine, chlorine, cyano or monosubstituted by cyclopropyl, of hetaryl which is optionally mono- or disubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy, dimethylaminocarbonyl, of hetaryl substituted by cyclopropyl, where the cyclopropyl radical is optionally mono- or disubstituted by methyl, fluorine, chlorine, cyano or monosubstituted by cyclopropyl, of aryl which is optionally mono-, di- or trisubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy and trifluoroethoxy, of aryl substituted by cyclopropyl, where the cyclopropyl radical is optionally mono- or disubstituted by methyl, fluorine, chlorine, cyano or monosubstituted by cyclopropyl, of arylmethyl and arylethyl, each of which is optionally mono-, di- or trisubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy, of arylmethyl and arylethyl in each case substituted by cyclopropyl, where the cyclopropyl radical is optionally mono- or disubstituted by methyl, fluorine, chlorine, cyano or monosubstituted by cyclopropyl, of hetarylmethyl and hetarylethyl, each of which is optionally mono- or disubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy, of hetarylmethyl and hetarylethyl in each case substitued by cyclopropyl, where the cyclopropyl radical is optionally mono- or disubstituted by methyl, fluorine, chlorine, cyano or monosubstituted by cyclopropyl,
R¹ also represents a radical from the group consisting of 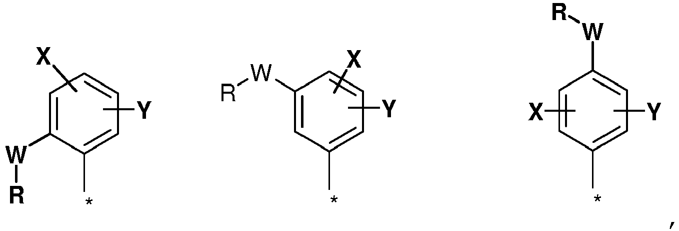 in which the bond to the nitrogen atom in the C(=V)-N-Q group in formula (I) is marked by the asterisk (*),
R represents NR⁷R⁸ or represents a radical from the group consisting of C₁-C₄-alkyl, C₃-C₄-alkenyl, C₃-C₄-alkynyl, C₁-C₂-alkoxy-C₁-C₂-alkyl and C₁-C₂-alkyl-S(O)ₘ-C₁-C₂-alkyl, each of which is optionally mono-, di-, tri-, tetra- or pentasubstituted by fluorine, chlorine or mono- or disubstituted by cyano, represents R⁷-CO-C₁-C₂-alkyl, represents NR⁷R⁸-CO-C₁-C₂-alkyl, represents C₃-C₆-cycloalkyl which is optionally mono- or disubstituted by C₁-C₂-alkyl, C₁-C₂-alkoxy or C₁-C₂-haloalkyl or by an oxygen atom (leads to C=O), represents C₃-C₆-cycloalkenyl which is optionally mono- or disubstituted by C₁-C₂-alkyl, C₁-C₂-alkoxy or C₁-C₂-haloalkyl or by an oxygen atom (leads to C=O), represents C₃-C₆-cycloalkyl-C₁-C₂-alkyl which is optionally mono- or disubstituted by C₁-C₂-alkyl, C₁-C₂-alkoxy or C₁-C₂-haloalkyl, represents C₃-C₆-cycloalkenyl-C₁-C₂-alkyl which is optionally mono-or disubstituted by C₁-C₂-alkyl, C₁-C₂-alkoxy or C₁-C₂-haloalkyl, represents heterocyclyl which is optionally mono- or disubstituted by C₁-C₂-alkyl, C₁-C₂-alkoxy or C₁-C₂-haloalkyl, represents heterocyclyl-C₁-C₂-alkyl which is optionally mono-or disubstituted by C₁-C₂-alkyl, C₁-C₂-alkoxy or C₁-C₂-haloalkyl or represents phenyl, benzyl, pyridyl, pyrimidyl, thiazolyl, oxazolyl, pyrazolyl, thienyl, furanyl, pyridinylmethyl or thiazolylmethyl, each of which is optionally mono- or disubstituted by fluorine, chlorine, bromine, cyano, methyl, ethyl, difluoromethyl, trifluoromethyl, methoxy, ethoxy, difluoromethoxy or trifluoromethoxy,
W represents a radical from the group consisting of S, SO and SO₂,
X represents a radical from the group consisting of hydrogen, fluorine, chlorine, bromine, cyano, methyl, ethyl, trifluoromethyl, methoxy, ethoxy, difluoromethoxy and trifluoromethoxy,
Y represents a radical from the group consisting of hydrogen, fluorine, chlorine, bromine, cyano, methyl, ethyl, trifluoromethyl, methoxy, ethoxy, difluoromethoxy and trifluoromethoxy,
A represents sulphur,
Q represents nitrogen or C-R³,
R³ represents hydrogen,
V represents oxygen,
R⁷ represents a radical from the group consisting of hydrogen, hydroxy, or represents C₁-C₄-alkyl, C₁-C₄-alkoxy, C₁-C₄-alkoxy-C₁-C₄-alkyl, C₁-C₄-alkyl-S(O)ₘ-C₁-C₂-alkyl, C₁-C₄-alkylcarbonyl, C₁-C₄-alkoxycarbonyl, C₃-C₆-cycloalkyl, C₃-C₆-cycloalkyl-C₁-C₃-alkyl, heterocyclyl or heterocyclyl-C₁-C₃-alkyl, each of which is optionally mono-, di-, tri-, tetra- or pentasubstituted by fluorine, chlorine or mono- or disubstituted by cyano, or represents phenyl, benzyl, pyridyl, pyrimidyl, thiazolyl, oxazolyl, pyrazolyl, thienyl, furanyl, pyridinylmethyl or thiazolylmethyl, each of which is optionally mono- to trisubstituted by C₁-C₄-alkyl, C₁-C₃-haloalkyl, C₁-C₃-alkoxy, C₁-C₃-haloalkoxy, cyclopropyl, fluorine, chlorine, bromine or cyano,
R⁸ represents hydrogen, an alkali or alkaline-earth metal ion, an ammonium ion which is optionally mono- to tetrasubstituted by C₁-C₄-alkyl, or represents a radical from the group consisting of C₁-C₄-alkyl, C₁-C₄-alkoxy, C₁-C₄-alkoxy-C₁-C₂-alkyl and C₁-C₄-alkyl-S(O)ₘ-C₁-C₂-alkyl, each of which is optionally mono-, di-, tri-, tetra- or pentasubstituted by fluorine, chlorine or is mono-or disubstituted by cyano,
m represents a number from the group consisting of 0, 1 and 2, and where
alkali metal ion represents an ion from the group consisting of lithium, sodium, potassium, rubidium, caesium,
alkaline earth metal ion represents an ion from the group consisting of beryllium, magnesium, calcium, strontium, barium,
halogen represents fluorine, chlorine, bromine and iodine,
heterocyclyl represents a radical from the group consisting of oxetanyl, thiethanyl, tetrahydrofuryl and morpholinyl,
aryl represents phenyl and
hetaryl (synonymous with heteroaryl, including as part of a larger unit such as, for example, hetarylalkyl) represents a radical from the group consisting of pyridyl, pyrimidyl, pyrazinyl, pyridazinyl, thiazolyl, pyrazolyl and benzothienyl.

5. Compounds of the formula (I) according to Claim 1, in which Het represents a radical from the group consisting of of 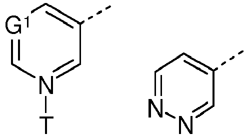 where the dotted line represents the bond to the carbon atom in the N=C-A group attached to Het,
G¹ represents N or C-A¹,
A¹ represents a radical from the group consisting of hydrogen, fluorine and chlorine,
T represents oxygen or an electron pair,
R¹ represents a radical from the group consisting of methyl, ethyl, isopropyl, n-propyl, n-butyl, isobutyl, sec-butyl, *tert*-butyl, 2,2,2-trifluoroethyl, 2,2-difluoroethyl, 2,2-difluoro-n-propyl, methylsulphanylmethyl, methylsulphanylethyl, methylsulphanyl-n-propyl, trifluoromethylsulphonylmethyl, ethylsulphonyl-methyl, 2,2,2-trifluoroethylsulphonylmethyl, 2,2-difluoroethylsulphonylmethyl, isopropylsulphanylmethyl, methylsulphinylmethyl, trifluoromethylsulphinylmethyl, ethylsulphinylmethyl, 2,2,2-trifluoroethylsulphinylmethyl, 2,2-difluoroethylsulphinylmethyl, isopropylsulphinylmethyl, methylsulphonylmethyl, trifluoromethylsulphonylmethyl, ethylsulphonylmethyl, 2,2,2-trifluoroethylsulphonylmethyl, 2,2-difluoroethylsulphonylmethyl, isopropylsulphonylmethyl, methoxycarbonyl, ethoxycarbonyl, methoxycarbonylmethyl, ethoxycarbonylmethyl, methoxycarbonylethyl, ethoxycarbonylethyl, dimethylaminocarbonyl, diethylaminocarbonyl, *N*-ethyl-*N-*methylaminocarbonyl, *N*-isopropyl-*N-*methylaminocarbonyl, dimethylaminocarbonylmethyl, diethylaminocarbonylmethyl, *N*-ethyl-*N-*methylaminocarbonylmethyl, *N*-isopropyl-*N-*methylaminocarbonylmethyl, dimethylaminocarbonyl-ethyl, diethylaminocarbonylethyl, *N*-ethyl-*N-*methylaminocarbonylethyl, *N*-isopropyl-*N-*methylaminocarbonylethyl, *N*-cyclopropyl-*N-*methylaminocarbonylmethyl, *N*-cyclopropyl-*N-*methylaminocarbonylethyl, methylsulphanyl, trifluoromethylsulphanyl, ethylsulphanyl, 2,2,2-trifluoroethylsulphanyl, 2,2-difluoroethylsulphanyl, isopropylsulphanyl, methylsulphinyl, trifluoromethylsulphinyl, ethylsulphinyl, 2,2,2-trifluoroethylsulphinyl, 2,2-difluoroethylsulphinyl, isopropylsulphinyl, methylsulphonyl, trifluoromethylsulphonyl, ethylsulphonyl, 2,2,2-trifluoroethylsulphonyl, 2,2-difluoroethylsulphonyl, isopropylsulphonyl, cyclopropyl, 1-cyanocyclopropyl, 1-chlorocyclopropyl, 1-fluorocyclopropyl, 2-cyanocyclopropyl, 2-chlorocyclopropyl, 2-fluorocyclopropyl, 2,2,3,3-tetrafluorocyclopropyl, 2-cyclopropylcyclopropyl, cyclobutyl, cyclopentyl, cyclohexyl, 4-trifluoromethylcyclohexyl, cyclopropylmethyl, cyclopropylethyl, cyclobutylmethyl, *N*-cyclopropyl-*N-*methylaminocarbonyl, morpholin-4-ylcarbonylmethyl, piperazin-1-ylcarbonylmethyl, 4-methylpiperazin-1-ylcarbonylmethyl, of heterocyclylmethyl and heterocyclylethyl, each of which is optionally mono-, di- or trisubtituted by identical or different substitutents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy and difluoromethoxy, of in each case cyclopropyl-substituted heterocyclylmethyl and heterocyclylethyl, where the cyclopropyl radical is optionally mono- or disubstituted by methyl, fluorine, chlorine, cyano or monosubstituted by cyclopropyl, of hetaryl which is optionally mono- or disubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy, dimethylaminocarbonyl, of hetaryl substituted by cyclopropyl, where the cyclopropyl radical is optionally mono- or disubstituted by methyl, fluorine, chlorine, cyano or monosubstituted by cyclopropyl, of aryl which is optionally mono-, di- or trisubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy and trifluoroethoxy, of aryl substituted by cyclopropyl, where the cyclopropyl radical is optionally mono- or disubstituted by methyl, fluorine, chlorine, cyano or monosubstituted by cyclopropyl, of arylmethyl and arylethyl, each of which is optionally mono-, di- or trisubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy, of arylmethyl and arylethyl in each case substituted by cyclopropyl, where the cyclopropyl radical is optionally mono- or disubstituted by methyl, fluorine, chlorine, cyano or monosubstituted by cyclopropyl, of hetarylmethyl and hetarylethyl, each of which is optionally mono- or disubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy, of hetarylmethyl and hetarylethyl in each case substituted by cyclopropyl, where the cyclopropyl radical is optionally mono- or disubstituted by methyl, fluorine, chlorine, cyano or monosubstituted by cyclopropyl,
R¹ also represents the radical of the formula 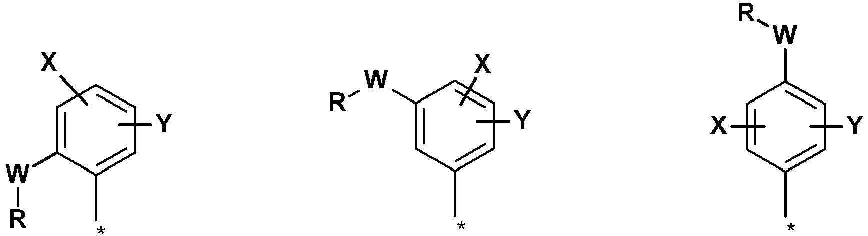 in which the bond to the nitrogen atom in the C(=V)-N-Q group in formula (I) is marked by the asterisk (*),
R represents methyl, ethyl, propyl, isopropyl, n-butyl, isobutyl, sec-butyl, tert-butyl, allyl, 2-butenyl, propargyl, 2-butynyl, each of which is optionally mono-, di- or trisubstituted by fluorine or monosubstituted by cyano, or represents C₃-C₆-cycloalkyl which is optionally monosubstituted by methyl, ethyl, methoxy, ethoxy or trifluoromethyl, or represents C₃-C₆-cycloalkylmethyl which is optionally monosubstituted by methyl, ethyl, methoxy or trifluoromethyl, or represents phenyl, benzyl, pyridyl, pyrimidyl, thiazolyl and pyridinylmethyl, each of which is optionally mono- or disubstituted by fluorine, chlorine, bromine, cyano, methyl, ethyl, isopropyl, tert-butyl, trifluoromethyl, difluoromethyl, methoxy, ethoxy, difluoromethoxy or trifluoromethoxy,
W represents a radical from the group consisting of S, SO and SO₂,
X represents a radical from the group consisting of hydrogen, fluorine, chlorine, bromine, cyano, methyl, ethyl, trifluoromethyl, methoxy, ethoxy, difluoromethoxy and trifluoromethoxy,
Y represents a radical from the group consisting of hydrogen, fluorine, chlorine, bromine, cyano, methyl, ethyl, trifluoromethyl, methoxy, ethoxy, difluoromethoxy and trifluoromethoxy,
A represents sulphur,
Q represents nitrogen or C-R³,
R³ represents hydrogen or methyl,
V represents oxygen and where
alkali metal ion represents an ion from the group consisting of lithium, sodium, potassium, rubidium, caesium,
alkaline earth metal ion represents an ion from the group consisting of beryllium, magnesium, calcium, strontium, barium,
halogen represents fluorine, chlorine, bromine and iodine,
heterocyclyl represents a radical from the group consisting of oxetanyl, thiethanyl, tetrahydrofuryl and morpholinyl,
aryl represents phenyl and
hetaryl (synonymous with heteroaryl, including as part of a larger unit such as, for example, hetarylalkyl) represents a radical from the group consisting of pyridyl, pyrimidyl, pyrazinyl, pyridazinyl, thiazolyl, pyrazolyl and benzothienyl.

6. Compounds of the formula (I) according to Claim 1 in which
Het represents 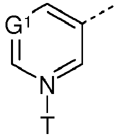 where the dotted line represents the bond to the carbon atom in the N=C-A group attached to Het,
G¹ represents C-A¹,
A¹ represents hydrogen,
T represents oxygen or an electron pair,
R¹ represents
methyl, ethyl, isopropyl, n-propyl, n-butyl, isobutyl, sec-butyl, *tert*-butyl, 2,2,2-trifluoroethyl, 2,2-difluoroethyl, 2,2-difluoro-n-propyl, dimethylaminocarbonyl, diethylaminocarbonyl, *N*-ethyl-*N-*methylaminocarbonyl, *N*-isopropyl-*N-*methylaminocarbonyl, dimethylaminocarbonylmethyl, diethylaminocarbonylmethyl, *N*-ethyl-*N-*methylaminocarbonylmethyl, *N*-isopropyl-*N-*methylaminocarbonylmethyl, dimethylaminocarbonylethyl, diethylaminocarbonylethyl, *N*-ethyl-*N-*methylaminocarbonylethyl, *N*-isopropyl-*N-*methylaminocarbonylethyl, *N*-cyclopropyl-*N-*methylaminocarbonylmethyl, *N*-cyclopropyl-*N-*methylaminocarbonylethyl, cyclopropyl, cyclobutyl, cyclopentyl, cyclohexyl, represents hetarylmethyl or hetarylethyl, each of which is optionally mono- or disubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy,
R¹ also represents 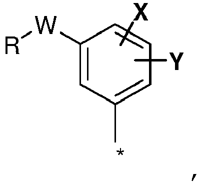 in which the bond to the nitrogen atom in the C(=V)-N-Q group in formula (I) is marked by the asterisk (*),
R represents C₁-C₄-alkyl which is optionally mono- to trisubstituted by fluorine or chlorine,
W represents a radical from the group S and SO,
X represents a radical from the group consisting of hydrogen, fluorine, chlorine, bromine, methyl, ethyl, difluoromethyl and trifluoromethyl,
Y represents a radical from the group consisting of hydrogen, fluorine, chlorine, bromine, iodine, methyl and ethyl,
A represents sulphur,
Q represents nitrogen or C-R³,
R³ represents hydrogen,
V represents oxygen and where
halogen represents fluorine, chlorine, bromine and iodine and
hetaryl represents a radical from the group consisting of pyridyl, pyrimidinyl, thiazolyl, pyrazolyl and benzothienyl.

7. Compounds of the formula (I) according to Claim 1 in which
Het represents 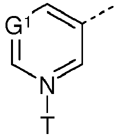 where the dotted line represents the bond to the carbon atom in the N=C-A group attached to Het,
G¹ represents C-A¹ or N,
A¹ represents hydrogen or fluorine,
T represents oxygen or an electron pair,
R¹ represents methyl, ethyl, isopropyl, n-propyl, n-butyl, isobutyl, sec-butyl, tert-butyl, 2,2,2-trifluoroethyl, 2,2-difluoroethyl, 2,2-difluoro-n-propyl, dimethylaminocarbonyl, diethylaminocarbonyl, N-ethyl-N-methylaminocarbonyl, N-isopropyl-N-methylaminocarbonyl, dimethylaminocarbonylmethyl, diethylaminocarbonylmethyl, N-ethyl-N-methylaminocarbonylmethyl, N-isopropyl-N-methylaminocarbonylmethyl, dimethylamino-carbonylethyl, diethylaminocarbonylethyl, N-ethyl-N-methylaminocarbonylethyl, N-isopropyl-N-methylaminocarbonylethyl, N-cyclopropyl-N-methylaminocarbonylmethyl, N-cyclopropyl-N-methylaminocarbonylethyl, cyclopropyl, cyclobutyl, cyclopentyl, cyclohexyl, cyclopropylmethyl, optionally dimethylaminocarbonyl-substituted benzothienyl, optionally halogen-, methyl- or trifluoroethoxy-substituted phenyl, optionally methyl-, methoxy-, difluoromethoxy- or halogen-substituted phenylmethyl, represents hetarylmethyl or hetarylethyl, each of which is optionally mono- or disubstituted by identical or different substituents from the group consisting of fluorine, chlorine, bromine, cyano, nitro, hydroxy, amino, methyl, ethyl, isopropyl, tert-butyl, trifluoromethyl, difluoromethyl, methoxy, trifluoromethoxy, difluoromethoxy,
R¹ also represents 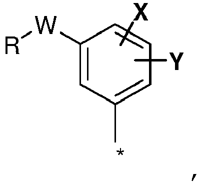 in which the bond to the nitrogen atom in the C(=V)-N-Q group in formula (I) is marked by the asterisk (*),
R represents C₁-C₄-alkyl which is optionally mono- to trisubstituted by fluorine or chlorine,
W represents a radical from the group consisting of S, SO and SO₂,
X represents a radical from the group consisting of hydrogen, fluorine, chlorine, bromine, methyl, ethyl, difluoromethyl and trifluoromethyl,
Y represents a radical from the group consisting of hydrogen, fluorine, chlorine, bromine, iodine, methyl and ethyl,
A represents sulphur,
Q represents nitrogen or C-R³,
R³ represents hydrogen or methyl,
V represents oxygen and where
halogen represents fluorine, chlorine, bromine and iodine and
hetaryl represents a radical from the group consisting of pyridyl, pyrimidinyl, thiazolyl, pyrazolyl and benzothienyl.

8. Compounds of the formula (I) according to any of Claims 1 to 3 in which Het represents the radical of the formula

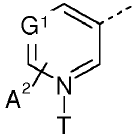

9. Compounds of the formula (I) according to any of Claims 1 to 8 in which T represents an electron pair.

10. Compounds of the formula (I) according to any of Claims 1 to 7 in which Het represents pyridin-3-yl.

11. Compounds of the formula (I) according to any of Claims 1 to 7 in which Het represents 5-fluoropyridin-3-yl.

12. Compounds of the formula (I) according to any of Claims 1 to 5 in which R¹ represents the radical

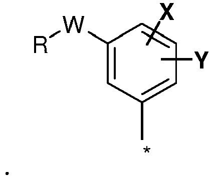

13. Composition, **characterized by** a content of at least one compound of the formula (I) according to any of Claims 1 to 12 and customary extenders and/or surfactants.

14. Non-therapeutic use of compounds of the formula (I) according to any of Claims 1 to 12 or of compositions according to Claim 13 for controlling pests.

15. Compounds of the formula (II) 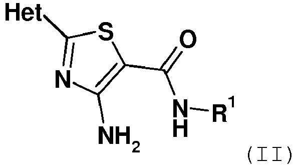 in which Het and R¹ have the meanings given in Claim 1.

## Revendications

1. Composés de formule (I) 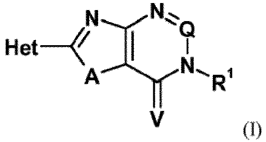 dans lesquels
Het représente un radical de la série constituée par : 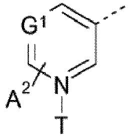 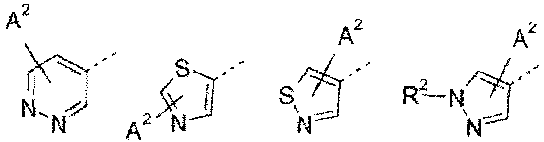 dans lesquels la ligne en pointillés représente la liaison à l'atome de carbone dans le groupe N=C-A relié à Het, et
G¹ représente N ou C-A¹,
A¹ représente hydrogène, halogène, cyano, nitro, alkyle, halogénoalkyle, alcoxy, halogénoalcoxy ; ou cycloalkyle ou cycloalcényle, chacun éventuellement substitués,
A² représente hydrogène, halogène, cyano, nitro, alkyle, halogénoalkyle, alcoxy, halogénoalcoxy ; ou cycloalkyle ou cycloalcényle, chacun éventuellement substitués,
T représente l'oxygène ou une paire d'électrons,
R² représente hydrogène, alkyle, halogénoalkyle ou cycloalkyle éventuellement substitué,
R¹ représente un radical de la série constituée par hydrogène, alkyle, halogénoalkyle, cyanoalkyle, alcényle, halogénoalcényle, alcynyle, halogénoalcynyle, alcoxy, halogénoalcoxy, alcoxyalkyle éventuellement substitué par halogène, bis(alcoxy)alkyle éventuellement substitué par halogène, alkylsulfanylalkyle éventuellement substitué par halogène, alkylcarbonylalkyle éventuellement substitué par halogène, alkylsulfinylalkyle éventuellement substitué par halogène, alkylsulfonylalkyle éventuellement substitué par halogène, dialkylaminosulfanylalkyle, dialkylaminosulfinylalkyle, dialkylaminosulfonylalkyle, alcoxycarbonyle éventuellement substitué par halogène, alcoxycarbonylalkyle éventuellement substitué par halogène, alcynyloxy éventuellement substitué par halogène, alcynyloxycarbonyle éventuellement substitué par halogène, dialkylaminocarbonyle, N-alkyl-N-cycloalkylaminocarbonyle, dialkylaminocarbonylalkyle, N-alkyl-N-cycloalkylaminocarbonylalkyle, hétérocyclyl-carbonylalkyle, alkylsulfanyle, halogénoalkylsulfanyle, alkylsulfinyle, halogénoalkylsulfinyle, alkylsulfonyle, halogénoalkylsulfonyle, cycloalkyle éventuellement substitué par halogène, cyano, nitro, alkyle, cycloalkyle, halogénoalkyle, alcoxy, halogénoalcoxy, alcoxycarbonyle, halogénoalcoxycarbonyle ou hétaryle (qui est lui-même éventuellement substitué par alkyle ou halogène), cycloalkylcarbonyle éventuellement substitué par halogène, cyano, nitro, alkyle, halogénoalkyle, cycloalkyle, alcoxy, halogénoalcoxy, alcoxycarbonyle, halogénoalcoxycarbonyle ou hétaryle (qui est lui-même éventuellement substitué par alkyle ou halogène), cycloalkylalkyle éventuellement substitué par halogène, cyano, nitro, alkyle, halogénoalkyle, cycloalkyle, alcoxy, halogénoalcoxy, alcoxycarbonyle, halogénoalcoxycarbonyle ou hétaryle (qui est lui-même éventuellement substitué par alkyle ou halogène), hétérocyclyle éventuellement substitué, hétérocyclylalkyle éventuellement substitué par halogène, cyano (également dans la partie alkyle), nitro, hydroxy, alkyle, halogénoalkyle, cycloalkyle (qui est éventuellement substitué), alcoxy, halogénoalcoxy, alkylthio, halogénoalkylthio, alkylsulfinyle, alkylsulfonyle, halogénoalkylsulfinyle, halogénoalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle ; aryle éventuellement substitué par halogène, cyano, nitro, hydroxy, amino, alkyle, halogénoalkyle, cycloalkyle (qui est éventuellement substitué), alcoxy ou halogénoalcoxy ; arylalkyle éventuellement substitué par halogène, cyano (également dans la partie alkyle), nitro, hydroxy, amino, alkyle, cycloalkyle (qui est éventuellement substitué), halogénoalkyle, alcoxy ou halogénoalcoxy ; hétaryle éventuellement substitué par halogène, cyano, nitro, hydroxy, alkyle, halogénoalkyle, cycloalkyle (qui est éventuellement substitué), alcoxy, halogénoalcoxy, alkylthio, halogénoalkylthio, alkylsulfinyle, alkylsulfonyle, halogénoalkylsulfinyle, halogénoalkylsulfonyle, amino, alkylamino, dialkylamino, alkylaminocarbonyle, dialkylaminocarbonyle, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle ; hétarylalkyle éventuellement substitué par halogène, cyano (également dans la partie alkyle), nitro, hydroxy, alkyle, halogénoalkyle, cycloalkyle (qui est éventuellement substitué), alcoxy, halogénoalcoxy, alkylthio, halogénoalkylthio, alkylsulfinyle, alkylsulfonyle, halogénoalkylsulfinyle, halogénoalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle,
R¹ représente également un radical de la série constituée par : 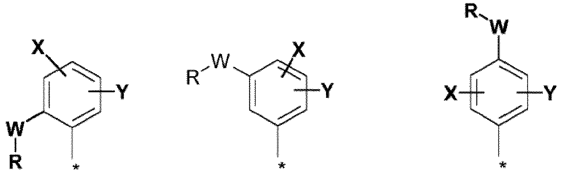 dans lesquels la liaison à l'atome d'azote dans le groupe C(=V)-N-Q dans la formule (I) est marquée avec l'étoile (*),
R représente NR⁷R⁸ ou un radical à chaque fois éventuellement substitué de la série constituée par alkyle, alcényle, alcynyle, alcoxyalkyle, alkyl-S(O)ₘ-alkyle, R⁷-CO-alkyle, NR⁷R⁸-CO-alkyle, cycloalkyle, cycloalcényle, cycloalkylalkyle, cycloalcénylalkyle, hétérocyclyle, hétérocyclylalkyle, phényle, phénylalkyle, hétaryle et hétarylalkyle,
W représente un radical de la série constituée par O, S, SO et SO₂,
X représente un radical de la série constituée par hydrogène, halogène, cyano, nitro, alkyle, halogénoalkyle, alcoxy, halogénoalcoxy et cycloalkyle,
Y représente un radical de la série constituée par hydrogène, halogène, alkyle, cycloalkyle, halogénoalkyle, alcoxy, halogénoalcoxy, cyano et NR⁵R⁶,
A représente oxygène ou soufre,
Q représente azote ou C-R³,
R³ représentant un radical de la série constituée par hydrogène, alkyle, cycloalkyle, halogénoalkyle, OH, alcoxy, halogénoalcoxy, SH, alkylsulfanyle, alkylsulfinyle, alkylsulfonyle, NH₂, alkylamino et dialkylamino,
V représente un radical de la série constituée par oxygène, soufre et NR⁴,
R⁴ représente un radical de la série constituée par hydrogène, cyano, alkyle, halogénoalkyle et cycloalkyle,
R⁵ représente un radical de la série constituée par hydrogène, alkyle, cycloalkyle et halogénoalkyle,
R⁶ représente un radical de la série constituée par hydrogène, alkyle, cycloalkyle et halogénoalkyle,
ou
R⁵ et R⁶ représentent ensemble avec l'azote auquel ils sont reliés un cycle de 3 à 6 chaînons saturé ou insaturé, éventuellement substitué et contenant éventuellement un ou plusieurs hétéroatomes supplémentaires,
R⁷ représente hydrogène, hydroxy ou un radical à chaque fois éventuellement substitué de la série constituée par alkyle, alcoxy, alcoxyalkyle, alkyl-S(O)ₘ-alkyle, alkylcarbonyle, alcoxycarbonyle, cycloalkyle, cycloalcényle, cycloalkylalkyle, cycloalcénylalkyle, hétérocyclyle, hétérocyclylalkyle, phényle, phénylalkyle, hétaryle et hétarylalkyle,
R⁸ représente l'hydrogène, un ion métallique, un ion ammonium éventuellement substitué ou un radical à chaque fois éventuellement substitué de la série constituée par alkyle, alcoxy, alcoxyalkyle, alkyl-S(O)ₘ-alkyle, et
m représente un nombre de la série constituée par 0, 1 et 2.

2. Composés de formule (I) selon la revendication 1, dans lesquels
Het représente un radical de la série constituée par : 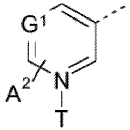 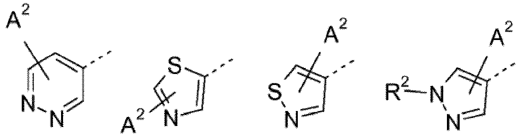 dans lesquels la ligne en pointillés représente la liaison à l'atome de carbone dans le groupe N=C-A relié à Het,
G¹ représente N ou C-A¹,
A¹ représente un radical de la série constituée par hydrogène, halogène, alkyle en C₁-C₆ et halogénoalkyle en C₁-C₄,
A² représente un radical de la série constituée par hydrogène, halogène, alkyle en C₁-C₆ et halogénoalkyle en C₁-C₄,
T représente l'oxygène ou une paire d'électrons,
R² représente un radical de la série constituée par hydrogène, alkyle en C₁-C₆, halogénoalkyle en C₁-C₆ ; et cycloalkyle en C₃-C₆ éventuellement substitué une fois à trois fois par halogène, cyano, nitro, alkyle en C₁-C₃, alcoxy en C₁-C₃, et une fois par cycloalkyle en C₃-C₆,
R¹ représente un radical de la série constituée par hydrogène, alkyle en C₁-C₆, halogénoalkyle en C₂-C₆, cyano-alkyle en C₁-C₆, alcényle en C₂-C₄, halogénoalcényle en C₂-C₄, alcynyle en C₂-C₄, halogénoalcynyle en C₂-C₄, alcoxy en C₁-C₆, halogénoalcoxy en C₁-C₆, alcoxy en C₁-C₆-alkyle en C₁-C₆ éventuellement substitué par halogène, bis(alcoxy en C₁-C₆)-alkyle en C₁-C₆ éventuellement substitué par halogène, alkylsulfanyle en C₁-C₆-alkyle en C₁-C₆ éventuellement substitué par halogène, alkylcarbonyle en C₁-C₄-alkyle en C₁-C₄ éventuellement substitué par halogène, alkylsulfinyle en C₁-C₆-alkyle en C₁-C₆ éventuellement substitué par halogène, alkylsulfonyle en C₁-C₆-alkyle en C₁-C₆ éventuellement substitué par halogène, di-(alkyle en C₁-C₆)-aminosulfanyl-alkyle en C₁-C₆, di-(alkyle en C₁-C₆)-aminosulfinyl-alkyle en C₁-C₆, di-(alkyle en C₁-C₆)-aminosulfonyl-alkyle en C₁-C₆, alcoxycarbonyle en C₁-C₆ éventuellement substitué par halogène, alcoxycarbonyle en C₁-C₆-alkyle en C₁-C₆ éventuellement substitué par halogène, alcynyloxy en C₂-C₄ éventuellement substitué par halogène, alcynyloxycarbonyle en C₂-C₄ éventuellement substitué par halogène, di-(alkyle en C₁-C₆)-aminocarbonyle, N-alkyle en C₁-C₆-N-cycloalkyle en C₃-C₆-aminocarbonyle, di-(alkyle en C₁-C₆) -aminocarbonyl-alkyle en C₁-C₆, N-alkyle en C₁-C₆-N-cycloalkyle en C₃-C₆-aminocarbonyl-alkyle en C₁-C₆, hétérocyclylcarbonyl-alkyle en C₁-C₆, alkylsulfanyle en C₁-C₆, halogénoalkylsulfanyle en C₁-C₆, alkylsulfinyle en C₁-C₆, halogénoalkylsulfinyle en C₁-C₆, alkylsulfonyle en C₁-C₆, halogénoalkylsulfonyle en C₁-C₆, cycloalkyle en C₃-C₆ éventuellement substitué par halogène, cyano, nitro, alkyle en C₁-C₆, halogénoalkyle en C₁-C₆, cycloalkyle en C₃-C₆, alcoxy en C₁-C₆, halogénoalcoxy en C₁-C₆, alcoxycarbonyle en C₁-C₆, halogénoalcoxycarbonyle en C₁-C₆ ou hétaryle (qui est lui-même éventuellement substitué par alkyle en C₁-C₆ ou halogène) ; cycloalkylcarbonyle en C₃-C₆ éventuellement substitué par halogène, cyano, nitro, alkyle en C₁-C₆, halogénoalkyle en C₁-C₆, cycloalkyle en C₃-C₆, alcoxy en C₁-C₆, halogénoalcoxy en C₁-C₆, alcoxycarbonyle en C₁-C₆, halogénoalcoxycarbonyle en C₁-C₆ ou hétaryle (qui est lui-même éventuellement substitué par alkyle en C₁-C₆ ou halogène) ; cycloalkyle en C₃-C₆-alkyle en C₁-C₆ éventuellement substitué par halogène, cyano, nitro, alkyle en C₁-C₆, halogénoalkyle en C₁-C₆, cycloalkyle en C₃-C₆, alcoxy en C₁-C₆, halogénoalcoxy en C₁-C₆, alcoxycarbonyle en C₁-C₆, halogénoalcoxycarbonyle en C₁-C₆ ou hétaryle (qui est lui-même éventuellement substitué par alkyle en C₁-C₆ ou halogène) ; hétérocyclyl-alkyle en C₁-C₆ éventuellement substitué par halogène, cyano (également dans la partie alkyle en C₁-C₆ de l'hétérocyclyl-alkyle en C₁-C₆), nitro, hydroxy, alkyle en C₁-C₆, halogénoalkyle en C₁-C₆, cycloalkyle en C₃-C₆ (qui est éventuellement substitué par halogène, cyano, alkyle en C₁-C₆ et cycloalkyle en C₃-C₆), alcoxy en C₁-C₆, halogénoalcoxy en C₁-C₆, alkylthio en C₁-C₆, halogénoalkylthio en C₁-C₆, alkylsulfinyle en C₁-C₆, alkylsulfonyle en C₁-C₆, halogénoalkylsulfinyle en C₁-C₆, halogénoalkylsulfonyle en C₁-C₆, amino, alkylamino en C₁-C₆, di-(alkyle en C₁-C₆)-amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle ; aryle éventuellement substitué par halogène, cyano, nitro, hydroxy, amino, alkyle en C₁-C₆, halogénoalkyle en C₁-C₆, cycloalkyle en C₃-C₆ (qui est éventuellement substitué par halogène, cyano, alkyle en C₁-C₆ et cycloalkyle en C₃-C₆), alcoxy en C₁-C₆ ou halogénoalcoxy en C₁-C₆; aryl-alkyle en C₁-C₆ éventuellement substitué par halogène, cyano (également dans la partie alkyle en C₁-C₆ de l'aryl-alkyle en C₁-C₆), nitro, hydroxy, amino, alkyle en C₁-C₆, halogénoalkyle en C₁-C₆, cycloalkyle en C₃-C₆ (qui est éventuellement substitué par halogène, cyano, alkyle en C₁-C₆ et cycloalkyle en C₃-C₆), alcoxy en C₁-C₆ ou halogénoalcoxy en C₁-C₆ ; hétaryle éventuellement substitué par halogène, cyano, nitro, hydroxy, alkyle en C₁-C₆, halogénoalkyle en C₁-C₆, cycloalkyle en C₃-C₆ (qui est éventuellement substitué par halogène, cyano, alkyle en C₁-C₆ et cycloalkyle en C₃-C₆), alcoxy en C₁-C₆, halogénoalcoxy en C₁-C₆, alkylthio en C₁-C₆, halogénoalkylthio en C₁-C₆, alkylsulfinyle en C₁-C₆, alkylsulfonyle en C₁-C₆, halogénoalkylsulfinyle en C₁-C₆, halogénoalkylsulfonyle en C₁-C₆, amino, alkylamino en C₁-C₆, di-(alkyle en C₁-C₆)-amino, alkylcarbonylamino en C₁-C₆, alkylaminocarbonyle en C₁-C₆, di-(alkyle en C₁-C₆)-aminocarbonyle, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle ; hétaryl-alkyle en C₁-C₆ éventuellement substitué par halogène, cyano (également dans la partie alkyle en C₁-C₆ de l'hétaryl-alkyle en C₁-C₆), nitro, hydroxy, alkyle en C₁-C₆, halogénoalkyle en C₁-C₆, cycloalkyle en C₃-C₆ (qui est éventuellement substitué par halogène, cyano, alkyle en C₁-C₆ et cycloalkyle en C₃-C₆), alcoxy en C₁-C₆, halogénoalcoxy en C₁-C₆, alkylthio en C₁-C₆, halogénoalkylthio en C₁-C₆, alkylsulfinyle en C₁-C₆, alkylsulfonyle en C₁-C₆, halogénoalkylsulfinyle en C₁-C₆, halogénoalkylsulfonyle en C₁-C₆, amino, alkylamino en C₁-C₆, di-(alkyle en C₁-C₆)-amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle,
R¹ représente également un radical de la série constituée par : 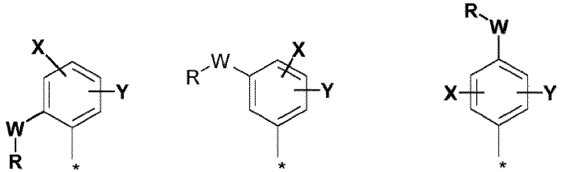 dans lesquels la liaison à l'atome d'azote dans le groupe C(=V)-N-Q dans la formule (I) est marquée avec l'étoile (*),
R représente NR⁷R⁸ ou alkyle en C₁-C₆, alcényle en C₃-C₆, alcynyle en C₃-C₆, alcoxy en C₁-C₆-alkyle en C₁-C₆, alkyle en C₁-C₆-S(O)ₘ-alkyle en C₁-C₄, chacun éventuellement substitués par halogène, oxygène (conduit à C=O) ou cyano ; R⁷-CO-alkyle en C₁-C₄, NR⁷R⁸-CO-alkyle en C₁-C₄ ; cycloalkyle en C₃-C₆ éventuellement substitué une fois ou deux fois par oxygène (conduit à C=O), alkyle en C₁-C₄, cycloalkyle en C₃-C₈, alcoxy en C₁-C₄ et halogénoalkyle en C₁-C₄ ; cycloalcényle en C₃-C₈ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; cycloalkyle en C₃-C₆-alkyle en C₁-C₄ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; cycloalcényle en C₃-C₆-alkyle en C₁-C₄ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; hétérocyclyle éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; hétérocyclyl-alkyle en C₁-C₄ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; ou phényle, phényl-alkyle en C₁-C₄, hétaryle et hétaryl-alkyle en C₁-C₄, chacun éventuellement substitués une fois à trois fois par halogène, cyano, alkyle en C₁-C₄, cycloalkyle en C₃-C₆, halogénoalkyle en C₁-C₄, alcoxy en C₁-C₄ ou halogénoalcoxy en C₁-C₄,
W représente un radical de la série constituée par O, S, SO et SO₂,
X représente un radical de la série constituée par hydrogène, halogène, cyano, nitro, alkyle en C₁-C₆, halogénoalkyle en C₁-C₆, cycloalkyle en C₃-C₆, alcoxy en C₁-C₆ et halogénoalcoxy en C₁-C₆,
Y représente un radical de la série constituée par hydrogène, halogène, alkyle en C₁-C₆, cycloalkyle en C₃-C₆, halogénoalkyle en C₁-C₆, alcoxy en C₁-C₆, halogénoalcoxy en C₁-C₆, cyano et NR⁵R⁶,
A représente le soufre,
Q représente l'azote ou C-R³,
R³ représentant un radical de la série constituée par hydrogène, alkyle en C₁-C₆, halogénoalkyle en C₁-C₆, cycloalkyle en C₃-C₆, OH, alcoxy en C₁-C₆, halogénoalcoxy en C₁-C₆, SH, alkylsulfanyle en C₁-C₆, alkylsulfinyle en C₁-C₆, alkylsulfonyle en C₁-C₆, NH₂, alkylamino en C₁-C₆ et di-(alkyle en C₁-C₆)-amino,
V représente un radical de la série constituée par oxygène, soufre et NR⁴,
R⁴ représente un radical de la série constituée par hydrogène, cyano, alkyle en C₁-C₆, halogénoalkyle en C₂-C₆ et cycloalkyle en C₃-C₆,
R⁵ représente un radical de la série constituée par hydrogène, alkyle en C₁-C₆, cycloalkyle en C₃-C₆ et halogénoalkyle en C₂-C₆,
R⁶ représente un radical de la série constituée par hydrogène, alkyle en C₁-C₆, cycloalkyle en C₃-C₆ et halogénoalkyle en C₂-C₆,
R⁵ et R⁶ peuvent également représenter ensemble avec l'azote auquel ils sont reliés un cycle de 3 à 6 chaînons saturé à trois fois insaturé, éventuellement substitué par halogène, cyano, alkyle en C₁-C₄, halogénoalkyle en C₁-C₄, alcoxy en C₁-C₄, halogénoalcoxy en C₁-C₄,
R⁷ représente un radical de la série constituée par hydrogène, hydroxy ; alkyle en C₁-C₆, alcoxy en C₁-C₆, alcoxy en C₁-C₆-alkyle en C₁-C₄, alkyle en C₁-C₆-S(O)ₘ-alkyle en C₁-C₄, alkylcarbonyle en C₁-C₆, alcoxycarbonyle en C₁-C₆, cycloalkyle en C₃-C₆, cycloalcényle en C₃-C₆, cycloalkyle en C₃-C₆-alkyle en C₁-C₃, cycloalcényle en C₃-C₆-alkyle en C₁-C₃, hétérocyclyle, hétérocyclyl-alkyle en C₁-C₃, chacun éventuellement substitués une fois ou plusieurs fois par halogène ou une fois ou deux fois par cyano ; et phényle, phényl-alkyle en C₁-C₃, hétaryle et hétaryl-alkyle en C₁-C₃, chacun éventuellement substitués une fois à quatre fois par alkyle en C₁-C₄, halogénoalkyle en C₁-C₄, alcoxy en C₁-C₄, halogénoalcoxy en C₁-C₄, cycloalkyle en C₃-C₆, halogène ou cyano,
R⁸ représente hydrogène, un ion de métal alcalin ou alcalino-terreux ou un ion ammonium éventuellement substitué une fois à quatre fois par alkyle en C₁-C₄, ou un radical à chaque fois éventuellement substitué une fois ou plusieurs fois par halogène ou une fois ou deux fois par cyano, de la série constituée par alkyle en C₁-C₄, alcoxy en C₁-C₄, alcoxy en C₁-C₄-alkyle en C₁-C₄ et alkyle en C₁-C₄-S(O)ₘ-alkyle en C₁-C₄,
m représente un nombre de la série constituée par 0, 1 et 2, et
ion alcalin est choisi dans la série constituée par le lithium, le sodium, le potassium, le rubidium, le césium,
ion alcalino-terreux est choisi dans la série constituée par le béryllium, le magnésium, le calcium, le strontium, le baryum,
halogène est choisi dans la série constituée par le fluor, le chlore, le brome et l'iode,
aryle (également en tant que partie d'une unité de plus grande taille, telle que par exemple arylalkyle) est choisi dans la série constituée par phényle, naphtyle, anthryle, phénanthrényle,
hétaryle (signifie hétéroaryle, également en tant que partie d'une unité de plus grande taille, telle que par exemple hétarylalkyle) est choisi dans la série constituée par furyle, thiényle, pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzisofuryle, benzothiényle, benzisothiényle, indolyle, isoindolyle, indazolyle, benzothiazolyle, benzisothiazolyle, benzoxazolyle, benzisoxazolyle, benzimidazolyle, 2,1,3-benzoxadiazole, quinolinyle, isoquinolinyle, cinnolinyle, phtalazinyle, quinazolinyle, quinoxalinyle, naphtyridinyle, benzotriazinyle, purinyle, ptéridinyle et indolizinyle, et
hétérocyclyle est un cycle saturé à 4, 5 ou 6 chaînons, qui contient 1 ou 2 atomes d'azote et/ou un atome d'oxygène et/ou un atome de soufre.

3. Composés de formule (I) selon la revendication 1, dans lesquels
Het représente un radical de la série constituée par : 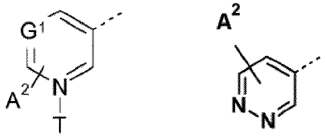 dans lesquels la ligne en pointillés représente la liaison à l'atome de carbone dans le groupe N=C-A relié à Het,
G¹ représente N ou C-A¹,
A¹ représente un radical de la série constituée par hydrogène, halogène, alkyle en C₁-C₄ et halogénoalkyle en C₁-C₄,
A² représente un radical de la série constituée par hydrogène, halogène, alkyle en C₁-C₄ et halogénoalkyle en C₁-C₄,
T représente l'oxygène ou une paire d'électrons,
R¹ représente un radical de la série constituée par hydrogène, alkyle en C₁-C₄, halogénoalkyle en C₂-C₄, cyano-alkyle en C₁-C₄, alcényle en C₂-C₄, halogénoalcényle en C₂-C₄, alcynyle en C₂-C₄, halogénoalcynyle en C₂-C₄, alcoxy en C₁-C₄, halogénoalcoxy en C₁-C₄, alcoxy en C₁-C₂-alkyle en C₁-C₄ éventuellement substitué par halogène, bis(alcoxy en C₁-C₂)alkyle en C₁-C₄ éventuellement substitué par halogène, alkylsulfanyle en C₁-C₄-alkyle en C₁-C₄ éventuellement substitué par halogène, alkylcarbonyle en C₁-C₄-alkyle en C₁-C₄ éventuellement substitué par halogène, alkylsulfinyle en C₁-C₄-alkyle en C₁-C₄ éventuellement substitué par halogène, alkylsulfonyle en C₁-C₄-alkyle en C₁-C₄ éventuellement substitué par halogène, di-(alkyle en C₁-C₄)-aminosulfanyl-alkyle en C₁-C₄, di-(alkyle en C₁-C₄) -aminosulfinyl-alkyle en C₁-C₄, di-(alkyle en C₁-C₄) -aminosulfonyl-alkyle en C₁-C₄, alcoxycarbonyle en C₁-C₄ éventuellement substitué par halogène, alcoxycarbonyle en C₁-C₄-alkyle en C₁-C₄ éventuellement substitué par halogène, alcynyloxy en C₂-C₄ éventuellement substitué par halogène, alcynyloxycarbonyle en C₂-C₄ éventuellement substitué par halogène, di-(alkyle en C₁-C₄)-aminocarbonyle, N-alkyle en C₁-C₄-N-cycloalkyle en C₃-C₆-aminocarbonyle, di-(alkyle en C₁-C₄) -aminocarbonyl-alkyle en C₁-C₄, N-alkyle en C₁-C₄-N-cycloalkyle en C₃-C₆-aminocarbonyl-alkyle en C₁-C₄, hétérocyclylcarbonyl-alkyle en C₁-C₄, alkylsulfanyle en C₁-C₄, halogénoalkylsulfanyle en C₁-C₄, alkylsulfinyle en C₁-C₄, halogénoalkylsulfinyle en C₁-C₄, alkylsulfonyle en C₁-C₄, halogénoalkylsulfonyle en C₁-C₄ ; cycloalkyle en C₃-C₆ éventuellement substitué par halogène, cyano, nitro, alkyle en C₁-C₄, halogénoalkyle en C₁-C₄, cycloalkyle en C₃-C₆, alcoxy en C₁-C₄, halogénoalcoxy en C₁-C₄, alcoxycarbonyle en C₁-C₄, halogénoalcoxycarbonyle en C₁-C₄ ou pyridyle (qui est lui-même éventuellement substitué par alkyle en C₁-C₄ ou halogène) ; cycloalkylcarbonyle en C₃-C₆ éventuellement substitué par halogène, cyano, nitro, alkyle en C₁-C₄, halogénoalkyle en C₁-C₄, cycloalkyle en C₃-C₆, alcoxy en C₁-C₄, halogénoalcoxy en C₁-C₄, alcoxycarbonyle en C₁-C₄, halogénoalcoxycarbonyle en C₁-C₄ ou pyridyle (qui est lui-même éventuellement substitué par alkyle en C₁-C₄ ou halogène) ; cycloalkyle en C₃-C₆-alkyle en C₁-C₄ éventuellement substitué par halogène, cyano, nitro, alkyle en C₁-C₄, halogénoalkyle en C₁-C₄, cycloalkyle en C₃-C₆, alcoxy en C₁-C₄, halogénoalcoxy en C₁-C₄, alcoxycarbonyle en C₁-C₄, halogénoalcoxycarbonyle en C₁-C₄, pyridyle, pyrimidyle, pyrazanyle, pyridazinyle, thiazolyle, isothiazolyle, thiadiazolyle, oxazolyle, isoxazolyle, oxadiazolyle, pyrazolyle, triazinyle ou triazolyle (les hétaryles mentionnés étant eux-mêmes éventuellement substitués par alkyle en C₁-C₄ ou halogène) ; hétérocyclyl-alkyle en C₁-C₄ éventuellement substitué par halogène, cyano (également dans la partie alkyle en C₁-C₄ de l'hétérocyclyl-alkyle en C₁-C₄), nitro, hydroxy, alkyle en C₁-C₄, halogénoalkyle en C₁-C₄, cycloalkyle en C₃-C₆, (qui est éventuellement substitué par halogène, cyano, alkyle en C₁-C₄ et cycloalkyle en C₃-C₆), alcoxy en C₁-C₄, halogénoalcoxy en C₁-C₄, alkylthio en C₁-C₄, halogénoalkylthio en C₁-C₄, alkylsulfinyle en C₁-C₄, alkylsulfonyle en C₁-C₄, halogénoalkylsulfinyle en C₁-C₄, halogénoalkylsulfonyle en C₁-C₄, amino, alkylamino en C₁-C₄, di-(alkyle en C₁-C₄)-amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle ; aryle éventuellement substitué par halogène, cyano, nitro, hydroxy, amino, alkyle en C₁-C₄, halogénoalkyle en C₁-C₄, cycloalkyle en C₃-C₆ (qui est éventuellement substitué par halogène, cyano, alkyle en C₁-C₄ et cycloalkyle en C₃-C₆), alcoxy en C₁-C₄ ou halogénoalcoxy en C₁-C₄ ; aryl-alkyle en C₁-C₄ éventuellement substitué par halogène, cyano (également dans la partie alkyle en C₁-C₄ de l'aryl-alkyle en C₁-C₄), nitro, hydroxy, amino, alkyle en C₁-C₄, halogénoalkyle en C₁-C₄, cycloalkyle en C₃-C₆ (qui est éventuellement substitué par halogène, cyano, alkyle en C₁-C₄ et cycloalkyle en C₃-C₆), alcoxy en C₁-C₄ ou halogénoalcoxy en C₁-C₄ ; hétaryle éventuellement substitué par halogène, cyano, nitro, hydroxy, alkyle en C₁-C₄, halogénoalkyle en C₁-C₄, cycloalkyle en C₃-C₆ (qui est éventuellement substitué par halogène, cyano, alkyle en C₁-C₄ et cycloalkyle en C₃-C₆), alcoxy en C₁-C4, halogénoalcoxy en C₁-C₄, alkylthio en C₁-C₄, halogénoalkylthio en C₁-C₄, alkylsulfinyle en C₁-C₄, alkylsulfonyle en C₁-C₄, halogénoalkylsulfinyle en C₁-C₄, halogénoalkylsulfonyle en C₁-C₄, amino, alkylamino en C₁-C₄, di-(alkyle en C₁-C₄)-amino, alkylcarbonylamino en C₁-C₄, alkylaminocarbonyle en C₁-C₄, di-(alkyle en C₁-C₄)-aminocarbonyle, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle ; hétaryl-alkyle en C₁-C₄ éventuellement substitué par halogène, cyano (également dans la partie alkyle en C₁-C₄ de l'hétaryl-alkyle en C₁-C₄), nitro, hydroxy, alkyle en C₁-C₄, halogénoalkyle en C₁-C₄, cycloalkyle en C₃-C₆ (qui est éventuellement substitué par halogène, cyano, alkyle en C₁-C₄ et cycloalkyle en C₃-C₆), alcoxy en C₁-C₄, halogénoalcoxy en C₁-C₄, alkylthio en C₁-C₄, halogénoalkylthio en C₁-C₄, alkylsulfinyle en C₁-C₄, alkylsulfonyle en C₁-C₄, halogénoalkylsulfinyle en C₁-C₄, halogénoalkylsulfonyle en C₁-C₄, amino, alkylamino en C₁-C₄, di-(alkyle en C₁-C₄) -amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle,
R¹ représente également un radical de la série constituée par : 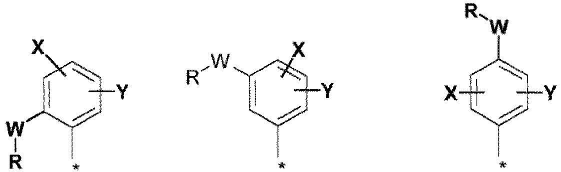 dans lesquels la liaison à l'atome d'azote dans le groupe C(=V)-N-Q dans la formule (I) est marquée avec l'étoile (*),
R représente NR⁷R⁸ ou alkyle en C₁-C₆, alcényle en C₃-C₆, alcynyle en C₃-C₆, alcoxy en C₁-C₄-alkyle en C₁-C₃, alkyle en C₁-C₄-S(O)ₘ-alkyle en C₁-C₃, chacun éventuellement substitués une fois à sept fois par halogène, une fois ou deux fois par oxygène (conduit à C=O) ou une fois ou deux fois par cyano ; R⁷-CO-alkyle en C₁-C₂, NR⁷R⁸-CO-alkyle en C₁-C₂ ; cycloalkyle en C₃-C₈ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; cycloalcényle en C₃-C₈ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; cycloalkyle en C₃-C₆-alkyle en C₁-C₄ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; cycloalcényle en C₃-C₆-alkyle en C₁-C₄ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; hétérocyclyle éventuellement substitué une fois ou deux fois par oxygène (conduit à C=0), alkyle en C₁-C₄, cycloalkyle en C3-C6, alcoxy en C₁-C₄ et halogénoalkyle en C₁-C₄ ; hétérocyclyl-alkyle en C₁-C₄ éventuellement substitué une fois ou deux fois par oxygène (conduit à C=O), alkyle en C₁-C₄, cycloalkyle en C₃-C₆, alcoxy en C₁-C₄ et halogénoalkyle en C₁-C₄ ; ou phényle, phényl-alkyle en C₁-C₄, hétaryle et hétaryl-alkyle en C₁-C₄, chacun éventuellement substitués une fois à trois fois par halogène, cyano, alkyle en C₁-C₄, halogénoalkyle en C₁-C4, cycloalkyle en C₃-C₆, alcoxy en C₁-C₄ ou halogénoalcoxy en C₁-C₄,
W représente un radical de la série constituée par S, SO et SO₂,
X représente un radical de la série constituée par hydrogène, fluor, chlore, brome, iode, cyano, nitro, alkyle en C₁-C₄, halogénoalkyle en C₁-C₄, cycloalkyle en C₃-C₆, alcoxy en C₁-C₄ et halogénoalcoxy en C₁-C₄,
Y représente un radical de la série constituée par hydrogène, fluor, chlore, brome, iode, alkyle en C₁-C₄, halogénoalkyle en C₁-C₄, cycloalkyle en C₃-C₆, alcoxy en C₁-C₄ et halogénoalcoxy en C₁-C₄,
A représente le soufre,
Q représente l'azote ou C-R³,
R³ représentant un radical de la série constituée par hydrogène, alkyle en C₁-C₄, halogénoalkyle en C₁-C₄, cycloalkyle en C₃-C₆, OH, alcoxy en C₁-C₄, halogénoalcoxy en C₁-C₄, SH, alkylsulfanyle en C₁-C₄, alkylsulfinyle en C₁-C₄, alkylsulfonyle en C₁-C₄, NH₂, alkylamino en C₁-C₄ et di-(alkyle en C₁-C₄)-amino,
V représente l'oxygène,
R⁷ représente un radical de la série constituée par hydrogène, hydroxy ; alkyle en C₁-C₄, alcoxy en C₁-C₄, alcoxy en C₁-C₄-alkyle en C₁-C₄, alkyle en C₁-C₄-S(O)ₘ-alkyle en C₁-C₃, alkylcarbonyle en C₁-C₄, alcoxycarbonyle en C₁-C₄, cycloalkyle en C₃-C₆, cycloalkyle en C₃-C₆-alkyle en C₁-C₃, hétérocyclyle, hétérocyclyl-alkyle en C₁-C₃, chacun éventuellement substitués une fois ou plusieurs fois par halogène ou une fois ou deux fois par cyano ; et phényle, benzyle, pyridyle, pyrimidyle, thiazolyle, oxazolyle, pyrazolyle, thiényle, furanyle, pyridinylméthyle et thiazolylméthyle, chacun éventuellement substitués une fois à trois fois par alkyle en C₁-C₄, halogénoalkyle en C₁-C₃, alcoxy en C₁-C₃, halogénoalcoxy en C₁-C₃, cyclopropyle, fluor, chlore, brome ou cyano,
R⁸ représente hydrogène, un ion de métal alcalin ou alcalino-terreux ou un ion ammonium éventuellement substitué une fois à quatre fois par alkyle en C₁-C₄, ou un radical à chaque fois éventuellement substitué une fois ou plusieurs fois par halogène ou une fois ou deux fois par cyano, de la série constituée par alkyle en C₁-C₄, alcoxy en C₁-C₄, alcoxy en C₁-C₄-alkyle en C₁-C₂ et alkyle en C₁-C₄-S(O)ₘ-alkyle en C₁-C₂,
m représente un nombre de la série constituée par 0, 1 et 2, et
ion alcalin est choisi dans la série constituée par le lithium, le sodium, le potassium, le rubidium, le césium,
ion alcalino-terreux est choisi dans la série constituée par le béryllium, le magnésium, le calcium, le strontium, le baryum,
halogène est choisi dans la série constituée par le fluor, le chlore, le brome et l'iode,
aryle (également en tant que partie d'une unité de plus grande taille, telle que par exemple arylalkyle) est choisi dans la série constituée par phényle, naphtyle, anthryle, phénanthrényle,
hétaryle (signifie hétéroaryle, également en tant que partie d'une unité de plus grande taille, telle que par exemple hétarylalkyle) est choisi dans la série constituée par pyrazolyle, imidazolyle, 1,2,3-triazolyle, 1,2,4-triazolyle, oxazolyle, isoxazolyle, thiazolyle, isothiazolyle, pyridyle, pyrimidinyle, pyridazinyle, pyrazinyle, 1,2,3-triazinyle, 1,2,4-triazinyle, 1,3,5-triazinyle, benzothiényle, et hétérocyclyle est choisi dans la série constituée par azétidinyle, azolidinyle, azinanyle, oxétanyle, oxolanyle, oxanyle, dioxanyle, thiéthanyle, thiolanyle, thianyle, tétrahydrofuryle, pipérazinyle, morpholinyle.

4. Composés de formule (I) selon la revendication 1, dans lesquels
Het représente un radical de la série constituée par : 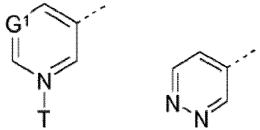 dans lesquels la ligne en pointillés représente la liaison à l'atome de carbone dans le groupe N=C-A relié à Het,
G¹ représente N ou C-A¹,
A¹ représente un radical de la série constituée par hydrogène, fluor, chlore,
T représente l'oxygène ou une paire d'électrons,
R¹ représente un radical de la série constituée par méthyle, éthyle, isopropyle, n-propyle, n-butyle, iso-butyle, sec-butyle, *tert*-butyle, 2,2,2-trifluoroéthyle, 2,2-difluoroéthyle, 2,2-difluoro-n-propyle, méthylsulfanyl-méthyle, méthylsulfanyléthyle, méthylsulfanyl-n-propyle, trifluorométhylsulfonylméthyle, éthylsulfonylméthyle, 2,2,2-trifluoroéthylsulfonylméthyle, 2,2-difluoroéthylsulfonylméthyle, isopropylsulfanylméthyle, méthylsulfinylméthyle, trifluorométhylsulfinylméthyle, éthylsulfinyl-méthyle, 2,2,2-trifluoroéthylsulfinylméthyle, 2,2-difluoroéthylsulfinylméthyle, isopropylsulfinylméthyle, méthylsulfonylméthyle, trifluorométhylsulfonylméthyle, éthylsulfonylméthyle, 2,2,2-trifluoroéthylsulfonylméthyle, 2,2-difluoroéthylsulfonylméthyle, isopropylsulfonylméthyle, méthoxycarbonyle, éthoxycarbonyle, méthoxycarbonylméthyle, éthoxycarbonylméthyle, méthoxycarbonyléthyle, éthoxycarbonyléthyle, diméthylaminocarbonyle, diéthylaminocarbonyle, *N*-éthyl-*N*-méthylaminocarbonyle, *N*-isopropyl-*N*-méthylaminocarbonyle, diméthylaminocarbonylméthyle, diéthylaminocarbonylméthyle, *N*-éthyl-*N*-méthylaminocarbonylméthyle, *N*-isopropyl-*N*-méthylaminocarbonylméthyle, diméthylaminocarbonyléthyle, diéthylaminocarbonyléthyle, *N*-éthyl-*N*-méthylaminocarbonyléthyle, *N*-isopropyl-*N-*méthylaminocarbonyléthyle, *N*-cyclopropyl-*N-*méthylaminocarbonylméthyle, *N*-cyclopropyl-*N-*méthylaminocarbonyléthyle, méthylsulfanyle, trifluorométhylsulfanyle, éthylsulfanyle, 2,2,2-trifluoroéthylsulfanyle, 2,2-difluoroéthylsulfanyle, isopropylsulfanyle, méthylsulfinyle, trifluorométhylsulfinyle, éthylsulfinyle, 2,2,2-trifluoroéthylsulfinyle, 2,2-difluoroéthylsulfinyle, isopropylsulfinyle, méthylsulfonyle, trifluorométhylsulfonyle, éthylsulfonyle, 2,2,2-trifluoroéthylsulfonyle, 2,2-difluoroéthylsulfonyle, isopropylsulfonyle, cyclopropyle, 1-cyanocyclopropyle, 1-chlorocyclopropyle, 1-fluorocyclopropyle, 2-cyanocyclopropyle, 2-chlorocyclopropyle, 2-fluorocyclopropyle, 2,2,3,3-tétrafluorocyclopropyle, 2-cyclopropylcyclopropyle, cyclobutyle, cyclopentyle, cyclohexyle, 4-trifluorométhylcyclohexyle, cyclopropylméthyle, cyclopropyléthyle, cyclobutylméthyle, *N-*cyclopropyl-*N*-méthylaminocarbonyle, morpholin-4-ylcarbonylméthyle, pipérazin-1-ylcarbonylméthyle, 4-méthyl-pipérazin-1-ylcarbonylméthyle ; hétérocyclylméthyle et hétérocyclyléthyle chacun éventuellement substitués une fois, deux fois ou trois fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy ; hétérocyclylméthyle et hétérocyclyléthyle, chacun substitués par cyclopropyle, le radical cyclopropyle étant éventuellement substitué une fois ou deux fois par méthyle, fluor, chlore, cyano ou une fois par cyclopropyle ; hétaryle éventuellement substitué une fois ou deux fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy, diméthylaminocarbonyle ; hétaryle substitué par cyclopropyle, le radical cyclopropyle étant éventuellement substitué une fois ou deux fois par méthyle, fluor, chlore, cyano ou une fois par cyclopropyle ; aryle éventuellement substitué une fois, deux fois ou trois fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy, trifluoroéthoxy ; aryle substitué par cyclopropyle, le radical cyclopropyle étant éventuellement substitué une fois ou deux fois par méthyle, fluor, chlore, cyano ou une fois par cyclopropyle ; arylméthyle et aryléthyle, chacun éventuellement substitués une fois, deux fois ou trois fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy ; arylméthyle et aryléthyle, chacun substitués par cyclopropyle, le radical cyclopropyle étant éventuellement substitué une fois ou deux fois par méthyle, fluor, chlore, cyano ou une fois par cyclopropyle ; hétarylméthyle et hétaryléthyle, chacun éventuellement substitués une fois ou deux fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy ; hétarylméthyle et hétaryléthyle, chacun substitués par cyclopropyle, le radical cyclopropyle étant éventuellement substitué une fois ou deux fois par méthyle, fluor, chlore, cyano ou une fois par cyclopropyle,
R¹ représente également un radical de la série constituée par : 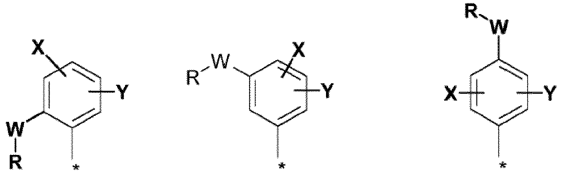 dans lesquels la liaison à l'atome d'azote dans le groupe C(=V)-N-Q dans la formule (I) est marquée avec l'étoile (*),
R représente NR⁷R⁸ ou un radical à chaque fois éventuellement substitué une fois, deux fois, trois fois, quatre fois ou cinq fois par fluor, chlore, ou une fois ou deux fois par cyano, de la série constituée par alkyle en C₁-C₄, alcényle en C₃-C₄, alcynyle en C₃-C₄, alcoxy en C₁-C₂-alkyle en C₁-C₂ et alkyle en C₁-C₂-S(O)ₘ-alkyle en C₁-C₂ ; R⁷-CO-alkyle en C₁-C₂, NR⁷R⁸-CO-alkyle en C₁-C₂ ; cycloalkyle en C₃-C₆ éventuellement substitué une fois ou deux fois par alkyle en C₁-C₂, alcoxy en C₁-C₂ ou halogénoalkyle en C₁-C₂ ou par un atome d'oxygène (conduit à C=O) ; cycloalcényle en C₃-C₆ éventuellement substitué une fois ou deux fois par alkyle en C₁-C₂, alcoxy en C₁-C₂ ou halogénoalkyle en C₁-C₂ ou par un atome d'oxygène (conduit à C=O) ; cycloalkyle en C₃-C₆-alkyle en C₁-C₂ éventuellement substitué une fois à deux fois par alkyle en C₁-C₂, alcoxy en C₁-C₂ ou halogénoalkyle en C₁-C₂ ; cycloalcényle en C₃-C₆-alkyle en C₁-C₂ éventuellement substitué une fois ou deux fois par alkyle en C₁-C₂, alcoxy en C₁-C₂ ou halogénoalkyle en C₁-C₂ ; hétérocyclyle éventuellement substitué une fois ou deux fois par alkyle en C₁-C₂, alcoxy en C₁-C₂ ou halogénoalkyle en C₁-C₂ ; hétérocyclyl-alkyle en C₁-C₂ éventuellement substitué une fois ou deux fois par alkyle en C₁-C₂, alcoxy en C₁-C₂ ou halogénoalkyle en C₁-C₂ ; ou phényle, benzyle, pyridyle, pyrimidyle, thiazolyle, oxazolyle, pyrazolyle, thiényle, furanyle, pyridinylméthyle ou thiazolylméthyle, chacun éventuellement substitués une fois ou deux fois par fluor, chlore, brome, cyano, méthyle, éthyle, difluorométhyle, trifluorométhyle, méthoxy, éthoxy, difluorométhoxy ou trifluorométhoxy,
W représente un radical de la série constituée par S, SO et SO₂,
X représente un radical de la série constituée par hydrogène, fluor, chlore, brome, cyano, méthyle, éthyle, trifluorométhyle, méthoxy, éthoxy, difluorométhoxy et trifluorométhoxy,
Y représente un radical de la série constituée par hydrogène, fluor, chlore, brome, cyano, méthyle, éthyle, trifluorométhyle, méthoxy, éthoxy, difluorométhoxy et trifluorométhoxy,
A représente le soufre,
Q représente l'azote ou C-R³,
R³ représentant l'hydrogène,
V représente l'oxygène,
R⁷ représente un radical de la série constituée par hydrogène, hydroxy ; alkyle en C₁-C₄, alcoxy en C₁-C₄, alcoxy en C₁-C₄-alkyle en C₁-C₄, alkyle en C₁-C₄-S(O)ₘ-alkyle en C₁-C₂, alkylcarbonyle en C₁-C₄, alcoxycarbonyle en C₁-C₄, cycloalkyle en C₃-C₆, cycloalkyle en C₃-C₆-alkyle en C₁-C₃, hétérocyclyle, hétérocyclyl-alkyle en C₁-C₃, chacun éventuellement substitués une fois, deux fois, trois fois, quatre fois ou cinq fois par fluor, chlore, ou une fois ou deux fois par cyano ; et phényle, benzyle, pyridyle, pyrimidyle, thiazolyle, oxazolyle, pyrazolyle, thiényle, furanyle, pyridinylméthyle et thiazolylméthyle, chacun éventuellement substitués une fois à trois fois par alkyle en C₁-C₄, halogénoalkyle en C₁-C₃, alcoxy en C₁-C₃, halogénoalcoxy en C₁-C₃, cyclopropyle, fluor, chlore, brome ou cyano,
R⁸ représente hydrogène, un ion alcalin ou alcalino-terreux, un ion ammonium éventuellement substitué une fois à quatre fois par alkyle en C₁-C₄, ou un radical à chaque fois éventuellement substitué une fois, deux fois, trois fois, quatre fois ou cinq fois par fluor, chlore, ou une fois ou deux fois par cyano, de la série constituée par alkyle en C₁-C₄, alcoxy en C₁-C₄, alcoxy en C₁-C₄-alkyle en C₁-C₂ et alkyle en C₁-C₄-S(O)ₘ-alkyle en C₁-C₂,
m représente un nombre de la série constituée par 0, 1 et 2, et
ion alcalin représente un ion de la série constituée par le lithium, le sodium, le potassium, le rubidium, le césium,
ion alcalino-terreux représente un ion de la série constituée par le béryllium, le magnésium, le calcium, le strontium et le baryum,
halogène représente le fluor, le chlore, le brome et l'iode,
hétérocyclyle représente un radical de la série constituée par oxétanyle, thiéthanyle, tétrahydrofuryle et morpholinyle,
aryle représente phényle et
hétaryle (signifie hétéroaryle, également en tant que partie d'une unité de plus grande taille, telle que par exemple hétarylalkyle) représente un radical de la série constituée par pyridyle, pyrimidyle, pyrazinyle, pyridazinyle, thiazolyle, pyrazolyle et benzothiényle.

5. Composés de formule (I) selon la revendication 1, dans lesquels
Het représente un radical de la série constituée par : 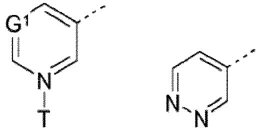 dans lesquels la ligne en pointillés représente la liaison à l'atome de carbone dans le groupe N=C-A relié à Het,
G¹ représente N ou C-A¹,
A¹ représente un radical de la série constituée par hydrogène, fluor, chlore,
T représente l'oxygène ou une paire d'électrons,
R¹ représente un radical de la série constituée par méthyle, éthyle, isopropyle, n-propyle, n-butyle, iso-butyle, sec-butyle, *tert*-butyle, 2,2,2-trifluoroéthyle, 2,2-difluoroéthyle, 2,2-difluoro-n-propyle, méthylsulfanyl-méthyle, méthylsulfanyléthyle, méthylsulfanyl-n-propyle, trifluorométhylsulfonylméthyle, éthylsulfonylméthyle, 2,2,2-trifluoroéthylsulfonylméthyle, 2,2-difluoroéthylsulfonylméthyle, isopropylsulfanylméthyle, méthylsulfinylméthyle, trifluorométhylsulfinylméthyle, éthylsulfinyl-méthyle, 2,2,2-trifluoroéthylsulfinylméthyle, 2,2-difluoroéthylsulfinylméthyle, isopropylsulfinylméthyle, méthylsulfonylméthyle, trifluorométhylsulfonylméthyle, éthylsulfonylméthyle, 2,2,2-trifluoroéthylsulfonylméthyle, 2,2-difluoroéthylsulfonylméthyle, isopropylsulfonylméthyle, méthoxycarbonyle, éthoxycarbonyle, méthoxycarbonylméthyle, éthoxycarbonylméthyle, méthoxycarbonyléthyle, éthoxycarbonyléthyle, diméthylaminocarbonyle, diéthylaminocarbonyle, *N*-éthyl-*N-*méthylaminocarbonyle, *N*-isopropyl-*N*-méthylaminocarbonyle, diméthylaminocarbonylméthyle, diéthylaminocarbonylméthyle, *N*-éthyl-*N*-méthylaminocarbonylméthyle, *N*-isopropyl-*N*-méthylaminocarbonylméthyle, diméthylaminocarbonyléthyle, diéthylaminocarbonyléthyle, *N*-éthyl-*N*-méthylaminocarbonyléthyle, *N*-isopropyl-*N*-méthylaminocarbonyléthyle, *N*-cyclopropyl-*N*-méthylaminocarbonylméthyle, *N*-cyclopropyl-*N*-méthylaminocarbonyléthyle, méthylsulfanyle, trifluorométhylsulfanyle, éthylsulfanyle, 2,2,2-trifluoroéthylsulfanyle, 2,2-difluoroéthylsulfanyle, isopropylsulfanyle, méthylsulfinyle, trifluorométhylsulfinyle, éthylsulfinyle, 2,2,2-trifluoroéthylsulfinyle, 2,2-difluoroéthylsulfinyle, isopropylsulfinyle, méthylsulfonyle, trifluorométhylsulfonyle, éthylsulfonyle, 2,2,2-trifluoroéthylsulfonyle, 2,2-difluoroéthylsulfonyle, isopropylsulfonyle, cyclopropyle, 1-cyanocyclopropyle, 1-chlorocyclopropyle, 1-fluorocyclopropyle, 2-cyanocyclopropyle, 2-chlorocyclopropyle, 2-fluorocyclopropyle, 2,2,3,3-tétrafluorocyclopropyle, 2-cyclopropylcyclopropyle, cyclobutyle, cyclopentyle, cyclohexyle, 4-trifluorométhylcyclohexyle, cyclopropylméthyle, cyclopropyléthyle, cyclobutylméthyle, *N-*cyclopropyl-*N*-méthylaminocarbonyle, morpholin-4-ylcarbonylméthyle, pipérazin-1-ylcarbonylméthyle, 4-méthyl-pipérazin-1-ylcarbonylméthyle, hétérocyclylméthyle et hétérocyclyléthyle chacun éventuellement substitués une fois, deux fois ou trois fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy ; hétérocyclylméthyle et hétérocyclyléthyle, chacun substitués par cyclopropyle, le radical cyclopropyle étant éventuellement substitué une fois ou deux fois par méthyle, fluor, chlore, cyano ou une fois par cyclopropyle ; hétaryle éventuellement substitué une fois ou deux fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy, diméthylaminocarbonyle ; hétaryle substitué par cyclopropyle, le radical cyclopropyle étant éventuellement substitué une fois ou deux fois par méthyle, fluor, chlore, cyano ou une fois par cyclopropyle ; aryle éventuellement substitué une fois, deux fois ou trois fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy, trifluoroéthoxy ; aryle substitué par cyclopropyle, le radical cyclopropyle étant éventuellement substitué une fois ou deux fois par méthyle, fluor, chlore, cyano ou une fois par cyclopropyle ; arylméthyle et aryléthyle, chacun éventuellement substitués une fois, deux fois ou trois fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy ; arylméthyle et aryléthyle, chacun substitués par cyclopropyle, le radical cyclopropyle étant éventuellement substitué une fois ou deux fois par méthyle, fluor, chlore, cyano ou une fois par cyclopropyle ; hétarylméthyle et hétaryléthyle, chacun éventuellement substitués une fois ou deux fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy ; hétarylméthyle et hétaryléthyle, chacun substitués par cyclopropyle, le radical cyclopropyle étant éventuellement substitué une fois ou deux fois par méthyle, fluor, chlore, cyano ou une fois par cyclopropyle,
R¹ représente également le radical de formule 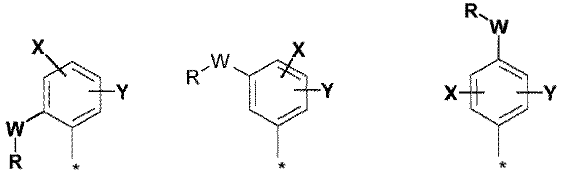 dans lesquels la liaison à l'atome d'azote dans le groupe C(=V)-N-Q dans la formule (I) est marquée avec l'étoile (*),
R représente méthyle, éthyle, propyle, iso-propyle, n-butyle, iso-butyle, sec-butyle, tert-butyle, allyle, 2-butényle, propargyle, 2-butinyle, chacun éventuellement substitués une fois, deux fois ou trois fois par fluor ou une fois par cyano ; cycloalkyle en C₃-C₆ éventuellement substitué une fois par méthyle, éthyle, méthoxy, éthoxy ou trifluorométhyle ; cycloalkylméthyle en C₃-C₆ éventuellement substitué une fois par méthyle, éthyle, méthoxy ou trifluorométhyle ; ou phényle, benzyle, pyridyle, pyrimidyle, thiazolyle et pyridinylméthyle, chacun éventuellement substitués une fois ou deux fois par fluor, chlore, brome, cyano, méthyle, éthyle, iso-propyle, tert.-butyle, trifluorométhyle, difluorométhyle, méthoxy, éthoxy, difluorométhoxy ou trifluorométhoxy,
W représente un radical de la série constituée par S, SO et SO₂,
X représente un radical de la série constituée par hydrogène, fluor, chlore, brome, cyano, méthyle, éthyle, trifluorométhyle, méthoxy, éthoxy, difluorométhoxy et trifluorométhoxy,
Y représente un radical de la série constituée par hydrogène, fluor, chlore, brome, cyano, méthyle, éthyle, trifluorométhyle, méthoxy, éthoxy, difluorométhoxy et trifluorométhoxy,
A représente le soufre,
Q représente l'azote ou C-R³,
R³ représentant hydrogène ou méthyle,
V représente l'oxygène, et
ion alcalin représente un ion de la série constituée par le lithium, le sodium, le potassium, le rubidium, le césium,
ion alcalino-terreux représente un ion de la série constituée par le béryllium, le magnésium, le calcium, le strontium et le baryum,
halogène représente le fluor, le chlore, le brome et l'iode,
hétérocyclyle représente un radical de la série constituée par oxétanyle, thiéthanyle, tétrahydrofuryle et morpholinyle,
aryle représente phényle et
hétaryle (signifie hétéroaryle, également en tant que partie d'une unité de plus grande taille, telle que par exemple hétarylalkyle) représente un radical de la série constituée par pyridyle, pyrimidyle, pyrazinyle, pyridazinyle, thiazolyle, pyrazolyle et benzothiényle.

6. Composés de formule (I) selon la revendication 1, dans lesquels
Het représente : 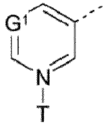 dans lesquels la ligne en pointillés représente la liaison à l'atome de carbone dans le groupe N=C-A relié à Het,
G¹ représente C-A¹,
A¹ représente l'hydrogène,
T représente l'oxygène ou une paire d'électrons,
R¹ représente méthyle, éthyle, isopropyle, n-propyle, n-butyle, iso-butyle, sec-butyle, *tert*-butyle, 2,2,2-trifluorooéthyle, 2,2-difluorooéthyle, 2,2-difluoro-n-propyle, diméthylaminocarbonyle, diéthylaminocarbonyle, *N*-éthyl-*N*-méthylaminocarbonyle, *N*-isopropyl-*N-*méthylaminocarbonyle, diméthylaminocarbonylméthyle, diéthylaminocarbonylméthyle, *N*-éthyl-*N*-méthylaminocarbonylméthyle, *N*-isopropyl-*N*-méthylaminocarbonylméthyle, diméthylaminocarbonyléthyle, diéthylaminocarbonyléthyle, *N*-éthyl-*N*-méthylaminocarbonyléthyle, *N*-isopropyl-*N*-méthylaminocarbonyléthyle, *N*-cyclopropyl-*N*-méthylaminocarbonylméthyle, *N*-cyclopropyl-*N*-méthylaminocarbonyléthyle, cyclopropyle, cyclobutyle, cyclopentyle, cyclohexyle ; hétarylméthyle et hetaryléthyle, chacun éventuellement substitués une fois ou deux fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy,
R¹ représente également 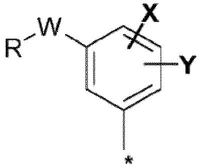 dans lesquels la liaison à l'atome d'azote dans le groupe C(=V)-N-Q dans la formule (I) est marquée avec l'étoile (*),
R représente alkyle en C₁-C₄ éventuellement substitué une fois à trois fois par fluor ou chlore,
W représente un radical de la série constituée par S et SO,
X représente un radical de la série constituée par hydrogène, fluor, chlore, brome, méthyle, éthyle, difluorométhyle et trifluorométhyle,
Y représente un radical de la série constituée par hydrogène, fluor, chlore, brome, iode, méthyle et éthyle,
A représente le soufre,
Q représente l'azote ou C-R³,
R³ représentant l'hydrogène,
V représente l'oxygène, et
halogène représente le fluor, le chlore, le brome et l'iode,
hétaryle représente un radical de la série constituée par pyridyle, pyrimidyle, thiazolyle, pyrazolyle et benzothiényle.

7. Composés de formule (I) selon la revendication 1, dans lesquels
Het représente : 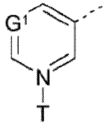 dans lesquels la ligne en pointillés représente la liaison à l'atome de carbone dans le groupe N=C-A relié à Het,
G¹ représente C-A¹ ou N,
A¹ représente l'hydrogène ou le fluor,
T représente l'oxygène ou une paire d'électrons,
R¹ représente méthyle, éthyle, isopropyle, n-propyle, n-butyle, iso-butyle, sec-butyle, *tert*-butyle, 2,2,2-trifluoroéthyle, 2,2-difluoroéthyle, 2,2-difluoro-n-propyle, diméthylaminocarbonyle, diéthylaminocarbonyle, *N*-éthyl-*N*-méthylaminocarbonyle, *N*-isopropyl-*N-*méthylaminocarbonyle, diméthylaminocarbonylméthyle, diéthylaminocarbonylméthyle, *N*-éthyl-*N*-méthylamino-carbonylméthyle, *N*-isopropyl-*N*-méthylaminocarbonyl-méthyle, diméthylaminocarbonyléthyle, diéthylamino-carbonyléthyle, *N*-éthyl-*N*-méthylaminocarbonyléthyle, *N*-isopropyl-*N*-méthylaminocarbonyléthyle, *N*-cyclopropyl-*N*-méthylaminocarbonylméthyle, *N*-cyclopropyl-*N-*méthylaminocarbonyléthyle, cyclopropyle, cyclobutyle, cyclopentyle, cyclohexyle, cyclopropylméthyle ; benzothiényle éventuellement substitué par diméthylaminocarbonyle ; phényle éventuellement substitué par halogène, méthyle ou trifluoroéthoxy ; phénylméthyle éventuellement substitué par méthyle, méthoxy, difluorométhoxy ou halogène ; hétarylméthyle et hétaryléthyle, chacun éventuellement substitués une fois ou deux fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy, difluorométhoxy,
R¹ représente également 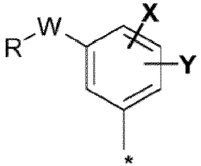 dans lesquels la liaison à l'atome d'azote dans le groupe C(=V)-N-Q dans la formule (I) est marquée avec l'étoile (*),
R représente alkyle en C₁-C₄ éventuellement substitué une fois à trois fois par fluor ou chlore,
W représente un radical de la série constituée par S, SO et SO₂,
X représente un radical de la série constituée par hydrogène, fluor, chlore, brome, méthyle, éthyle, difluorométhyle et trifluorométhyle,
Y représente un radical de la série constituée par hydrogène, fluor, chlore, brome, iode, méthyle et éthyle,
A représente le soufre,
Q représente l'azote ou C-R³,
R³ représentant hydrogène ou méthyle,
V représente l'oxygène, et
halogène représente le fluor, le chlore, le brome et l'iode,
hétaryle représente un radical de la série constituée par pyridyle, pyrimidyle, thiazolyle, pyrazolyle et benzothiényle.

8. Composés de formule (I) selon les revendications 1 à 3, dans lesquels Het représente le radical de formule

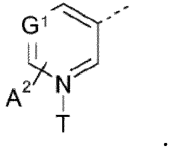

9. Composés de formule (I) selon les revendications 1 à 8, dans lesquels T représente une paire d'électrons.

10. Composés de formule (I) selon les revendications 1 à 7, dans lesquels Het représente pyridin-3-yle.

11. Composés de formule (I) selon les revendications 1 à 7, dans lesquels Het représente 5-fluoro-pyridin-3-yle.

12. Composés de formule (I) selon les revendications 1 à 5, dans lesquels R¹ représente le radical

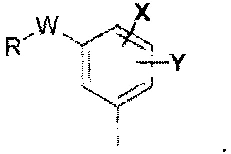

13. Agent, **caractérisé par** une teneur en au moins un composé de formule (I) selon l'une quelconque des revendications 1 à 12 et en des extendeurs et/ou substances tensioactives usuels.

14. Utilisation non thérapeutique de composés de formule (I) selon l'une quelconque des revendications 1 à 12 ou d'agents selon la revendication 13 pour lutter contre des nuisibles.

15. Composés de formule (II) 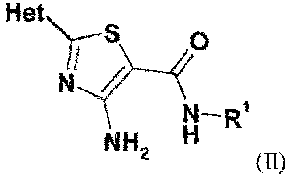 dans laquelle Het et R¹ ont les significations indiquées dans la revendication 1.
